(19) Europäisches Patentamt / European Patent Office / Office européen des brevets

(11) **EP 2 311 842 A2**

(12) **EUROPEAN PATENT APPLICATION**


(43) Date of publication: **20.04.2011 Bulletin 2011/16**

(21) Application number: **11153222.2**

(22) Date of filing: **23.06.2009**

(51) Int Cl.: ***C07D 513/04*** *(2006.01)* ***C07D 519/00*** *(2006.01)* ***A61K 31/437*** *(2006.01)* ***A61P 35/00*** *(2006.01)*

(84) Designated Contracting States: **AT BE BG CH CY CZ DE DK EE ES FI FR GB GR HR HU IE IS IT LI LT LU LV MC MK MT NL NO PL PT RO SE SI SK TR**
Designated Extension States: **AL BA RS**

(30) Priority: **24.06.2008 US 75134 P**

(62) Document number(s) of the earlier application(s) in accordance with Art. 76 EPC: **09789905.8 / 2 303 886**



(71) Applicant: **Takeda Pharmaceutical Company Limited**
**Osaka-shi,**
**Osaka 541-0045 (JP)**

(72) Inventors:
- **Dong, Qing**
  **San Diego, CA 92121 (US)**
- **Gong, Xianchang**
  **San Diego, CA 92121 (US)**
- **Hirose, Masaaki**
  **Osaka-shi Osaka 532-8686 (JP)**
- **Jin, Bohan**
  **San Diego, CA 92121 (US)**
- **Zhou, Feng**
  **San Diego, CA 92121 (US)**

(74) Representative: **Duncan, Garreth Andrew**
**D Young & Co LLP**
**120 Holborn**
**London**
**EC1N 2DY (GB)**



Remarks:
This application was filed on 03-02-2011 as a divisional application to the application mentioned under INID code 62.


(54) **PI3K/M TOR inhibitors**


(57) Compounds of the formula:

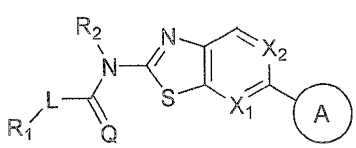

wherein ring A is heteroaryl, Q is O or S; L is selected from the group consisting of $-CHR_3-$, $-NR_4-$ and -O-; $X_1$ and $X_2$ are each independently selected from the group consisting of $CR_5$, and N; and $R_1$, $R_2$, $R_3$, $R_4$, $R_5$ and $R_{5'}$ are defined herein, are provided. The compounds are P13K/mTOR inhibitors and are useful in the treatment of a number of conditions, including cancer and inflammatory diseases.


EP 2 311 842 A2

## Description

## FIELD OF THE INVENTION

The present invention relates to compounds that may be used to inhibit kinases as well as compositions of matter, kits and articles of manufacture comprising these compounds. The present invention also relates to methods for inhibiting kinases as well as treatment methods using compounds according to the present invention. In addition, the present invention relates to methods of making the compounds of the present invention, as well as intermediates useful in such methods. In particular, the present invention relates to enzyme inhibitors of the mammalian target of rapamycin kinase (mTOR) and/or one or more phosphatidylinositol-3-kinase (PBK); compositions of matter, kits and articles of manufacture comprising these compounds; methods for inhibiting MOTOR and/or one or more PI3K enzyme; and methods of making inhibitors of mTOR and/or one or more PI3K enzyme.

## BACKGROUND OF THE INVENTION

The phosphatidylinositol 3-kinase (PI3K) family comprises 15 kinases that display sequence homology within their kinase domains, but with distinct substrate specificities, expression patterns, and modes of regulation (reviewed in Vanhaesebroeck, B., et al. (2001) Ann. Rev. Biochem. 70:535-602). The class I PBKs (p110α, p110β, p110δ and p110γ) are subdivided into IA or IB according to the regulatory subunit, and catalyze formation of the lipid second messenger PI(3,4,5)P3 (phosphatidylinositol-3,4,5-trisphosphate), which engages downstream effectors such as the Akt/PDK1 pathway, the Tec family kinases, and the Rho family GTPases. Class IA enzymes (α, β, δ) have a p85 regulatory subunit containing two SH2 domains that is activated by interaction with a receptor tyrosine kinase (e.g., growth factor receptors such as InsR and IGF-1R), whereas Class IB (γ) has a p101 regulatory subunit that is activated by GPCRs. As a second messenger, $PI(3,4,5)P_3$ then acts as a docking site to recruit and activate proteins containing 3-phosphoinositide binding domains, such as the pleckstrin homology (PH) domain and the recently identified FYVE and phox domains. Examples of downstream targets affected by PI3K-mediated production of PI(3,4,5)P3 include: 1) protein kinases that promote cell growth, survival and proliferation, such as Akt (PKB), PDK1 (phosphoinositide-dependent kinase 1) and the Tec family kinases; 2) GAPs (GTPase-activating proteins) and GEFs (guanine nucleotide exchange factors) that regulate GTPases mediating cell motility and enzyme trafficking; and 3) scaffolding proteins that nucleate the assembly of key signaling complexes. Thus, through this single lipid second messenger the Class IPI3Ks are able to link extracellular hormonal input to intracellular signaling proteins that control diverse cellular processes.

Class II (CIIα, CIIβ, and CIIδ) and III (Vps34) PI3Ks appear to play a role in intracellular trafficking through the formation of PI(3)P and $PI(3,4)P_2$, but their physiological significance remains to be elucidated. Class IV enzymes, the so-called PIKKs (PI3K-related protein kinase) are a subfamily of serine/threonine protein kinases that share homology with PI3K, but phosphorylate proteins rather than phosphoinositide lipids. The PIKKs include key kinases that monitor genomic integrity (ATM or ataxia-telangiectasia mutated), ATR (ATP-and Rad3-related), Smg-1 and DNA-PK (DNA-dependent protein kinase), as well as mTOR (mammalian target of rapamycin), a kinase that integrates nutritional and growth factor signals in order to control protein synthesis and cell growth. The role of mTOR singalling is discussed in more detail below.

There is now considerable evidence that Class IA PI3K enzymes contribute to tumorigenesis in a variety of human cancers, either directly or indirectly (Vivanco, I., and Sawyers, C.L. (2002) Nature Rev. Cancer 2:489-501). Aberrations in $PI(3,4,5)P_3$ levels occur frequently in numerous forms of cancer, caused either by activation of PI3Ks or through inactivation of the phosphoinositide 3-phosphate phosphatase that removes the 3-phosphate from PI(3,4,5)P3 to regenerate $PI(4,5)P_2$, namely PTEN (phosphatase and tensin homologue deleted on chromosome ten). For example, recent data suggest that at least 50% of human breast cancers involve mutations in either PI3Kα or PTEN (Luo, J., et al. (2003) Cancer Cell 4:257-262; Saal, L.H., et al. (2005) Cancer Res. 65:2554-2559). The p110α subunit is amplified in some tumors such as those of the ovary (Shayesteh, L. et al., (1999) Nature Genetics, 21:99-102) and cervix (Ma, Y.-Y. et al., Oncogene (2000) 19:2739-2744). More recently, activating mutations within the catalytic site of the p110α catalytic subunit have been associated with various other tumors such as those of the colorectal region and of the breast and lung (Samuel, Y., et al., Science (2004) 304:554; Bader, A.G., et al. (2005) Nature Rev. Cancer 5:921-929; Parsons, W.D., et al. (2005) Nature 36:792). Tumor-related mutations in the p85α regulatory subunit have also been identified in cancers such as those of the ovary and colon (Philp, A.J., et al., Cancer Res. (2001) 61:7426-7429). In most cases, an increased lipid kinase activity *in vitro* and the ability to induce oncogenic transformation *in vivo* were shown for both the most frequently mutated, so-called 'hotspot' residues (Samuels, Y., et al., Science (2004) 304:554; Kang, S., et al. (2005) Proc. Natl. Acad. Sci USA 102:802-807; Ikenoue, T., et al. (2005) Cancer Res. 65:4562-4567; Isakof, S.J., et al. (2005) Cancer Res. 65:10992-11000), and for 14 rare cancer-specific mutations in p110α (Gymnopoulos, M. et al. (2007) Proc. Natl. Acad. Sci. USA 104:5569-5574).

In addition to the direct effects described above, Class IA PI3Ks may contribute to tumorigenic events that

occur upstream in the signaling pathways via ligand-dependent or ligand-independent activation of receptor tyrosine kinases, GPCRs or integrins (Fresno Vara, J.A., et al. (2004) Cancer Treatment Rev. 30:193-204). Over-expression of the receptor tyrosine kinase ERBB2 leading to activation of the PI3K-Akt signaling pathway in variety of tumors (Harari, D., et al. (2000) Oncogene 19:6102-6114) and overexpression of the ras oncogene (Kauffmann-Zeh, A., et al. (1997) Nature 385:544-548) are just two examples. Similarly, Class IA PI3Ks may contribute indirectly to tumorigenesis via downstream signaling events, such as occurs when the PTEN tumor suppressor phosphatase is inactivated in a broad range of tumors (Simpson, L., and Parsons, R. (2001) Exp. Cell. Res. 264:29-41), resulting in a loss of counterbalancing hydrolysis of PI(3,4,5)$P_3$ to the less active PI(4,5)$P_2$. There is also evidence that Class IA PI3K enzymes contribute to tumorigenesis in tumor-associated stromal cells, given the demonstrated role for these enzymes in mediating angiogenic events in endothelial cells in response to pro-angiogenic factors such as VEFG (Abid, M.R., et al. (2004) Arterioscler. Thromb. Vasc. Biol. 24:294-300), and their role in cell motility and migration (Sawyer, T.K. (2004) Expert Opin. Investig. Drugs 13:1-19).

These findings suggest that pharmacological inhibitors of Class IA PI3K kinases will be of therapeutic value for the treatment of various diseases including different forms of the disease of cancer comprising solid tumors such as carcinomas and sarcomas and the leukemias and lymphoid malignancies. In particular, Class IA PI3K kinases should be of therapeutic value for treatment of, for example, cancer of the breast, colorectum, lung (including small cell lung cancer, non-small cell lung cancer and bronchioalveolar cancer) and prostate, and of cancer of the bile duct, bone, bladder, head and neck, kidney, liver, gastrointestinal tissue, esophagus, ovary, pancreas, skin, testes, thyroid, uterus, cervix and vulva, and of leukemias (including ALL and CML), multiple myeloma and lymphomas.

mTOR (mammalian target of rapamycin, also known as FRAP, RAFT1 or RAPT), a member of the PIKK family of serine/threonine protein kinases and also involved in PI3K-mediated signaling, has recently been recognized as a critical component in cell-signaling pathways often found to be dysregulated in human cancers (Guertin, D.A., et al. (2007) Cancer Cell 12:9-22; Wan, X. and Helman, L.J. (2007) The Oncologist 12:1007-1018). mTOR is a key regulator of cell growth and has been shown to regulate a wide range of cellular functions including translation, transcription, mRNA turnover, protein stability, actin cytoskeleton reorganization and autophagy (Jacinto, E. and Hall, M.N. (2003) Nature Rev. Mol. Cell Biol. 4:117-126; Wullschleger, S., et al. (2006) Cell 124:471-484).

mTOR integrates signals from growth factors (such as insulin or insulin-like growth factor-1) and nutrients (such as amino acids and glucose) to regulate cell growth. Recent results have shown that mTOR also integrates signals related to the energy status of the cell via AMPK (AMP-activated protein kinase) (Gwinn, D.M., et al. (2008) Molecular Cell 30:214-226). mTOR responds to growth factors via the PI3K-Akt pathway, thus providing a link to the Class IA PI3K kinases described above. mTOR is connected to the PI3K-Akt pathway through a rather complex scheme that involves the tuberous sclerosis proteins TSC1 and TSC2, which act in concert as a heterodimer, to regulate the small GTPase, Rheb, which binds to and activates the kinase domain of mTOR in a GTP-dependent manner (Smith, E.M., at al. (2005) J. Biol. Chem. 280:18717-18727; Long, X., et al. (2005) Curr. Biol. 15:702-713). Downstream, mTOR regulates translation via phosphorylation and activation of S6K1 (also know as p70-S6K1), and via phosphorylation of 4E-BP1 (elF4E binding protein-1) resulting in release of elF4E, which is then free to associate with elF4G to stimulate translation initiation (Hay, N. and Sonenberg, N. (2004) Genes Dev. 18:1926-1945).

Much of the knowledge of mTOR signaling is based on the use of rapamycin (sirolimus), a natural product originally isolated as an anti-fungal agent from a strain of *Streptomyces hygroscopicus* (Vezina, C., et al. (1975) J. Antibiot. (Tokyo) 28:721-726). Rapamycin was approved in the US as an immunosuppressant for use after renal transplantation in the 1990s, and subsequently was shown to have potent inhibitory activity against several human cancers *in vitro* and *in vivo.* The most notable of the rapamycin analogues currently in preclinical and clinical development as anticancer agents, including CCI-779 (temsirolimus), RAD001 (everolimus), and AP23573, have shown significant efficacy in a subset of patients (Wan, X. and Helman, L.J. (2007) The Oncologist 12:1007-1018; Easton, J.B. and Houghton, P.J. (2006) Oncogene 25:6436-6446; Faivre, S., et al. (2006) Nature Rev. Drug Discovery 5:671-688; Granville, C.A., et al. (2006) Clin. Cancer Res. 12:679-689)

Recent evidence has revealed a rapamycin-insensitive path for mTOR signaling which may help explain some of the limitations seen with rapamycin analogue anticancer therapeutics. Thus, in man the mTOR signaling network consists of two major branches, each mediated by a specific mTOR multi-protein complex (mTORC). Rapamysin-sensitive mTORC1 controls several pathways that collectively determine the mass of the cell. Rapamycin-insensitive mTORC2 controls the actin cytoskeleton and thereby determines the shape of the cell (Loewith, R., et al. (2002) Molecular Cell 10:457-468; Wullschleger, S., et al. (2006) Cell 124:471-484). The rapamycin complex with FKBP12 (12 kDa immunophilin FK506-binding protein) binds to and inhibits mTOR catalytic activity of the mTORC1 complex both in vitro and in vivo, but has no effect on mTORC2 (Hara, K., et al. (2002) Cell 110:177-189; Jacinto, E., et al. (2004) Nature Cell. Biol. 6:1122-1128; Kim, D.H., et al. (2002) Cell 110:163-175; Sarbassov, D.D., et al. (2004) Curr. Biol. 14:1296-1302). Unlike rapamycin analogues, ATP-competitive small molecule inhibitors directed to the mTOR active site of both mTORC1 and mTORC2 will have the advantage of blocking mTOR signaling along both branches of the path. This will be of particular importance for anticancer therapy, since recent results demonstrate that rapamycin-mediated inhibition of

only the mTORC1 branch can lead to activation of Akt both directly (via mTORC2-catalyzed phosphorylation of Akt at Ser473) and indirectly (via release of feedback inhibition of Akt by S6K1), resulting in partial abrogation of the therapeutic effect (Guertin, D.A. and Sabatini, D.M. (2007) Cancer Cell 12:9-22).

In addition there is evidence that endothelial cell proliferation may also be dependent upon mTOR signaling. Endothelial cell proliferation is stimulated by vascular endothelial cell growth factor (VEGF) activation of the PI3K-Akt-mTOR signaling pathway (Dancey, J.E. (2005) Expert Opinion on Invest. Drugs 14:313-328). Moreover, mTOR signaling is believed to partially control VEGF synthesis through effects on the expression of hypoxia-inducible factor-1$\alpha$ (HIF-1$\alpha$) (Hudson, C.C., et al. (2002) Molec. Cell. Biol. 22:7004-7014). Therefore, tumor angiogenesis may depend on mTOR signaling in two ways, through hypoxia-induced synthesis of VEGF by tumor and stromal cells, and through VEGF stimulation of endothelial proliferation and survival through PI3K-Akt-mTOR signaling.

These findings suggest that pharmacological inhibitors of mTOR should be of therapeutic value for treatment of the various forms of cancer comprising solid tumors such as carcinomas and sarcomas and the leukemias and lymphoid malignancies. In particular, inhibitors of mTOR should be of therapeutic value for treatment of, for example, cancer of the breast, colorectum, lung (including small cell lung cancer, non-small cell lung cancer and bronchioalveolar cancer) and prostate, and of cancer of the bile duct, bone, bladder, head and neck, kidney, liver, gastrointestinal tissue, esophagus, ovary, pancreas, skin, testes, thyroid, uterus, cervix and vulva, and of leukemias (including ALL and CML), multiple myeloma and lymphomas.

In addition to tumorigenesis, there is evidence that mTOR plays a role in an array of hamartoma syndromes. Recent studies have shown that the tumor suppressor proteins such as TSC1, TSC2, PTEN and LKB1 tightly control mTOR signaling. Loss of these tumor suppressor proteins leads to a range of hamartoma conditions as a result of elevated mTOR signaling (Tee, A.R. and Blenis, J. (2005) Semin. Cell Dev. Biol. 16:29-37). Syndromes with an established molecular link to dysregulation of mTOR include Peutz-Jeghers syndrome, Cowden disease, neurofibromatosis, Birt-Hogg-Dube syndrome, Bannayan-Riley-Ruvalcaba syndrome, Proteus syndrome, Lheimitte-Duclos disease and Tuberous Sclerosis (Inoki, K., et al. (2005) Nature Genetics 37:19-24). Patients with these syndromes characteristically develop benign hamartomatous tumors in multiple organs.

Recent studies have revealed a role for mTOR in other diseases (Easton, J.B. and Houghton, P.J. (2004) Expert Opin. Ther. Targets 8:551-564). Rapamycin has been demonstrated to be a potent immunosuppressant by inhibiting antigen-induced proliferation of T cells, B cells and antibody production (Sehgal, S.N. (2003) Transplantation Proceedings 35:7S-14S) and thus mTOR inhibitors may also be useful as immunosuppressive agents.

Inhibition of the kinase activity of mTOR may also be useful in the prevention of restenosis, which is the control of undesired proliferation of normal cells in the vasculature in response to the introduction of stents in the treatment of vascular disease (Morice, M.C., et al. (2007) J. Amer. Coll. Cardiol. 50:1299-1304). Furthermore, the rapamycin analogue, everolimus, can reduce the severity and incidence of cardiac allograft vasculopathy (Eisen, H.J., et al., (2003) New England Journal of Medicine, 349:847-858; Vigano, M., et al. (2007) J. Heart Lung Transplant 26:584-592). Elevated mTOR activity has been associated with cardiac hypertrophy, which is of clinical importance as a major risk factor for heart failure and is a consequence of increased cellular size of cardiomyocytes (Tee, A.R. and Blenis, J. (2005) Semin. Cell Dev. Biol. 16:29-37). Thus mTOR inhibitors are expected to be of value in the prevention and treatment of a wide variety of diseases in addition to cancer.

Recent studies point to the potential benefit of dual mTOR and Class IA PI3K kinase inhibitors (Fan, Q-W., et al. (2006) Cancer Cell 9:341-349; Fan, Q-W., et al. (2007) Cancer Res. 67:7960-7965). Thus, the ATP-competitive small molecule inhibitor, PI-103, effected proliferative arrest in glioma cells and showed significant activity in xenografted tumors with no observable toxicity. The efficacy of Pi-103 in xenograft models was ascribed to its unique ability to inhibit both mTOR and PI3K$\alpha$ in a broad range of glioma cell lines, and that this ability persists irrespective of the PTEN, p53 and EGFR status (Fan, Q-W., et al. (2006) Cancer Cell 9:341-239). Subsequent studies have demonstrated the further utility of combining a dual mTOR/PI3K$\alpha$ inhibitor (PI-103) with erlotinib, and EGFR (epidermal growth factor receptor) tyrosine kinase inhibitor approved for use in non-small cell lung cancer patients, in PTEN-mutant glioma cells (Fan, Q-W., et al. (2007) Cancer Res. 67:7960-7965). Similarly, the combination of a dual mTOR/PI3K$\alpha$ inhibitor (PI-103) with ionizing radiation treatment proved effective against glioblastoma cells both in vitro and in vivo, when compared to either treatment alone (Chen, J.S., et al. (2008) Mol. Cancer Ther. 7:841-850). Thus, dual mTOR/Class IA PI3K inhibitors are expected to be of value in the prevention and treatment of a wide variety of diseases in addition to cancer.

There is a continued need to find new therapeutic agents to treat human diseases. The protein and lipid kinases, specifically but not limited to mTOR and the Class 1A PI3Ks, are especially attractive targets for the discovery of new therapeutics due to their important role in cancer and a range of other human diseases.

## SUMMARY OF THE INVENTION

The present invention relates to compounds that have activity for inhibiting PI3K and/or mTOR (PI3K/mTOR). The present invention also provides compositions, articles of manufacture and kits comprising these compounds. In

addition, the invention relates to methods of making the compounds of the present invention, as well as intermediates useful in such methods.

In one aspect, the invention is directed to compounds having the formula:

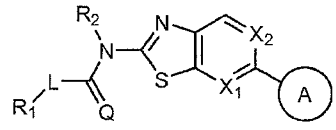

wherein

ring A is aryl or heteroaryl, each unsubstituted or substituted with 1-5 substituents which is independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, carbonylamino, sulfonylamino, $(C_{1-10})$alkylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$Cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl $(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl $(C_{1-6})$alkyl, $(C_{4-6})$aryl $(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero $(C_{1-5})$aryl;

Q is O or S;

L is selected from the group consisting of $-CHR_3-$, $-NR_4-$ and -O-;

$X_1$ is selected from the group consisting of $CR_5$ and N;

$X_2$ is selected from the group consisting of $CR_5$, and N;

$R_1$ and $R_3$ are each independently selected from the group consisting of hydrogen, halo, for nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1}{-}_{10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloalyl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, betero$(C_{3-12})$bicycloalicyclyl, $(C_{4-12})$aryl, hetero$(C_{1-10})$aryl, $(C_{9-12})$bicycloaryl, and heteno$(C_{4-12})$bicycloaryl, each substituted or unsubstituted, or $R_1$ and $R_3$ are taken together to form a cyclopropyl;

$R_2$ is selected from the group consisting of hydrogen, $(C_{1-6})$alkoxy, carbonyl, oxycarbonyl, aminocarbonyl, amino, $(C_{1-6})$alkylamino, sulfonyl, sulfinyl, $(C_{1-6})$alkyl, $(C_{1-10})$alkenyl, $(C_{1-10})$alkynyl, hetero$(C_{1-6})$alkyl, halo$(C_{1-6})$alkyl, hydroxy$(C_{1-6})$alkyl, carbonyl$(C_{1-6})$alkyl, $(C_{3-6})$cycloalkyl$(C_{1-3})$alkyl, hetero$(C_{1-5})$cycloalkyl$(C_{1-3})$alkyl, $(C_{3-6})$alicyclyl, and hetero$(C_{1-5})$alicyclyl, each substituted or unsubstituted;

$R_4$ is selected from the group consisting of hydrogen, oxy, hydroxy, carbonyloxy, $(C_{1-10})$alkoxy, $(C_{4-12})$aryloxy, hetero$(C_{1-10})$alyloxy, carbonyl, oxycarbonyl, aminocarbonyl, amino, $(C_{1-10})$alkylamino, imino, sulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl $(C_{1-10})$alkyl, sulfonyl$(C_{1-10})$alkyl, sulfinyl$(C_{1-10})$alkyl, aza$(C_{1-10})$allcyl, $(C_{1-10})$oxaalkyl, $(C_{1-10})$oxoalkyl, imino$(C_{1-10})$akyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-10})$alkyl, $(C_{9-12})$bicycloaryl$(C_{1-5})$alkyl, hetero$(C_{8-12})$bicycloaryl$(C_{1-5})$alicyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted; and

$R_5$ and $R_{5'}$ are each independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, sulfonylamino, imino, sulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alky $(C_{1-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or

unsubstituted;

provided that

when $X_1$ is N or CH, $X_2$ is CH, Q is O, L is-$CH_2$-, -NH- or O, $R_1$ is hydrogen, $(C_{1-4})$alkyl or hetero$(C_6)$cycloalkyl, and ring A is a pyridyl, pyrimidinyl or pyrazinyl, ring A does not have an amino, substituted amino, substituted oxy, or fluoro attached to position 4 of ring A where the ring atom through which ring A attaches to the ring containing $X_1$ and $X_2$ is counted as position 1;

when $X_1$ is CH, $X_2$ is CH, Q is O, L is -$CHR_3$-, $R_1$ and $R_3$ are both hydrogen, and ring A is a five- or six-membered heteroaryl comprising one nitrogen ring atom, said one nitrogen ring atom is not located at a position adjacent to the ring atom through which said heteroaryl attaches to the ring containing $X_1$ and $X_2$;

when $X_1$ is CH, $X_2$ is CH, Q is O, and L is -$CHR_3$-, $R_1$ and $R_3$ are both hydrogen, and ring A is a pyrimidinyl, the ring atom located at position 4 of said pyrimidinyl is not a nitrogen where the ring atom through which said pyrimidinyl attaches to the ring containing $X_1$ and $X_2$ is counted as at position 1;

when $X_1$ is CH, $X_2$ is CH, Q is O, L is -NH-, and ring A is as defined above, $R_1$ is not selected from the group consisting of alkyl, carbonyl, and phenoxy substituted pyridinyl;

when $X_1$ is N, $X_2$ is CH, Q is O, L is -$CHR_3$-, and ring A is as defined above, $R_1$ and $R_3$ are both not a substituted phenyl;

when $X_1$ is CH, $X_2$ is CH, Q is O, L is -$CH_2$-, and ring A is substituted or unsubstituted $(C_{6-10})$aryl, $R_1$ is not a halo, or a thiocarbonyl substituted amino group; and

the compounds of the invention do not include

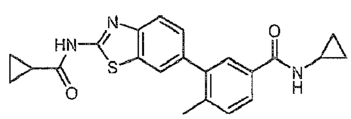

(3-(2-(cyclopropanecarboxamido)benzo[*d*]thiazol-6-yl)-*N*-cyclopropyl-4-methylbenzamide).

In another aspect, the invention is directed to pharmaceutical compositions that comprise a PI3K/mTOR inhibitor according to the present invention as an active ingredient. Pharmaceutical compositions according to the invention may optionally comprise 0.001%-100% of one or more inhibitors of this invention. These pharmaceutical compositions may be administered or coadministered by a wide variety of routes, including for example, orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, or intrathecally. The compositions may also be administered or coadministered in slow release dosage forms.

In another aspect, the invention is directed to kits and other articles of manufacture for treating disease states associated with PI3K and/or mTOR. In one embodiment, the kit comprises a composition comprising at least one PI3K/mTOR inhibitor of the present invention in combination with instructions. The instructions may indicate the disease state for which the composition is to be administered, storage information, dosing information and/or instructions regarding how to administer the composition. The kit may also comprise packaging materials. The packaging material may comprise a container for housing the composition. The kit may also optionally comprise additional components, such as syringes for administration of the composition. The kit may comprise the composition in single or multiple dose forms.

In another aspect, the invention is directed to articles of manufacture that comprise a composition comprising at least one PI3K/mTOR inhibitor of the present invention in combination with packaging materials. The packaging material may comprise a container for housing the composition. The container may optionally comprise a label indicating the disease state for which the composition is to be administered, storage information, dosing information and/or instructions regarding how to administer the composition. The article of manufacture may also optionally comprise additional components, such as syringes for administration of the composition. The article of manufacture may comprise the composition in single or multiple dose forms.

In yet another aspect of the invention is directed to methods for preparing compounds, compositions, kits, and articles of manufacture according to the present invention. For example, several synthetic schemes are provided herein for synthesizing compounds according to the present invention.

In yet other aspect, the invention is directed to methods of using compounds, compositions, kits and articles of manufacture according to the present invention.

In one embodiment, the compounds, compositions, kits and articles of manufacture are used to inhibit PI3K/mTOR.

In another embodiment, the compounds, compositions, kits and articles of manufacture are used to treat a disease state for which PI3K/mTOR possess activity that contributes to the pathology and/or symptomology of the disease state.

In another embodiment, a compound is administered to a subject wherein PI3K/mTOR activity within the subject is altered, preferably reduced.

In another embodiment, a prodrug of a compound is administered to a subject that is converted to the compound *in vivo* where it inhibits PI3K/mTOR.

In another embodiment, a method of inhibiting PI3K/mTOR is provided that comprises contacting a PI3K/mTOR with a compound according to the present invention.

In another embodiment, a method of inhibiting PI3K/mTOR is provided that comprises causing a compound according to the present invention to be present in a subject in order to inhibit PI3K/mTOR *in vivo.*

In another embodiment, a method of inhibiting a PI3K/mTOR is provided that comprises administering a first compound to a subject that is converted *in vivo* to a second compound wherein the second compound inhibits PI3K/mTOR *in vivo*. It is noted that the compounds of the present invention may be the first or second compounds.

In another embodiment, a therapeutic method is provided that comprises administering a compound according to the present invention.

In another embodiment, a method of treating a condition in a patient that is known to be mediated by PI3K/mTOR, or which is known to be treated by PI3K/mTOR inhibitors, comprising administering to the patient a therapeutically effective amount of a compound according to the present invention.

In another embodiment, a method is provided for treating a disease state for which PI3K/mTOR possess activity that contributes to the pathology and/or symptomology of the disease state, the method comprising: causing a compound according to the present invention to be present in a subject in a therapeutically effective amount for the disease state.

In another embodiment, a method is provided for treating a disease state for which PI3K/mTOR possess activity that contributes to the pathology and/or symptomology of the disease state, the method comprising: administering a first compound to a subject that is converted *in vivo* to a second compound such that the second compound is present in the subject in a therapeutically effective amount for the disease state. It is noted that the compounds of the present invention may be the first or second compounds.

In another embodiment, a method is provided for treating a disease state for which PI3K/mTOR possess activity that contributes to the pathology and/or symptomology of the disease state, the method comprising: administering a compound according to the present invention to a subject such that the compound is present in the subject in a therapeutically effective amount for the disease state.

In another embodiment, a method is provided for using a compound according to the present invention in order to manufacture a medicament for use in the treatment of a disease state that is known to be mediated by PI3K/mTOR, or that is known to be treated by PI3K/mTOR inhibitors.

It is noted in regard to all of the above embodiments that the present invention is intended to encompass all pharmaceutically acceptable ionized forms (e.g., salts) and solvates (e.g., hydrates) of the compounds, regardless of whether such ionized forms and solvates are specified since it is well known in the art to administer pharmaceutical agents in an ionized or solvated form. It is also noted that unless a particular stereochemistry is specified, recitation of a compound is intended to encompass all possible stereoisomers (*e.g.*, enantiomers or diastereomers depending on the number of chiral centers), independent of whether the compound is present as an individual isomer or a mixture of isomers. Further, unless otherwise specified, recitation of a compound is intended to encompass all possible resonance forms and tautomers. With regard to the claims, the language "compound comprising the formula," "compound having the formula" and "compound of the formula" is intended to encompass the compound and all pharmaceutically acceptable ionized forms and solvates, all possible stereoisomers, and all possible resonance forms and tautomers unless otherwise specifically specified in the particular claim.

It is further noted that prodrugs may also be administered which are altered *in vivo* and become a compound according to the present invention. The various methods of using the compounds of the present invention are intended, regardless of whether prodrug delivery is specified, to encompass the administration of a prodrug that is converted *in vivo* to a compound according to the present invention. It is also noted that certain compounds of the present invention may be altered *in vivo* prior to inhibiting PI3K/mTOR and thus may themselves be prodrugs for another compound. Such prodrugs of another compound may or may not themselves independently having PI3K/mTOR inhibitory activity.

## DEFINITIONS

Unless otherwise stated, the following terms used in the specification and claims shall have the following meanings for the purposes of this Application.

It is noted that, as used in the specification and the appended claims, the singular forms "a," "an" and "the" include plural referents unless the context clearly dictates otherwise. Further, definitions of standard chemistry terms

may be found in reference works, including Carey and Sundberg "ADVANCED ORGANIC CHEMISTRY 5TH ED." Vols. A (2007) and B (2007), Springer Science+Business Media, New York. Also, unless otherwise indicated, conventional methods of mass spectroscopy, NMR, HPLC, protein chemistry, biochemistry, recombinant DNA techniques and pharmacology, within the skill of the art are employed.

"Alicyclyl" refers to a monocyclic or polycyclic radical; where when the alicyclyl is monocyclic, it is a saturated or partially unsaturated ring and the ring atoms of which are all carbon atoms, and when the alicyclyl is a polycyclic assembly of rings, the ring through which the radical attaches to the parent molecule is a saturated or partially unsaturated ring and the ring atoms of which are all carbon atoms, and the other rings of the polycyclic assembly may be an aromatic, partially unsaturated, or saturated other ring and the ring atoms of which may be all carbon atoms or optionally includes heteroatoms. It is understood that alicyclyl encompasses cycloalkyl. $(C_X)$alicyclyl and $(C_{X-Y})$alicyclyl are typically used where X and Y indicate the number of carbon atoms in the ring assembly. Examples of alicyclic moieties include, but are not limited to, cyclopropyl, cyclohexane, cyclopentyl, cyclopentenyl, cyclopentadienyl, cyclohexyl, cyclohexenyl, cyclohexadienyl, cycloheptyl, cycloheptenyl, cycloheptadienyl, cyclooctanyl, cyclooctenyl, cyclooctadienyl, inden-1-y1, inden-6-yl, fluorin-1-yl, fluroin-9-yl, 2,3-dihydroquinolin-6-yl, decalin, norbornene, norbornadiene, and the like.

"Aliphatic" means a moiety characterized by a straight or branched chain arrangement of constituent carbon atoms and may be saturated or partially unsaturated with one, two or more double or triple bonds.

"Alkenyl" means a straight or branched, carbon chain that contains at least one carbon-carbon double bond (-CR=CR'- or -CR=CR'R", wherein R, R' and R" are each independently hydrogen or further substituents). Examples of alkenyl include vinyl, allyl, isopropenyl, pentenyl, hexenyl, heptenyl, 1-propenyl, 2-butenyl, 2-methyl-2-butenyl, and the like. In particular embodiments, "alkenyl," either alone or represented along with another radical, can be a $(C_{2-20})$alkenyl, a $(C_{1-15})$alkenyl, a $(C_{2-10})$alkenyl, a $(C_{2-5})$alkenyl or a $(C_{2-3})$alkenyl. Alternatively, "alkenyl," either alone or represented along with another radical, can be a $(C_2)$alkenyl, a $(C_3)$alkenyl or a $(C_4)$alkenyl.

"Alkenylene" means a straight or branched, divalent carbon chain having one or more carbon-carbon double bonds (-CR=CR'-, wherein R and R' are each independently hydrogen or further substituents). Examples of alkenylene include ethene-1,2-diyl, propene-1,3-diyl, methylene-1,1-diyl, and the like. In particular embodiments, "alkenylene," either alone or represented along with another radical, can be a $(C_{2-20})$ alkenylene, a $(C_{2-15})$ alkenylene, a $(C_{2-10})$ alkenylene, a $(C_{2-5})$ alkenylene or a $(C_{2-3})$ alkenylene. Alternatively, "alkenylene," either alone or represented along with another radical, can be a $(C_2)$ alkenylene, a $(C_3)$ alkenylene or a $(C_4)$ alkenylene.

"Alkoxy" means an oxygen moiety having a further alkyl substituent. The alkoxy groups of the present invention can be optionally substituted.

"Alkyl" represented by itself means a straight or branched, saturated, aliphatic radical having a chain of carbon atoms, optionally with one or more of the carbon atoms being replaced with oxygen (See "oxaalkyl"), a carbonyl group (See "oxoalkyl"), sulfur (See "thioalkyl"), and/or nitrogen (See "azaalkyl"). $(C_X)$alkyl and $(C_{X-Y})$alkyl are typically used where X and Y indicate the number of carbon atoms in the chain. For example, $(C_{1-6})$alkyl includes alkyls that have a chain of between 1 and 6 carbons (e.g., methyl, ethyl, propyl, isopropyl, butyl, *sec*-butyl, isobutyl, *tert*-butyl, pentyl, hexyl, and the like). Alkyl represented along with another radical (e.g., as in arylalkyl, heteroarylalkyl and the like) means a straight or branched, saturated aliphatic divalent radical having the number of atoms indicated or when no atoms are indicated means a bond (e.g., $(C_{6-10})$aryl$(C_{1-3})$alkyl includes, benzyl, phenethyl, 1-phenylethyl, 3-phenylpropyl, 2-thienylmethyl, 2-pyridinylmethyl and the like). In particular embodiments, "alkyl," either alone or represented along with another radical, can be a $(C_{1-20})$alkyl, a $(C_{1-15})$alkyl, a $(C_{1-10})$alkyl, a $(C_{1-5})$alkyl or a $(C_{1-3})$alkyl. Alternatively, "alkyl," either alone or represented along with another radical, can be a $(C_1)$alkyl, a $(C_2)$alkyl or a $(C_3)$alkyl.

"Alkylene", unless indicated otherwise, means a straight or branched, saturated or unsaturated, aliphatic, divalent radical. $(C_X)$alkylene and $(C_{X-Y})$alkylene are typically used where X and Y indicate the number of carbon atoms in the chain. For example, $(C_{1-6})$alkylene includes methylene ($-CH_2-$), ethylene ($-CH_2CH_2-$), trimethylene ($-CH_2CH_2CH_2-$), tetramethylene ($-CH_2CH_2CH_2CH_2-$), 2-methyltetramethylene ($-CH_2CH(CH_3)CH_2CH_2-$), pentamethylene ($-CH_2CH_2CH_2CH_2CH_2-$) and the like. In particular embodiments, "alkylene," either alone or represented along with another radical, can be a $(C_{1-20})$alkylene, a $(C_{1-15})$alkylene, a $(C_{1-10})$alkylene, a $(C_{1-5})$alkylene or a $(C_{1-3})$alkylene. Alternatively, "alkylene," either alone or represented along with another radical, can be a $(C_1)$alkylene, a $(C_2)$alkylene or a $(C_3)$alkylene.

"Alkylidene" means a straight or branched, saturated, aliphatic radical connected to the parent molecule by a double bond. $(C_X)$alkylidene and $(C_{X-Y})$alkylidene are typically used where X and Y indicate the number of carbon atoms in the chain. For example, $(C_{1-6})$alkylidene includes methylidene ($=CH_2$), ethylidene ($=CHCH_3$), isopropylidene ($=C(CH_3)_2$), propylidene ($=CHCH_2CH_3$), allylidene ($=CH-CH=CH_2$), and the like. In particular embodiments, "alkylidene," either alone or represented along with another radical, can be a $(C_{1-20})$alkylidene, a $(C_{1-15})$alkylidene, a $(C_{1-10})$alkylidene, a $(C_{1-5})$alkylidene or a $(C_{1-3})$alkylidene. Alternatively, "alkylidene," either alone or represented along with another radical, can be a $(C_1)$alkylidene, a $(C_2)$alkylidene or a $(C_3)$alkylidene.

"Alkynyl" means a straight or branched, carbon chain that contains at least one carbon-carbon triple bond (-C≡C- or -C=CR, wherein R is hydrogen or a further substituent). Examples of alkynyl include ethynyl, propargyl, 3-methyl-1-pentynyl, 2-heptynyl and the like. In particular, embodiments, "alkynyl," either alone or represented along with

another radical, can be a $(C_{2-20})$alkynyl, a $(C_{2-15})$alkynyl, a $(C_{2-10})$alkynyl, a $(C_{2-5})$alkynyl or a $(C_{2-3})$alkynyl. Alternatively, "alkynyl," either alone or represented along with another radical, can be a $(C_2)$alkynyl, a $(C_3)$alkynyl or a $(C_4)$alkynyl.

"Alkynylene" means a straight or branched, divalent carbon chain having one or more carbon-carbon triple bonds (-CR≡CR'-, wherein R and R' are each independently hydrogen or further substituents). Examples of alkynylene include ethyne-1,2-diyl, propyne-1,3-diyl, and the like. In particular embodiments, "alkynylene," either alone or represented along with another radical, can be a $(C_{2-20})$ alkynylene, a $(C_{2-15})$ alkynylene, a $(C_{2-10})$ alkynylene, a $(C_{2-5})$ alkynylene or a $(C_{2-3})$ alkynylene. Alternatively, "alkynylene," either alone or represented along with another radical, can be a $(C_2)$ alkynylene, a $(C_3)$ alkynylene or a $(C_4)$ alkynylene.

"Amido" means the radical -C(=O)-NR-, -C(=O)-NRR', -NR-C(=O)- and/or -NR-C(=O)R', wherein each R and R' are independently hydrogen or a further substituent.

"Amino" means a nitrogen moiety having two further substituents where, for example, a hydrogen or carbon atom is attached to the nitrogen. For example, representative amino groups include $-NH_2$, $-NHCH_3$, $-N(CH_3)_2$, $-NH((C_{1-10})$alkyl), $-N((C_{1-10})alkyl)_2$, -NH(aryl), -NH(heteroaryl), $-N(aryl)_2$, $-N(heteroaryl)_2$, and the like. Optionally, the two substituents together with the nitrogen may also form a ring. Unless indicated otherwise, the compounds of the invention containing amino moieties may include protected derivatives thereof. Suitable protecting groups for amino moieties include acetyl, *tert*-butoxycarbonyl, benzyloxycarbonyl, and the like.

"Animal" includes humans, non-human mammals (e.g., dogs, cats, rabbits, cattle, horses, sheep, goats, swine, deer, and the like) and non-mammals (e.g., birds, and the like).

"Aromatic" means a moiety wherein the constituent atoms make up an unsaturated ring system, where all ring atoms are in continuous pi (π) bonding and the total number of π electrons is equal to 4n+2. An aromatic ring may be such that the ring atoms are only carbon atoms or may include carbon and non-carbon atoms (See "heteroaryl"). Examples of all carbon aromatic rings include benzene, naphthalene and anthracene, and the like. Examples of heteoaromatic rings include pyrrole, thiophene, furan, oxazole, thiazole, imidazole, pyrazole, isoxazole, isothiazole, 1,2,3-oxadiazole, 1,2,3-triazole, 1,3,4-thiadiazole, pyridine, pyrimidine, pyrazine, 1,3,5-triazine, infolizine, indole, isoindole, benzo[*b*]furan, benzo[*b*]thiophene, benzimidazole, benzthiazole, purine, quinoline, isoquinoline, cinnoline, phthalazine, quinazoline, quinoxaline, 1,8-naphthyridine, pteridine, acridine, phenazine, and the like.

"Aryl" refers to a monocyclic or polycyclic radical; when the aryl is monocyclic, it is an aromatic ring and the ring atoms of which are all carbon atoms., and when the aryl is a polycyclic assembly of rings, the ring through which the radical attaches to the parent molecule is an aromatic ring and the ring atoms of which are all carbon atoms, and the other rings of the polycyclic assembly may be an aromatic, partially unsaturated, or saturated ring and the ring atoms of which may be all carbon atoms or optionally includes heteroatoms. Examples of aryl include phenyl, naphthalenyl, anthracenyl, acridin-2-yl, quinolin-6-yl, benzo[b]furan-5-yl, 2,3,4,5-tetrahydro-1H-benzo[e][1,4]diazepin-8-yl, indolinyl, fluorinyl, 2,3-dihydroquinolin-6-yl, decalin, and the like. $(C_X)$aryl and $(C_{X-Y})$aryl are typically used where X and Y indicate the number of carbon atoms in the ring. In particular embodiments, "aryl," either alone or represented along with another radical, can be a $(C_{3-14})$alyl, a $(C_{3-10})$aryl, a $(C_{3-7})$aryl, a $(C_{8-10})$aryl or a $(C_{5-7})$aryl. Alternatively, "aryl," either alone or represented along with another radical, can be a $(C_5)$aryl, a $(C_6)$aryl, a $(C_7)$aryl, a $(C_8)$aryl., a $(C_9)$aryl or a $(C_{10})$aryl.

"Azaalkyl" means an alkyl, as defined above, except where one or more of the carbon atoms forming the alkyl chain are replaced with substituted or unsubstituted nitrogen atoms (-NR- or -NRR', wherein R and R' are each independently hydrogen or further substituents). For example, a $(C_{1-10})$azaalkyl refers to a chain comprising between 1 and 10 carbons and one or more nitrogen atoms.

"Aza-cyclyl" means a heterocyclyl moiety containing at least one nitrogen atom and the point of attachment of the cyclyl is through the nitrogen atom.

"Bicycloalicyclyl" refers to a bicyclic radical comprising two fused, spiro or bridged rings; where the ring through which the radical attaches to the parent molecule is a saturated or partially unsaturated ring and the ring atoms of which are all carbon atoms, and the other ring of the bicyclic assembly may be an aromatic, partially unsaturated, or saturated ring and the ring atoms of which may be all carbon or optionally includes heteroatoms. Examples of bicycloalicyclyl include inden-5-yl, indolin-5-yl, 4,5-dihydrobenzo[*b*]thiophen-5-yl, and the like.

"Bicycloalkyl" refers to a bicyclic radical comprising two fused, spiro or bridged rings; where both rings are saturated rings, and where the ring atoms of the ring through which the radical attaches to the parent molecule are all carbon atoms, and the ring atoms of the other ring may be all carbons or optionally includes heteroatoms. In particular embodiments, "bicycloalkyl," either alone or represented along with another radical, can be a $(C_{4-15})$bicycloalkyl, a $(C_{4-10})$bicycloalkyl, a $(C_{6-10})$bicycloalkyl, or a $(C_{8-10})$bicycloalkyl. Alternatively, "bicycloalkyl," either alone or represented along with another radical, can be a $(C_8)$bicycloalkyl, a $(C_9)$bicycloalkyl or a $(C_{10})$bicycloalkyl. Examples of bicycloalkyl include decahydronaphthalen-2-yl, octahydro-1H-inden-1-yl, decahydroquinolin-6-yl, octahydro-1H-indol-5-yl, and the like.

"Bicycloaryl" refers to a monocyclic or polycyclic radical comprising two fused, spiro or bridged rings; where the ring through which the radical attaches to the parent molecule is an aromatic ring and the ring atoms of which are all carbon atoms, and where the other ring of the bicyclic assembly may be an aromatic, partially unsaturated, or saturated ring and the ring atoms of which may be all carbon or optionally includes heteroatoms. $(C_X)$bicycloaryl and $(C_{X-Y})$

bicycloaryl are typically used where X and Y indicate the number of carbon atoms in the bicyclic ring assembly and directly attached to the ring. In particular embodiments, "bicycloaryl," either alone or represented along with another radical, can be a (a ($C_{4-15}$)bicycloaryl, a ($C_{4-10}$)bicycloaryl, a ($C_{6-10}$)bicycloaryl or a ($C_{8-10}$)bicycloaryl. Alternatively, "bicycloalkyl," either alone or represented along with another radical, can be a ($C_8$)bicycloaryl, a ($C_9$)bicycloaryl or a ($C_{10}$)bicycloaryl. Examples of bicycloaryl include naphthalenyl, quinolin-6-yl, quinoxalin-6-yl, benzimidazol-5-yl, indol-5-yl, and the like.

"Bridging ring" and "bridged ring" as used herein refer to a ring that is bonded to another ring to form a compound having a bicyclic or polycyclic structure where two ring atoms that are common to both rings are not directly bound to each other. Non-exclusive examples of common compounds having a bridging ring include borneol, norbornane, 7-oxabicyclo[2.2.1]heptane, and the like. One or both rings of the bicyclic system may also comprise heteroatoms.

"Carbamoyl" means the radical -OC(O)NRR', wherein R and R' are each independently hydrogen or further substituents.

"Carbocyclic" or "carbocyclyl" refers to a monocyclic or polycyclic radical where all the ring atoms are carbon atoms.

"Carbonyl" means the radical -C(=O)- and/or -C(=O)R, wherein R is hydrogen or a further substituent. It is noted that the carbonyl radical may be further substituted with a variety of substituents to form different carbonyl groups including acids, acid halides, aldehydes, amides, esters, and ketones.

"Carboxy" means the radical -C(=O)-O- and/or -C(=O)-OR, wherein R is hydrogen or a further substituent. It is noted that compounds of the invention containing carboy moieties may include protected derivatives thereof, *i.e.*, where the oxygen is substituted with a protecting group. Suitable protecting groups for carboxy moieties include benzyl, *tert*-butyl, and the like.

"Cyano" means the radical -CN.

"Cycloalkyl" refers to a saturated, monocyclic or polycyclic radical, where when the cycloalky is monocyclic, the ring atoms of the cycloalkyl all carbon atoms, and when the cycloalkyl is a polycyclic assembly of rings, the ring atoms of the ring through which the radical attaches to the parent molecule may be all carbon atoms or optionally include heteroatoms. ($C_X$)cycloalkyl and ($C_{X-Y}$)cycloalkyl are typically used where X and Y indicate the number of carbon atoms in the ring assembly. For example, ($C_{3-10}$)cycloalkyl includes cyclopropyl, cyclobutyl, cyclopentyl, cyclohexyl, cycloheptyl, bicyclo[2.2.2]octyl, adamantan-1-yl, octahydro-1H-inden-2-yl, decahydronaphthyl, oxocyclohexyl, dioxocyclohexyl, thiocyclohexyl, 2-oxobicyclo[2.2.1]hept-1-yl, octahydro-1H-indol-5-yl, decahydroquinolinyl, and the like. In particular embodiments, "cycloalkyl," either alone or represented along with another radical, can be a ($C_{3-14}$)cycloalkyl, a ($C_{3-10}$) cycloalkyl, a ($C_{3-7}$)cycloalkyl, a ($C_{8-10}$)cycloalkyl or a ($C_{5-7}$)cycloalkyl. Alternatively, "cycloalkyl," either alone or represented along with another radical, can be a ($C_5$)cycloalkyl, a ($C_6$)cycloalkyl, a ($C_7$)cycloalkyl, a ($C_8$)cycloalkyl, a ($C_9$) cycloalkyl or a ($C_{10}$)cycloalkyl.

"Cycloalkylene" refers to a divalent cycloalkyl as defined in this Application ($C_X$)cycloalkylene and ($C_{X-Y}$)cycloalkylene are typically used where X and Y indicate the number of carbon atoms in the ring assembly. In particular embodiments, "cycloalkylene," either alone or represented along with another radical, can be a ($C_{3-14}$)cycloaklene, a ($C_{3-10}$)cycloalkylene, a ($C_{3-7}$)cycloalkylene, a ($C_{8-10}$)cycloalkylene or a ($C_{5-7}$)ccloalkylene. Alternatively, "cycloalkylene," either alone or represented along with another radical, can be a ($C_5$)cycloalkylene, a ($C_6$)cycloalkylene, a ($C_7$)cycloalkylene, a ($C_8$)cycloalkylene., a ($C_9$)cycloalkylene or a ($C_{10}$)cycloalkylene.

"Cyclyl" and "cyclic" refers to a radical comprising an array of atoms in continuous arrangement. Cyclyl and cyclic as defined herein may be monocyclic, bicyclic or polycyclic, and include fused, spiro, or bridged polycyclics, and each of the cyclic components may be aromatic, partially unsaturated, or saturated, and the ring atoms are all carbon atoms or optionally one or more of the ring atoms are heteroatoms.

"Disease" specifically includes any unhealthy condition of an animal or part thereof and includes an unhealthy condition that may be caused by, or incident to, medical or veterinary therapy applied to that animal, *i.e.*, the "side effects" of such therapy.

"$EC_{50}$" means the molar concentration of an agonist that produces 50% of the maximal possible effect of that agonist. The action of the agoinst may be stimulatory or inhibitory.

"Fused ring" as used herein refers to a ring that is bonded to another ring to form a compound having a bicyclic or polycyclic structure where the ring atoms that are common to both rings are directly bound to each other. Non-exclusive examples of common fused rings include decalin, naphthalene, anthracene, phenanthrene, indole, furan, benzofuran, quinoline, and the like. Compounds having fused ring systems may be saturated, partially saturated, carbocyclics, heterocyclics, aromatics, heteroaromatics, and the like.

"Halo" means fluoro, chloro, bromo or iodo.

"Heteroalkyl" means alkyl, as defined in this Application, provided that one or more of the atoms within the alkyl chain is a heteroatom. In particular embodiments, "heteroalkyl," either alone or represented along with another radical, can be a hetero($C_{1-20}$)alkyl, a hetero($C_{1-15}$)alkyl, a hetero($C_{1-10}$)alkyl, a hetero($C_{1-5}$)alkyl, a hetero($C_{1-3}$)alkyl or a hetero($C_{1-2}$)alkyl. Alternatively, "heteroalkyl," either alone or represented along with another radical, can be a hetero

$(C_1)$alkyl, a hetero$(C_2)$alkyl or a hetero$(C_3)$alkyl.

"Heteroalicyclyl" or "heterocyclic" means an alicyclyl, as defined in this Application, provided at least one of the ring atoms of the ring through which the heteroalicyclyl attaches is a heteroatom such as nitrogen, oxygen and sulfur. The nitrogen atoms can be optionally quaternerized or oxidized and the sulfur atoms can be optionally oxidized. $(C_X)$ heteroalicyclyl and hetero$(C_{X-Y})$alicyclyl are typically used where X and Y indicate the number of carbon atoms in the ring assembly. Examples of heteroalicyclyl include pyrroline, imidazoline, pyrazoline, pyran, indene, indoline, fluorine, and the like.

"Heteroaryl" refers to an aryl as defined in this Application provided that at least one of the ring atoms of the ring through which the heteroaryl attaches to the parent molecule is a heteroatom. Monocyclic heteroaryl groups include, but are not limited to, cyclic aromatic groups having five or six ring atoms, wherein at least one ring atom is a heteroatom and the remaining ring atoms are carbon. The nitrogen atoms can be optionally quaternerized and the sulfur atoms can be optionally oxidized. Heteroaryl groups of this Application include, but are not limited to, furanyl, imidazolyl, isothiazolyl, isoxazolyl, oxadiazolyl, oxazolyl, 1,2,3-oxadiazolyl, pyrazinyl, pyrazolyl, pyridazinyl, pyridinyl, pyrimidinyl, pyrrolinyl, thiazolyl, 1,3,4-thiadiazolyl, triazolyl and tetrazolyl. "Bicyclic or tricyclic heteroaryls" include, but are not limited to, benzo [*b*]furan-3-yl, benzo[b]thiophen-3-yl, benzimidazol-2-yl, imidazo[4,5-c]pyridinyl, quinazolin-2-yl, and the likes. The heteroaryl groups of this invention can be substituted or unsubstituted. In particular embodiments, "heteroaryl," either alone or represented along with another radical, can be a hetero$(C_{1-13})$aryl, a hetero$(C_{2-13})$aryl, a hetero$(C_{2-6})$aryl, a hetero $(C_{3-9})$aryl or a hetero$(C_{5-9})$aryl. Alternatively, "heteroaryl," either alone or represented along with another radical, can be a hetero$(C_3)$aryl, a hetero$(C_4)$aryl, a hetero$(C_5)$aryl, a hetero$(C_6)$aryl, a hetero$(C_7)$aryl, a hetero$(C_8)$aryl or a hetero $(C_9)$aryl.

"Heteroatom" refers to an atom that is not a carbon atom. Particular examples of heteroatoms include, but are not limited to, nitrogen, oxygen, and sulfur.

"Heteroatom moiety" includes a moiety where the atom by which the moiety is attached is not a carbon. Examples of heteroatom moieties include -NR-, $-N^+(O^-)=$, -O-, -S- or $-S(O)_2-$, wherein R is hydrogen or a further substituent.

"Heterobicycloalicyclyl refers to a bicycloalicyclic radical as defined in this Application provided that at least one of the ring atoms of the ring through which the heterobicycloalicyclic radical attaches to the parent molecule is a heteroatom.

"Heterobicycloalkyl" means a bicycloalkyl radical as defined in this Application, provided that at least one of the ring atoms of the ring that is a component of the bicyclic radical and through which the heterocycloalkyl ring assembly attaches is a heteroatom. For example hetero$(C_{9-12})$bicycloalkyl as used in this application includes, but is not limited to, 3-aza-bicyclo[4.1.0]hept-3-yl, 2-aza-bicyclo[3.1.0]hex-2-yl, 3-aza-bicyclo[3.1.0]hex-3-yl, and the like. In particular embodiments, "heterobicycloalkyl," either alone or represented along with another radical, can be a hetero$(C_{1-14})$bicycloalkyl, a hetero$(C_{4-14})$bicycloalkyl, a hetero$(C_{4-9})$bicycloalkyl or a hetero$(C_{5-9})$bicycloalkyl. Alternatively, "heterobicycloalkyl," either alone or represented along with another radical, can be a hetero$(C_5)$bicycloalkyl, hetero$(C_6)$bicycloalkyl, hetero$(C_7)$bicycloalkyl, hetero$(C_8)$bicycloalkyl or a hetero$(C_9)$bicycloalkyl.

"Heterobicycloaryl" means a bicycloaryl radical as defined in this Application, provided that at least one of the ring atoms of the ring which is a component of the bicyclic radical and through which the heterobicycloaryl radical attaches to the parent atom is a heteroatom. For example, hetero$(C_{4-12})$bicycloaryl as used in this Application includes, but is not limited to, 2-amino-4-oxo-3,4-dihydropteridin-6-yl, quinolin-2-yl, and the like. In particular embodiments, "heterobicycloaryl," either alone or represented along with another radical, can be a hetero$(C_{1-14})$bicycloaryl, a hetero$(C_{4-14})$ bicycloaryl, a hetero$(C_{4-9})$bicycloarylor a hetero$(C_{5-9})$bicycloaryl. Alternatively, "heterobicycloaryl," either alone or represented along with another radical, can be a hetero$(C_5)$bicycloaryl, hetero$(C_6)$bicycloaryl, hetero$(C_7)$bicycloaryl, hetero $(C_8)$bicycloaryl or a hetero$(C_9)$bicycloaryl.

"Heterocycloalkyl" means a cycloalkyl radical as defined in this Application, provided that at least one of the ring atoms of the ring through which the heterocycloalkyl radical attaches to the parent atom is a heteroatom. The heteroatom is independently selected from N, O, or S. Non-exclusive examples of heterocycloalkyl include piperidyl, 4-morpholyl, 4-piperazinyl, pyrrolidinyl, perhydropyrrolizinyl, 1,2-pyrazolidin-4-yl, 1,3-dioxanyl, 1,4-dioxoianyl and the like. In particular embodiments, "heterocycloalkyl," either alone or represented along with another radical, can be a hetero $(C_{1-13})$cycloalkyl, a hetero$(C_{1-9})$cycloalkyl, a hetero$(C_{1-6})$cycloalkyl, a hetero$(C_{5-9})$cycloalkyl or a hetero$(C_{2-6})$cycloalkyl. Alternatively, "heterocycloalkyl," either alone or represented along with another radical, can be a hetero$(C_2)$cycloalkyl, a hetero$(C_3)$cycloalkyl, a hetero$(C_4)$cycloalkyl, a hetero$(C_5)$cycloalkyl, a hetero$(C_6)$cycloalkyl, hetero$(C_7)$cycloalkyl, hetero$(C_8)$cycloalkyl or a hetero$(C_9)$cycloalkyl.

"Heterocycloalkylene" means a divalent heterocycloalkyl, wherein the heterocycloalkyl is as defined in this Application. In particular embodiments, "heterocycloalkylene," either alone or represented along with another radical, can be a hetero$(C_{1-13})$cycloalkylene, a hetero$(C_{1-9})$cycloalkylene, a hetero$(C_{1-6})$cycloalkylene, a hetero$(C_{5-9})$cycloalkylene or a hetero$(C_{2-6})$cycloalkylene. Alternatively, "heterocycloalkylene," either alone or represented along with another radical, can be a hetero$(C_2)$cycloalkylene, a hetero$(C_3)$cycloalkylene, a hetero$(C_4)$cycloalkylene, a hetero$(C_5)$ cycloalkylene, a hetero$(C_6)$cycloalkylene, hetero$(C_7)$cycloalkylene, hetero$(C_8)$cycloalkylene or a hetero$(C_9)$cy-

cloalkylene.

"heterocyclyl" means a cyclyl as defined in this Application provided that at least one of the ring atoms is a heteroatom.

"Hydroxy" means the radical -OH.

"$IC_{50}$" means the molar concentration of an inhibitor that produces 50% inhibition of the target enzyme.

"Imino" means the radical -CR(=NR') and/or -C(-NR')-, wherein R and R' are each independently hydrogen or a further substituent.

"Iminoketone derivative" means a derivative comprising the moiety -C(NR)-, wherein R is hydrogen or a further substituent.

"Isomers" means compounds having identical molecular formulae but differing in the nature or sequence of bonding of their atoms or in the arrangement of their atoms in space. Isomers that differ in the arrangement of their atoms in space are termed "stereoisomers." Stereoisomers that are not mirror images of one another are termed "diastereomers" and stereoisomers that are nonsuperimposable mirror images are termed "enantiomers" or sometimes "optical isomers." A carbon atom bonded to four nonidentical substituents is termed a "chiral center." A compound with one chiral center has two enantiomeric forms of opposite chirality. A mixture of the two enantiomeric forms is termed a "racemic mixture." A compound that has more than one chiral center has $2^{n-1}$ enantiomeric pairs, where *n* is the number of chiral centers. Compounds with more than one chiral center may exist as ether an individual diastereomer or as a mixture of diastereomers, termed a "diastereomeric mixture." When one chiral center is present a stereoisomer may be characterized by the absolute configuration of that chiral center. Absolute configuration refers to the arrangement in space of the substituents attached to the chiral center. Enantiomers are characterized by the absolute configuration of their chiral centers and described by the *R*- and *S*-sequencing rules of Cahn, Ingold and Prelog. Conventions for stereochemical nomenclature, methods for the determination of stereochemistry and the separation of stereoisomers are well known in the art (*e.g*., see "Advanced Organic Chemistry", 5th edition, March, Jerry, John Wiley & Sons, New York, 2001).

"Leaving group" means the group with the meaning conventionally associated with it in synthetic organic chemistry, *i.e*., an atom or group displaceable under reaction (*e.g*., alkylating) conditions. Examples of leaving groups include, but are not limited to, halo (*e.g*., F, Cl, Br and I), alkyl (*e.g*., methyl and ethyl) and sulfonyloxy (*e.g*., mesyloxy, ethanesulfonyloxy, benzenesulfonyloxy and tosyloxy), thiomethyl, thienyloxy, dihalophosphinoyloxy, tetrahalophosphoxy, benzyloxy, isopropyloxy, acyloxy, and the like.

"mTOR" refers to the enzyme that is the mammalian target of rapamycin (also known as FRAP, RAFT1 or RAPT). mTOR is a member of the PIKK family (PI3K-related protein kinase) of serine/threonine protein kinases and also involved in PI3K-mediated signaling.

"Nitro" means the radical $-NO_2$.

"Oxaalkyl" means an alkyl, as defined above, except where one or more of the carbon atoms forming the alkyl chain are replaced with oxygen atoms (-O- or -OR, wherein R is hydrogen or a further substituent). For example, an oxa($C_{1-10}$)alkyl refers to a chain comprising between 1 and 10 carbons and one or more oxygen atoms.

"Oxoalkyl" means an alkyl, as defined above, except where one or more of the carbon atoms forming the alkyl chain are replaced with carbonyl groups (-C(=O)- or -C(=O)-R, wherein R is hydrogen or a further substituent). The carbonyl group may be an aldehyde, ketone, ester, amide, acid or acid halide. For example, an oxo($C_{1-10}$)alkyl refers to a chain comprising between 1 and 10 carbon atoms and one or more carbonyl groups.

"Oxy" means the radical -O- or -OR, wherein R is hydrogen or a further substituent. Accordingly, it is noted that the oxy radical may be further substituted with a variety of substituents to form different oxy groups including hydroxy, alkoxy, aryloxy, heteroaryloxy or carbonyloxy.

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

"Pharmaceutically acceptable salts" means salts of compounds of the present invention which are pharmaceutically acceptable, as defined above, and which possess the desired pharmacological activity. Such salts include acid addition salts formed with inorganic acids such as hydrochloric acid, hydrobromic acid, sulfuric acid, nitric acid, phosphoric acid, and the like; or with organic acids such as acetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, *o*-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, 2-naphthalenesulfonic acid, p-toluenesulfonic acid, camphorsulfonic acid, 4-methylbicyclo[2.2.2]oct-2-ene-1-carboxylic acid, glucoheptonic acid, 4,4'-methylenebis(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid and the like.

Pharmaceutically acceptable salts also include base addition salts which may be formed when acidic protons present are capable of reacting with inorganic or organic bases. Acceptable inorganic bases include sodium hydroxide,

sodium carbonate, potassium hydroxide, aluminum hydroxide and calcium hydroxide. Acceptable organic bases include ethanolamine, diethanolamine, triethanolamine, tromethamine, *N*-methylglucamine and the like.

"PI3K" refers to the phosphatidylinositol-3-kinase (PI3K) family of kinases. These kinases display sequence homology within their kinase domains, but with distinct substrate specificities, expression patterns, and modes of regulation. The kinases of the PI3K family are subdivided into four classes. Classes I, II and III are lipid kinases that phosphorylate phosphoinositide lipids. Class I PI3Ks include four isoforms: p110α (PI3Kα), p110β (PI3Kβ), p110δ (PI3Kδ), and p110γ (PI3Kγ), and are further subdivided into IA or IB according to their regulatory subunit. Class IA, which includes PI3Kα, PI3Kβ and PI3Kδ, has a p85 regulatory subunit containing two SH2 domains that is activated by interaction with a receptor tyrosine kinase. Class IB, which includes PI3Kγ, has a p101 regulatory subunit that is activated by GPCRs. Class II composes of four members (CIIα, CIIβ, and CIIδ) and Class III compose of a single member (Vps34). Class IV kinases, also call PIKKs (PI3K-related protein kinase) are a subfamily of serine/threonine protein kinases that share homology with PI3K, but phosphorylate proteins rather than phosphoinositide lipids. The PIKKs include mTOR (mammalian target of rapamycin).

"PI3K and/or mTOR" when refers to inhibitory compounds, means that the compound is a PI3K inhibitor, an mTOR inhibitor, or a dual PI3K and mTOR inhibitor. "PI3K/mTOR" is used as an abbreviation for "PI3K and/or mTOR".

"Polycyclic ring" includes bicyclic and multi-cyclic rings. The individual rings comprising the polycyclic ring can be fused, spiro or bridging rings.

"Prodrug" means a compound that is convertible *in vivo* metabolically into an inhibitor according to the present invention. The prodrug itself may or may not also have activity with respect to a given target protein. For example, a compound comprising a hydroxy group may be administered as an ester that is converted by hydrolysis *in vivo* to the hydroxy compound. Suitable esters that may be converted *in vivo* into hydroxy compounds include acetates, citrates, lactates, phosphates, tartrates, malonates, oxalates, salicylates, propionates, succinates, fumarates, maleates, methylene-bis-b-hydroxynaphthoates, gentisates, isethionates, di-*p*-toluoyltartrates, methanesulfonates, ethanesulfonates, benzenesulfonates, *p*-toluenesulfonates, cyclohexylsulfamates, quinates, esters of amino acids, and the like. Similarly, a compound comprising an amine group may be administered as an amide that is converted by hydrolysis *in* vivo to the amine compound.

"Protected derivatives" means derivatives of inhibitors in which a reactive site or sites are blocked with protecting groups. Protected derivatives are useful in the preparation of inhibitors or in themselves may be active as inhibitors. A comprehensive list of suitable protecting groups can be found in P.G.M. Wuts and T.W. Greene, "Greene's Protecting Groups in Organic Synthesis, 4th edition, John Wiley & Sons, Inc. 2007.

"Ring" and "ring assembly" means a carbocyclic or a heterocyclic system and includes aromatic and non-aromatic systems. The system can be monocyclic, bicyclic or polycyclic. In addition, for bicyclic and polycyclic systems, the individual rings comprising the polycyclic ring can be fused, spiro or bridging rings.

"Subject" and "patient" includes humans, non-human mammals (*e.g.*, dogs, cats, rabbits, cattle, horses, sheep, goats, swine, deer, and the like) and non-mammals (*e.g.*, birds, and the like).

"Substituted or unsubstituted" means that a given moiety may consist of only hydrogen substituents through available valencies (unsubstituted) or may further comprise one or more non-hydrogen substituents through available valencies (substituted) that are not otherwise specified by the name of the given moiety. For example, isopropyl is an example of an ethylene moiety that is substituted by $-CH_3$. In general, a non-hydrogen substituent may be any substituent that may be bound to an atom of the given moiety that is specified to be substituted. Examples of substituents include, but are not limited to, aldehyde, alicyclic, aliphatic, $(C_{1-10})$alkyl, alkylene, alkylidene, amide, amino, aminoalkyl, aromatic, aryl, bicycloalkyl, bicycloaryl, carbamoyl, carbocyclyl, carboxyl, carbonyl group, cycloalkyl, cycloalkylene, ester, halo, heterobicycloalkyl, heterocycloalkylene, heteroaryl, heterobicycloaryl, heterocycloalkyl, oxo, hydroxy, iminoketone, ketone, nitro, oxaalkyl, and oxoalkyl moieties, each of which may optionally also be substituted or unsubstituted. In one particular embodiment, examples of substituents include, but are not limited to, hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, sulfonylamino, sulfinyl, $(C_{1-10})$alkyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, $(C_{1-10})$azaalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, $(C_{3-12})$cycloalkyl, hetero$(C_{3-12})$cycloalkyl, $(C_{9-12})$bicycloalkyl, hetero$(C_{3-12})$bicycloalkyl, $(C_{4-12})$aryl, hetero$(C_{1-10})$aryl, $(C_{9-12})$bicycloaryl and hetero$(C_{4-12})$bicycloaryl. In addition, the substituent is itself optionally substituted by a further substituent. In one particular embodiment, examples of the further substituent include, but are not limited to, hydrogen, halo, nitro, cyano, hydroxy, $(C_{1-10})$alkoxy, $(C_{4-12})$aryloxy, hetero$(C_{1-10})$aryloxy, amino, $(C_{1-10})$alkylamino, $(C_{1-10})$alkyl, halo$(C_{1-10})$alkyl, hydroxy$(C_{1-10})$alkyl, carbonyl$(C_{1-10})$alkyl, thiocarbonyl$(C_{1-10})$akyl, sulfonyl$(C_{1-10})$alkyl, sulfinyl$(C_{1-10})$alkyl, $(C_{1-10})$azaalkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, $(C_{3-12})$cycloalkyl, hetero$(C_{3-12})$cycloalkyl, $(C_{9-12})$bicycloalkyl, hetero$(C_{3-12})$bicycloalkyl, $(C_{4-12})$aryl, hetero$(C_{1-10})$aryl, $(C_{9-12})$bicycloaryl and hetero$(C_{4-12})$bicycloaryl.

"Sulfinyl" means the radical -SO- and/or -SO-R, wherein R is hydrogen or a further substituent. It is noted that the sulfinyl radical may be further substituted with a variety of substituents to form different sulfinyl groups including sulfinic acids, sulfinamides, sulfinyl esters, and sulfoxides.

"Sulfonyl" means the radical $-SO_2-$ and/or $-SO_2-R$, wherein R is hydrogen or a further substituent. It is noted that the sulfonyl radical may be further substituted with a variety of substituents to form different sulfonyl groups including sulfonic acids, sulfonamides, sulfonate esters, and sulfones.

"Therapeutically effective amount" means that amount which, when administered to an animal for treating a disease, is sufficient to effect such treatment for the disease.

"Thio" denotes replacement of an oxygen by a sulfur and includes, but is not limited to, -SR, -S- and =S containing groups.

"Thioalkyl" means an alkyl, as defined above, except where one or more of the carbon atoms forming the alkyl chain are replaced with sulfur atoms (-S- or -S-R, wherein R is hydrogen or a further substituent). For example, a thio $(C_{1-10})$alkyl refers to a chain comprising between 1 and 10 carbons and one or more sulfur atoms.

"Thiocarbonyl" means the radical -C(=S)- and/or -C(=S)-R, wherein R is hydrogen or a further substituent. It is noted that the thiocarbonyl radical may be further substituted with a variety of substituents to form different thiocarbonyl groups including thioacids, thioamides, thioesters, and thioketones.

"Treatment" or "treating" means any administration of a compound of the present invention and includes:

(1) preventing the disease from occurring in an animal which may be predisposed to the disease but does not yet experience or display the pathology or symptomatology of the disease,
(2) inhibiting the disease in an animal that is experiencing or displaying the pathology or symptomatology of the diseased (*i.e.*, arresting further development of the pathology and/or symptomatology), or
(3) ameliorating the disease in an animal that is experiencing or displaying the pathology or symptomatology of the diseased (*i.e.*, reversing the pathology and/or symptomatology).

It is noted in regard to all of the definitions provided herein that the definitions should be interpreted as being open ended in the sense that further substituents beyond those specified may be included. Hence, a $C_1$ alkyl indicates that there is one carbon atom but does not indicate what are the substituents on the carbon atom. Hence, a $(C_1)$alkyl comprises methyl (*i.e.*, $-CH_3$) as well as -CRR'R" where R, R', and R" may each independently be hydrogen or a further substituent where the atom attached to the carbon is a heteroatom or cyano. Hence, $CF_3$, $CH_2OH$ and $CH_2CN$, for example, are all $(C_1)$alkyls. Similarly, terms such as alkylamino and the like comprise dialkylamino and the like.

A compound having a formula that is represented with a dashed bond is intended to include the formulae optionally having zero, one or more double bonds, as exemplified and shown below:

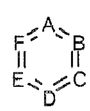

represents

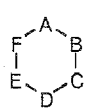 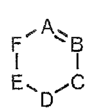 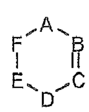 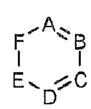 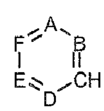 ,

etc.

In addition, atoms making up the compounds of the present invention are intended to include all isotopic forms of such atoms. Isotopes, as used herein, include those atoms having the same atomic number but different mass numbers. By way of general example and without limitation, isotopes of hydrogen include tritium and deuterium, and isotopes of carbon include $^{13}C$ and $^{14}C$.

## DETAILED DESCRIPTION OF THE INVENTION

The present invention relates to compounds that may be used to inhibit PI3K/mTOR. The present invention also relates to pharmaceutical compositions, kits and articles of manufacture comprising such compounds. In addition,

the present invention relates to methods and intermediates useful for making the compounds. Further, the present invention relates to methods of using said compounds. It is noted that the compounds of the present invention may also possess activity for other members of the same protein family and thus may be used to address disease states associated with these other family members.

It is noted that the compounds of the present invention may also possess inhibitory activity for other kinase family members and thus may be used to address disease states associated with these other family members.

## Compound of the Invention

In one of its aspects, the present invention relates to compounds that are useful as PI3K and/or mTOR (PI3K/mTOR) inhibitors.

In one embodiment, PI3K/mTOR inhibitors of the present invention consist of the formula:

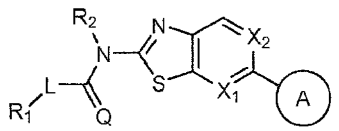

or a hydrate, solvate, stereoisomer, tautomer, or a pharmaceutically acceptable salt thereof, wherein

ring A is aryl or heteroaryl, each unsubstituted or substituted with 1-5 substituents which is independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, carbonylamino, sulfonylamino, $(C_{1-10})$alkylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl;

Q is O or S;

L is selected from the group consisting of $-CHR_3-$, $-NR_4-$ and -O-;

$X_1$ is selected from the group consisting of $CR_5$ and N;

$X_2$ is selected from the group consisting of $CR_5$, and N;

$R_1$ and $R_3$ are each independently selected from the group consisting of hydrogen, halo, for nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted, or $R_1$ and $R_3$ are taken together to form a cyclopropyl;

$R_2$ is selected from the group consisting of hydrogen, $(C_{1-6})$alkoxy, carbonyl, oxycarbonyl, aminocarbonyl, amino, $(C_{1-6})$alkylamino, sulfonyl, sulfinyl, $(C_{1-6})$alkyl, $(C_{1-10})$alkenyl, $(C_{1-10})$alkynyl, hetero$(C_{1-6})$alkyl, halo$(C_{1-6})$alkyl, hydroxy$(C_{1-6})$alkyl, carbonyl$(C_{1-6})$alkyl, $(C_{3-6})$cycloalkyl$(C_{1-3})$alkyl, hetero$(C_{1-5})$cycloalkyl$(C_{1-3})$alkyl, $(C_{3-6})$alicyclyl, and hetero$(C_{1-5})$alicyclyl, each substituted or unsubstituted;

$R_4$ is selected from the group consisting of hydrogen, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, imino, sulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl,

($C_{9-12}$)bicycloaryl($C_{1-5}$)alkyl, hetero($C_{8-12}$)bicycloaryl($C_{1-5}$)alkyl, hetero($C_{1-10}$)akyl, ($C_{3-12}$)alicyclyl, hetero($C_{3-12}$)alicyclyl, ($C_{9-12}$)bicycloalicyclyl, hetero($C_{3-12}$)bicycloalicyclyl, ($C_{4-12}$)aryl, hetero($C_{1-10}$)aryl, ($C_{9-12}$)bicycloaryl, and hetero($C_{4-12}$)bicycloaryl, each substituted or unsubstituted; and

$R_5$ and $R_{5'}$ are each independently selected from the group consisting of hydrogen, halo, nitro, cyano, thio, oxy, hydroxy, carbonyloxy, ($C_{1-10}$)alkoxy, ($C_{4-12}$)aryloxy, hetero($C_{1-10}$)aryloxy, carbonyl, oxycarbonyl, aminocarbonyl, amino, ($C_{1-10}$)alkylamino, sulfonylamino, imino, sulfonyl, sulfinyl, ($C_{1-10}$)alkyl, ($C_{1-10}$)alkenyl, ($C_{1-10}$)alkynyl, halo($C_{1-10}$)alkyl, hydroxy($C_{1-10}$)alkyl, carbonyl($C_{1-10}$)alkyl, thiocarbonyl($C_{1-10}$)alkyl, sulfonyl($C_{1-10}$)alkyl, sulfinyl($C_{1-10}$)alkyl, aza($C_{1-10}$)alkyl, ($C_{1-10}$)oxaalkyl, ($C_{1-10}$)oxoalkyl, imino($C_{1-10}$)alkyl, ($C_{3-12}$)cycloalkyl($C_{1-5}$)alkyl, hetero($C_{3-12}$)cycloalkyl($C_{1-10}$)alkyl, aryl($C_{1-10}$)alkyl, hetero($C_{1-10}$)aryl($C_{1-5}$)alkyl, ($C_{9-12}$)bicycloaryl($C_{1-5}$)alkyl, hetero($C_{8-12}$)bicycloaryl($C_{1-5}$)alkyl, hetero($C_{1-10}$)alkyl, ($C_{3-12}$)alicyclyl, hetero($C_{3-12}$)alicyclyl, ($C_{9-12}$)bicycloalicyclyl, hetero($C_{3-12}$)bicycloalicyclyl, ($C_{4-12}$)aryl, hetero($C_{1-10}$)aryl, ($C_{9-12}$)bicycloaryl, and hetero($C_{4-12}$)bicycloaryl, each substituted or unsubstituted;

provided that

when $X_1$ is N or CH, $X_2$ is CH, Q is O, L is-$CH_2$-, -NH- or O, $R_1$ is hydrogen, ($C_{1-4}$)alkyl or hetero($C_6$)cycloalkyl, and ring A is a pyridyl, pyrimidinyl or pyrazinyl, ring A does not have an amino, substituted amino, substituted oxy, or fluoro attached to position 4 of ring A where the ring atom through which ring A attaches to the ring containing $X_1$ and $X_2$ is counted as position 1;

when $X_1$ is CH, $X_2$ is CH, Q is O, L is -$CHR_3$-, $R_1$ and $R_3$ are both hydrogen, and ring A is a five- or six-membered heteroaryl comprising one nitrogen ring atom, said one nitrogen ring atom is not located at a position adjacent to the ring atom through which said heteroaryl attaches to the ring containing $X_1$ and $X_2$;

when $X_1$ is CH, $X_2$ is CH, Q is O, and L is -$CHR_3$-, $R_1$ and $R_3$ are both hydrogen, and ring A is a pyrimidinyl, the ring atom located at position 4 of said pyrimidinyl is not a nitrogen where the ring atom through which said pyrimidinyl attaches to the ring containing $X_1$ and $X_2$ is counted as at position 1;

when $X_1$ is CH, $X_2$ is CH, Q is O, L is -NH-, and ring A is as defined above, $R_1$ is not selected from the group consisting of alkyl, carbonyl, and phenoxy substituted pyridinyl;

when $X_1$ is N, $X_2$ is CH, Q is O, L is -$CHR_3$-, and ring A is as defined above, $R_1$ and $R_3$ are both not a substituted phenyl;

when $X_1$ is CH, $X_2$ is CH, Q is O, L is -$CH_2$-, and ring A is substituted or unsubstituted ($C_{6-10}$)aryl, $R_1$ is not a halo, or a thiocarbonyl substituted amino group; and

the compounds of the invention do not include

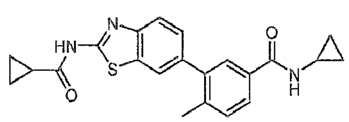

(3-(2-(cyclopropanecarboxamido)benzo[*d*]thiazol-6-yl)-*N*-cyclopropyl-4-methylbenzamide).

In another embodiment, PI3K/mTOR inhibitors of the present invention consisting the formula:

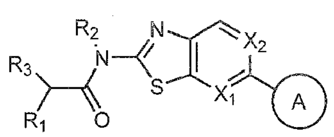

or a hydrate, solvate, stereoisomer, tautomer, or a pharmaceutically acceptable salt thereof,
wherein

ring A is aryl or heteroaryl, each unsubstituted or substituted with 1-5 substituents which is independently selected from the group consisting of hydrogen, halo, nitro, cyano, thio, oxy, hydroxy, carbonyloxy, ($C_{1-10}$)alkoxy, ($C_{4-12}$)aryloxy, hetero($C_{1-10}$)aryloxy, carbonyl, oxycarbonyl, aminocarbonyl, amino, carbonylamino, sulfonylamino, ($C_{1-10}$)alkylamino, imino, sulfonyl, aminosulfonyl, sulfinyl, ($C_{1-10}$)alkyl, ($C_{1-10}$)alkenyl, ($C_{1-10}$)alkynyl, halo($C_{1-10}$)alkyl, hydroxy($C_{1-10}$)alkyl, carbonyl($C_{1-10}$)alkyl, thiocarbonyl($C_{1-10}$)alkyl, sulfonyl($C_{1-10}$)alkyl, sulfinyl($C_{1-10}$)alkyl, aza($C_{1-10}$)alkyl, ($C_{1-10}$)oxaalkyl, ($C_{1-10}$)oxoalkyl, imino($C_{1-10}$)alkyl, ($C_{3-12}$)cycloalkyl($C_{1-5}$)alkyl, hetero($C_{3-12}$)cycloalkyl($C_{1-10}$)alkyl, aryl($C_{1-10}$)alkyl, hetero($C_{1-10}$)aryl($C_{1-5}$)alkyl, ($C_{9-12}$)bicycloaryl($C_{1-5}$)alkyl, hetero($C_{8-12}$)bicycloaryl($C_{1-5}$)alkyl,

hetero($C_{1-10}$)alkyl, ($C_{3-12}$)alicyclyl, hetero($C_{3-12}$)alicyclyl, ($C_{9-12}$)bicycloalicyclyl, hetero($C_{3-12}$)bicycloalicyclyl, ($C_{4-12}$)aryl, hetero($C_{1-10}$)aryl, ($C_{9-12}$)bicycloaryl, and hetero($C_{4-12}$)bicycloaryl, each unsubstituted or further substituted with 1-2 substituents independently selected from the group consisting of halo, cyano, ($C_{1-6}$)alkyl, hydroxyl ($C_{1-6}$)alkyl, halo($C_{1-6}$)alkyl, amino($C_{1-6}$)alkyl, ($C_{3-6}$)alicyclyl($C_{1-6}$)alkyl, hetero($C_{1-5}$)alicyclyl($C_{1-6}$)alkyl, ($C_{4-6}$)aryl ($C_{1-6}$)alkyl, hetero($C_{1-5}$)aryl($C_{1-6}$)alkyl, amino, ($C_{1-6}$)alkoxy, ($C_{3-6}$)alicyclyl, hetero($C_{1-5}$)alicyclyl, ($C_{4-6}$)aryl, hetero ($C_{1-5}$)aryl;

$X_1$ is selected from the group consisting of $CR_5$ and N;

$X_2$ is selected from the group consisting of $CR_{5'}$ and N;

$R_1$ and $R_3$ are each independently selected from the group consisting of hydrogen, halo, for nitro, cyano, thio, oxy, hydroxy, carbonyloxy, ($C_{1-10}$)alkoxy, ($C_{4-12}$)aryloxy, hetero($C_{1-10}$)aryloxy, carbonyl, oxycarbonyl, aminocarbonyl, amino, ($C_{1-10}$)alkylamino, sulfonylamino, imino, sulfonyl, sulfinyl, ($C_{1-10}$)alkyl, ($C_{1-10}$)alkenyl, ($C_{1-10}$)alkynyl, halo ($C_{1-10}$)alkyl, hydroxy($C_{1-10}$)alkyl, carbonyl($C_{1-10}$)alkyl, thiocarbonyl($C_{1-10}$)alkyl, sulfonyl($C_{1-10}$)alkyl, sulfinyl($C_{1-10}$) alkyl, aza($C_{1-10}$)alkyl, ($C_{1-10}$)oxaalkyl, ($C_{1-10}$)oxoalkyl, imino($C_{1-10}$)alkyl, ($C_{3-12}$)cycloalkyl($C_{1-5}$)alkyl, hetero($C_{3-12}$) cycloalkyl($C_{1-10}$)alkyl, aryl($C_{1-10}$)alkyl, hetero($C_{1-10}$)aryl($C_{1-5}$)alkyl, ($C_{9-12}$)bicycloaryl($C_{1-5}$)alkyl, hetero($C_{8-12}$)bicycloaryl($C_{1-5}$)alkyl, hetero($C_{1-10}$)alkyl, ($C_{3-12}$)alicyclyl, hetero($C_{3-12}$)alicyclyl, ($C_{9-12}$)bicycloalicyclyl, hetero($C_{3-12}$)bicycloalicyclyl, ($C_{4-12}$)aryl, hetero($C_{1-10}$)aryl, ($C_{9-12}$)bicycloaryl, and hetero($C_{4-12}$)bicycloaryl, each substituted or unsubstituted, or $R_1$ and $R_3$ are taken together to form a cyclopropyl;

$R_2$ is selected from the group consisting of hydrogen, ($C_{1-6}$)alkoxy, carbonyl, oxycarbonyl, aminocarbonyl, amino, ($C_{1-6}$)alkylamino, sulfonyl, sulfinyl, ($C_{1-6}$)alkyl, ($C_{1-10}$)alkenyl, ($C_{1-10}$)alkynyl, hetero($C_{1-6}$)alkyl, halo($C_{1-6}$)alkyl, hydroxy($C_{1-6}$)alkyl, carbonyl($C_{1-6}$)alkyl, ($C_{3-6}$)cycloalkyl($C_{1-3}$)alkyl, hetero($C_{1-5}$)cycloalkyl($C_{1-3}$)alky, ($C_{1-6}$)alicyclyl, and hetero($C_{1-5}$)alicyclyl, each substituted or unsubstituted; and

$R_5$ and $R_{5'}$ are each independently selected from the group consisting of hydrogen, halo, nitro, cyano, thio, oxy, hydroxy, carbonyloxy, ($C_{1-10}$)alkoxy, ($C_{4-12}$)aryloxy, hetero($C_{1-10}$)aryloxy, carbonyl, oxycarbonyl, aminocarbonyl, amino, ($C_{1-10}$)alkylamino, sulfonylamino, imino, sulfonyl, sulfonyl, ($C_{1-10}$)alkyl, ($C_{1-10}$)alkenyl, ($C_{1-10}$)alkynyl, halo ($C_{1-10}$)alkyl, hydroxy($C_{1-10}$)alkyl, carbonyl($C_{1-10}$)alkyl, thiocarbonyl($C_{1-10}$)alkyl, sulfonyl($C_{1-10}$)alkyl, sulfinyl($C_{1-10}$) alkyl, aza($C_{1-10}$)alkyl, ($C_{1-10}$)oxaalkyl, ($C_{1-10}$)oxoalkyl, imino($C_{1-10}$)alkyl, ($C_{3-12}$)cyctoalkyl($C_{1-5}$)alkyl, hetero($C_{3-12}$) cycloalkyl($C_{1-10}$)alkyl, aryl($C_{1-10}$)alkyl, hetero($C_{1-10}$)aryl($C_{1-5}$)alkyl, ($C_{9-12}$)bicycloaryl($C_{1-5}$)alkyl, hetero($C_{8-12}$)bicycloaryl($C_{1-5}$)alkyl, hetero($C_{1-10}$)alkyl, ($C_{3-12}$)alicyclyl, hetero($C_{3-12}$)alicyclyl, ($C_{9-12}$)bicycloalicyclyl, hetero($C_{3-12}$)bicycloalicyclyl, ($C_{4-12}$)aryl, hetero($C_{1-10}$)aryl, ($C_{9-12}$)bicycloaryl, and hetero($C_{4-12}$)bicycloaryl, each substituted or unsubstituted;

provided that

when $X_1$ is N or CH, $X_2$ is CH, $R_3$ is hydrogen, $R_1$ is hydrogen, unsubstituted or substituted ($C_{1-4}$)alkyl, or hetero ($C_6$)cycloalkyl, and ring A is a pyridyl, pyrimidinyl or pyrazinyl, ring A does not have an amino, substituted amino, substituted oxy, or fluoro attached to position 4 of ring A where the ring atom through which ring A attaches to the ring containing $X_1$ and $X_2$ is counted as position 1;

when $X_1$ is CH, $X_2$ is CH, $R_1$ and $R_3$ are both hydrogen, and ring A is a five- or six-member heteroaryl comprising one nitrogen ring atom, said one nitrogen ring atom is not located at a position adjacent to the ring atom through which said heteroaryl attaches to the ring containing $X_1$ and $X_2$;

when $X_1$ is CH, $X_2$ is CH, $R_1$ and $R_3$ are both hydrogen, and ring A is a pyrimidinyl, the ring atom located at position 4 of said pyrimidinyl is not a nitrogen where the ring atom through which said pyrimidinyl attaches to the ring containing $X_1$ and $X_2$ is counted as at position 1;

when $X_1$ is N, $X_2$ is CH, and ring A is as defined in claim 1, $R_1$ and $R_3$ are both not a substituted phenyl;

when $X_1$ is CH, $X_2$ is CH, $R_3$ is hydrogen, and ring A is substituted or unsubstituted ($C_{6-10}$)aryl, $R_1$ is not a halo, or a thiocarbonyl substituted amino group; and

the compounds of the invention do not include

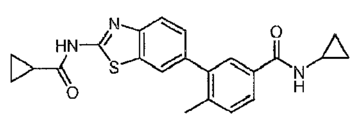

(3-(2-(cyclopropanecarboxamido)benzo[*d*]thiazol-6-yl)-*N*-cyclopropyl-4-methylbenzamide).

In yet another embodiment, P13K/mTOR inhibitors of the present invention consist of the formula:

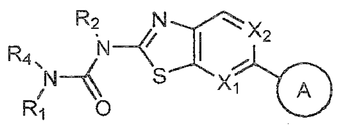

or a hydrate, solvate, stereoisomer, tautomer, or a pharmaceutically acceptable salt thereof, wherein

ring A is aryl or heteroaryl, each unsubstituted or substituted with 1-5 substituents which is independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, carbonylamino, sulfonylamino, $(C_{1-10})$alkylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1}$-$_{10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl;

$X_1$ is selected from the group consisting of $CR_5$ and N;

$X_2$ is selected from the group consisting of $CR_{5'}$ and N;

$R_1$ is selected from the group consisting of hydrogen, halo, for nitro, cyano, thio, oxy, hydroxy, carbonyloxy, $(C_{1-10})$alkoxy, $(C_{4-12})$alyloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicyclomyl$(C_{1-5})$alkyl, hetero$(C_{8-12})$bicycloaryl$(C_{1-5})$alky hetero$(C_{1-10})$alky $(C_{3-12})$alicyclyl, hetero$(C_{3-12})$alicyclyl, $(C_{9}$-$_{12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted;

$R_2$ is selected from the group consisting of hydrogen, $(C_{1-6})$alkoxy, carbonyl, oxycarbonyl, aminocarbonyl, amino, $(C_{1-6})$alkylamino, sulfonyl, sulfinyl, $(C_{1-6})$alkyl, $(C_{1-10})$alkenyl, $(C_{1-10})$alkynyl, hetero$(C_{1-6})$alkyl, halo$(C_{1-6})$alkyl, hydroxy$(C_{1-6})$alkyl, carbonyl$(C_{1-6})$alkyl, $(C_{3}$-$_{6})$cycloalkyl$(C_{1-3})$alkyl, hetero$(C_{1-5})$cycloalkyl$(C_{1-3})$alkyl, $(C_{3}$-$_{6})$alicyclyl, and hetero$(C_{1-5})$alicyclyl, each substituted or unsubstituted;

$R_4$ is selected from the group consisting of hydrogen, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, imino, sulfonyl, sulfinyl, $(C_{1-10})$atkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3}$-$_{12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted; and

$R_5$ and $R_5$, are each independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, sulfonylamino, imino, sulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alky $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hotero$(C_{4-12})$bicycloaryl, each substituted or unsubstituted;

provided that

when $X_1$ is N or CH, $X_2$ is CH, $R_4$ is hydrogen, $R_1$ is unsubstituted or substituted $(C_{1-4})$alkyl, and ring A is a pyridyl,

pyrimidinyl or pyrazinyl, ring A does not have an amino, substituted amino, substituted oxy, or fluoro attached to position 4 of ring A where the ring atom through which ring A attaches to the ring containing $X_1$ and $X_2$ is counted as position 1; and

when $X_1$ is CH and $X_2$ is CH, and ring A is as defined in claim 1, $R_1$ is not selected from the group consisting of alkyl, carbonyl, and phenoxy substituted pyridinyl.

In yet another embodiment, PI3K/mTOR inhibitors of the present invention consist of the formula:

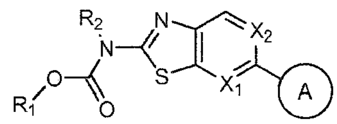

wherein

ring A is aryl or heteroaryl, each unsubstituted or substituted with 1-5 substituents which is independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, carbonylamino, sulfonylamino, $(C_{1-10})$alkylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{1-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicydyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{1-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl;

$X_1$ is selected from the group consisting of $CR_5$ and N;

$X_2$ is selected from the group consisting of $CR_5$, and N;

$R_1$ is selected from the group consisting of hydrogen, halo, for nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$allcyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$blcycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted;

$R_2$ is selected from the group consisting of hydrogen, $(C_{1-6})$alkoxy, carbonyl, oxycarbonyl, aminocarbonyl, amino, $(C_{1-6})$alkylamino, sulfonyl, sulfinyl, $(C_{1-6})$alkyl, $(C_{1-10})$alkenyl, $(C_{1-10})$alkynyl, hotero$(C_{1-6})$alkyl, halo$(C_{1-6})$alkyl, hydroxy$(C_{1-6})$alkyl, carbonyl$(C_{1-6})$alkyl, $(C_{3-6})$cycloalkyl$(C_{1-3})$alkyl, hetero$(C_{1-5})$cycloalkyl$(C_{1-3})$alkyl, $(C_{3-6})$alicyclyl, and hetero$(C_{1-5})$alicyclyl, each substituted or unsubstituted; and

$R_5$ and $R_{5'}$ are each independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, sulfonylamino, imino, sulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alky hetero$(C_{1-10})$alkyl, $(C_{3-12})$alicyclyl, hetero$(C_{3-12})$alicyclyl, $(C_{9-12})$blcycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted;

provided that

when $X_1$ is N or CH, $X_2$ is CH, $R_1$ is unsubstituted or substituted $(C_{1-4})$alkyl, and ring A is a pyridyl, pyrimidinyl or

pyrazinyl, ring A does not have an amino, substituted amino, substituted oxy, or fluoro attached to position 4 of ring A where the ring atom through which ring A attaches to the ring containing $X_1$ and $X_2$ is counted as position 1.

**Ring A**

In some variations of the above embodiments of the compounds of the invention, ring A is selected from six membered monocyclic and fused bicyclic aryl and heteroaryl of the formula:

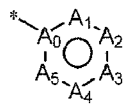

wherein

$A_0$ is C or N;
$A_1$ is $CR_6$ or N;
$A_2$ is $CR_7$ or N;
$A_3$ is $CR_8$ or N;
$A_4$ is $CR_9$ or N;
$A_5$ is $CR_{10}$ orN;
each $R_6$, $R_7$, $R_8$, $R_9$, and $R_{10}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl $(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$ alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted, each unsubstituted or further substituted with 1-2 substituents independently selected from the group consisting of halo, cyano, $(C_{1-6})$alkyl, hydroxyl$(C_{1-6})$alkyl, halo$(C_{1-6})$ alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$ aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl, or one of $R_6$ and $R_7$, $R_7$ and $R_8$, $R_8$ and $R_9$, and $R_9$ and $R_{10}$, are taken together to from a saturated, unsaturated, or aromatic ring, each unsubstituted or substituted;

provided that

when $X_1$ is N or CH, $X_2$ is CH, Q is O, L is-$CH_2$-, -NH- or -O-, $R_1$ is hydrogen, unsubstituted or substituted $(C_{1-4})$ alkyl, or hetero$(C_6)$cycloalkyl, and ring A is a pyridyl, pyrimidinyl or pyrazinyl, $R_8$ is not amino, substituted amino, substituted oxy, or fluoro;
when $X_1$ is CH, $X_2$ is CH, Q is O, L is -$CHR_3$-, $R_1$ and $R_3$ are both hydrogen, and ring A is a pyridyl, $A_1$ is $CR_6$ and $A_5$ is $CR_{10}$;
when $X_1$ is CH, $X_2$ is CH, Q is O, L is -$CH_2$-, $R_1$ is hydrogen, and ring A is a pyrimidinyl, $A_3$ is not N;
when $X_1$ is CH, $X_2$ is CH, Q is O, and L is -NH-, $R_1$ is not selected from the group consisting of alkyl, carbonyl, and phenoxy substituted pyridinyl;
when $X_1$ is N, $X_2$ is CH, Q is O, L is -$CHR_3$-, $R_1$ and $R_3$ are both not a substituted phenyl;
when $X_1$ is CH, $X_2$ is CH, Q is O, L is -$CH_2$-, and ring A is substituted or unsubstituted $(C_{6-10})$aryl, $R_1$ is not a halo, or a thiocarbonyl substituted amino group; and
the compounds of the invention do not include

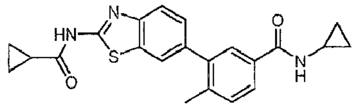

(3-(2-(cyclopropanecarboxamido)benzo[*d*]thiazol-6-yl)-*N*-cyclopropyl-4-methylbenzamide).

In some variations, ring A is selected from the group consisting of

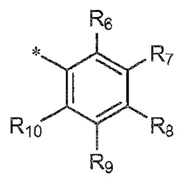
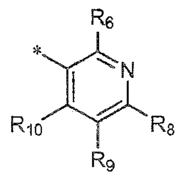
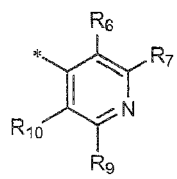
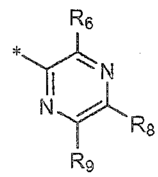

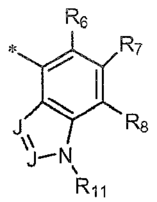
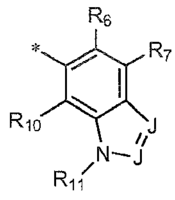
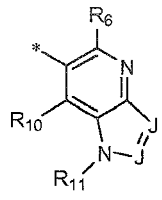
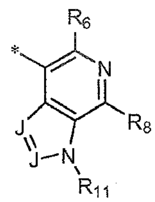
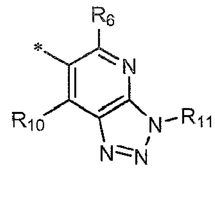

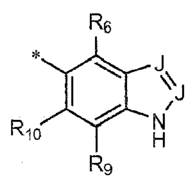
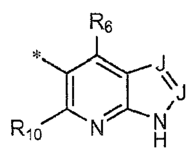
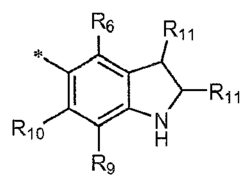
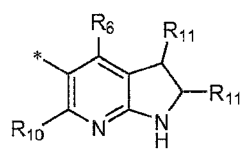

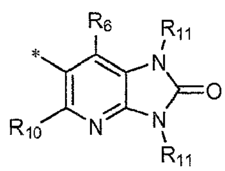
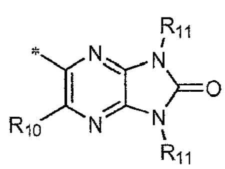
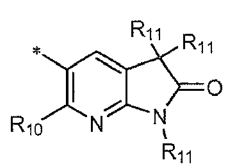
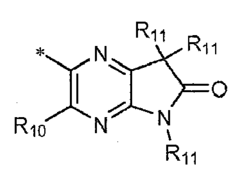

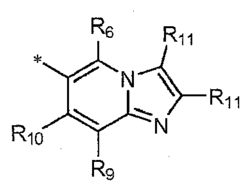
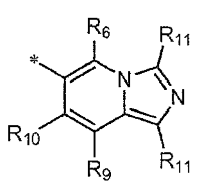
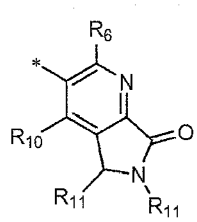
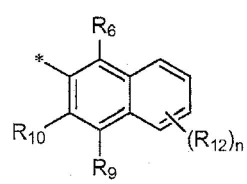

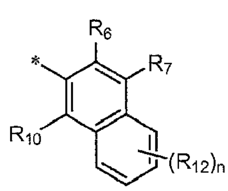
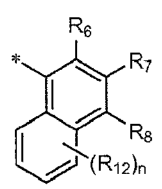
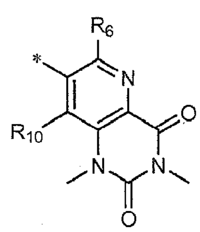
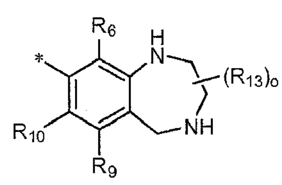

wherein

n is 1, 2, 3, or 4;

o is 1, 2, 3, 4, 5, or 6;

each J is independently N or $CR_{11}$; and

each $R_{11}$, $R_{12}$ and $R_{13}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$akyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cydoaikyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicycly hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero $(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl $(C_{1-6})$alkyl hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{1-6})$alicyclyl, hetero$(C_{1-5})$alicyclyl, $(C_{4-6})$aryl, hetero$(C_{1-5})$aryl.

In other variations, ring A is selected from the group consisting of

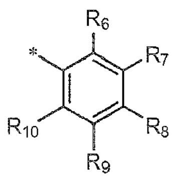
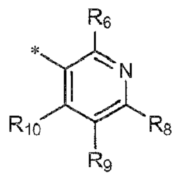
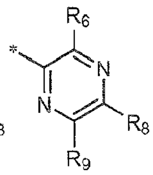
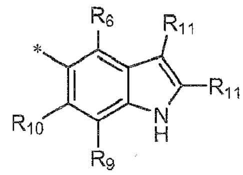

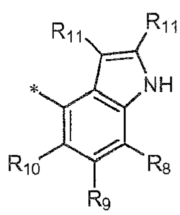
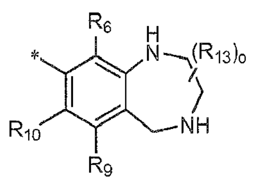

wherein

o is 1, 2, 3, 4, 5, or 6; and

each $R_{11}$ and $R_{13}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$akynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicyeloalicyclyl, hetero$(C_{3-12})$bicycloalicyclyl, $(C_{4-12})$aryl, hetero$(C_{1-10})$aryl, $(C_{9-12})$bicycloaryl, and hetero $(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl $(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl.

In still other variations, ring A is selected from the group consisting of

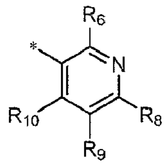
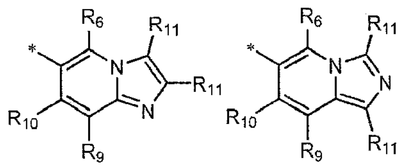
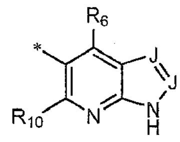
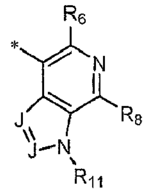
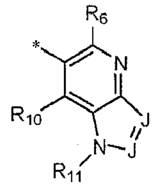

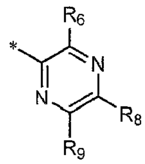
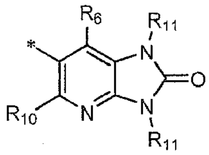
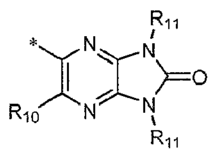
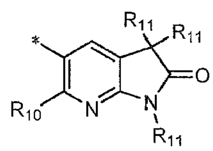
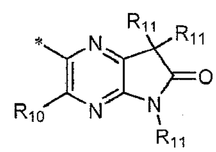

wherein

each J is independently N or $CR_{13}$; and

$R_{11}$ is selected from the group consisting of halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl.

In still other variations, ring A is

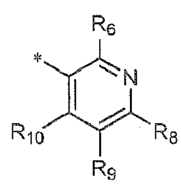

.

In some variations of the above three embodiments of the compounds of the invention, ring A is selected from five-membered monocyclyls and fused bicyclyls of the formula:

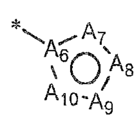

wherein

$A_6$ is C or N;

$A_7$ is $CR_{22}$, $NR_{22}$, N, O, or S;

$A_8$ is $CR_{23}$, $NR_{23}$, N, O, or S;

$A_9$ is $CR_{24}$, $NR_{24}$, N, O, or S;

$A_{10}$ is $CR_{25}$, $NR_{25}$, N, O, or S; and

each $R_{22}$, $R_{23}$, $R_{24}$, and $R_{25}$ is independently selected from the group consisting of hydrogen, halo, nitro, cyano,

thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, sulfonylamino, imino, sulfonyl, carbonylamino, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaiyl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and betero $(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl $(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl, or one of $R_{22}$ and $R_{23}$, $R_{23}$ and $R_{24}$, and $R_{24}$ and $R_{25}$, are taken together to form an unsubstituted or substituted 5-, 6- or 7-membered ring;

provided that

when $X_1$ is CH, $X_2$ is CH, Q is O, L is $-CHR_3-$, $R_1$ and $R_3$ are both hydrogen, and ring A is a heteroaryl comprising one nitrogen ring atom, $A_7$ is not N or $NR_{22}$ and $A_{10}$ is not N or $NR_{25;}$ and
when $X_1$ is CH, $X_2$ is CH, Q is O, and L is -NH-, $R_1$ is not an unsubstituted or substituted $(C_{1-4})$alkyl.

In some variations, ring A is selected from the group consisting of furanyl, thiophenyl, pyrrolyl, oxazolyl, thiazolyl, imidazolyl, pyrazolyl, isoxazolyl, isothiazolyl, oxadiazolyl, triazolyl, thiadiazolyl, indolizinyl, indolyl, isoindolyl, benzofuranyl, benzothiophenyl, indazolyl, benzimidazolyl, benzthiazolyl, pyridinylfuranyl, pyridinylthiophenyl, pyridinylpyrazolyl, pyridinylimidazolyl, pyridinylthiazolyl, pyrimidinylfuranyl, pyrimidinylthiophenyl, pyrimidinylpyrazolyl, pyrimidinylimidazolyl, pyrimidinylthiazolyl, and purinyl, wherein one of $R_{22}$, $R_{23}$, $R_{24}$, and $R_{25}$ is bonded to each carbon ring atom or nitrogen ring atom of the five-membered cyclyl according to claim 9, and wherein each of the cycly is unsubstituted or further substituted with said 1-2 substituents. In other variations, ring A is

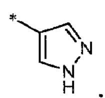

In one particular embodiment, where the compounds of the invention consisting the formula

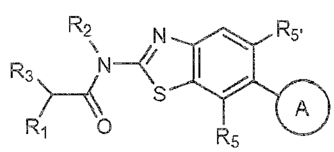

wherein

$R_1$ and $R_3$ are each independently selected from the group consisting of hydrogen, halo, for nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, sulfonylamino, imino, sulfonyl, sulfinyl, $(C_{1-10})$alkyl, $(C_{1-10})$alkenyl, $(C_{1-10})$alkynyl, halo $(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_1{-}_{10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12)}$alicyclyl, $(C_{9-12})$blcycloalicyclyl, hotero$(C_{3-12})$blcycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted, or $R_1$ and $R_3$ are taken together to form a $(C_{3-6})$cycloalkyl;
$R_2$ is selected from the group consisting of hydrogen, $(C_{1-6})$alkoxy, carbonyl, oxycarbonyl, aminocarbonyl, amino, $(C_{1-6})$alkylamino, sulfonyl, sulfinyl, $(C_{1-6})$alkyl, $(C_{1-10})$alkenyl, $(C_{1-10})$alkynyl, hetero$(C_{1-6})$alkyl, halo$(C_{1-6})$alkyl, hydroxy$(C_{1-6})$alkyl, carbonyl$(C_{1-6})$alkyl, $(C_{3-6})$cycloalkyl$(C_{1-3})$alkyl, hetero$(C_{1-5})$cycloalkyl$(C_{1-3})$alkyl, $(C_{3-6})$alicyclyl, and hetero$(C_{1-5})$alicyclyl, each substituted or unsubstituted;
$R_5$ and $R_{5'}$ are each independently selected from the group consisting of hydrogen, halo, nitro, cyano, thio, oxy,

hydroxy, carbonyloxy, $(C_{1-10})$alkoxy, $(C_{4-12})$aryloxy, hetero$(C_{1-10})$myloxy, carbonyl, oxycarbonyl, aminocarbonyl, amino, $(C_{1-10})$alkylamino, sulfonylamino, imino, sulfonyl, sulfinyl, $(C_{1-10})$alkyl, $(C_{1-10})$alkenyl, $(C_{1-10})$alkynyl, halo $(C_{1-10})$alkyl, hydroxy$(C_{1-10})$alkyl, carbonyl$(C_{1-10})$alkyl, tblocarbonyl$(C_{1-10})$alkyl, sulfonyl$(C_{1-10})$alkyl, sulfinyl$(C_{1-10})$ alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$ cycloalkyl$(C_{1-10})$alkyl, aryl$(C_{1-10})$alkyl, hetero$(C_{1-10})$alyl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted; and

ring A is selected from the group consisting of

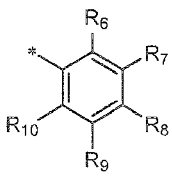
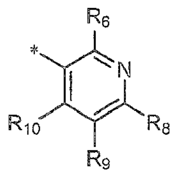
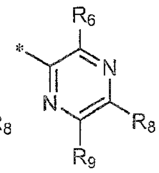
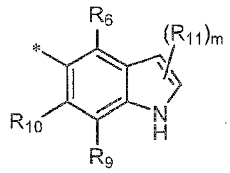

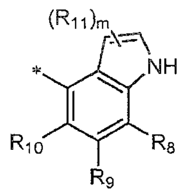
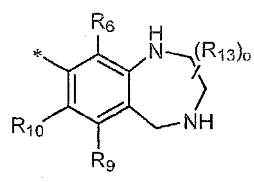

where

ois 1, 2, 3, 4, 5, or 6;

each $R_6$, $R_7$, $R_8$, $R_9$, and $R_{10}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$alyloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$ alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$ oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero $(C_{1-10})$aryl, $(C_{9-12})$bicycloaiyl, and hetero$(C_{4-12})$bicycloaryl, each unsubstituted or further substituted with a substituent selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl; and

each $R_{11}$ and $R_{13}$ is independently selected from the group consisting of halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$atkyl, hydroxy$(C_{1-10})$alkyl, carbonyl$(C_{1-10})$alkyl, thiocarbonyl$(C_{1-10})$ alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$ alkyl, $(C_{3-12})$cycloalkyl$(C_{1-5})$alkyl, hetero$(C_{3-12})$cydoalkyl$(C_{1-10})$alkyl, aryl$(C_{1-10})$alkyl, hetero$(C_{1-10})$aryl$(C_{1-5})$ alkyl, $(C_{9-12})$bicycloaryl$(C_{1-5})$alkyl, hetero$(C_{8-12})$bicycloaryl$(C_{1-8})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl;

provided that

when ring A is a pyridinyl or pyrazinyl, $R_8$ is not an amino, substituted amino, substituted oxy, or fluoro; and

when ring A is substituted or unsubstituted phenyl, $R_1$ is not a halo or a thiocarbonyl substituted amino; and

the compounds of the invention do not include

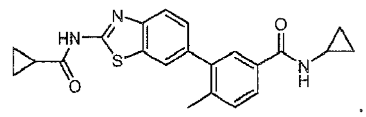

In some variations of the preceding particular embodiment, ring A is of the formula:

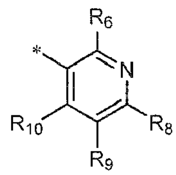

In another particular embodiment, where the compounds of the invention consisting the formula

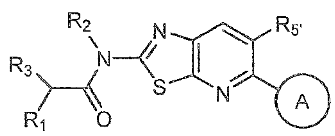

wherein

$R_1$ and $R_3$ are each independently selected from the group consisting of hydrogen, halo, for nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, sulfonylamino, imino, sulfonyl, sulfinyl, $(C_{1-10})$alkyl, $(C_{1-10})$alkenyl, $(C_{1-10})$alkynyl, halo $(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$ alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$ cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted, or $R_1$ and $R_3$ are taken together to form a $(C_{3-6})$cycloalkyl;

$R_2$ is selected from the group consisting of hydrogen, $(C_{1-6})$alkoxy, carbonyl, oxycarbonyl, aminocarbonyl, amino, $(C_{1-6})$alkylamino, sulfonyl, sulfinyl, $(C_{1-6})$alkyl, $(C_{1-10})$alkenyl, $(C_{1-10})$alkynyl, hetero$(C_{1-6})$alkyl, halo$(C_{1-6})$alkyl, hydroxy$(C_{1-6})$alkyl, carbonyl$(C_{1-6})$alkyl, $(C_{3-6})$cycloalkyl$(C_{1-3})$alkyl, hetero$(C_{1-5})$cycloalkyl$(C_{1-3})$alkyl, $(C_{3-6})$alicyclyl, and hetero$(C_{1-5})$alicyclyl, each substituted or unsubstituted;

$R_{5'}$ is selected from the group consisting of hydrogen, halo, nitro, cyano, thio, oxy, hydroxy, carbonyloxy, $(C_{1-10})$ alkoxy, $(C_{4-12})$aryloxy, hetero$(C_{1-10})$aryloxy, carbonyl, oxycarbonyl, aminocarbonyl, amino, $(C_{1-10})$alkylamino, sulfonylamino, imino, sulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloatkyl$(C_{1-5})$alkyl, hetero$(C_{3-12})$cycloalkyl$(C_{1-10})$alkyl, aryl$(C_{1-10})$ alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetoro$(C_{1-10})$ alkyl, $(C_{3-12})$alicyclyl, hetero$(C_{3-12})$alicyclyl, $(C_{9-12})$bicycloalicyclyl, hetero$(C_{3-12})$bicycloalicydyl, $(C_{4-12})$aryl, hetero $(C_{1-10})$aryl, $(C_{9-12})$bicycloaryl, and hetero$(C_{4-12})$bicycloaryl, each substituted or unsubstituted;

ring A is selected from the group consisting of

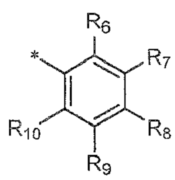 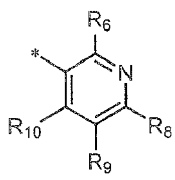 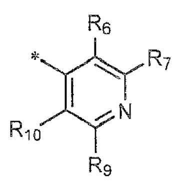 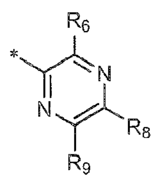 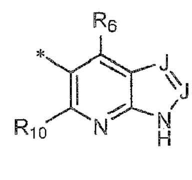

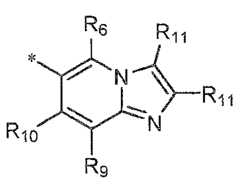 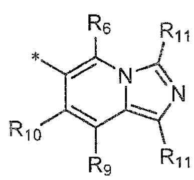 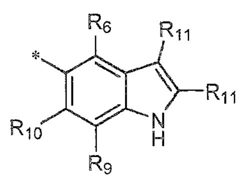 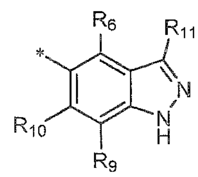

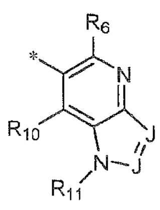 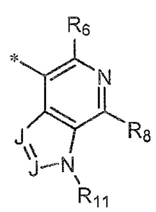 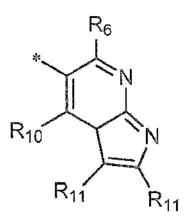 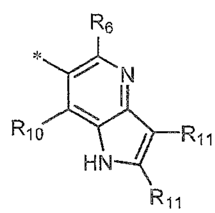 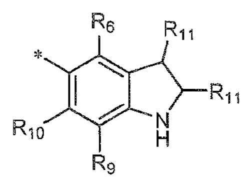

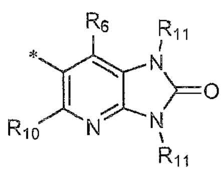 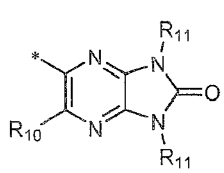 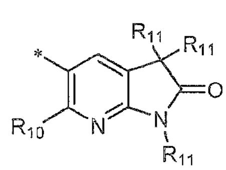 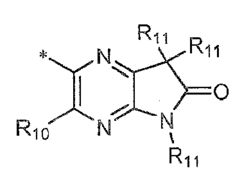

where

each $R_6$, $R_7$, $R_8$, $R_9$, and $R_{10}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl $(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$ alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$ alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero $(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, aMino$(C_{1-6})$ alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl; and

each $R_{11}$ is selected from the group consisting of halo, nitro, cyano, thio, oxy, hydroxy, oxo, carbonyloxy, $(C_{1-10})$ alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$ alkyl, hetero$(C_{3-12})$cycloalkyl$(C_{1-10})$alky aryl$(C_{1-10})$alkyl, hetero$(C_{1-10})$aryl$(C_{1-5})$alkyl, $(C_{9-12})$bicycloaryl$(C_{1-5})$ alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or substituted with 1-2 substituents independently selected from the group consisting of $(C_{1-6})$alkyl, hydroxyl$(C_{1-6})$alkyl, halo$(C_{1-6})$alkyl, amino$(C_{1-6})$alkyl, amino, $(C_{1-6})$alkoxy, $(C_{3-6})$alicyclyl, hetero$(C_{1-5})$alicyclyl, $(C_{4-6})$aryl, and hetero$(C_{1-5})$aryl;

provided that

when $R_2$ and $R_5$, are both hydrogen, $R_1$ and $R_3$ are both not a substituted phenyl.

In some variations of the immediately preceding particular embodiment, ring A is of the formula:

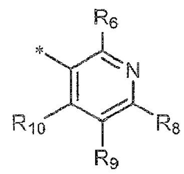

In still another particular embodiment, the compounds of the invention consisting the formula

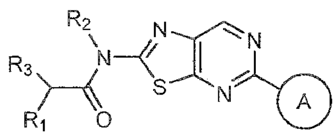

wherein

$R_1$ and $R_3$ are each independently selected from the group consisting of hydrogen, halo, for nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, sulfonylamino, imino, sulfonyl, sulfinyl, $(C_{1-10})$alkyl, $(C_{1-10})$alkenyl, $(C_{1-10})$alkynyl, halo $(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$ alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$ cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloatyl$(C_{1-5})$alkyl, hetero$(C_{1-10})$alkyl, $(C_{3-12})$alicyclyl, hetero$(C_{1-12})$alicyclyl, $(C_{9-12})$bicycloalicyclyl, hetero$(C_{3-12})$bicycloalicyclyl, $(C_{4-12})$aryl, hetero$(C_{1-10})$alyl, $(C_{9-12})$bicycloaryl, and hetero$(C_{4-12})$bicycloaryl, each substituted or unsubstituted, or $R_1$ and $R_3$ are taken together to form a $(C_{3-6})$cycloalkyl;
$R_2$ is selected from the group consisting of hydrogen, $(C_{1-6})$alkoxy, carbonyl, oxycarbonyl, aminocarbonyl, amino, $(C_{1-6})$alkylamino, sulfonyl, sulfinyl, $(C_{1-6})$alkyl, $(C_{1-10})$alkenyl, $(C_{1-10})$alkynyl, hetero$(C_{1-6})$alkyl, halo$(C_{1-6})$alkyl, hydroxy$(C_{1-6})$alkyl, carbonyl$(C_{1-6})$alkcy $(C_{3-6})$cycloalkyl$(C_{1-3})$alkyl, hetero$(C_{1-5})$cycloalkyl$(C_{1-3})$alkyl, $(C_{3-6})$alicyclyl, and hetero$(C_{1-5})$alicyclyl, each substituted or unsubstituted;
ring A is selected from the group consisting of

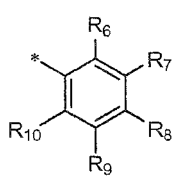
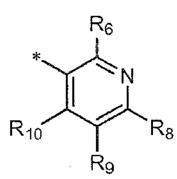
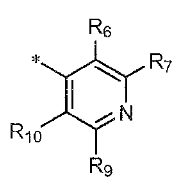
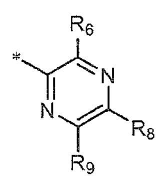

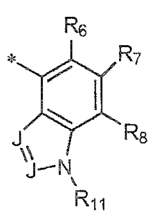
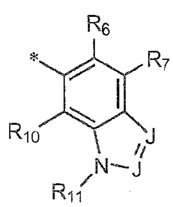
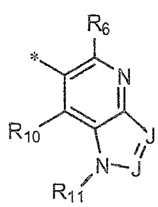
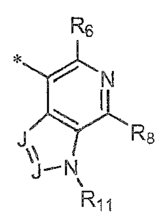
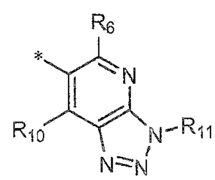

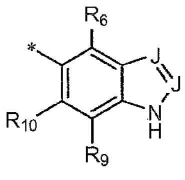 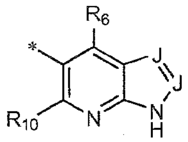 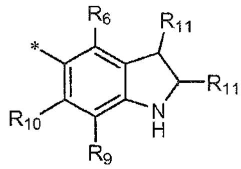 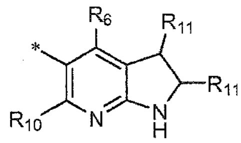

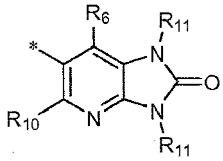 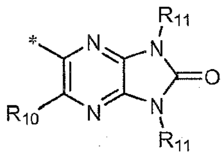 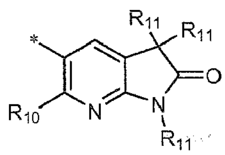 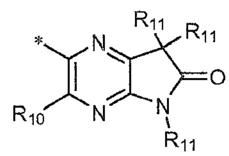

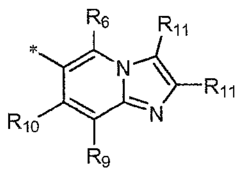 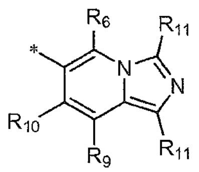 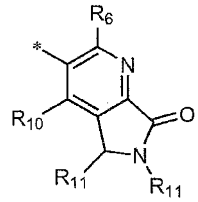 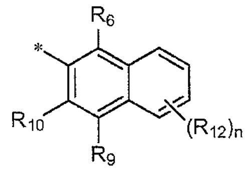

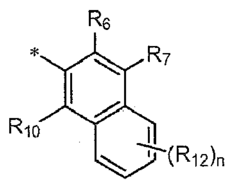 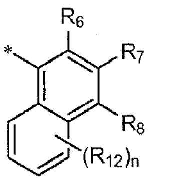 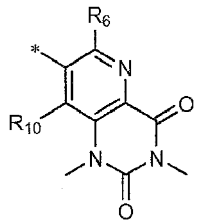 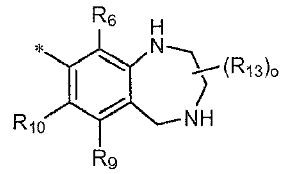

where

n is 1, 2, 3, or 4;

o is 1, 2, 3, 4, 5, or 6;

each $R_6$, $R_7$, $R_8$, $R_9$, and $R_{10}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl $(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$ alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$ alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero $(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicy-cloaiyl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$ alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl; and

each $R_{11}$, $R_{12}$ and $R_{13}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, sulfonylamino, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino $(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl $(C_{1-5})$alkyl, $(C_{9-12})$bicycloaryl$(C_{1-5})$alkyl, hetero$(C_{8-12})$bicydoaiyl$(C_{1-5})$alkyl, hetero$(C_{1-10})$alkyl, $(C_{3-12})$alicyclyl,

hetero($C_{3-12}$)allcyclyl, ($C_{9-12}$)bicycloalicyclyl, hetero($C_{3-12}$)bicycloalicyclyl, ($C_{4-12}$)aryl, hetero($C_{1-10}$)aryl, ($C_{9-12}$) bicycloaryl, and hetero($C_{4-12}$)bicycloaryl, each unsubstituted or further substituted with 1-2 substituents independently selected from the group consisting of halo, cyano, ($C_{1-6}$)alkyl, hydroxyl($C_{1-6}$)alkyl, halo($C_{1-6}$)alkyl, amino($C_{1-6}$)alkyl, ($C_{3-6}$)alicyclyl($C_{1-6}$)alkyl, hetero($C_{1-5}$)alicyclyl($C_{1-6}$)alkyl, ($C_{4-6}$)aryl($C_{1-6}$)alkyl, hetero($C_{1-5}$)aryl($C_{1-6}$)alkyl, amino, ($C_{1-6}$)alkoxy, ($C_{3-6}$)alicyclyl, betero($C_{1-5}$)alicyclyl, ($C_{4-6}$)aryl, hetero($C_{1-5}$)aryl.

In some variations of the immediately preceding embodiment, ring A is of the formula:

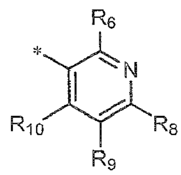

## $\underline{R_2}$

In the embodiments and particular embodiments, and their variations of the compounds of the invention, in some variations, $R_2$ is selected from the group consisting of hydrogen, ($C_{1-6}$)alkyl, ($C_{1-10}$)alkenyl, ($C_{1-10}$)alkynyl, hetero ($C_{1-6}$)alkyl, ($C_{3-6}$)cycloalkyl, and hetero($C_{1-5}$)cycloalkyl, each unsubstituted or substituted. In other variations, $R_2$ is hydrogen. In other variations, $R_2$ is ($C_{1-6}$)alkyl. In other variations, $R_2$ is cycopropyl. In other variations, $R_2$ is methyl. In still other variations, $R_2$ is ethyl.

## $\underline{R_1}$ and $\underline{R_3}$

In the embodiments, particular embodiments and their various variations of the compounds of the invention, in some variations, $R_1$ is independently selected from the group consisting of hydrogen, hydroxy, halo, nitro, cyano, thio, oxy, amino, carbonyloxy, ($C_{1-6}$)alkoxy, ($C_{4-12}$)aiyloxy, hetero($C_{1-10}$)arytoxy, carbonyl, oxycarbonyl, sulfonyl, sulfinyl, ($C_{1-6}$)alkyl, hetero($C_{1-6}$)alkyl, halo($C_{1-6}$)alkyl, ($C_{1-10}$)alkenyl, ($C_{1-10}$)alkynyl, hydroxy($C_{1-6}$)alkyl, carbonyl($C_{1-10}$)alkyl, sulfonyl($C_{1-6}$)alkyl, ($C_{3-6}$)cycloalkyl($C_{1-3}$)alkyl, hetero($C_{1-5}$)cycloalkyl($C_{1-3}$)alkyl, aryl($C_{1-3}$)alkyl, hetero($C_{1-5}$)aryl($C_{1-3}$)alkyl, ($C_{3-6}$)alicyclyl, hetero($C_{1-5}$)alicyclyl, ($C_{4-6}$)aryl, and hetero($C_{1-5}$)aryl, each unsubstituted or substituted.

In other variations, $R_1$ is selected from the group consisting of hydrogen, ($C_{1-6}$)alkyl, ($C_{1-10}$)alkenyl, ($C_{1-10}$) alkynyl, hetero($C_{1-6}$)alkyl, ($C_{1-6}$)alkoxy, and amino, each unsubstituted or substituted. In other variations, $R_1$ is ($C_{1-6}$) alkoxy. In other variations, $R_1$ is ($C_{1-6}$)alkyl. In still other variations, $R_1$ is amino, unsubstituted or substituted. In still other variations, $R_1$ is hydrogen.

In the embodiments, particular embodiments, and their various variations of the compounds of the invention, in some variations, $R_3$ is selected from the group consisting of hydrogen, hydroxy, halo, nitro, cyano, thio, oxy, amino, carbonyloxy, ($C_{1-6}$)alkoxy, ($C_{4-12}$)aryloxy, hetero($C_{1-10}$)aryloxy, carbonyl, oxycarbonyl, sulfonyl, sulfinyl, ($C_{1-6}$)alkyl, ($C_{1-10}$)alkenyl, ($C_{1-10}$)alkynyl, hetero($C_{1-6}$)alkyl, halo($C_{1-6}$)alkyl, hydroxy($C_{1-6}$)alkyl, carbonyl($C_{1-10}$)alkyl, sulfonyl($C_{1-6}$) alky ($C_{3-6}$)cycloalkyl($C_{1-3}$)alkyl, hetero($C_{1-5}$)cycloalkyl($C_{1-3}$)alkyl, aryl($C_{1-3}$)alkyl, hetero($C_{1-5}$)aryl($C_{1-3}$)alkyl, ($C_{3-6}$)alicyclyl, hetero($C_{1-5}$)alicyclyl, ($C_{4-6}$)aryl, and hetero($C_{1-5}$)aryl, each unsubstituted or substituted.

In other variations, $R_3$ is selected from the group consisting of hydrogen, ($C_{1-6}$)alkyl, hetero($C_{1-6}$)alkyl, ($C_{1-10}$) alkenyl, ($C_{1-10}$)alkynyl, ($C_{1-6}$)alkoxy, and amino, each unsubstituted or substituted. $R_3$ is selected from the group consisting of hydrogen, ($C_{1-6}$)alkyl, hetero($C_{1-6}$)alkyl, ($C_{1-6}$)alkoxy, and amino, each unsubstituted or substituted. In other variations, $R_3$ is ($C_{1-6}$)alkoxy. In other variations, $R_3$ is ($C_{1-6}$)alkyl. In still other variations, $R_3$ is amino, unsubstituted or substituted. In other variations, $R_3$ is hydrogen. In still other variations,

In some variations, $R_1$ and $R_3$ are taken together to form a cyclopropyl, unsubstituted or substituted.

## $\underline{R_4}$

In the embodiments, particular embodiments and their various variations of the compounds of the invention, in some variations, when present, $R_4$ is selected from the group consisting of hydrogen, ($C_{1-6}$)alkyl, ($C_{1-10}$)alkenyl, ($C_{1-10}$)alkynyl, hetero($C_{1-6}$)alkyl, ($C_{3-6}$)alicyclyl, hetero($C_{1-5}$)alicyclyl, phenyl, and ($C_{1-5}$)heteroaryl, each unsubstituted or substituted.

In other variations, when present, $R_4$ is selected from the group consisting of hydrogen, ($C_{1-6}$)alkyl, hetero ($C_{1-6}$)alkyl, ($C_{3-6}$)eycloalkyl, ($C_{1-5}$)heterocycloalkyl, phenyl, ($C_{1-5}$)heteroaryl, each unsubstituted or substituted.

In still other variations, when present, $R_4$ is ($C_{1-6}$)alkyl.

In yet still other variations, when present, $R_4$ is cyclopropyl.

In other variations, $R_4$ is hydrogen.

**$R_5$ and $R_{5'}$**

In the embodiments, particular embodiments, and their various variations of the compounds of the invention, in some variations, when present, $R_5$ and $R_5$, are each independently selected from the group consisting of hydrogen, $(C_{1-6})$alkyl, $(C_{1-10})$alkenyl, $(C_{1-10})$alkynyl, hetero$(C_{1-6})$alkyl, $(C_{3-6})$alicyclyl, and hetero$(C_{1-5})$alicyciyl, each unsubstituted or substituted

In other variations, $R_5$ and $R_{5'}$, when present, are each independently selected from the group consisting of hydrogen, $(C_{1-6})$alkyl, hetero$(C_{1-6})$alkyl, $(C_{3-6})$cycloalkyl, and $(C_{1-5})$heterocycloalkyl, each unsubstituted or substituted.

In still other variations, one of $R_5$ and $R_{5'}$, when present, is $(C_{1-6})$alkyl.

In yet still other variations, one of $R_5$ and $R_{5'}$, when present, is cyclopropyl.

In still other variations, one of $R_5$ and $R_{5'}$ is hydrogen.

In other variations, $R_5$ and $R_5$', are both hydrogen.

**$R_6$ $R_7$ $R_8$ $R_9$ and $R_{10}$**

In the embodiments, particular embodiments, and their various variations of the compounds of the invention, in some variations, when present, each $R_6$, $R_7$, $R_8$, $R_9$, and $R_{10}$ is independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, sulfonyl, aminosulfonyl, sulfinyl, $(C_1\text{-}_{10})$alkyl, $(C_{1-10})$alkenyl, $(C_{1-10})$alkynyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, hetero$(C_{1-10})$alkyl, $(C_{3-6})$ alicyclyl, and hetero$(C_{1-5})$alicyclyl, $(C_{4-12})$aryl, and hetero$(C_{1-10})$aryl, each unsubstituted or further substituted with 1-2 substituents selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alky betero$(C_{1-5})$aiyl$(C_{1-6})$alkyl, amino, $(C_{1-6})$ alkoxy, $(C_{3-6})$alicyclyl, hetero$(C_{1-5})$alicydyl, $(C_{4-6})$aryl, hetero$(C_{1-5})$aryl.

In some variations, when present, $R_6$ is selected from the group consisting of hydrogen, halo, cyano, hydroxyl, amino, $(C_{1-6})$alkoxy, $(C_{1-6})$alkyl, hetero$(C_{1-6})$alkyl, halo$(C_{1-6})$alkyl, $(C_{3-6})$cycloalkyl, and $(C_{1-5})$heterocycloalkyl, each unsubstituted or substituted with said 1-2 substituents.

In other variations, when present, $R_6$ is hydrogen or $-NH_2$. In other variations, when present, $R_6$ is halo.

In other variations, when present, $R_7$ is selected from the group consisting of hydrogen, halo, cyano, hydroxyl, $(C_{1-6})$alkoxy, $(C_{1-6})$alkyl, $(C_{1-10})$alkenyl, $(C_{1-10})$alkynyl, hetero$(C_{1-6})$alkyl, halo$(C_{1-6})$alkyl, carbonyl, aminocarbonyl, carbonylamino, $(C_{3-6})$ alicyclyl, and hetero$(C_{1-5})$alicyclyl, each unsubstituted or substituted with said 1-2 substituents. In other variations, $R_7$ is selected from the group consisting of hydrogen, halo, cyano, hydroxyl, $(C_{1-6})$alkoxy, $(C_{1-6})$alkyl, hetero$(C_{1-6})$alkyl, halo$(C_{1-6})$alkyl, carbonyl, aminocarbonyl, carbonylamino, $(C_{3-6})$cycyloalkyl, and $(C_{1-5})$heterocycloalkyl, each unsubstituted or substituted with said 1-2 substituents. In other variations, when present, $R_7$ is $(C_{1-6})$alkyl. In other variations, when present, $R_7$ is hydrogen, trifluoromethyl, and $-C(O)NHOR_{14}$ where $R_{14}$ is $(C_{1-6})$alkyl.

In some variations, $R_8$, when present, is selected from the group consisting of hydrogen, halo, cyano, hydroxyl, $(C_{1-6})$alkoxy, $(C_{1-6})$alkyl, $(C_{1-10})$alkenyl, $(C_{1-10})$alkynyl, hetero$(C_{1-6})$alkyl, halo$(C_{1-6})$alkyl, carbonyl, aminocarbonyl, carbonylamino, amino, sulfonyl, $(C_{3-6})$ alicyclyl, and hetero$(C_{1-5})$alicyclyl, $(C_{4-6})$aryl, and hetero$(C_{1-5})$aryl, each unsubstituted or substituted with said 1-2 substituents.

In some other variations, $R_8$, when present, is selected from the group consisting of hydrogen, halo, cyano, $(C_{1-6})$alkoxy, $(C_{1-6})$alkyl, hetero$(C_{1-6})$alkyl, halo$(C_{1-6})$alkyl, carbonyl, aminocarbonyl, carbonylamino, amino, sulfonyl, $(C_{3-6})$cycloalkyl, $(C_{1-5})$heterocycloalkyl, $(C_{4-6})$aryl, and $(C_{1-5})$heteroaryl, each unsubstituted or substituted with said 1-2 substituents.

In some other variations, when present, $R_8$ is hydrogen, hydroxyl, chloro, cyano, triazolyl, $(C_{1-6})$alkylsulfonyl, $(C_{4-6})$arylsulfonyl, $(C_{1-6})$alkylaminosulfonyl, $(C_{1-6})$alkylsulfonylamino, (C4-6)arylaminosulfonyl, $(C_{1-6})$alkylsulfonylamino, $(C_{1-6})$alkyl, cyano$(C_{1-6})$alkyl, $-C(O)NHOR_{14}$, $-C(O)NHR_{14}$, and $-NHC(O)R_{14}$, where $R_{14}$ is selected from the group consisting of hydrogen, $(C_{1-6})$allcyl, and $(C_{3-6})$cycloalkyl, each unsubstituted or substituted with said 1-2 substituents.

In still other variations, when present, $R_8$ is selected from the group consisting of hydrogen, chloro, cyano, hydroxyl, $-NH_2$, $-NHC(O)CH_3$, $-C(O)NHCH_3$, $-(CH_3)_2CCN$, $-NHS(O)_2CH_3$, $-S(O)_2NHCH_3$, $-S(O)_2CH_3$, 4*H*-1,2,4-triazol-3-yl, $-C(O)NH_2$, and cyclopropylaminocarbonyl.

In yet still other variations, when present, $R_8$ is selected from the group consisting of hydrogen, chloro, and $-C(O)NHCH_3$.

In all of the above embodiments and variations, insome variations, when present, $R_9$ is selected from the group

consisting of hydrogen, halo, cyano, hydroxyl, $(C_{1-6})$alkoxy, $(C_{1-6})$alkyl, $(C_{1-10})$alkenyl, $(C_{1-10})$alkynyl, hetero$(C_{1-6})$alkyl, balo$(C_{1-6})$atkyl, carbonyl, aminocarbonyl, carbonylamino, amino, sulfonyl, $(C_{3-6})$ alicyclyl, hetero$(C_{1-5})$alicyclyl, $(C_{4-6})$ aryl, and $(C_{1-5})$heteroaryl, each unsubstituted or substituted.

In other variations, when present, $R_9$ is selected from the group consisting of hydrogen, halo, cyano, $(C_{1-6})$ alkoxy, $(C_{1-6})$alkyl, hetero$(C_{1-6})$alkyl, halo$(C_{1-6})$alkyl, carbonyl, aminocarbonyl, carbonylamino, amino, sulfonyl, $(C_{3-6})$ cycloalkyl, $(C_{1-5})$heterocycloalkyl, $(C_{4-6})$aryl, and $(C_{1-5})$heteroaryl, each unsubstituted or substituted with said 1-2 substituents.

In other variations, $R_9$ is hydrogen, hydroxyl, chloro, cyano, triazolyl, $(C_{1-6})$alkylsulfonyl, $(C_{4-6})$arylsulfonyl, $(C_{1-6})$alkylaminosulfonyl, $(C_{1-6})$alkylsulfonylamino, $(C_{4-6})$arylaminosulfonyl, $(C_{1-6})$alkyl, $(C_{1-6})$alkylsulfonylamino, cyano $(C_{1-6})$alkyl, $-C(O)NHOR_{14}$, $-C(O)NHR_{14}$, $-NHC(O)R_{14}$ where $R_{14}$ is selected from the group consisting of hydrogen, and $(C_{1-6})$alkyl, and $(C_{3-6})$cycloalkyl, each unsubstituted or substituted with said 1-2 substituents. In still other variations, when present, $R_9$ is selected from the group consisting of hydrogen, chloro, cyano, hydroxyl, $-NH_2$, $-NHC(O)CH_3$, - C (O)$NHCH_3$, $-(CH_3)_2CCN$, $-NHS(O)_2CH_3$, $-S(O)_2NHCH_3$, $-S(O)_2CH_3$, $-C(O)NH_2$, and phenylsulfonylamino.

In still other variations, when present, $R_9$ is selected from the group consisting of hydrogen, $-NHC(O)CH_3$, $-NHS(O)_2CH_3$, and $-S(O)_2CH_3$,

In all the above embodiments and variations of the compound of the invention, in some variations, when present, $R_{10}$ is selected from the group consisting of hydrogen, halo, cyano, hydroxyl, amino, $(C_{1-6})$alkoxy, $(C_{1-6})$alkyl, $(C_{1-10})$ alkenyl, $(C_{1-10})$alkynyl, hetero$(C_{1-6})$alkyl, halo$(C_{1-6})$alkyl, $(C_{3-6})$ alicyclyl, and hetero$(C_{1-5})$alicyclyl, each unsubstituted or substituted with said 1-2 substituents.

In other variations, when present, $R_{10}$ is selected from the group consisting of hydrogen, halo, cyanoamino, $(C_{1-6})$alkoxy, $(C_{1-6})$alkyl, hetero$(C_{1-6})$alkyl, halo$(C_{1-6})$alkyl, $(C_{3-6})$cycloalkyl, and hetero$(C_{1-5})$cydoafiyl, each unsubstituted or substituted with said 1-2 substituents.

In other variations, when present, $R_{10}$ is $(C_{1-6})$alkyl. In other variations, when present, $R_{10}$ is hydrogen. In other variations, when present, $R_{10}$ is $-NH_2$. In other variations, when present, $R_{10}$ is halo.

## $R_{11}$, $R_{12}$ and $R_{13}$

In all the above embodiments, particular embodiments, and their various variations of the compounds of the invention, in some variations, when present, each $R_{11}$, $R_{12}$ and $R_{13}$ is independently selected from the group consisting of hydrogen, halo, nitro, cyano, carbonyl, oxo, carbonyloxy, $(C_{1-10})$alkoxy, $(C_{4-12})$aryloxy, hetero$(C_{1-10})$aryloxy, oxycarbonyl, aminocarbonyl, amino, aminocarbonyl, carbonylamino, $(C_{1-10})$alkylaniino, sulfonylamino, sulfonyl, aminosulfonyl, sulfinyl, $(C_{1-10})$alkyl, $(C_{1-10})$alkenyl, $(C_{1-10})$alkynyl, carbonyl$(C_{1-10})$alkyl, sulfonyl$(C_{1-10})$alkyl, aza$(C_{1-10})$alkyl, $(C_{3-12})$cycloalkyl$(C_{1-5})$alkyl, hetero$(C_{3-12})$cycloalkyl$(C_{1-10})$altyl, aryl$(C_{1-10})$alkyl, hetero$(C_{1-10})$aryl$(C_{1-5})$alkyl, hetero$(C_{1-10})$alkyl, $(C_{3-6})$alicyclyl, and hetero$(C_{1-5})$alicyclyl, $C_{4-12})$aryl, and hetero$(C_{1-10})$aryl, each unsubstituted or further substituted with a substituent selected from the group consisting of halo, cyano, $(C_{1-6})$alkyl, liydroxyl$(C_{1-6})$aflyl, halo$(C_{1-6})$alkyl, amino $(C_{1-6})$alkyl, $(C_{3-6})$alieyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl.

In some variations, when present, $R_{11}$ is selected from the group consisting of halo, cyano, oxo, carbonyl, aminocarbonyl, and carbonylamino, each unsubstituted or substituted with said 1-2 substituents. In other variations, $R_{11}$ is selected from the group consisting of hydrogen, $-C(O)NH_2$, $-C(O)H$, cyano, carbonyl, aminocarbonyl, and carbonylamino, each unsubstituted or substituted with said one substituent. In still other variations, $R_{11}$ is $-C(O)NH_2$. In yet still other variations, $R_{11}$ is cyano.

In some variations, when present, $R_{12}$ is selected from the group consisting of halo, cyano, oxo, carbonyl, aminocarbonyl, and carbonylamino, each unsubstituted or substituted with said 1-2 substituents. In other variations, $R_{12}$ is from the group consisting of $-C(O)NH_2$, $-C(O)H$, cyano, carbonyl, aminocarbonyl, and carbonylamino, each unsubstituted or substituted.

In some variations, when present, $R_{13}$ is selected from the group consisting of halo, cyano, oxo, carbonyl, aminocarbonyl, and carbonylamino, each unsubstituted or substituted with said 1-2 substituents. In other variations, $R_{13}$ is selected from the group consisting of hydrogen and oxo, each unsubstituted or substituted.

## $R_{22}$, $R_{23}$, $R_{24}$, and $R_{25}$

In the embodiments, particular embodiments, and their various variations of the compounds of the invention, in some variations, when present, $R_{22}$, $R_{23}$, $R_{24}$, and $R_{25}$ are each independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thlocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, $(C_{3-12})$cy-

cloalkyl($C_{1-5}$)alkyl, hetero($C_{3-12}$)cycloalkyl($C_{1-10}$)alkyl, aryl($C_{1-10}$)alkyl, hetero($C_{1-10}$)aryl($C_{1-5}$)alkyl, hetero($C_{1-10}$)alkyl, ($C_{3-6}$)alicyclyl, and hetero($C_{1-5}$)alicyclyl, ($C_{4-12}$)aryl, and hetero($C_{1-10}$)aryl, each unsubstituted or further substituted with 1-2 substituents independently selected from the group consisting of halo, cyano, ($C_{1-6}$)alkyl, hydroxyl($C_{1-6}$)alkyl, halo ($C_{1-6}$)alkyl, amino($C_{1-6}$)alkyl, ($C_{3-6}$)alicyelyl($C_{1-6}$)alkyl, hetero($C_{1-5}$)alicyclyl($C_{1-6}$)alkyl, ($C_{4-6}$)aryl($C_{1-6}$)alkyl, hetero($C_{1-5}$) aryl($C_{1-6}$)alkyl, amino, ($C_{1-6}$)alkoxy, ($C_{3-6}$)alicyclyl, hetero($C_{1-5}$)alicyclyl, ($C_{4-6}$)aryl, hetero($C_{1-5}$)aryl.

In some other variations, when present, $R_{22}$, $R_{23}$, $R_{24}$, and $R_{25}$ are each independently selected from the group consisting of hydrogen, halo, oxy, carbonyloxy, ($C_{1-10}$)alkoxy, ($C_{4-12}$)aryloxy, hetero($C_{1-10}$)aryloxy, carbonyl, oxycarbonyl, aminocarbonyl, amino, ($C_{1-10}$)alkylamino, carbonylamino, sulfonylamino, sulfonyl, aminosulfonyl, ($C_{1-10}$)alkyl, halo ($C_{1-10}$)alkyl, bydroxy($C_{1-10}$)alkyl, carbonyl($C_{1-10}$)alkyl, sulfonyl($C_{1-10}$)alkyl, ($C_{3-12}$)cycloalkyl($C_{1-5}$)alkyl, hetero($C_{3-12}$) cyctoalkyl($C_{1-10}$)alkyl, aryl($C_{1-10}$)alkyl, hetero($C_{1-10}$)aryl($C_{1-5}$)alkyl, hetero($C_{1-10}$)alkyl, ($C_{3-6}$)cycloalkyl, and hetero($C_{1-5}$) cycloalkyl, ($C_{4-12}$)aryl, and hetero($C_{1-10}$)aryl, each unsubstituted or substituted with said 1-2 substituents.

In still other variations, when present, $R_{22}$, $R_{23}$, $R_{24}$, and $R_{25}$ are each independently selected from the group consisting of hydrogen, halo, oxy, carbonyloxy, ($C_{1-10}$)alkoxy, ($C_{4-12}$)aryloxy, hetero($C_{1-10}$)aryloxy, carbonyl, oxycarbonyl, aminocarbonyl, amino, ($C_{1-10}$)alkylamino, sulfonylamino, sulfonyl.

In still other variations, when present, $R_{22}$, $R_{23}$, $R_{24}$, and $R_{25}$ are each independently selected from the group consisting of ($C_{1-10}$)alkyl, halo($C_{1-10}$)alkyl, hydroxy($C_{1-10}$)alkyl, carbonyl($C_{1-10}$)alkyl, sulfonyl($C_{1-10}$)alkyl, ($C_{3-12}$)cycloalkyl($C_{1-5}$)alkyl, betero($C_{3-12}$)cycloalkyl($C_{1-10}$)alkyl, aryl($C_{1-10}$)alkyl, betero($C_{1-10}$)aryl($C_{1-5}$)alkyl, hetero($C_{1-10}$)alkyl, ($C_{4-12}$)aryl, and hetero($C_{1-10}$)aryl, each unsubstituted or substituted with said 1-2 substituents.

Particular examples of compounds according to the present invention include, but are not limited to:

*N*-(5-(1*H*-Indol-5-yl)thiazolo[5,4-*b*]pyridin-2-yl)acetamide;
*N*-(5-(1*H*-Indazol-5-yl)thiazolo[5,4-*b*]pyridin-2-yl)acetamide;
*N*-(5-(6-Acetamidopyridin-3-yl)thiazolo[5,4-*b*]pyridin-2-yl)acetamide;
*N*-(5-(4-(2-Cyanopropan-2-yl)phenyl)thiazolo[5,4-*b*]pyridin-2-yl) acetamide;
5-(2-Acetamidothiazolo[5,4-*b*]pyridin-5-yl)-N-methylpicolinamide;
*N*-(5-(6-Chloro-5-(phenylsulfonamido)pyridin-3-yl)thiazolo[5,4-*b*] pyridin-2-yl) acetamide;
*N*-(5-(6-Chloro-5-(methylsulfonamido)pyridin-3-yl)thiazolo[5,4-*b*] pyridin-2-yl)acetamide;
*N*-(5-(6-Chloro-5-(methylsulfonamido)pyridin-3-yl)thiazolo[5,4-*b*] pyridin-2-yl)cyclopropanecarboxamide;
*N*-(6-Phenylbenzo[*d*]thiazol-2-yl)acetamide;
*N*-(6-(Pyridin-3-yl)benzo[*d*]thiazol-2-yl)acetamide;
*N*-(6-(1*H*-Indol-5-yl)benzo[*d*]thiazol-2-yl)acetamide;
*N*-(6-(4-Aminophenyl)benzo[*d*]thiazol-2-yl)acetamide;
*N*-(6-(1*H*-Indol-4-yl)benzo[*d*]thiazol-2-yl)acetamide;
*N*-(6-(3-Formyl-1*H*-indol-5-yl)benzo[*d*]thiazol-2-yl)acetamide;
*N*-(6-(3-Cyano-1*H*-indol-5-yl)benzo[d]thiazol-2-yl)acetamide;
5-(2-Acetamidobenzo[*d*]thiazol-6-yl)-1*H*-indole-3-carboxamide;
*N*-(6-(6-Chloro-5-(phenylsulfonamido)pyridin-3-yl)benzo [*d*]thiazol-2-yl)acetamide;
*N*-(6-(4-Amino-2-(trifluoromethyl)phenyl)benzo[*d*]thiazol-2-yl)acetamide;
*N*-(6-(5-Aminopyrazin-2-yl)benzo[*d*]thiazol-2-yl)acetamide;
*N*-(6-(2,5-Dioxo-2,3,4,5-tetrahydro-1*H*-benzo[*e*][1,4]diazepin-8-yl) benzo[*d*]thiazol-2-yl)acetamide;
*N*-(5-(Imidazo[1,2-*a*]pyridin-6-yl)thiazolo[5,4-*b*]pyridin-2-yl)acetamide;
*N*-(5-(6-Cyanopyridin-3-yl)thiazolo[5,4-*b*]pyridin-2-yl)acetamide;
*N*-(5-(1*H*-Pyrrolo[2,3-*b*]pyridin-5-yl)thiazolo[5,4-*b*]pyridin-2-yl)acetamide;
*N*-(5-(1*H*-Pyrrolo[3,2-*b*]pyridin-6-yl)thiazolo[5,4-*b*]pyridin-2-yl)acetamide;
*N*-(5-(4-(Methylsulfonyl)phenyl)thiazolo[5,4-*b*]pyridine-2-yl)acetamide;
3-(2-Acetamidothiazolo[5,4-*b*]pyridin-5-yl)-*N*-methoxybenzamide;
*N*-(5-(4-(*N*-Methylsulfamoyl)phenyl)thiazolo[5,4-*b*]pyridine-2-yl)acetamide;
*N*-(5-(4-(Methylsulfonamido)phenyl)thiazolo[5,4-*b*]pyridin-2-yl)acetamide;
4-(2-Acetamidothiazolo[5,4-*b*]pyridin-5-yl)-*N*-methoxybenzamide;
*N*-(5-(2-Oxoindolin-5-yl)thiazolo[5,4-*b*]pyridin-2-yl)acetamide;
*N*-(6-(7-(Methylsulfonamido)-1*H*-indol-5-yl)benzo[*d*]thiazol-2-yl)acetamide;
*N*-(5-(Pyridin-4-yl)thiazolo[5,4-*b*]pyridin-2-yl)acetamide;
*N*-(5-(2-Aminophenyl)thiazolo[5,4-*b*]pyridin-2-yl)acetamide;
4-(2-Acetamidothiazolo[5,4-*b*]pyridin-5-yl)-*N*-methylbenzamide;
*N*-(5-(4-Acetamidophenyl)thiazolo[5,4-*b*]pyridin-2-yl)acetamide;
4-(2-Acetamidothiazolo[5,4-*b*]pyridin-5-yl)-*N*-cyclopropylbenzamide;
4-(2-Acetamidothiazolo[5,4-*b*]pyridin-5-yl)benzamide;
*N*-(4-(2-Acetamidobenzo[*d*]thiazol-6-yl)phenyl)acetamide ;

3-(2-Acetamidobenzo[*d*]thiazol-6-yl)-*N*-methylbenzamide;
*N*-(3-(2-Acetamidobenzo[*d*]tHiazol-6-yl)phenyl)acetamide;
*N*-(5-(4-(4*H*-1,2,4-Triazol-3-yl)phenyl)thiazolo[5,4-*b*]pyridin-2-yl)acetamide;
*N*-(5-(5-Amino-6-chloropyridin-3-yl)thiazolo[5,4-d]pyrimidin-2-yl)acetamide;
*N*-(5-(6-Chloro-5-(methylsulfonamido)pyridin-3-yl)thiazolo[5,4-*d*]pyrimidin-2-yl)acetamide;
*N*-(5-(6-Aminopyridin-3-yl)thiazolo[5,4-*d*]pyrimidin-2-yl)acetamide;
*N*-(5-(6-Amino-5-(trifluoromethyl)pyridin-3-yl)thiazolo[5,4-*d*]pyrimidin-2-yl)acetamide;
*N*-(5-(6-chloro-5-(ethylsulfonamido)pyridin-3-yl)thiazolo[5,4-*b*] pyridin-2-yl)acetamide;
*N*-(6-(6-chloro-5-(methylsulfonamido)pyridin-3-yl)benzo [*d*]thiazol-2-yl)acetamide;
*N*-(5-(6-chloro-5-(cyclopropanesulfonamido)pyridin-3-yl)thiazolo[5,4-*b*]pyridin-2-yl)acetamide;
*N*-(5-(6-Chloro-5-(phenylsulfonamido)pyridin-3-yl)thiazolo[5,4-*d*]pyrimidin-2-yl)acetamide; and
*N*-(5-(6-chloro-5-(methylsulfonamido)pyridin-3-yl)thiazolo[5,4-*b*]pyridin-2-yl)-2-hydroxyacetamide;
*N*-(5-(6-chloro-5-(methylsulfonamido)pyridin-3-yl)thiazolo[5,4-b]pyridin-2-yl)-2-(1-methylpiperidin-4-yloxy)acetamide
*N*-(5-(6-chloro-5-(methylsulfonamido)pyridin-3-yl)thiazolo[5,4-*b*]pyridin-2-yl)-2-(dimethylamino)acetamide;
*N*-(5-(6-chloro-5-(methylsulfonamido)pyridin-3-yl)thiazolo[5,4-*b*]pyridin-2-yl)-3-hydroxypropanamide;
(R)-*N*-(5-(6-chloro-5-(methylsulfonamido)pyridin-3-yl)thiazolo[5,4-*b*]pyridin-2-yl)-2,3-dihydroxypropanamide;
(S)-*N*-(5-(6-chloro-5-(methylsulfonamido)pyridin-3-yl)thiazolo[5,4-*b*]pyridin-2-yl)-2,3-dihydroxypropanamide;
(S)-*N*-(5-(6-chloro-5-(methylsulfonamido)pyridin-3-yl)thiazolo[5,4-*b*]pyridin-2-yl)-3,4-dihydroxybutanamide;
(R)-*N*-(5-(6-chloro-5-(methylsulfonamido)pyridin-3-yl)thiazolo[5,4-*b*]pyridin-2-yl)-3,4-dihydroxybutanamide;
*N*-(5-(6-chloro-5-(2-hydroxyethylsulfonamido)pyridin-3-yl)thiazolo[5,4-*b*]pyridin-2-yl)acetamide;
*N*-(5-(6-chloro-5-(3-hydroxypropylsulfonamido)pyridin-3-yl)thiazolo[5,4-*b*]pyridin-2-yl)acetamide;
*N*-(5-(6-chloro-5-(2-(dimethylamino)ethylsulfonamido)pyridin-3-yl)thiazolo[5,4-b]pyridin-2-yl)acetamide;
*N*-(5-(6-chloro-5-(3-(dimethylamino)propylsulfonamido)pyridin-3-yl)thiazolo[5,4-*b*]pyridin-2-yl)acetamide;
*N*-(5-(6-chloro-5-(1-methylpiperldine-4-sulfonamido)pyridin-3-yl)thiazolo[5,4-*b*]pyridin-2-yl)acetamide;
*N*-(5-(6-chloro-5-(methylsulfonamido)pyridin-3-yl)thiazolo[5,4-*b*]pyridin-2-yl)-1-methylpiperidine-4-carboxamide;
*N*-(5-(6-chloro-5-(methylsulfonamido)pyridin-3-yl)thiazolo[5,4-*b*]pyridin-2-yl)-2-(1-methylpiperidin-4-yl)acetamide;
*N*-(5-(6-chloro-5-(methylsulfonamido)pyridin-3-yl)thiazolo[5,4-*b*]pyridin-2-yl)-3-hydroxy-2-(hydroxymethyl)propanamide;
*N*-(5-(6-chloro-5-(methylsulfonamido)pyridin-3-yl)thiazolo[5,4-*b*]pyridin-2-yl)-4-hydroxy-3-(hydroxymethyl)butanamide;
*N*-(5-(6-chloro-5-(2-hydroxy-1-(hydroxymethyl)ethylsulfonamido)pyridin-3-yl)thiazolo[5,4-*b*]pyridin-2-yl)acetamide;
*N*-(5-(6-chloro-5-(3-hydroxy-2-(hydroxymethyl)propylsulfonamido)pyridin-3-yl)thiazolo[5,4-*b*]pyridin-2-yl)acetamide;
*N*-(5-(6-chloro-5-(3-(4-methylpiperazin-1-yl)propylsulfonamido)pyridin-3-yl)thiazolo[5,4-*b*]pyridin-2-yl)acetamide;
*N*-(5-(6-chloro-5-((1-methylpiperidin-4-yl)methylsulfonamido)pyridin-3-yl)thiazolo[5,4-*b*]pyridin-2-yl)acetamide;
*N*-(5-(1*H*-indazol-4-yl)thiazolo[5,4-*b*]pyridin-2-yl)acetamide;
*N*-(5-(1*H*-pyrazolo[3,4-*b*]pyridin-5-yl)thiazolo[5,4-*b*]pyridin-2-yl)acetamide;
*N*-(5-(3*H*-imidazo[4,5-b]pyridin-6-yl)thiazolo[5,4-*b*]pyridin-2-yl)acetamide;
*N*-(5-(1*H*-pyrazolo[4,3-b]pyridin-6-yl)thiazolo[5,4-*b*]pyridin-2-yl)acetamide;
*N*-(5-(6-chloro-5-(methylsulfonamido)pyridin-3-yl)thiazolo[5,4-*b*]pyridin-2-yl)propionamide;
*N*-(5-(5-chloro-6-(methylsulfonamido)imidazo[1,5-*a*]pyridin-8-yl)thiazolo[5,4-*b*]pyridin-2-yl)acetamide;
*N*-(5-(1-(methylsulfonyl)-1*H*-pyrrolo[3,4-*b*]pyridin-6-yl)thiazolo[5,4-*b*]pyridin-2-yl)acetamide;
*N*-(5-(1-(methylsulfonyl)-1*H*-pyrrolo[3,4-*b*]pyridin-3-yl)thiazolo[5,4-*b*]pyridin-2-yl)acetamide;
*N*-(5-(1,3-dimethyl-2-oxo-2,3-dihydro-1*H*-imidazo[4,5-*b*]pyridin-6-yl)thiazolo[5,4-*b*]pyridin-2-yl)acetamide;
*N*-(5-(oxazolo[5,4-*b*]pyridin-6-yl)thiazolo[5,4-*b*]pyridin-2-yl)acetamide;
*N*-(5-(oxazolo[4,5-*b*]pyridin-6-yl)thiazolo[5,4-*b*]pyridin-2-yl)acetamide;
*N*-(5-(7-oxo-6,7-dihydro-5*H*-pyrrolo[3,4-*b*]pyridin-3-yl)thiazolo[5,4-*b*]pyridin-2-yl)acetamide;
*N*-(5-(5-oxo-6,7-dihydro-5*H*-pyrrolo[3,4-*b*]pyridin-3-yl)thiazolo[5,4-*b*]pyridin-2-yl)acetamide;
*N*-(5-(7-(methylsulfonamido)benzo[*d*]oxazol-5-yl)thiazolo[5,4-*b*]pyridin-2-yl)acetamide; and
*N*-(5-(7-(methylsulfonamido)-1*H*-benzo[*d*]imidazol-5-yl)thiazolo[5,4-*b*]pyridin-2-yl)acetamide.

In another of its aspects, the present invention relates to process of making compounds that are useful as PI3K/mTOR inhibitors.

In one embodiment, the process comprises the steps:

reacting an cyclized intermediate having the formula

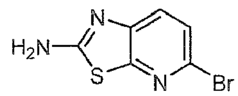

with acetyl anhydride under conditions that form a second intermediate having the formula

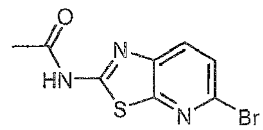

reacting the second intermediate with a compound having the formula

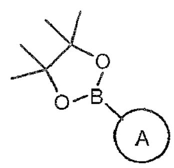

under conditions that form product having the formula

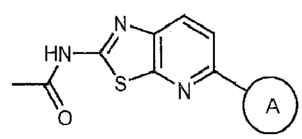

wherein

ring A is aryl or heteroaryl, each unsubstituted or substituted with 1-5 substituents which is independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cydoalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl;;

provided that

when ring A is a pyridyl, pyrimidinyl or pyrazinyl, ring A does not have an amino, substituted amino, substituted oxy, or fluoro attached to position 4 of ring A where the ring atom through which ring A attaches to the ring containing $X_1$ and $X_2$ is counted as position 1.

In some variations of the above embodiment, the process further comprises reacting 6-bromo-3-aminopyridine with potassium thiocyanate and bromine in acetic acid to form the cyclized intermediate.

In some variations of the above embodiment of the process of the invention, ring A is selected from the group consisting of

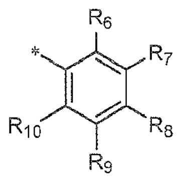
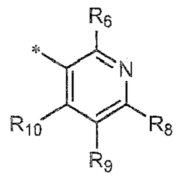
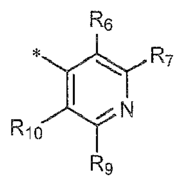
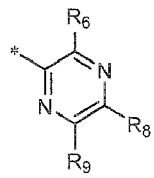
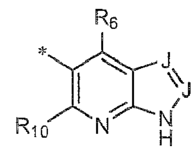

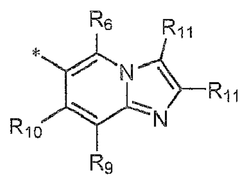
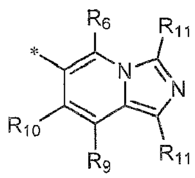
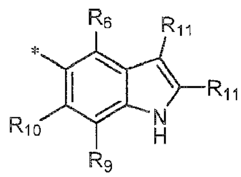
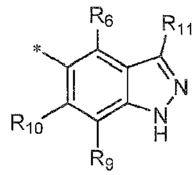

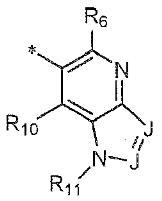
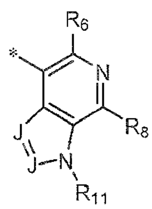
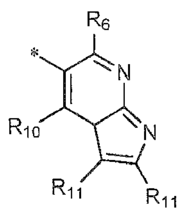
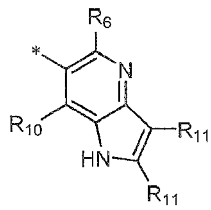
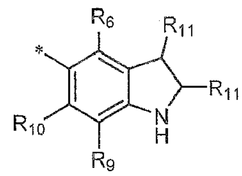

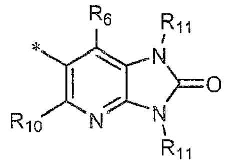
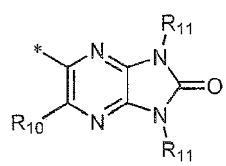
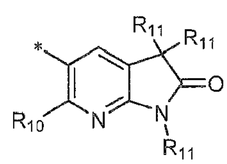
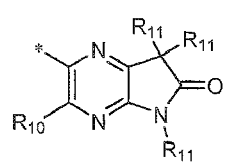

where

each $R_6$, $R_7$, $R_8$, $R_9$, and $R_{10}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$aklyl, hetero$(C_{8-12})$bicycloaryl$(C_{1-5})$alkyl, hetero$(C_{1-10})$alkyl, $(C_{3-12})$cydoalkyl, hetero$(C_{3-12})$cycloalkyl, $(C_{9-12})$bicycloalkyl, hetero$(C_{3-12})$blcycloalkyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl; and

$R_{11}$ is selected from the group consisting of halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$cycloalkyl, hetero$(C_{3-12})$cycloalkyl, $(C_{9-12})$bicycloalkyl, hetero$(C_{3-12})$bicycloalkyl, $(C_{4-12})$aryl, hetero$(C_{1-10})$aryl, $(C_{9-12})$bicycloaryl, and hetero$(C_{4-12})$bicycloaryl, each unsubstituted or further substituted with a substituent selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl.

In some other variations, ring A is of the formula:

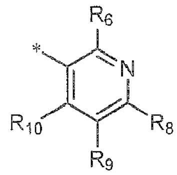

In another embodiment, the process of the invention comprising:

reacting a starting material having the formula

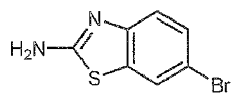

with a compound of the formula

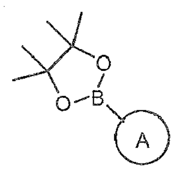

under conditions that form an intermediate of the formula

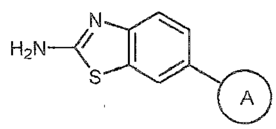

reacting said intermediate with acetyl anhydride under conditions that form a product having the formula

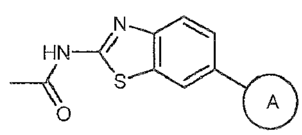

wherein

ring A is aryl or heteroaryl, each unsubstituted or substituted with 1-5 substituents which is independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, heiero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl

$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero $(C_{1-5})$aryl;

provided that

when ring A is a pyridyl, pyrimidinyl or pyrazinyl, ring A does not have an amino, substituted amino, substituted oxy, or fluoro attached to position 4 of ring A where the ring atom through which ring A attaches to the ring containing $X_1$ and $X_2$ is counted as position 1.

In some variations of the above embodiment of the process of the invention, ring A is selected from the group consisting of

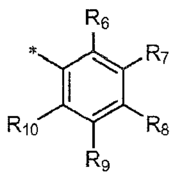
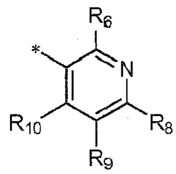
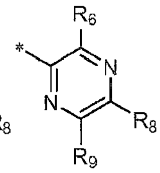
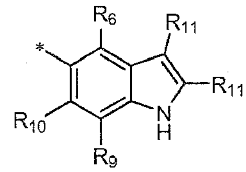

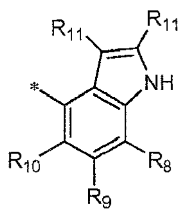
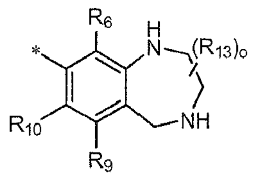

where

o is 0, 1, 2, 3, 4, 5, or 6;

each $R_6$, $R_7$, $R_8$, $R_9$, and $R_{10}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, sulfinyl, $(C_{1-10})$alkyl, $(C_{1-10})$ alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl $(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl $(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$ alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$ alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$ alicyclyl, $(C_{4-6})$aryl, hetero$(C_{1-5})$aryl; and

each $R_{11}$ and $R_{13}$ is independently selected from the group consisting of halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$ alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$ alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents selected from the group consisting of halo, cyano, $(C_{1-6})$ alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$ alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl;

provided that

when ring A is a pyridinyl or pyrazinyl, $R_8$ is not an amino, substituted amino, substituted oxy, or fluoro.

In some other variations, ring A is of the formula:

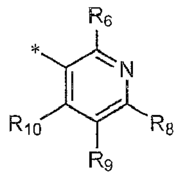

It is noted that the compounds of the present invention may be in the form of a pharmaceutically acceptable salt, biohydrolyzable ester, biohydrolyzable amide, biohydrolyzable carbamate, solvate, hydrate or prodrug thereof. For example, the compound optionally comprises a substituent that is convertible *in vivo* to a different substituent such as hydrogen.

It is further noted that the compound may be present as a mixture of stereoisomers, or the compound may present as a single stereoisomer.

In another of its aspects, there is provided a pharmaceutical composition comprising as an active ingredient a compound according to any one of the above embodiments and variations. In one particular variation, the composition is a solid formulation adapted for oral administration. In another particular variation, the composition is a liquid formulation adapted for oral administration. In yet another particular variation, the composition is a tablet. In still another particular variation, the composition is a liquid formulation adapted for parenteral administration.

The present invention also provides a pharmaceutical composition comprising a compound according to any one of the above embodiments and variations, wherein the composition is adapted for administration by a route selected from the group consisting of orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, and intrathecally.

In yet another of its aspects, there is provided a kit comprising a compound of any one of the above embodiments and variations; and instructions which comprise one or more forms of information selected from the group consisting of indicating a disease state for which the composition is to be administered, storage information for the composition, dosing information and instructions regarding how to administer the composition. In one particular variation, the kit comprises the compound in a multiple dose form.

In still another of its aspects, there is provided an article of manufacture comprising a compound of any one of the above embodiments and variations; and packaging materials. In one variation, the packaging material comprises a container for housing the compound. In one particular variation, the container comprises a label indicating one or more members of the group consisting of a disease state for which the compound is to be administered, storage information, dosing information and/or instructions regarding how to administer the compound. In another variation, the article of manufacture comprises the compound in a multiple dose form.

In a further of its aspects, there is provided a therapeutic method comprising administering a compound of any one of the above embodiments and variations to a subject.

In another of its aspects, the invention is related to methods of inhibiting PI3K, mTOR and PI3K and mTOR together.

In one embodiment, the invention is related to a method of inhibiting PI3K comprising contacting PI3K with a compound of any one of the above embodiments and variations.

In another embodiment, the invention is related to a method of inhibiting PI3K comprising causing a compound of any one of the above embodiments and variations to be present in a subject in order to inhibit PI3K *in vivo.*

In yet another embodiment, the invention is related to a method of inhibiting PI3K comprising administering a first compound to a subject that is converted *in vivo* to a second compound wherein the second compound inhibits PI3K *in vivo,* the second compound being a compound according to any one of the above embodiments and variations.

In yet another embodiment, the invention is related to a method of treating a disease state for which PI3K possesses activity that contributes to the pathology and/or symptomology of the disease state, the method comprising causing a compound of any one of the above embodiments and variations to be present in a subject in a therapeutically effective amount for the disease state.

In yet another embodiment, the invention is related to a method of treating a disease state for which PI3K possesses activity that contributes to the pathology and/or symptomology of the disease state, the method comprising administering a compound of any one of the above embodiments and variations to a subject, wherein the compound is present in the subject in a therapeutically effective amount for the disease state.

In yet another embodiment, the invention is related to a method of treating a disease state for which PI3K possesses activity that contributes to the pathology and/or symptomology of the disease state, the method comprising administering a first compound to a subject that is converted *in vivo* to a second compound wherein the second compound inhibits PI3K *in vivo*, the second compound being a compound according to any one of the above embodiments and variations.

In one embodiment, the invention is related to a method of inhibiting mTOR comprising contacting mTOR with a compound of any one of the above embodiments and variations.

In another embodiment, the invention is related to a method of inhibiting mTOR comprising causing a compound of any one of the above embodiments and variations to be present in a subject in order to inhibit mTOR *in vivo.*

In yet another embodiment, the invention is related to a method of inhibiting mTOR comprising administering a first compound to a subject that is converted *in vivo* to a second compound wherein the second compound inhibits mTOR *in vivo,* the second compound being a compound according to any one of the above embodiments and variations.

In yet another embodiment, the invention is related to a method of treating a disease state for which mTOR possesses activity that contributes to the pathology and/or symptomology of the disease state, the method comprising causing a compound of any one of the above embodiments and variations to be present in a subject in a therapeutically effective amount for the disease state.

In yet another embodiment, the invention is related to a method of treating a disease state for which mTOR possesses activity that contributes to the pathology and/or symptomology of the disease state, the method comprising administering a compound of any one of the above embodiments and variations to a subject, wherein the compound is present in the subject in a therapeutically effective amount for the disease state.

In yet another embodiment, the invention is related to a method of treating a disease state for which mTOR possesses activity that contributes to the pathology and/or symptomology of the disease state, the method comprising administering a first compound to a subject that is converted *in vivo* to a second compound wherein the second compound inhibits mTOR *in vivo,* the second compound being a compound according to any one of the above embodiments and variations.

In one embodiment, the invention is related to a method of inhibiting both PI3K and mTOR comprising contacting both PI3K and mTOR with a compound of any one of the above embodiments and variations.

In another embodiment, the invention is related to a method of inhibiting both PI3K and mTOR comprising causing a compound of any one of the above embodiments and variations to be present in a subject in order to inhibit both PI3K and mTOR *in vivo.*

In yet another embodiment, the invention is related to a method of inhibiting both PI3K and mTOR comprising administering a first compound to a subject that is converted *in vivo* to a second compound wherein the second compound inhibits both PI3K and mTOR *in vivo,* the second compound being a compound according to any one of the above embodiments and variations.

In yet another embodiment, the invention is related to a method of treating a disease state for which PI3K and mTOR each possesses activity that contributes to the pathology and/or symptomology of the disease state, the method comprising causing a compound of any one of the above embodiments and variations to be present in a subject in a therapeutically effective amount for the disease state.

In yet another embodiment, the invention is related to a method of treating a disease state for which PI3K and mTOR each possesses activity that contributes to the pathology and/or symptomology of the disease state, the method comprising administering a compound of any one of the above embodiments and variations to a subject, wherein the compound is present in the subject in a therapeutically effective amount for the disease state.

In yet another embodiment, the invention is related to a method of treating a disease state for which PI3K and mTOR each possesses activity that contributes to the pathology and/or symptomology of the disease state, the method comprising administering a first compound to a subject that is converted *in vivo* to a second compound wherein the second compound inhibits both PI3K and mTOR *in vivo,* the second compound being a compound according to any one of the above embodiments and variations.

In some variations of each of the above embodiments and variations of the methods of the invention, the disease state is selected from the group consisting of tumors, cancers, cancer metastasis, allergy/asthma, diseases and conditions of the immune system, inflammation, disease and conditions of the central nervous system (CNS), cardiovascular diseases, viral infections, dermatological diseases, and diseases and conditions related to uncontrolled angiogenesis.

In other variations, the disease state is cancers. In some variations, the cancer is selected from the group consisting of squamous cell carcinoma, astrocytoma, Kaposi's sarcoma, glioblastoma, non small-cell lung cancer, bladder cancer, head and neck cancer, melanoma, ovarian cancer, prostate cancer, breast cancer, small-cell lung cancer, glioma, colorectal cancer, genitourinary cancer, gastrointestinal cancer, renal cancer, hematological cancers, non-Hodgkin's lymphoma, lymphoma, multiple myeloma, leukemia (including acute myelogenous leukemia, chronic myelogenous leukemia, chronic lymphocytic leukemia), myelodysplastic syndrome, and mesothelioma. In other variations, the cancer

is selected from the group consisting of cancer of the breast, colorectum, lung (including small cell lung cancer, non-small cell lung cancer and bronchioalveolar cancer) and prostate, cancer of the bile duct, bone, bladder, head and neck, kidney, liver, gastrointestinal tissue, oesophagus, ovary, pancreas, skin, testes, thyroid, uterus, cervix and vulva, and of leukaemias (including ALL and CML), multiple myeloma and lymphomas.

In other variations, the disease state is inflammation.

In other variations, the disease state is chronic inflammation. In some variations, the chronic inflammation disease is selected from the group consisting of inflammatory bowel diseases, psoriasis, sarcoidois, and rheumatoid arthritis. In other variation, the chronic inflammation is inflammatory bowel diseases. In other variation, the chronic inflammation is rheumatoid arthritis.

In another aspect, the invention is related to use of the compounds of each of the above embodiments and variations as a medicament.

In some variations, the use is for the manufacture of a medicament for inhibiting mTOR. In other variations, the use is for the manufacture of a medicament for inhibiting a PI3K. In other variations, the use is for the manufacture of a medicament for inhibiting a PI3K and mTOR. In still other variations, the use is for the manufacture of a medicament for treating a disease state for which a PI3K possesses activity that contributes to the pathology and/or symptomology of the disease state. In still other variations, the use is for the manufacture of a medicament for treating a disease state for which mTOR possesses activity that contributes to the pathology and/or symptomology of the disease state. In still other variations, the use is for the manufacture of a medicament for treating a disease state for which a PI3K and mTOR each possesses activity that contributes to the pathology and/or symptomology of the disease state.

In still other variations, the use is for the manufacture of a medicament for treating cancer. In still other variations, the use is in the manufacture of a medicament for treating chromic inflammatory diseases.

It is noted that in all of the above embodiments and variations of the uses of the compound related to the inhibition of PI3K only, or both PI3K and mTOR, in some variations, the PI3K is a Class I PI3K. In some variations, the Class I PI3K is a Class IA PI3K. In other variations, Class I PI3K is a Class IB PI3K. In some variations, the Class IA PI3K is PI3K$\alpha$. In other variations, the Class IA PI3K is PI3K$\beta$. In other variations, the Class IA PI3K is PI3K$\delta$. In other variations, the Class I PI3K is a Class IB PI3K$\gamma$.

## Salts, Hydrates, and Prodrugs of PI3K/mTOR Inhibitors

It should be recognized that the compounds of the present invention may be present and optionally administered in the form of salts, hydrates and prodrugs that are converted *in vivo* into the compounds of the present invention. For example, it is within the scope of the present invention to convert the compounds of the present invention into and use them in the form of their pharmaceutically acceptable salts derived from various organic and inorganic acids and bases in accordance with procedures well known in the art.

When the compounds of the present invention possess a free base form, the compounds can be prepared as a pharmaceutically acceptable acid addition salt by reacting the free base form of the compound with a pharmaceutically acceptable inorganic or organic acid, *e.g.,* hydrohalides such as hydrochloride, hydrobromide, hydroiodide; other mineral acids and their corresponding salts such as sulfate, nitrate, phosphate, *etc*.; and alkyl and monoarylsulfonates such as ethanesulfonate, toluenesulfonate and benzenesulfonate; and other organic acids and their corresponding salts such as acetate, tartrate, maleate, succinate, citrate, benzoate, salicylate and ascorbate. Further acid addition salts of the present invention include, but are not limited to: adipate, alginate, arginate, aspartate, bisulfate, bisulfite, bromide, butyrate, camphorate, camphorsulfonate, caprylate, chloride, chlorobenzoate, cyclopentanepropionate, digluconate, dihydrogenphosphate, dinitrobenzoate, dodecylsulfate, fumarate, galacterate (from mucic acid), galacturonate, glucoheptonate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate and phthalate. It should be recognized that the free base forms will typically differ from their respective salt forms somewhat in physical properties such as solubility in polar solvents, but otherwise the salts are equivalent to their respective free base forms for the purposes of the present invention.

When the compounds of the present invention possess a free acid form, a pharmaceutically acceptable base addition salt can be prepared by reacting the free acid form of the compound with a pharmaceutically acceptable inorganic or organic base. Examples of such bases are alkali metal hydroxides including potassium, sodium and lithium hydroxides; alkaline earth metal hydroxides such as barium and calcium hydroxides; alkali metal alkoxides, *e.g.,* potassium ethanolate and sodium propanolate; and various organic bases such as ammonium hydroxide, piperidine, diethanolamine and *N*-methylglutamine. Also included are the aluminum salts of the compounds of the present invention. Further base salts of the present invention include, but are not limited to: copper, ferric, ferrous, lithium, magnesium, manganic, manganous, potassium, sodium and zinc salts. Organic base salts include, but are not limited to, salts of primary, secondary and

tertiary amines, substituted amines including naturally occurring substituted amines, cyclic amines and basic ion exchange resins, *e.g.*, arginine, betaine, caffeine, chloroprocaine, choline, *N,N'*-dibenzylethylenediamine (benzathine), dicyclohexylamine, diethanolamine, 2-diethylaminoethanol, 2-dimethylaminoethanol, ethanolamine, ethylenediamine, *N*-ethylmorpholine, *N*-ethylpiperidine, glucamine, glucosamine, histidine, hydrabamine, iso-propylamine, lidocaine, lysine, meglumine, *N*-methyl-D-glucamine, morpholine, piperazine, piperidine, polyamine resins, procaine, purines, theobromine, triethanolamine, triethylamine, trimethylamine, tripropylamine and tris-(hydroxymethyl)-methylamine (tromethamine). It should be recognized that the free acid forms will typically differ from their respective salt forms somewhat in physical properties such as solubility in polar solvents, but otherwise the salts are equivalent to their respective free acid forms for the purposes of the present invention.

Compounds of the present invention that comprise basic nitrogen containing groups may be quaternized with such agents as $(C_{1-4})$ alkyl halides, *e.g.*, methyl, ethyl, isopropyl and tert-butyl chlorides, bromides and iodides; di $(C_{1-4})$ alkyl sulfates, *e.g.*, dimethyl, diethyl and diamyl sulfates; $(C_{10-18})$ alkyl halides, *e.g.*, decyl, dodecyl, lauryl, myristyl and stearyl chlorides, bromides and iodides; and aryl $(C_{1-4})$ alkyl halides, *e.g.*, benzyl chloride and phenethyl bromide. Such salts permit the preparation of both water-soluble and oil-soluble compounds of the present invention.

*N*-oxides of compounds according to the present invention can be prepared by methods known to those of ordinary skill in the art. For example, *N*-oxides can be prepared by treating an unoxidized form of the compound with an oxidizing agent (*e.g.*, trifluoroperacetic acid, permaleic acid, perbenzoic acid, peracetic acid, meta-chloroperoxybenzoic acid, or the like) in a suitable inert organic solvent (*e.g.*, a halogenated hydrocarbon such as dichloromethane) at approximately 0 °C. Alternatively, the *N*-oxides of the compounds can be prepared from the *N*-oxide of an appropriate starting material.

Prodrug derivatives of compounds according to the present invention can be prepared by modifying substituents of compounds of the present invention that are then *converted in vivo* to a different substituent. It is noted that in many instances, the prodrugs themselves also fall within the scope of the range of compounds according to the present invention. For example, prodrugs can be prepared by reacting a compound with a carbamylating agent (*e.g.*, 1,1-acyloxyalkylcarbonochloridate, para-nitrophenyl carbonate, or the like) or an acylating agent. Further examples of methods of making prodrugs are described in Saulnier et al.(1994), Bioorganic and Medicinal Chemistry Letters, Vol. 4, p. 1985.

Protected derivatives of compounds of the present invention can also be made. Examples of techniques applicable to the creation of protecting groups and their removal can be found in P.G.M. Wuts and T.W. Greene in "Greene's Protective Groups in Organic Synthesis" 4th edition, John Wiley and Sons, 2007.

Compounds of the present invention may also be conveniently prepared, or formed during the process of the invention, as solvates (*e.g.*, hydrates). Hydrates of compounds of the present invention may be conveniently prepared by recrystallization from an aqueous/organic solvent mixture, using organic solvents such as dioxin, tetrahydrofuran or methanol.

A "pharmaceutically acceptable salt", as used herein, is intended to encompass any compound according to the present invention that is utilized in the form of a salt thereof, especially where the salt confers on the compound improved pharmacokinetic properties as compared to the free form of compound or a different salt form of the compound. The pharmaceutically acceptable salt form may also initially confer desirable pharmacokinetic properties on the compound that it did not previously possess, and may even positively affect the pharmacodynamics of the compound with respect to its therapeutic activity in the body. An example of a pharmacokinetic property that may be favorably affected is the manner in which the compound is transported across cell membranes, which in turn may directly and positively affect the absorption, distribution, biotransformation and excretion of the compound. While the route of administration of the pharmaceutical composition is important, and various anatomical, physiological and pathological factors can critically affect bioavailability, the solubility of the compound is usually dependent upon the character of the particular salt form thereof, which it utilized. One of skill in the art will appreciate that an aqueous solution of the compound will provide the most rapid absorption of the compound into the body of a subject being treated, while lipid solutions and suspensions, as well as solid dosage forms, will result in less rapid absorption of the compound.

## Uses of the Compounds of the Invention

It is well known that signaling pathways mediated by the PI3K-mTOR family of kinases have a central role in a number of cell processes including translation, cell growth, proliferation and survival, and deregulation of these pathways is a causative factor in a wide spectrum of human disorders.

There is now considerable evidence that Class I PI3K enzymes contribute to tumorigenesis in a variety of human cancers, either directly or indirectly (Vivanco, I., and Sawyers, C.L. (2002) Nature Rev. Cancer 2:489-501). There is evidence that PI3K signaling plays an important role in mediating angiogenic events in response to pro-angiogenic factors (Abid et al., Arterioscler. Thromb. Vasco Biol., 2004, 24, 294-300). Class I PI3K enzymes are shown to involve in motility and migration (Sawyer, Expert Opinion Investing. Drugs, 2004, 13, 1-19). Recent data suggest that PI3Kgamma is involved in the path finding process in tumor metastasis (Hannigan et al., Proc. Nat. Acad. of Sciences of U.S.A.,

2002, 99(6), 3603-8). In addition, Class I PI3K enzymes play an important role in the regulation of immune cells contributing to protumourigenic effects of inflammatory cells (Coussens and Werb, Nature, 2002, 420, 860867). Class I PI3K enzymes were also shown to have important roles in cells of the immune system (Koyasu, Nature Immunology, 2003, 4, 313-319) and thus they are therapeutic targets for inflammatory and allergic indications.

There is evidence that mTOR signaling effects cell proliferation (Dancey, Expert Opinion on Investigational Drugs, 2005, 14,313-328; Hudson et al., Molecular and Cellular Biology, 2002, 22, 7004-7014). In addition to tumourigenesis, there is evidence that elevated mTOR kinase signaling plays a role in an array of hamartoma syndromes (Tee and Blenis, Seminars in Cell and Developmental Biology, 2005, 16, 29-37). Recent studies indicate that mTOR kinase may have a role in antigen-induced proliferation of T cells, B cells and antibody production (Sehgal, Transplantation Proceedings, 2003, 35, 7S-14S) (Easton & Houghton, Expert Opinion on Therapeutic Targets, 2004, 8, 551-564). Elevated mTOR kinase activity has been associated with cardiac hypertrophy, which is of clinical importance as a major risk factor for heart failure and is a consequence of increased cellular size of cardiomyocytes (Tee & Blenis, Seminars in Cell and Developmental Biology, 2005, 16, 29-37).

Thus the PI3K inhibitors, mTOR inhibitors, and PI3K and mTOR dual inhibitors of the present invention may be used for the treatment and prevention of diseases and conditions that are mediated by PI3K and/or mTOR kinases; these diseases and conditions include, but are not limited to, tumorigenesis, cancers, cancer metastasis, allergy/asthma, diseases and conditions of the immune system, inflammation, disease and conditions of the central nervous system (CNS), cardiovascular disease, viral infections, dermatological disease, and diseases and conditions related to uncontrolled angiogenesis.

**Diseases and conditions related to Cell Proliferation**

One set of indications for the PI3K and/or mTOR inhibitors of the invention are those involving undesirable or uncontrolled cell proliferation. Such indications include benign tumors, hamartoma conditions, various types of cancers such as primary tumors and tumor metastasis, restenosis (e.g. coronary, carotid, and cerebral lesions), abnormal stimulation of endothelial cells (atherosclerosis), insults to body tissue due to surgery, abnormal wound healing, diseases that produce fibrosis of tissue, repetitive motion disorders, disorders of tissues that are not highly vascularized, proliferative responses associated with organ transplants, neurodegenerative disorders including Parkinson's, Alzheimer's, Huntington's and prion-related disease, inflammation and inflammation related disorders such as pain, headaches, fever, arthritis, asthma, bronchitis, tendonitis, eczema, inflammatory bowel disease, and the like, and diseases dependent on angiogenesis such as, cancer, arthritis, diabetic retinopathy, age associated macular degeneration (AMD) and infectious diseases in particular fungal infections, viral diseases including but not limited to diseases caused by hepatitis B virus (HBV), hepatitis C virus (HCV) and herpes simplex virus type-1 (HSV-1), cardiovascular and central nervous system diseases.

Benign tumors and related conditions that may be treated by the PI3K and/or mTOR inhibitors of the present include, but are not limited to, hemangiomas, hepatocellular adenoma, cavernous haemangioma, focal nodular hyperplasia, acoustic neuromas, neurofibroma, bile duct adenoma, bile duct cystanoma, fibroma, lipomas, leiomyomas, mesotheliomas, teratomas, myxomas, nodular regenerative hyperplasia, trachomas, pyogenic granulomas, and the like, and hamartoma conditions such asPeutz-Jeghers syndrome (PJS), Cowden disease, BaImaYaII-Riley-Ruvalcaba syndrome (BRRS), Proteus syndrome, Lhermitte-Duclos disease and Tuberous Sclerosis (TSC).

Specific types of cancers or malignant tumors, either primary (from the tissue in which they are found) or secondary (metastases), that can be treated using the PI3K/mTOR inhibitors of the present invention include solid tumors such as carcinomas and sarcomas and the leukemias and lymphoid malignancies. Particularly, the cancers that can be treated include, but are not limited to, leukemia, breast cancer, genitourinary cancer, skin cancer, bone cancer, prostate cancer, liver cancer, lung cancer (including small-cell lung tumor, non small-cell lung cancer and bronchioalveolar cancer), brain cancer, cancer of the larynx, gall bladder, pancreas, rectum, bile duct, parathyroid, thyroid, adrenal, neural tissue, bladder, head and neck, colon, stomach, testes, oesophagus, uterus, cervix and vulva, colorectal, bronchi, kidneys, basal cell carcinoma, squamous cell carcinoma of both ulcerating and papillary type, metastatic skin carcinoma, osteo sarcoma, Ewing's sarcoma, veticulum cell sarcoma, myeloma, giant cell tumor, gallstones, islet cell tumor, primary brain tumor, acute and chronic lymphocytic and granulocytic tumors, hairy-cell tumor, adenoma, hyperplasia, medullary carcinoma, pheochromocytoma, mucosal neuronms, intestinal ganglioneuromas, hyperplastic corneal nerve tumor, marfanoid habitus tumor, Wilm's tumor, seminoma, ovarian tumor, leiomyomater tumor, cervical dysplasia and in situ carcinoma, neuroblastoma, retinoblastoma, soft tissue sarcoma, malignant carcinoid, topical skin lesion, gastrointestinal cancer, hematological cancers, myelodysplastic syndrome, mycosis fungoide, rhabdomyosarcoma, astrocytoma, non-Hodgkin's lymphoma, Kaposi's sarcoma, osteogenic and other sarcoma, malignant hypercalcemia, renal cell tumor, polycythermia vera, adenocarcinoma, glioblastoma multiforma, glioma, leukemia (including acute myelogenous leukemia, chronic myelogenous leukemia (CML), acute lymphocytic leukemia (ALL), chronic lymphocytic leukemia), lymphomas, malignant melanomas, epidermoid carcinomas, and other carcinomas and sarcomas.

More particularly, PI3K and/or mTOR inhibitors of the invention may be used for the treatment of cancer of the breast, colorectum, lung (including small cell lung cancer, non-small cell lung cancer and bronchioalveolar cancer) and prostate, and of cancer of the bile duct, bone, bladder, head and neck, kidney, liver, gastrointestinal tissue, oesophagus, ovary, pancreas, skin, testes, thyroid, uterus, cervix and vulva, and of leukaemias (including ALL and CML), multiple myeloma and lymphomas.

The PI3K and/or mTOR inhibitors of the present invention may also be used to treat abnormal cell proliferation due to insults to body tissue during surgery. These insults may arise as a result of a variety of surgical procedures such as joint surgery, bowel surgery, and cheloid scarring. Diseases that produce fibrotic tissue include emphysema. Repetitive motion disorders that may be treated using the present invention include carpal tunnel syndrome.

mTOR inhibitors of the invention may also be useful in the prevention of restenosis that is the control of undesired proliferation of normal cells in the vasculature in response to the introduction of stents in the treatment of vasculature disease (Morice et al, New England Journal of Medicine, 2002, 346, 1773-1780).

Proliferative responses associated with organ transplantation that may be treated using PI3K and/or mTOR inhibitors of the invention include proliferative responses contributing to potential organ rejections or associated complications. Specifically, these proliferative responses may occur during transplantation of the heart, lung, liver, kidney, and other body organs or organ systems.

**Diseases and conditions related to Angiogenesis**

Abnormal angiogenesis that may be treated using this invention include those abnormal angiogenesis accompanying rheumatoid arthritis, ischemic-reperfusion related brain edema and injury, conical ischemia, ovarian hyperplasia and hypervascularity, (polycystic ovary syndrom), endometriosis, psoriasis, diabetic retinopaphy, and other ocular angiogenic diseases such as retinopathy of prematurity (retrolental fibroplastic), macular degeneration, corneal graft rejection, neuroscular glaucoma and Oster Webber syndrome.

Examples of diseases associated with uncontrolled angiogenesis that may be treated according to the present invention include, but are not limited to retinal/choroidal neuvascularization and corneal neovascularization. Examples of retinal/choroidal neuvascularization include, but are not limited to, Bests diseases, myopia, optic pits, Stargarts diseases, Pagets disease, vein occlusion, artery occlusion, sickle cell anemia, sarcoid, syphilis, pseudoxanthoma elasticum carotid abostructive diseases, chronic uveitis/vitritis, mycobacterial infections, Lyme's disease, systemic lupus erythematosis, retinopathy of prematurity, Eales disease, diabetic retinopathy, macular degeneration, Bechets diseases, infections causing a retinitis or chroiditis, presumed ocular histoplasmosis, pars planitis, chronic retinal detachment, hyperviscosity syndromes, toxoplasmosis, trauma and post-laser complications, diseases associated with rubesis (neovascularization of the angle) and diseases caused by the abnormal proliferation of fibrovascular or fibrous tissue including all forms of proliferative vitreoretinopathy. Examples of corneal neuvascularization include, but are not limited to, epidemic keratoconjunctivitis, vitamin A deficiency, contact lens overwear, atopic keratitis, superior limbic keratitis, pterygium keratitis sicca, sjogrens, acne rosacea, phylectenulosis, diabetic retinopathy, retinopathy of prematurity, corneal graft rejection, Mooren ulcer, Terrien's marginal degeneration, marginal keratolysis, polyarteritis, Wegener sarcoidosis, scleritis, periphigoid radial keratotomy, neovascular glaucoma and retrolental fibroplasia, syphilis, Mycobacteria infections, lipid degeneration, chemical bums, bacterial ulcers, fungal ulcers, Herpes simplex infections, Herpes zoster infections, protozoan infections and Kaposi sarcoma.

**Neurodegenerative Diseases and Conditions**

Neurodegenerative diseases which may be treated or prevented by the compounds of this invention include, but are not limited to, Alzheimer's disease (Nakagawa et al. Nature 2000, 403, 98-103), Parkinson's disease (Imai et al. Gell 2001, 105, 891-902), amyotrophic lateral sclerosis (ALS), epilepsy, seizures, Huntington's disease, polyglutamine diseases (Nishitoh et al. Genes Dev. 2002, 16, 1345-1355), traumatic brain injury, ischemic and hemorrhaging stroke, cerebral ischemias or neurodegenerative disease, including apoptosis-driven neurodegenerative disease, caused by traumatic injury, acute hypoxia, ischemia or glutamate neurotoxicity.

**Inflammatory Diseases and Conditions**

Inflammatory diseases which may be treated or prevented by the PI3K/mTOR inhibitors of this invention include, but are not limited to, acute pancreatitis, chronic pancreatitis, asthma, allergies, chronic obstructive pulmonary disease, adult respiratory distress syndrome.

Chronic inflammatory diseases associated with uncontrolled angiogenesis may also be treated using PI3K/mTOR inhibitors of the present invention. Chronic inflammation depends on continuous formation of capillary sprouts to maintain an influx of inflammatory cells. The influx and presence of the inflammatory cells produce granulomas and

thus maintains the chronic inflammatory state. Inhibition of angiogenesis using a PI3K/mTOR inhibitor alone or in conjunction with other anti-inflammatory agents may prevent the formation of the granulosmas and thus alleviate the disease. Examples of Chronic inflammatory diseases include, but are not limited to, inflammatory bowel diseases such as Crohn's disease and ulcerative colitis, psoriasis, sarcoidois, and rheumatoid arthritis.

Inflammatory bowel diseases such as Crohn's disease and ulcerative colitis are characterized by chronic inflammation and angiogenesis at various sites in the gastrointestinal tract. For example, Crohn's disease occurs as a chronic transmural inflammatory disease that most commonly affects the distal ileum and colon but may also occur in any part of the gastrointestinal tract from the mouth to the anus and perianal area. Patients with Crohn's disease generally have chronic diarrhea associated with abdominal pain, fever, anorexia, weight loss and abdominal swelling. Ulcerative colitis is also a chronic, nonspecific, inflammatory and ulcerative disease arising in the colonic mucosa and is characterized by the presence of bloody diarrhea. These inflammatory bowel diseases are generally caused by chronic granulomatous inflammation throughout the gastrointestinal tract, involving new capillary sprouts surrounded by a cylinder of inflammatory cells. Inhibition of angiogenesis by these inhibitors should inhibit the formation of the sprouts and prevent the formation of granulomas. Inflammatory bowel diseases also exhibit extra intestinal manifestations, such as skin lesions. Such lesions are characterized by inflammation and angiogenesis and can occur at many sites other the gastrointestinal tract. Inhibition of angiogenesis by PI3K/mTOR inhibitors according to the present invention can reduce the influx of inflammatory cells and prevent lesion formation.

Sarcoidosis, another chronic inflammatory disease, is characterized as a multisystem granulomatous disorder. The granulomas of this disease can form anywhere in the body. Thus, the symptoms depend on the site of the granulomas and whether the disease is active. The granulomas are created by the angiogenic capillary sprouts providing a constant supply of inflammatory cells. By using PI3K/mTOR inhibitors according to the present invention to inhibit angiogenesis, such granulomas formation can be inhibited. Psoriasis, also a chronic and recurrent inflammatory disease, is characterized by papules and plaques of various sizes. Treatment using these inhibitors alone or in conjunction with other anti-inflammatory agents should prevent the formation of new blood vessels necessary to maintain the characteristic lesions and provide the patient relief from the symptoms.

Rheumatoid arthritis (RA) is also a chronic inflammatory disease characterized by non-specific inflammation of the peripheral joints. It is believed that the blood vessels in the synovial lining of the joints undergo angiogenesis. In addition to forming new vascular networks, the endothelial cells release factors and reactive oxygen species that lead to pannus growth and cartilage destruction. The factors involved in angiogenesis may actively contribute to, and help maintain, the chronically inflamed state of rheumatoid arthritis. Treatment using PI3K/mTOR inhibitors according to the present invention alone or in conjunction with other anti-RA agents may prevent the formation of new blood vessels necessary to maintain the chronic inflammation and provide the RA patient relief from the symptoms.

### Autoimmune Diseases and Conditions

Autoimmune diseases which may be treated or prevented by the compounds of this invention include, but are not limited to, glomerulonephritis, rheumatoid arthritis, systemic lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, graft vs. host disease, multiple sclerosis, or Sjoegren's syndrome.

### Infectious Diseases and Conditions

Infectious diseases which may be treated or prevented by the compounds of this invention include, but are not limited to, sepsis, septic shock, and Shigellosis.

Diseases and conditions that are mediated by inducible pro-inflammatory proteins which may be treated or prevented by the compounds of this invention include, but are not limited to, edema, analgesia, fever and pain, such as neuromuscular pain, headache, cancer pain, dental pain and arthritis pain.

### Cardiovascular Diseases and Conditions

Elevated mTOR kinase activity has been associated with cardiac hypertrophy, which is of clinical importance as a major risk factor for heart failure and is a consequence of increased cellular size of cardiomyocytes (Tee & Blenis, Seminars in Cell and Developmental Biology, 2005, 16, 29-37). Inhibitors of the invention may be used to reduce the severity and incidence of cardiac allograft vasculopathy. Other conditions that are mediated by PI3KlmTOR and may be treated or prevented by the compounds of this invention include, but are not limited to, ischemia/reperfusion in stroke, heart attacks, myocardial ischemia, organ hypoxia, vascular hyperplasia, cardiac hypertrophy, hepatic ischemia, liver disease, congestive heart failure, pathologic immune responses such as that caused by T cell activation, and thrombin-induced platelet aggregation.

## Combination Therapy

A wide variety of therapeutic agents may have a therapeutic additive or synergistic effect with PI3K/mTOR inhibitors according to the present invention. Combination therapies that comprise one or more compounds of the present invention with one or more other therapeutic agents can be used, for example, to: 1) enhance the therapeutic effect(s) of the one or more compounds of the present invention and/or the one or more other therapeutic agents; 2) reduce the side effects exhibited by the one or more compounds of the present invention and/or the one or more other therapeutic agents; and/or 3) reduce the effective dose of the one or more compounds of the present invention and/or the one or more other therapeutic agents. It is noted that combination therapy is intended to cover when agents are administered before or after each other (sequential therapy) as well as when the agents are administered at the same time.

Examples of such therapeutic agents that may be used in combination with PI3K/mTOR inhibitors include, but are not limited to, anti-cell proliferation agents, anticancer agents, alkylating agents, antibiotic agents, antimetabolic agents, hormonal agents, plant-derived agents, and biologic agents.

Anti-cell proliferation agents are those which inhibit undesirable and uncontrolled cell proliferation. Examples of anti-cell proliferation agents that may be used in conjunction with the PI3K/mTOR inhibitors of the present invention include, but are not limited to, retinoid acid and derivatives thereof, 2-methoxyestradiol, ANGIOSTATIN™ protein, ENDOSTATIN™ protein, suramin, squalamine, tissue inhibitor of metalloproteinase-I, tissue inhibitor of metalloproteinase-2, plasminogen activator inhibitor-1, plasminogen activator inhibitor-2, cartilage-derived inhibitor, paclitaxel, platelet factor 4, protamine sulphate (clupeine), sulphated chitin derivatives (prepared from queen crab shells), sulphated polysaccharide peptidoglycan complex (sp-pg), staurosporine, modulators of matrix metabolism, including for example, proline analogs ((1-azetidine-2-carboxylic acid (LACA), cishydroxyproline, d,1-3,4-dehydroproline, thiaproline, beta-aminopropionitrile fumarate, 4-propyl-5-(4-pyridinyl)-2(3H)-oxazolone, methotrexate, mitoxantrone, heparin, interferons, 2 macroglobulin-serum, chimp-3, chymostatin, beta.-cyclodextrin tetradecasulfate, eponemycin; fumagillin, gold sodium thiomalate, d-penicillamine (CDPT), beta-1-anticollagenase-serum, alpha-2-antiplasmin, bisantrene, lobenzarit disodium, *n*-(2-carboxyphenyl-4-chloroanthronilic acid disodium or "CCA", thalidomide, angostatic steroid, cargboxynaminolmidazole, metalloproteinase inhibitors such as BB94. Other anti-angiogenesis agents that may be used include antibodies, preferably monoclonal antibodies against these angiogenic growth factors: bFGF, aFGF, FGF-5, VEGF isoforms, VEGF-C, HGF/SF and Ang-1/Ang-2. Ferrara N. and Alitalo, K. "Clinical application of angiogenic growth factors and their inhibitors" (1999) Nature Medicine 5:1359-1364.

Alkylating agents are polyfunctional compounds that have the ability to substitute alkyl groups for hydrogen ions. Examples of alkylating agents include, but are not limited to, bischloroethylamines (nitrogen mustards, e.g. chlorambucil, cyclophosphamide, ifosfamide, mechlorethamine, melphalan, uracil mustard), aziridines (e.g. thiotepa), alkyl alkone sulfonates (e.g. busulfan), nitrosoureas (e.g. carmustine, lomustine, streptozocin), nonclassic alkylating agents (altretamine, dacarbazine, and procarbazine), platinum compounds (carboplastin and cisplatin). These compounds react with phosphate, amino, hydroxyl, sulfihydryl, carboxyl, and imidazole groups. Under physiological conditions, these drugs ionize and produce positively charged ion that attach to susceptible nucleic acids and proteins, leading to cell cycle arrest and/or cell death. Combination therapy including a PI3K/mTOR inhibitor and an alkylating agent may have therapeutic synergistic effects on cancer and reduce sides affects associated with these chemotherapeutic agents.

Antibiotic agents are a group of drugs that produced in a manner similar to antibiotics as a modification of natural products. Examples of antibiotic agents include, but are not limited to, anthracyclines (e.g. doxorubicin, daunorubicin, epirubicin, idarubicin and anthracenedione), mitomycin C, bleomycin, dactinomycin, plicatomycin. These antibiotic agents interfere with cell growth by targeting different cellular components. For example, anthracyclines are generally believed to interfere with the action of DNA topoisomerase II in the regions of transcriptionally active DNA, which leads to DNA strand scissions. Bleomycin is generally believed to chelate iron and forms an activated complex, which then binds to bases of DNA, causing strand scissions and cell death. Combination therapy including a PI3K/mTOR Inhibitor and an antibiotic agent may have therapeutic synergistic effects on cancer and reduce sides affects associated with these chemotherapeutic agents.

Antimetabolic agents are a group of drugs that interfere with metabolic processes vital to the physiology and proliferation of cancer cells. Actively proliferating cancer cells require continuous synthesis of large quantities of nucleic acids, proteins, lipids, and other vital cellular constituents. Many of the antimetabolites inhibit the synthesis of purine or pyrimidine nucleosides or inhibit the enzymes of DNA replication. Some antimetabolites also interfere with the synthesis of ribonucleosides and RNA and/or amino acid metabolism and protein synthesis as well. By interfering with the synthesis of vital cellular constituents, antimetabolites can delay or arrest the growth of cancer cells. Examples of antimetabolic agents include, but are not limited to, fluorouracil (5-FU), floxuridine (5-FUdR), methotrexate, leucovorin, hydroxyurea, thioguanine (6-TG), mercaptopurine (6-MP), cytarabine, pentostatin, fludarabine phosphate, cladribine (2-CDA), asparaginase, and gemcitabine. Combination therapy including a PI3K/mTOR inhibitor and an antimetabolic agent may have

therapeutic synergistic effects on cancer and reduce sides affects associated with these chemotherapeutic agents.

Hormonal agents are a group of drug that regulate the growth and development of their target organs. Most of the hormonal agents are sex steroids and their derivatives and analogs thereof, such as estrogens, androgens, and progestins. These hormonal agents may serve as antagonists of receptors for the sex steroids to down regulate receptor expression and transcription of vital genes. Examples of such hormonal agents are synthetic estrogens (*e.g.* diethylstibestrol), antiestrogens (*e.g.* tamoxifen, toremifene, fluoxymesterol and raloxifene), antiandrogens (bicalutamide, nilutamide, flutamide), aromatase inhibitors (e.g., aminoglutethimide, anastrozole and tetrazole), ketoconazole, goserelin acetate, leuprolide, megestrol acetate and mifepristone. Combination therapy including a PI3K/mTOR inhibitor and a hormonal agent may have therapeutic synergistic effects on cancer and reduce sides affects associated with these chemotherapeutic agents.

Plant-derived agents are a group of drugs that are derived from plants or modified based on the molecular structure of the agents. Examples of plant-derived agents include, but are not limited to, vinca alkaloids (*e.g.*, vincristine, vinblastine, vindesine, vinzolidine and vinorelbine), podophyllotoxins (*e.g.*, etoposide (VP-16) and teniposide (VM-26)), taxanes (e.g., paclitaxel and docetaxel). These plant-derived agents generally act as antimitotic agents that bind to tubulin and inhibit mitosis. Podophyllotoxins such as etoposide are believed to interfere with DNA synthesis by interacting with topoisomerase II, leading to DNA strand scission. Combination therapy including a PI3K/mTOR inhibitor and a plant-derived agent may have therapeutic synergistic effects on cancer and reduce sides affects associated with these chemotherapeutic agents.

Biologic agents are a group of biomolecules that elicit cancer/tumor regression when used alone or in combination with chemotherapy and/or radiotherapy. Examples of biologic agents include, but are not limited to, immunomodulating proteins such as cytokines, monoclonal antibodies against tumor antigens, tumor suppressor genes, and cancer vaccines. Combination therapy including a PI3K/mTOR inhibitor and a biologic agent may have therapeutic synergistic effects on cancer, enhance the patient's immune responses to tumorigenic signals, and reduce potential sides affects associated with this chemotherapeutic agent.

Cytokines possess profound immunomodulatory activity. Some cytokines such as interleukin-2 (IL-2, aldesleukin) and interferon have demonstrated antitumor activity and have been approved for the treatment of patients with metastatic renal cell carcinoma and metastatic malignant melanoma. IL-2 is a T-cell growth factor that is central to T-cell-mediated immune responses. The selective antitumor effects of IL-2 on some patients are believed to be the result of a cell-mediated immune response that discriminate between self and nonself. Examples of interleukins that may be used in conjunction with PI3K/mTOR inhibitors include, but are not limited to, interleukin 2 (IL-2), and interleukin 4 (IL-4), interleukin 12 (IL-12).

Interferon include more than 23 related subtypes with overlapping activities, all of the IFN subtypes within the scope of the present invention. IFN has demonstrated activity against many solid and hematologic malignancies, the later appearing to be particularly sensitive.

Other cytokines that may be used in conjunction with a PI3K/mTOR inhibitor include those cytokines that exert profound effects on hematopoiesis and immune functions. Examples of such cytokines include, but are not limited to erythropoietin, granulocyte-CSF (filgrastin), and granulocyte, macrophage-CSF (sargramostim). These cytokines may be used in conjunction with a PI3K/mTOR inhibitor to reduce chemotherapy-induced myelopoietic toxicity.

Other immuno-modulating agents other than cytokines may also be used in conjunction with a PI3K/mTOR inhibitor to inhibit abnormal cell growth. Examples of such immuno-modulating agents include, but are not limited to bacillus Calmette-Guerin, levamisole, and octreotide, a long-acting octapeptide that mimics the effects of the naturally occurring hormone somatostatin.

Monoclonal antibodies against tumor antigens are antibodies elicited against antigens expressed by tumors, preferably tumor-specific antigens. For example, monoclonal antibody HERCEPTIN® (Trastruzumab) is raised against human epidermal growth factor receptor2 (HER2) that is overexpressed in some breast tumors including metastatic breast cancer. Overexpression of HER2 protein is associated with more aggressive disease and poorer prognosis in the clinic. HERCEPTIN® is used as a single agent for the treatment of patients with metastatic breast cancer whose tumors over express the HER2 protein. Combination therapy including PI3K/mTOR inhibitor and HERCEPTIN® may have therapeutic synergistic effects on tumors, especially on metastatic cancers.

Another example of monoclonal antibodies against tumor antigens is RITUXAN® (Rituximab) that is raised against CD20 on lymphoma cells and selectively deplete normal and malignant $CD20^+$ pre-B and mature B cells. RITUXAN® is used as single agent for the treatment of patients with relapsed or refractory low-grade or follicular, $CD20^+$, B cell non-Hodgkin's lymphoma. Combination therapy including a PI3K/mTOR inhibitor and RITUXAN® may have therapeutic synergistic effects not only on lymphoma, but also on other forms or types of malignant tumors.

Tumor suppressor genes are genes that function to inhibit the cell growth and division cycles, thus preventing the development of neoplasia. Mutations in tumor suppressor genes cause the cell to ignore one or more of the components of the network of inhibitory signals, overcoming the cell cycle check points and resulting in a higher rate of controlled cell growth-cancer. Examples of the tumor suppressor genes include, but are not limited to, *DPC-4, NF-1,*

*NF-2, RB, p53, WT1, BRCA1* and *BRCA2. DPC-4* is involved in pancreatic cancer and participates in a cytoplasmic pathway that inhibits cell division. *NF-1* codes for a protein that inhibits Ras, a cytoplasmic inhibitory protein. *NF-1* is involved in neuro fibroma and pheochromocytomas of the nervous system and myeloid leukemia. *NF-2* encodes a nuclear protein that is involved in meningioma, schwanoma, and ependymoma of the nervous system. *RB* codes for the pRB protein, a nuclear protein that is a major inhibitor of cell cycle. *RB* is involved in retinoblastoma as well as bone, bladder, small cell lung and breast cancer. *P53* codes for p53 protein that regulates cell division and can induce apoptosis. Mutation and/or inaction of p53 are found in wide ranges of cancers. *WT1* is involved in Wilms tumor of the kidneys. *BRCA1* is involved in breast and ovarian cancer, and *BRCA2* is involved in breast cancer. The tumor suppressor gene can be transferred into the tumor cells where it exerts its tumor suppressing functions. Combination therapy including a PI3K/mTOR inhibitor and a tumor suppressor may have therapeutic synergistic effects on patients suffering from various forms of cancers.

Cancer vaccines are a group of agents that induce the body's specific immune response to tumors. Most of cancer vaccines under research and development and clinical trials are tumor-associated antigens (TAAs). TAA are structures (i.e. proteins, enzymes or carbohydrates) which are present on tumor cells and relatively absent or diminished on normal cells. By virtue of being fairly unique to the tumor cell, TAAs provide targets for the immune system to recognize and cause their destruction. Example of TAAs include, but are not limited to gangliosides (GM2), prostate specific antigen (PSA), $\alpha$-fetoprotein (AFP), carcinoembryonic antigen (CEA) (produced by colon cancers and other adenocarcinomas, *e.g.* breast, lung, gastric, and pancreas cancers), melanoma associated antigens (MART-1, gp100, MAGE 1,3 tyrosinase), papillomavirus E6 and E7 fragments, whole cells or portions/lysates of antologous tumor cells and allogeneic tumor cells.

An adjuvant may be used to augment the immune response to TAAs. Examples of adjuvants include, but are not limited to, bacillus Calmette-Guerin (BCG), endotoxin lipopolysaccharides, keyhole limpet hemocyanin (GKLH), interleukin-2 (IL-2), granulocyte-macrophage colony-stimulating factor (GM-CSF) and cytoxan, a chemotherapeutic agent which is believe to reduce tumor-induced suppression when given in low doses.

## Compositions Comprising PI3K/mTOR Inhibitors

A wide variety of compositions and administration methods may be used in conjunction with the compounds of the present invention. Such compositions may include, in addition to the compounds of the present invention, conventional pharmaceutical excipients, and other conventional, pharmaceutically inactive agents. Additionally, the compositions may include active agents in addition to the compounds of the present invention. These additional active agents may include additional compounds according to the invention, and/or one or more other pharmaceutically active agents.

The compositions may be in gaseous, liquid, semi-liquid or solid form, formulated in a manner suitable for the route of administration to be used. For oral administration, capsules and tablets are typically used. For parenteral administration, reconstitution of a lyophilized powder, prepared as described herein, is typically used.

Compositions comprising compounds of the present invention may be administered or coadministered orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly,or intrathecally. The compounds and/or compositions according to the invention may also be administered or coadministered in slow release dosage forms.

The PI3K/mTOR inhibitors and compositions comprising them may be administered or coadministered in any conventional dosage form. Co-administration in the context of this invention is intended to mean the administration of more than one therapeutic agent, one of which includes a PI3K/mTOR inhibitor, in the course of a coordinated treatment to achieve an improved clinical outcome. Such co-administration may also be coextensive, that is, occurring during overlapping periods of time.

Solutions or suspensions used for parenteral, intradermal, subcutaneous, or topical application may optionally include one or more of the following components: a sterile diluent, such as water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; agents for the adjustment of tonicity such as sodium chloride or dextrose, and agents for adjusting the acidity or alkalinity of the composition, such as alkaline or acidifying agents or buffers like carbonates, bicarbonates, phosphates, hydrochloric acid, and organic acids like acetic and citric acid. Parenteral preparations may optionally be enclosed in ampules, disposable syringes or single or multiple dose vials made of glass, plastic or other suitable material.

When compounds according to the present invention exhibit insufficient solubility, methods for solubilizing the compounds may be used. Such methods are known to those of skill in this art, and include, but are not limited to, using cosolvents, such as dimethylsulfoxide (DMSO), using surfactants, such as TWEEN, or dissolution in aqueous sodium bicarbonate. Derivatives of the compounds, such as prodrugs of the compounds may also be used in formulating effective

pharmaceutical compositions.

Upon mixing or adding compounds according to the present invention to a composition, a solution, suspension, emulsion or the like may be formed. The form of the resulting composition will depend upon a number of factors, including the intended mode of administration, and the solubility of the compound in the selected carrier or vehicle. The effective concentration needed to ameliorate the disease being treated may be empirically determined.

Compositions according to the present invention are optionally provided for administration to humans and animals in unit dosage forms, such as tablets, capsules, pills, powders, dry powders for inhalers, granules, sterile parenteral solutions or suspensions, and oral solutions or suspensions, and oil-water emulsions containing suitable quantities of the compounds, particularly the pharmaceutically acceptable salts, preferably the sodium salts, thereof. The pharmaceutically therapeutically active compounds and derivatives thereof are typically formulated and administered in unit-dosage forms or multiple-dosage forms. Unit-dose forms, as used herein, refers to physically discrete units suitable for human and animal subjects and packaged individually as is known in the art. Each unit-dose contains a predetermined quantity of the therapeutically active compound sufficient to produce the desired therapeutic effect, in association with the required pharmaceutical carrier, vehicle or diluent. Examples of unit-dose forms include ampoules and syringes individually packaged tablet or capsule. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pint or gallons. Hence, multiple dose form is a multiple of unit-doses that are not segregated in packaging.

In addition to one or more compounds according to the present invention, the composition may comprise: a diluent such as lactose, sucrose, dicalcium phosphate, or carboxymethylcellulose; a lubricant, such as magnesium stearate, calcium stearate and talc; and a binder such as starch, natural gums, such as gum acaciagelatin, glucose, molasses, polyvinylpyrrolidine, celluloses and derivatives thereof, povidone, crospovidones and other such binders known to those of shill in the art. Liquid pharmaceutically administrable compositions can, for example, be prepared by dissolving, dispersing, or otherwise mixing an active compound as defined above and optional pharmaceutical adjuvants in a carrier, such as, for example, water, saline, aqueous dextrose, glycerol, glycols, ethanol, and the like, to form a solution or suspension. If desired, the pharmaceutical composition to be administered may also contain minor amounts of auxiliary substances such as wetting agents, emulsifying agents, or solubilizing agents, pH buffering agents and the like, for example, acetate, sodium citrate, cyclodextrine derivatives, sorbitan monolaurate, triethanolamine sodium acetate, triethanolamine oleate, and other such agents. Actual methods of preparing such dosage forms are known in the art, or will be apparent, to those skilled in this art; for example, see Remington: The Science and Practices of Pharmacy, Lippincott Williams, and Wilkins Publisher, 21st edition, 2005. The composition or formulation to be administered will, in any event, contain a sufficient quantity of an inhibitor of the present invention to reduce PI3K/mTOR activity in vivo, thereby treating the disease state of the subject.

Dosage forms or compositions may optionally comprise one or more compounds according to the present invention in the range of 0.005% to 100% (weight/weight) with the balance comprising additional substances such as those described herein. For oral administration, a pharmaceutically acceptable composition may optionally comprise any one or more commonly employed excipients, such as, for example pharmaceutical grades of mannitol, lactose, starch, magnesium stearate, talcum, cellulose derivatives, sodium crosscarmellose, glucose, sucrose, magnesium carbonate, sodium saccharin, talcum. Such compositions include solutions, suspensions, tablets, capsules, powders, dry powders for inhalers and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparing these formulations are known to those skilled in the art. The compositions may optionally contain 0.01%-100% (weight/weight) of one or more PI3K/mTOR inhibitors, optionally 0.1-95%, and optionally 1-95%.

Salts, preferably sodium salts, of the inhibitors may be prepared with carriers that protect the compound against rapid elimination from the body, such as time release formulations or coatings. The formulations may further include other active compounds to obtain desired combinations of properties.

### A. Formulations for Oral Administration

Oral pharmaceutical dosage forms may be as a solid, gel or liquid. Examples of solid dosage forms include, but are not limited to tablets, capsules, granules, and bulk powders. More specific examples of oral tablets include compressed, chewable lozenges and tablets that may be enteric-coated, sugar-coated or film-coated. Examples of capsules include hard or soft gelatin capsules. Granules and powders may be provided in non-effervescent or effervescent forms. Each may be combined with other ingredients known to those skilled in the art.

In certain embodiments, compounds according to the present invention are provided as solid dosage forms, preferably capsules or tablets. The tablets, pills, capsules, troches and the like may optionally contain one or more of the following ingredients, or compounds of a similar nature: a binder; a diluent; a disintegrating agent; a lubricant; a

glidant; a sweetening agent; and a flavoring agent.

Examples of binders that may be used include, but are not limited to, microcrystalline cellulose, gum tragacanth, glucose solution, acacia mucilage, gelatin solution, sucrose and starch paste.

Examples of lubricants that may be used include, but are not limited to, talc, starch, magnesium or calcium stearate, lycopodium and stearic acid.

Examples of diluents that may be used include, but are not limited to, lactose, sucrose, starch, kaolin, salt, mannitol and dicalcium phosphate.

Examples of glidants that may be used include, but are not limited to, colloidal silicon dioxide.

Examples of disintegrating agents that may be used include, but are not limited to, crosscarmellose sodium, sodium starch glycolate, alginic acid, corn starch, potato starch, bentonite, methylcellulose, agar and carboxymethylcellulose.

Examples of coloring agents that may be used include, but are not limited to, any of the approved certified water-soluble FD and C dyes, mixtures thereof; and water insoluble FD and C dyes suspended on alumina hydrate.

Examples of sweetening agents that may be used include, but are not limited to, sucrose, lactose, mannitol and artificial sweetening agents such as sodium cyclamate and saccharin, and any number of spray-dried flavors.

Examples of flavoring agents that may be used include, but are not limited to, natural flavors extracted from plants such as fruits and synthetic blends of compounds that produce a pleasant sensation, such as, but not limited to peppermint and methyl salicylate.

Examples of wetting agents that may be used include, but are not limited to, propylene glycol monostearate, sorbitan monooleate, diethylene glycol monolaurate and polyoxyethylene lauryl ether.

Examples of anti-emetic coatings that may be used include, but are not limited to, fatty acids, fats, waxes, shellac, ammoniated shellac and cellulose acetate phthalates.

Examples of film coatings that may be used include, but are not limited to, hydroxyethylcellulose, sodium carboxymethylcellulose, polyethylene glycol 4000 and cellulose acetate phthalate.

If oral administration is desired, the salt of the compound may optionally be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

When the dosage unit form is a capsule, it may optionally additionally comprise a liquid carrier such as a fatty oil. In addition, dosage unit forms may optionally additionally comprise various other materials that modify the physical form of the dosage unit, for example, coatings of sugar and other enteric agents.

Compounds according to the present invention may also be administered as a component of an elixir, suspension, syrup, wafer, sprinkle, chewing gum or the like. A syrup may optionally comprise, in addition to the active compounds, sucrose as a sweetening agent and certain preservatives, dyes and colorings and flavors.

The compounds of the present invention may also be mixed with other active materials that do not impair the desired action, or with materials that supplement the desired action, such as antacids, H2 blockers, and diuretics. For example, if a compound is used for treating asthma or hypertension, it may be used with other bronchodilators and antihypertensive agents, respectively.

Examples of phamaceutically acceptable carriers that may be included in tablets comprising compounds of the present invention include, but are not limited to binders, lubricants, diluents, disintegrating agents, coloring agents, flavoring agents, and wetting agents. Enteric-coated tablets, because of the enteric-coating, resist the action of stomach acid and dissolve or disintegrate in the neutral or alkaline intestines. Sugar-coated tablets may be compressed tablets to which different layers of pharmaceutically acceptable substances are applied. Film-coated tablets may be compressed tablets that have been coated with polymers or other suitable coating. Multiple compressed tablets may be compressed tablets made by more than one compression cycle utilizing the pharmaceutically acceptable substances previously mentioned. Coloring agents may also be used in tablets. Flavoring and sweetening agents may be used in tablets, and are especially useful in the formation of chewable tablets and lozenges.

Examples of liquid oral dosage forms that may be used include, but are not limited to, aqueous solutions, emulsions, suspensions, solutions and/or suspensions reconstituted from non-effervescent granules and effervescent preparations reconstituted from effervescent granules.

Examples of aqueous solutions that may be used include, but are not limited to, elixirs and syrups. As used herein, elixirs refer to clear, sweetened, hydroalcoholic preparations. Examples of pharmaceutically acceptable carriers that may be used in elixirs include, but are not limited to solvents. Particular examples of solvents that may be used include glycerin, sorbitol, ethyl alcohol and syrup. As used herein, syrups refer to concentrated aqueous solutions of a sugar, for example, sucrose. Syrups may optionally further comprise a preservative.

Emulsions refer to two-phase systems in which one liquid is dispersed in the form of small globules throughout another liquid. Emulsions may optionally be oil-in-water or water-in-oil emulsions. Examples of pharmaceutically acceptable carriers that may be used in emulsions include, but are not limited to non-aqueous liquids, emulsifying agents

and preservatives.

Examples of pharmaceutically acceptable substances that may be used in non-effervescent granules, to be reconstituted into a liquid oral dosage form, include diluents, sweeteners and wetting agents.

Examples of pharmaceutically acceptable substances that may be used in effervescent granules, to be reconstituted into a liquid oral dosage form, include organic acids and a source of carbon dioxide.

Coloring and flavoring agents may optionally be used in all of the above dosage forms.

Particular examples of preservatives that may be used include glycerin, methyl and propylparaben, benzoic add, sodium benzoate and alcohol.

Particular examples of non-aqueous liquids that may be used in emulsions include mineral oil and cottonseed oil.

Particular examples of emulsifying agents that may be used include gelatin, acacia, tragacanth, bentonite, and surfactants such as polyoxyethylene sorbitan monooleate.

Particular examples of suspending agents that may be used include sodium carboxymethylcellulose, pectin, tragacanth, Veegum and acacia. Diluents include lactose and sucrose. Sweetening agents include sucrose, syrups, glycerin and artificial sweetening agents such as sodium cyclamate and saccharin.

Particular examples of wetting agents that may be used include propylene glycol monostearate, sorbitan monooleate, diethylene glycol monolaurate and polyoxyethylene lauryl ether.

Particular examples of organic acids that may be used include citric and tartaric acid.

Sources of carbon dioxide that may be used in effervescent compositions include sodium bicarbonate and sodium carbonate. Coloring agents include any of the approved certified water soluble FD and C dyes, and mixtures thereof.

Particular examples of flavoring agents that may be used include natural flavors extracted from plants such fruits, and synthetic blends of compounds that produce a pleasant taste sensation.

For a solid dosage form, the solution or suspension, in for example propylene carbonate, vegetable oils or triglycerides, is preferably encapsulated in a gelatin capsule. Such solutions, and the preparation and encapsulation thereof, are disclosed in U.S. Pat. Nos. 4,328,245; 4,409,239; and 4,410,545. For a liquid dosage form, the solution, *e.g.*, for example, in a polyethylene glycol, may be diluted with a sufficient quantity of a pharmaceutically acceptable liquid carrier, *e.g.*, water, to be easily measured for administration.

Alternatively, liquid or semi-solid oral formulations may be prepared by dissolving or dispersing the active compound or salt in vegetable oils, glycols, triglycerides, propylene glycol esters (*e.g.*, propylene carbonate) and other such carriers, and encapsulating these solutions or suspensions in hard or soft gelatin capsule shells. Other useful formulations include those set forth in U.S. Pat. Nos. Re 28,819 and 4,358,603.

**B. Injectables, Solutions, and Emulsions**

The present invention is also directed to compositions designed to administer the compounds of the present invention by parenteral administration, generally characterized by subcutaneous, intramuscular or intravenous injection. Injectables may be prepared in any conventional form, for example as liquid solutions or suspensions, solid forms suitable for solution or suspension in liquid prior to injection, or as emulsions.

Examples of excipients that may be used in conjunction with injectables according to the present invention include, but are not limited to water, saline, dextrose, glycerol or ethanol. The injectable compositions may also optionally comprise minor amounts of non-toxic auxiliary substances such as wetting or emulsifying agents, pH buffering agents, stabilizers, solubility enhancers, and other such agents, such as for example, sodium acetate, sorbitan monolaurate, triethanolamine oleate and cyclodextrins. Implantation of a slow-release or sustained-release system, such that a constant level of dosage is maintained (see, *e.g.*, U.S. Pat. No. 3,710,795) is also contemplated herein. The percentage of active compound contained in such parenteral compositions is highly dependent on the specific nature thereof, as well as the activity of the compound and the needs of the subject.

Parenteral administration of the formulations includes intravenous, subcutaneous and intramuscular administrations. Preparations for parenteral administration include sterile solutions ready for injection, sterile dry soluble products, such as the lyophilized powders described herein, ready to be combined with a solvent just prior to use, including hypodermic tablets, sterile suspensions ready for injection, sterile dry insoluble products ready to be combined with a vehicle just prior to use and sterile emulsions. The solutions may be either aqueous or nonaqueous.

When administered intravenously, examples of suitable carriers include, but are not limited to physiological saline or phosphate buffered saline (PBS), and solutions containing thickening and solubilizing agents, such as glucose, polyethylene glycol, and polypropylene glycol and mixtures thereof.

Examples of pharmaceutically acceptable carriers that may optionally be used in parenteral preparations include, but are not limited to aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

Examples of aqueous vehicles that may optionally be used include Sodium Chloride Injection, Ringers Injection, Isotonic Dextrose Injection, Sterile Water Injection, Dextrose and Lactated Ringers Injection.

Examples of nonaqueous parenteral vehicles that may optionally be used include fixed oils of vegetable origin, cottonseed oil, corn oil, sesame oil and peanut oil.

Antimicrobial agents in bacteriostatic or fungistatic concentrations may be added to parenteral preparations, particularly when the preparations are packaged in multiple-dose containers and thus designed to be stored and multiple aliquots to be removed. Examples of antimicrobial agents that may be used include phenols or cresols, mercurials, benzyl alcohol, chlorobutanol, methyl and propyl *p*-hydroxybenzoic acid esters, thimerosal, benzalkonium chloride and benzethonium chloride.

Examples of isotonic agents that may be used include sodium chloride and dextrose. Examples of buffers that may be used include phosphate and citrate. Examples of antioxidants that may be used include sodium bisulfate. Examples of local anesthetics that may be used include procaine hydrochloride. Examples of suspending and dispersing agents that may be used include sodium carboxymethylcellulose, hydroxypropyl methylcellulose and polyvinylpyrrolidone. Examples of emulsifying agents that may be used include Polysorbate 80 (TWEEN 80). A sequestering or chelating agent of metal ions includes EDTA.

Pharmaceutical carriers may also optionally include ethyl alcohol, polyethylene glycol and propylene glycol for water miscible vehicles and sodium hydroxide, hydrochloric acid, citric acid or lactic acid for pH adjustment.

The concentration of an inhibitor in the parenteral formulation may be adjusted so that an injection administers a pharmaceutically effective amount sufficient to produce the desired pharmacological effect. The exact concentration of an inhibitor and/or dosage to be used will ultimately depend on the age, weight and condition of the patient or animal as is known in the art.

Unit-dose parenteral preparations may be packaged in an ampoule, a vial or a syringe with a needle. All preparations for parenteral administration should be sterile, as is known and practiced in the art.

Injectables may be designed for local and systemic administration. Typically a therapeutically effective dosage is formulated to contain a concentration of at least about 0.1% w/w up to about 90% w/w or more, preferably more than 1% w/w of the PI3K/mTOR inhibitor to the treated tissue(s). The inhibitor may be administered at once, or may be divided into a number of smaller doses to be administered at intervals of time. It is understood that the precise dosage and duration of treatment will be a function of the location of where the composition is parenterally administered, the carrier and other variables that may be determined empirically using known testing protocols or by extrapolation from *in vivo* or *in vitro* test data. It is to be noted that concentrations and dosage values may also vary with the age of the individual treated. It is to be further understood that for any particular subject, specific dosage regimens may need to be adjusted over time according to the individual need and the professional judgment of the person administering or supervising the administration of the formulations. Hence, the concentration ranges set forth herein are intended to be exemplary and are not intended to limit the scope or practice of the claimed formulations.

The PI3K/mTOR inhibitor may optionally be suspended in micronized or other suitable form or may be derivatized to produce a more soluble active product or to produce a prodrug. The form of the resulting mixture depends upon a number of factors, including the intended mode of administration and the solubility of the compound in the selected carrier or vehicle. The effective concentration is sufficient for ameliorating the symptoms of the disease state and may be empirically determined.

### C. Lyophilized Powders

The compounds of the present invention may also be prepared as lyophilized powders, which can be reconstituted for administration as solutions, emulsions and other mixtures. The lyophilized powders may also be formulated as solids or gels.

Sterile, lyophilized powder may be prepared by dissolving the compound in a sodium phosphate buffer solution containing dextrose or other suitable excipient. Subsequent sterile filtration of the solution followed by lyophilization under standard conditions know to those of skill in the art provides the desired formulation. Briefly, the lyophilized powder may optionally be prepared by dissolving dextrose, sorbitol, fructose, corn syrup, xylitol, glycerin, glucose, sucrose or other suitable agent, about 1-20%, preferably about 5 to 15%, in a suitable buffer, such as citrate, sodium or potassium phosphate or other such buffer known to those of skill in the art at, typically, about neutral pH. Then, a PI3K/mTOR inhibitor is added to the resulting mixture, preferably above room temperature, more preferably at about 30-35 °C, and stirred until it dissolves. The resulting mixture is diluted by adding more buffer to a desired concentration. The resulting mixture is sterile filtered or treated to remove particulates and to insure sterility, and apportioned into vials for lyophilization. Each vial may contain a single dosage or multiple dosages of the inhibitor.

### D. Formulation for Topical Administration

The compounds of the present invention may also be administered as topical mixtures. Topical mixtures may be used for local and systemic administration. The resulting mixture may be a solution, suspension, emulsions or the

like and are formulated as creams, gels, ointments, emulsions, solutions, elixirs, lotions, suspensions, tinctures, pastes, foams, aerosols, irrigations, sprays, suppositories, bandages, dermal patches or any other formulations suitable for topical administration.

The PI3K/mTOR inhibitors may be formulated as aerosols for topical application, such as by inhalation (see, U.S. Pat. Nos. 4,044,126, 4,414,209, and 4,364,923, which describe aerosols for delivery of a steroid useful for treatment of inflammatory diseases, particularly asthma). These formulations for administration to the respiratory tract can be in the form of an aerosol or solution for a nebulizer, or as a microfine powder for insufflation, alone or in combination with an inert carrier such as lactose. In such a case, the particles of the formulation will typically have diameters of less than 50 microns, preferably less than 10 microns.

The inhibitors may also be formulated for local or topical application, such as for topical application to the skin and mucous membranes, such as in the eye, in the form of gels, creams, and lotions and for application to the eye or for intracisternal or intraspinal application. Topical administration is contemplated for transdermal delivery and also for administration to the eyes or mucosa, or for inhalation therapies. Nasal solutions of the PI3K/mTOR inhibitor alone or in combination with other pharmaceutically acceptable excipients can also be administered.

### E. Formulations for Other Routes of Administration

Depending upon the disease state being treated, other routes of administration, such as topical application, transdermal patches, and rectal administration, may also be used. For example, pharmaceutical dosage forms for rectal administration are rectal suppositories, capsules and tablets for systemic effect. Rectal suppositories are used herein mean solid bodies for insertion into the rectum that melt or soften at body temperature releasing one or more pharmacologically or therapeutically active ingredients. Pharmaceutically acceptable substances utilized in rectal suppositories are bases or vehicles and agents to raise the melting point. Examples of bases include cocoa butter (theobroma oil), glycerin-gelatin, carbowax, (polyoxyethylene glycol) and appropriate mixtures of mono-, di- and triglycerides of fatty acids. Combinations of the various bases may be used. Agents to raise the melting point of suppositories include spermaceti and wax. Rectal suppositories may be prepared either by the compressed method or by molding. The typical weight of a rectal suppository is about 2 to 3 gm. Tablets and capsules for rectal administration may be manufactured using the same pharmaceutically acceptable substance and by the same methods as for formulations for oral administration.

### F. Examples of Formulations

The following are particular examples of oral, intravenous and tablet formulations that may optionally be used with compounds of the present invention. It is noted that these formulations may be varied depending on the particular compound being used and the indication for which the formulation is going to be used.

ORAL FORMULATION

| | |
|---|---|
| Compound of the Present Invention | 10-100 mg |
| Citric Acid Monohydrate | 105 mg |
| Sodium Hydroxide | 18 mg |
| Flavoring | |
| Water | q.s. to 100 mL |

INTRAVENOUS FORMULATION

| | |
|---|---|
| Compound of the Present Invention | 0.1-10 mg |
| Dextrose Monohydrate | q.s. to make isotonic |
| Citric Acid Monohydrate | 1.05 mg |
| Sodium Hydroxide | 0.18 mg |
| Water for Injection | q.s. to 1.0 mL |

TABLET FORMULATION

| | |
|---|---|
| Compound of the Present Invention | 1% |
| Microcrystalline Cellulose | 73% |

(continued)

| TABLET FORMULATION | |
|---|---|
| Stearic Acid | 25% |
| Colloidal Silica | 1%. |

## Dosage, Host and Safety

The compounds of the present invention are stable and can be used safely. In particular, the compounds of the present invention are useful as PI3K/mTOR inhibitors for a variety of subjects (*e.g.*, humans, non-human mammals and non-mammals).

The optimal dose may vary depending upon such conditions as, for example, the type of subject, the body weight of the subject, on the severity of the condition, the route of administration, and specific properties of the particular compound being used. Generally, acceptable and effective daily doses are amounts sufficient to effectively slow or eliminate the condition being treated. Typically, the daily dose for oral administration to an adult (body weight of about 60 kg) is about 1 to 1000 mg, about 3 to 300 mg, or about 10 to 200 mg. It will be appreciated that the daily dose can be given in a single administration or in multiple (*e.g.*, 2 or 3) portions a day.

## Kits and Articles of manufacture Comprising PI3K/mTOR Inhibitors

The invention is also directed to kits and other articles of manufacture for treating diseases associated with PI3K/mTOR. It is noted that diseases are intended to cover all conditions for which the PI3K/mTOR possess activity that contributes to the pathology and/or symptomology of the condition.

In one embodiment, a kit is provided that comprises a composition comprising at least one inhibitor of the present invention in combination with instructions. The instructions may indicate the disease state for which the composition is to be administered, storage information, dosing information and/or instructions regarding how to administer the composition. The kit may also comprise packaging materials. The packaging material may comprise a container for housing the composition. The kit may also optionally comprise additional components, such as syringes for administration of the composition. The kit may comprise the composition in single or multiple dose forms.

In another embodiment, an article of manufacture is provided that comprises a composition comprising at least one inhibitor of the present invention in combination with packaging materials. The packaging material may comprise a container for housing the composition. The container may optionally comprise a label indicating the disease state for which the composition is to be administered, storage information, dosing information and/or instructions regarding how to administer the composition. The kit may also optionally comprise additional components, such as syringes for administration of the composition. The kit may comprise the composition in single or multiple dose forms.

It is noted that the packaging material used in kits and articles of manufacture according to the present invention may form a plurality of divided containers such as a divided bottle or a divided foil packet. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container that is employed will depend on the exact dosage form involved, for example a conventional cardboard box would not generally be used to hold a liquid suspension. It is feasible that more than one container can be used together in a single package to market a single dosage form. For example, tablets may be contained in a bottle that is in turn contained within a box. Typically the kit includes directions for the administration of the separate components. The kit form is particularly advantageous when the separate components are preferably administered in different dosage forms (*e.g.*, oral, topical, transdermal and parenteral), are administered at different dosage intervals, or when titration of the individual components of the combination is desired by the prescribing physician.

One particular example of a kit according to the present invention is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the

recesses whereby an opening is formed in the sheet at the place of the recess. The tablet or capsule can then be removed via said opening.

Another specific embodiment of a kit is a dispenser designed to dispense the daily doses one at a time in the order of their intended use. Preferably, the dispenser is equipped with a memory-aid, so as to further facilitate compliance with the regimen. An example of such a memory-aid is a mechanical counter that indicates the number of daily doses that has been dispensed. Another example of such a memory-aid is a battery-powered microchip memory coupled with a liquid crystal readout, or audible reminder signal which, for example, reads out the date that the last daily dose has been taken and/or reminds one when the next dose is to be taken.

## Preparation of PI3/mTOR Inhibitors

Various methods may be developed for synthesizing compounds according to the present invention. Representative methods for synthesizing these compounds are provided in the Examples. It is noted, however, that the compounds of the present invention may also be synthesized by other synthetic routes that others may devise.

### Synthetic Schemes for Compounds of the Present Invention

Compounds according to the present invention may be synthesized according to the reaction schemes shown below. Other reaction schemes could be readily devised by those skilled in the art. It should also be appreciated that a variety of different solvents, temperatures and other reaction conditions can be varied to optimize the yields of the reactions.

In the reactions described hereinafter it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see P.G.M. Wuts and T.W. Greene in "Greene's Protective Groups in Organic Synthesis" 4th edition, John Wiley and Sons, 2007.

*Scheme A: General Synthetic Route I*

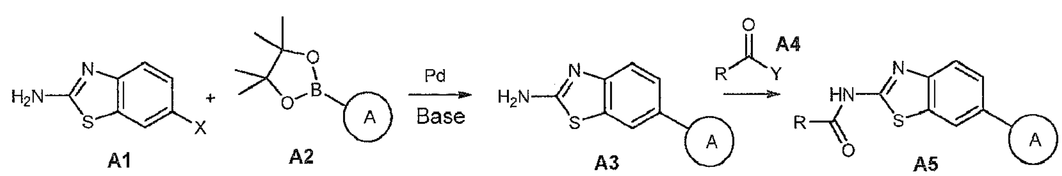


A1 A2 A3 A4 A5

where R is -$LR_1$, X is halo and Y is Cl or OH.

Compounds of the present invention having a benzothiazole core may be prepared as described in Scheme A above. Reacting commercially available 6-bromobenzo[d]thiazol-2-amine (**A1**) to a boronic acid or ester of ring A, e.g., **A2,** in the presence of palladium catalyst, (such as $PdCl_2$(dppf) and $Pd(PPh_3)_4$) in a base (such as $NaHCO_3$) and solvent (such as 1,4-dioxane) at elevated temperature would afford the desired intermediate **A3.** Acylation of **A3** with an acid chloride or an acid **A4** in the presence of a coupling reagent such as EDCI to yield product **A5.** Alternatively, for products with simple R groups, the acylation may be accomplished by reacting intermediate **A3** with an anhydride $(R(O))_2O$ in the presence of a base (such as pyridine) to yield product **A5.**

*Scheme B: General Synthetic Route II*

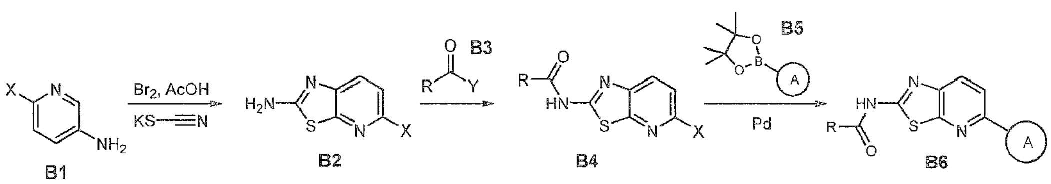


B1 B2 B3 B4 B5 B6

where R is -$LR_1$, X is halo and Y is Cl or OH.

A general synthetic route for producing compounds of the present invention comprising a thiazolopyridine is

shown in Scheme B. When commercially available 6-bromopyridin-3-amine **(B1)** is reacted with potassium thiocyanate and bromine in acetic acid, cyclized product **B2** would be obtained. Subsequent reaction of **B2** with an appropriate acid chloride or acid **B3** in a solvent such as DMA, or with an appropriate acid anhydride in the presence of a base such as pyridine would produce the desired intermediate **B4.** Palladium catalyzed coupling of a boronic acid or ester **B5** would afford the desired compound B6.

*Scheme C: General Synthetic Route III*

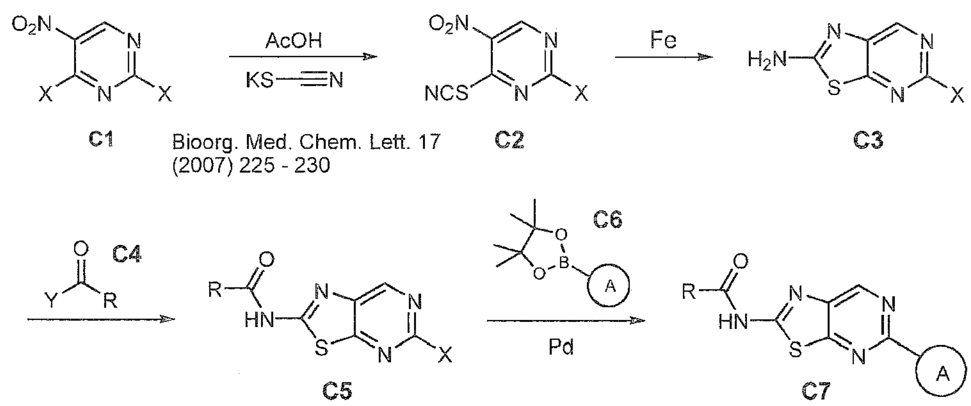


where R is $-LR_1$, X is halo and Y is Cl or OH.

Compounds of the present invention can be prepared as described in Scheme C. Reacting commercially available 2,4-dichloro-5-nitropyrimidine (**C1**) with potassium isothiocyanate in acetic acid would give intermediate **C2.** Reduction of the nitro group, followed by cyclization *in situ,* would give the precursor **C3.** Acylation with an acid chloride, acid or anhydride **C4** would yield intermediate **C5.** Coupling of **C5** with a boronic acid or ester, **C6** in the presence of palladium catalyst such as $PdCl_3$(pddf) or $Pd(PPh_3)4$ would then produce the desired product **C7.**

*Scheme D: General Synthetic Route IV*

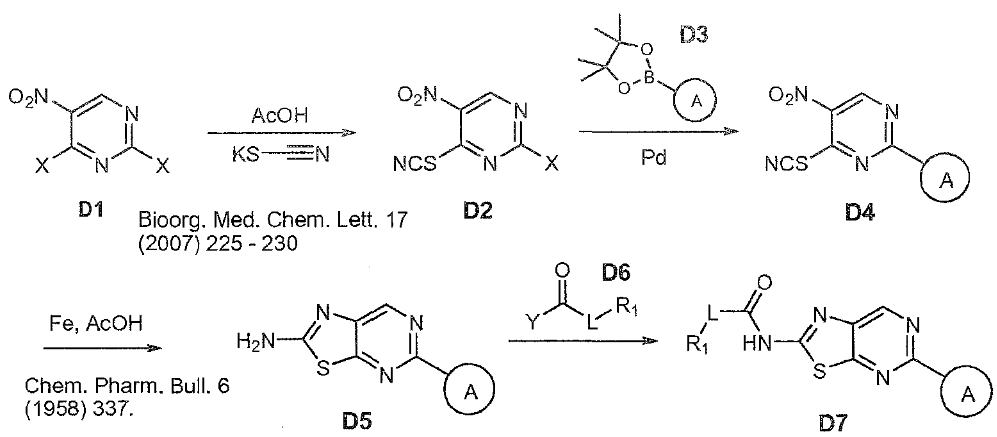


where R is $-LR_1$, X is halo and Y is Cl or OH.

Alternatively, some compounds of the present invention can be prepared as described in Scheme D. Reacting commercially available 2,4-dichloro-5-nitropyrimidine (**D1**) with potassium isothiocyanate and bromine in acetic acid would give intermediate **D2.** Suzuki coupling of **D2** with a boronic acid or ester **D3** in the presence of palladium catalyst such as $PdCl_3$(pddf) or $Pd(PPh_3)4$ would then produce intermediate **D4.** Reduction of the nitro group, followed by cyclization *in situ,* would give the precursor **D5,** which can subsequently be converted to the desired compounds **D7** by acylation with an acid chloride **D6,** for example.

In each of the above reaction procedures or schemes, the various substituents may be selected from among

the various substituents otherwise taught herein.

**Routine Procedures for Preparing the Compounds of the Invention**

It will be readily recognized that certain compounds according to the present invention have atoms with linkages to other atoms that confer a particular stereochemistry to the compound (*e.g.*, chiral centers). It is recognized that synthesis of compounds according to the present invention may result in the creation of mixtures of different stereoisomers (i.e., enantiomers and diastereomers). Unless a particular stereochemistry is specified, recitation of a compound is intended to encompass all of the different possible stereoisomers.

Compounds according to the present invention can also be prepared as their individual stereoisomers by reacting a racemic mixture of the compound with an optically active resolving agent to form a pair of diastereoisomeric compounds, separating the diastereomers and recovering the optically pure enantiomer. While resolution of enantiomers can be carried out using covalent diastereomeric derivatives of compounds, dissociable complexes are preferred (*e.g.*, crystalline diastereoisomeric salts).

Compounds according to the present invention can also be prepared as a pharmaceutically acceptable acid addition salt by reacting the free base form of the compound with a pharmaceutically acceptable inorganic or organic acid. Alternatively, a pharmaceutically acceptable base addition salt of a compound can be prepared by reacting the free acid form of the compound with a pharmaceutically acceptable inorganic or organic base. Inorganic and organic acids and bases suitable for the preparation of the pharmaceutically acceptable salts of compounds are set forth in the definitions section of this Application. Alternatively, the salt forms of the compounds can be prepared using salts of the starting materials or intermediates.

The free acid or free base forms of the compounds can be prepared from the corresponding base addition salt or acid addition salt form. For example, a compound in an acid addition salt form can be converted to the corresponding free base by treating with a suitable base (*e.g.*, ammonium hydroxide solution, sodium hydroxide, and the like). A compound in a base addition salt form can be converted to the corresponding free acid by treating with a suitable acid (*e.g.*, hydrochloric acid, *etc).*

The *N*-oxides of compounds according to the present invention can be prepared by methods known to those of ordinary skill in the art. For example, *N*-oxides can be prepared by treating an unoxidized form of the compound with an oxidizing agent (*e.g.*, trifluoroperacetic acid, permaleic acid, perbenzoic acid, peracetic acid, meta-chloroperoxybenzoic acid, or the like) in a suitable inert organic solvent (*e.g.*, a halogenated hydrocarbon such as dichloromethane) at approximately 0 ˚C. Alternatively, the *N*-oxides of the compounds can be prepared from the *N*-oxide of an appropriate starting material.

Compounds in an unoxidized form can be prepared from *N*-oxides of compounds by treating with a reducing agent (*e.g.*, sulfur, sulfur dioxide, triphenyl phosphine, lithium borohydride, sodium borohydride, phosphorus trichloride, tribromide, or the like) in an suitable inert organic solvent (*e.g.*, acetonitrile, ethanol, aqueous dioxane, or the like) at 0 to 80 ˚C.

Prodrug derivatives of the compounds can be prepared by methods known to those of ordinary skill in the art (*e.g.*, for further details see Saulnier et al. (1994), Bioorganic and Medicinal Chemistry Letters, Vol. 4, p. 1985). For example, appropriate prodrugs can be prepared by reacting a non-derivatized compound with a suitable carbamylating agent (*e.g.*, 1,1-acyloxyalkylcarbonochloridate, para-nitrophenyl carbonate, or the like).

Protected derivatives of the compounds can be made by methods known to those of ordinary skill in the art. A detailed description of the techniques applicable to the creation of protecting groups and their removal can be found in P.G.M. Wuts and T.W. Greene, "Greene's Protecting Groups in Organic Synthesis", 4th edition, John Wiley & Sons, Inc. 2007.

Compounds according to the present invention may be conveniently prepared, or formed during the process of the invention, as solvates (*e.g.*, hydrates). Hydrates of compounds of the present invention may be conveniently prepared by recrystallization from an aqueous/organic solvent mixture, using organic solvents such as dioxin, tetrahydrofuran or methanol.

As used herein the symbols and conventions used in these processes, schemes and examples are consistent with those used in the contemporary scientific literature, for example, the Journal of the American Chemical Society or the Journal of Biological Chemistry. Standard single-letter or three-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:

| | |
|---|---|
| μL (microliters) | Ac (acetyl) |
| atm (atmosphere) | ATP Adenosine tripliosphate |

(continued)

| | |
|---|---|
| ATPase (adenosine triphosphatase) | BOC (tert-bulyloxycarbonyl) |
| BSA (Bovine Serum Albumin) | BOP (bis(2-oxo-3-oxazolidinyl)phosphinic chloride) |
| CDI (1,1-carbonyldiimidazole) | CBZ (benzyloxycarbonyl) |
| DCE (dichloroethane) | DCC (dicyclohexylcarbodiimide) |
| DMAP (4-dimethylaminopyridine) | DCM (dichloromethane) |
| DMF (N,N-dimethylformamide) | DME (1,2-dimethoxyethane) |
| DMSO (dimethylsulfoxide) | DMPU (N,N' -dimethylpropyleneurea) |
| EDTA (ethylenediaminetetraacetic acid) | DTT (1,4-dithiothreitol) |
| $Et_2O$ (diethyl ether) | EDCI (ethylcarbodiimide hydrochloride) |
| FMOC (9-fluorenylmethoxycarbonyl) | Et (ethyl) |
| h (hours) | EtOAc(ethyl acetate) |
| HEPES (4-(2-hydroxyethyl)-1-piperazineethane-sulfonic acid) | g (grams) |
| HOBT (1-hydroxybenzotriazole) | HOAc or AcOH (acetic acid) |
| HPLC (high pressure liquid chromatography) | HOSu (N-hydroxysuccinimide) |
| i.v. (intravenous) | Hz(Hertz) |
| i-PrOH (isopropanol) | IBCF (isobutyl chloroformate) |
| M (molar) | L(liters) |
| Me (methyl) | mCPBA (meta-chloroperbenzoic acid) |
| mg (milligrams) | MeOH (methanol) |
| Min (minutes) | MHz (megahertz) |
| mM (millimolar) | mL (milliliters) |
| mol (moles) | mmol (millimoles) |
| mp (melting point) | MOPS (Morpholinepropanesulfonic acid) |
| OMe (methoxy) | NaOAc (sodium acetate) |
| RP (reverse phase) | psi (pounds per square inch) |
| SPA (Scintillation Proximity Assay) | r.t. (ambient temperature) |
| TBS (t-butyldimethylsilyl) | TBAF (tetra-n-butylammonium fluoride) |
| TEA (triethylamine) | tBu (tert-butyl) |
| TFAA (trifluoroacetic anhydride) | TFA(trifluoroacetic acid) |
| TIPS (triisopropylsilyl) | THF (tetrahydrofuran) |
| TMS (trimethylsilyl) | TLC (thin layer chromatography) |
| Tr (retention time) | TMSE (2-(trimethylsilyl)ethyl) |

All references to ether or $Et_2O$ are to diethyl ether; and brine refers to a saturated aqueous solution of NaCl. Unless otherwise indicated, all temperatures are expressed in ˚C (degrees Centigrade). All reactions are conducted under an inert atmosphere at RT unless otherwise noted.

$^1$H NMR spectra were recorded on a Bruker Avance 400. Chemical shifts are expressed in parts per million (ppm). Coupling constants are in units of Hertz (Hz). Splitting patterns describe apparent multiplicities and are designated as s (singlet), d (doublet), t (triplet), q (quartet), m (multiplet), br (broad).

Low-resolution mass spectra (MS) and compound purity data were acquired on a Waters ZQ LC/MS single quadrupole system equipped with electrospray ionization (ESI) source, UV detector (220 and 254 nm), and evaporative

light scattering detector (ELSD). Thin-layer chromatography was performed on 0.25 mm E. Merck silica gel plates (60F-254), visualized with UV light, 5% ethanolic phosphomolybdic acid, Ninhydrin or *p*-anisaldehyde solution. Flash column chromatography was performed on silica gel (230-400 mesh, Merck).

The starting materials and reagents used in preparing these compounds are either available from commercial suppliers such as the Aldrich Chemical Company (Milwaukee, WI), Bachem (Torrance, CA), Sigma (St. Louis, MO), or may be prepared by methods well known to a person of ordinary skill in the art, following procedures described in such standard references as Fieser and Fieser's Reagents for Organic Synthesis, vols. 1-23, John Wiley and Sons, New York, NY, 2006; Rodd's Chemistry of Carbon Compounds, vols. 1-5 and supps., Elsevier Science Publishers, 1998; Organic Reactions, vols. 1-68, John Wiley and Sons, New York, NY, 2007; March J.: Advanced Organic Chemistry, 5th ed., 2001, John Wiley and Sons, New York, NY; and Larock: Comprehensive Organic Transformations, 2nd edition, John Wiley and Sons, New York, 1999. The entire disclosures of all documents cited throughout this application are incorporated herein by reference.

Various methods for separating mixtures of different stereoisomers are known in the art. For example, a racemic mixture of a compound may be reacted with an optically active resolving agent to form a pair of diastereoisomeric compounds. The diastereomers may then be separated in order to recover the optically pure enantiomers. Dissociable complexes may also be used to resolve enantiomers (*e.g.*, crystalline diastereoisomeric salts). Diastereomers typically have sufficiently distinct physical properties (*e.g.*, melting points, boiling points, solubilities, reactivity, *etc.*) and can be readily separated by taking advantage of these dissimilarities. For example, diastereomers can typically be separated by chromatography or by separation/resolution techniques based upon differences in solubility. A more detailed description of techniques that can be used to resolve stereoisomers of compounds from their racemic mixture can be found in Jean Jacques, Andre Collet, and Samuel H. Wilen, Enantiomers, Racemates and Resolutions, John Wiley & Sons, Inc. (1981).

Diastereomers have distinct physical properties (*e.g.*, melting points, boiling points, solubilities, reactivity, etc.) and can be readily separated by taking advantage of these dissimilarities. The diastereomers can be separated by chromatography or, preferably, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixture can be found in Jean Jacques, Andre Collet, and Samuel H. Wilen, Enantiomers, Racemates and Resolutions, John Wiley & Sons, Inc. (1981).

Chiral components can be separated and purified using any of a variety of techniques known to those skilled in the art. For example, chiral components can be purified using supercritical fluid chromatography (SFC). In one particular variation, chiral analytical SFC/MS analyses are conducted using a Berger analytical SFC system (AutoChem, Newark, DE) which consists of a Berger SFC dual pump fluid control module with a Berger FCM 1100/1200 supercritical fluid pump and FCM 1200 modifier fluid pump, a Berger TCM 2000 oven, and an Alcott 718 autosampler. The integrated system can be controlled by BI-SFC Chemstation software version 3.4. Detection can be accomplished with a Waters ZQ 2000 detector operated in positive mode with an ESI interface and a scan range from 200-800 Da with 0.5 second per scan. Chromatographic separations can be performed on a ChiralPak AD-H, ChiralPak AS-H, ChiralCel OD-H, or ChiralCel OJ-H column (5$\mu$, 4.6 x 250 mm; Chiral Technologies, Inc. West Chester, PA) with 10 to 40% methanol as the modifier and with or without ammonium acetate (10 mM). Any of a variety of flow rates can be utilized including, for example, 1.5 or 3.5 mL/min with an inlet pressure set at 100 bar. Additionally, a variety of sample injection conditions can be used including, for example, sample injections of either 5 or 10$\mu$L in methanol at 0.1 mg/mL in concentration.

In another variation, preparative chiral separations are performed using a Berger MultiGram II SFC purification system. For example, samples can be loaded onto a ChiralPak AD column (21 x 250 mm, 10$\mu$). In particular variations, the flow rate for separation can be 70 mL/min, the injection volume up to 2 mL, and the inlet pressure set at 130 bar. Stacked injections can be applied to increase the efficiency.

Descriptions of the syntheses of particular compounds according to the present invention based on the above reaction schemes and variations thereof are set forth in the Example section.

## Assaying the Biological Activity of the compounds of the Invention

The inhibitory effect of the compound of the invention on PI3K/mTOR may be evaluated by a variety of binding assays and functional assays. Example A provides fluorescence based binding essay for determining the inhibition of mTOR. The assay is based on the differential sensitivity of phosphorylated and non-phosphorylated peptides to proteolytic cleavage; quanitation of the binding is by fluorescence. Example B provides a fluorescent based immunoassay for the determination of the inhibition of PI3K$\alpha$. Using these assays, some of the exemplified compounds were shown to have $IC_{50}$ of greater than 1 $\mu$M, some others less than about 1 $\mu$M, and most others of the compounds have an $IC_{50}$ value of less than about 0.1 $\mu$M. For example, Example 29 exhibits an $IC_{50}$ value for PI3K of 310 nM and an $IC_{50}$ value for mTOR of 790 nM. The $IC_{50}$ values of the exemplified compounds of the present invention are given in Table 1.

It will be apparent to those skilled in the art that various modifications and variations can be made in the compounds, compositions, kits, and methods of the present invention without departing from the spirit or scope of the invention. Thus, it is intended that the present invention cover the modifications and variations of this invention provided they come within the scope of the appended claims and their equivalents.

## EXAMPLE

### <u>Example 1:</u> Preparation of *N*-(5-Bromothiazolo[5,4-*b*]pyridin-2-yl)acetamide (1)

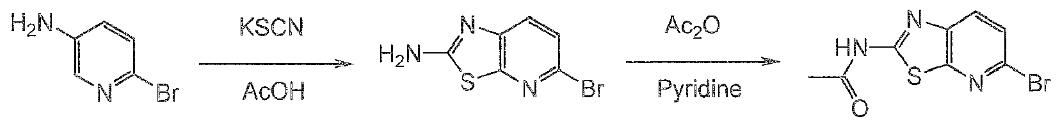


1A 1B Example 1

To a 500 ml 3-neck under $N_2$ was added KSCN (42.1 g, 433 mmol), followed by 225 mL of HOAC. It was cooled to -5 ˚C and 6-bromo-3-aminopyidine (15 g, 86.7 mmol) was added in portions. The mixture was further cooled to -15 ˚C, and a solution of $Br_2$ (5.7 mL) in 12 mL of HOAc was added dropwise while keeping the bath temperature below 10˚C. It was then allowed to slowly warm up to room temperature and stirred overnight. The small amount of precipitate was filtered off. The mother liquor was cooled to 0 ˚C, and water was added (200 mL). It was stirred for 5 min, and the precipitate formed was collected and washed with cold MeOH/$Et_2O$ (1:3, 50 mL, 2 times). The solid was dried under vacuum overnight to give 5-bromothiazolo[5,4-b]pyridin-2-amine the titled compound as a yellow solid (10.2 g, 51%). The solvent was stripped from the filtrate to half the volume, and the precipitation was collected, washed with cold MeOH-$Et_2O$, and dried to give another 2 g of product with a combined yield of 12.2 g (61% yield). To the above obtained compound (11.3 g, 40 mmol) in pyridine (88 mL) was slowly added $Ac_2O$ (44 mL) at 0 ˚C. The mixture was then stirred at room temperature for 16 h. Solvent was removed, and the residue was subjected to vacuum for 20 h to give N-(5-bromothiazolo[5,4-b]pyridin-2-yl)acetamide as a light brown solid (13.3 g, 100%). [M+H] calc'd for $C_8H_6BrN_3OS$, 272; found, 272.

### <u>Example 2:</u> Preparation of *N*-(5-(1*H*-Indol-5-yl)thiazolo[5,4-*b*]pyridin-2-yl)acetamide (2)

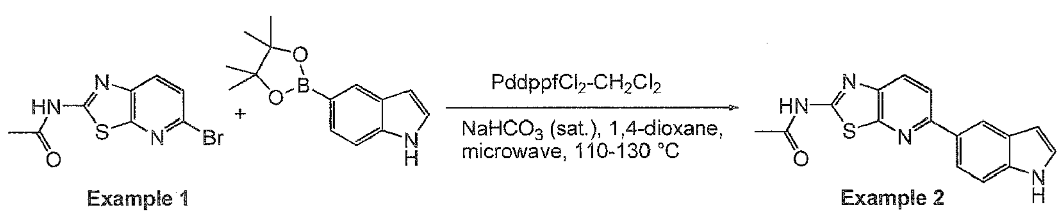


Example 1 Example 2

A mixture of **Example 1** (64 mg, 0.23 mmol), 5-(4,4,5,5-tetrametlayl-1,3,2-dioxaborolan-2-yl)-1H-indole (85 mg, 0.35 mmol), $PddppfCl_2$-DCM (cat.), Sat. $NaHCO_3$ (1 mL) and 1,4-dioxane (2 mL) was heated in a microwave at 130 ˚C for 30 min. The reaction mixture was purified by HPLC to give the titled compound N-(5-(1H-indol-5-yl)thiazolo [5,4-b]pyridin-2-yl)acetamide as a white solid (3.8 mg). $^1H$ NMR (400 MHz, MeOD) δ ppm 8.13 (s, 1H), 7.98 (d, *J*=6 Hz, 1H), 7.85 (d, *J*=6 Hz, 1H), 7.72 (d, *J*=6 Hz, 1H), 7.39 (d, *J*=6 Hz, 1H), 7.19 (d, *J*=4 Hz, 1H), 6.46 (d, *J*=4 Hz, 1H), 2.18 (s, 3H); [M+H] calc'd for $C_{16}H_{12}N_4OS$, 309; found, 309.

### <u>Example 3:</u> Preparation of *N*-(5-(1*H*-Indazol-5-yl)thiazolo[5,4-*b*]pyridin-2-yl)acetamide (3)

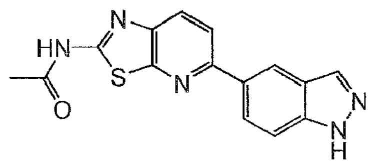

The titled compound was prepared following the procedures for the preparation of **Example 2,** except 5-(4,4,5,5-tetramethyl-1,3,2-dioxaborolan-2-yl)-1H-indazole was used as starting material. $^1$H NMR (400 MHz, MeOD) δ ppm 8.49 (s, 1H), 8.20-8.14 (m, 3H), 8.04 (d, *J*=6 Hz, 1H), 7.69 (d, *J*=6 Hz, 1H), 2.31 (s, 3H); [M+H] calc'd for $C_{15}H_{12}N_5OS$, 310; found, 310.

**<u>Example 4:</u> Preparation of *N*-(5-(6-Acetamidopyridin-3-yl)thiazolo[5,4-*b*]pyridin-2-yl)acetamide (4)**

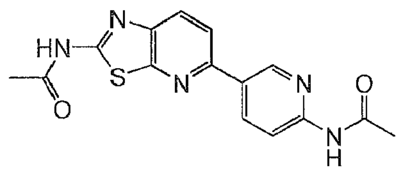

The titled compound was prepared following the procedures for the preparation of **Example 2,** except N-(5-(4,4,5,5-tetramethyl-1,3,2-dioxaborolan-2-yl)pyridin-2-yl)acetamide was used as starting material. $^1$H NMR (400 MHz, DMSO-$d_6$) δ ppm 13.34 (s, 1H), 11.53 (s, 1H), 9.88 (s, 1H), 9.30 (dd, *J*=6, 4 Hz, 1H), 9.02 (d, *J*=6 Hz, 1H), 8.99 (d, *J*=6 Hz, 1H), 8.91 (*J*=6 Hz, 1H), 3.05 (s, 3H), 2.94 (s, 3H); [M+H] calc'd for $C_{15}H_{13}N_5O_2S$, 328; found, 328.

**<u>Example 5:</u> Preparation of *N*-(5-(4-(2-Cyanopropan-2-yl)phenyl)thiazolo[5,4-*b*]pyridin-2-yl) acetamide (5)**

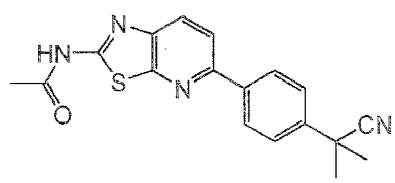

The titled compound was prepared following the procedures for the preparation of **Example 2,** except 2-methyl-2-(4-(4,4,5,5-tetramethyl-1,3,2-dioxaborolan-2-yl)phenyl)propanenitrile. $^1$H NMR (400 MHz, MeOD) δ ppm 8.02 (m, 3H), 7.88 (d, *J*=6 Hz, 1H), 7.57 (d, *J*=6 Hz, 2H), 2.19 (s, 3H), 1.68 (s, 6H); [M+H] calc'd for $C_{18}H_{16}N_4OS$, 337; found, 337.

**<u>Example 6:</u> Preparation of 5-(2-Acetamidothiazolo[5,4-*b*]pyridm-5-yl)-N-methylpicolinamide (6)**

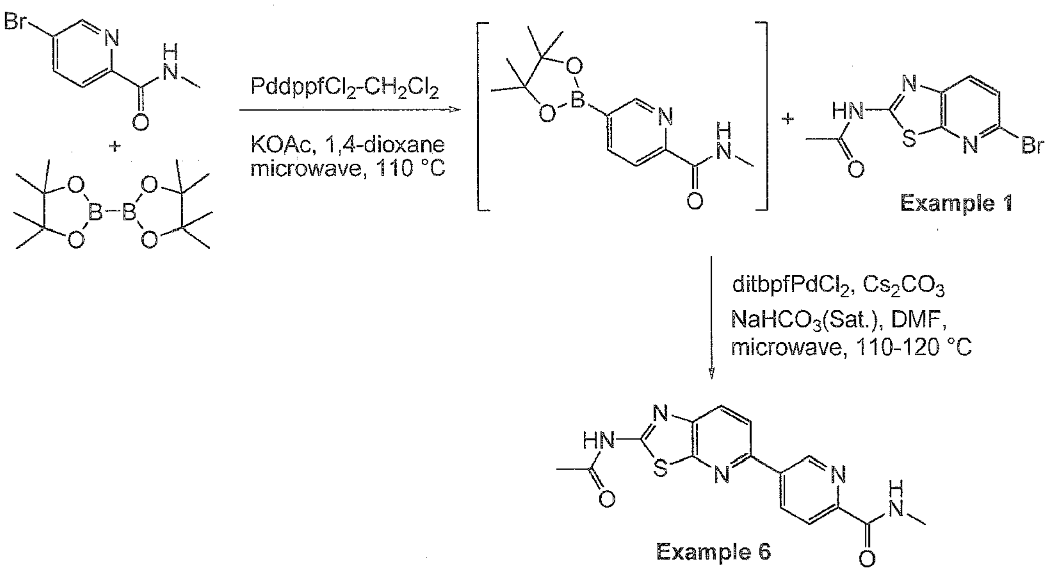

Example 1

Example 6

A mixture of 5-bromo-N-methylpicolinamide (320 mg, 1.48 mmol), bispinacolatodiboron (752 mg, 2.96 mmol), $PddppfCl_2$-DCM (cat.), KOAc (436 mg, 4.44 mmol) in 1,4-dioxane (5 mL, degassed) was heated at 110 ˚C in a microwave for 1 h. To this mixture was then added **Example 1** (608 mg, 2.22 mmol), ditbpfPdCl2 (cat.), $Cs_2CO_3$ (1.45 g, 4.44 mmol), $NaHCO_3$ (sat., 1 mL) and DMF (dry, degassed, 1 mL). It was heated in a microwave at 110˚C for 1 h, then at 120 ˚C for another hour. The reaction mixture was purified by HPLC to give the titled compound as a white solid. $^1$H NMR (400 MHz, DMSO-$d_6$) δ ppm 12.53 (s, 1H), 9.28 (d, *J*=4 Hz, 1H), 8.79 (m, 1H), 8.61 (dd, *J*=8 Hz, 1H), 8.18 (m, 2H), 8.08 (d, *J*=8 Hz, 1H), 2.78 (d, *J*=4 Hz, 1H), 2.18 (s, 3H); [M+H] calc'd for $C_{15}H_{13}NsO_2S$, 328; found, 328.

**<u>Example 7:</u> Preparation of *N*-(5-(6-Chloro-5-(phenylsulfonamido)pyridin-3-yl)thiazolo[5,4-*b*] pyridin-2-yl) acetamide (7)**

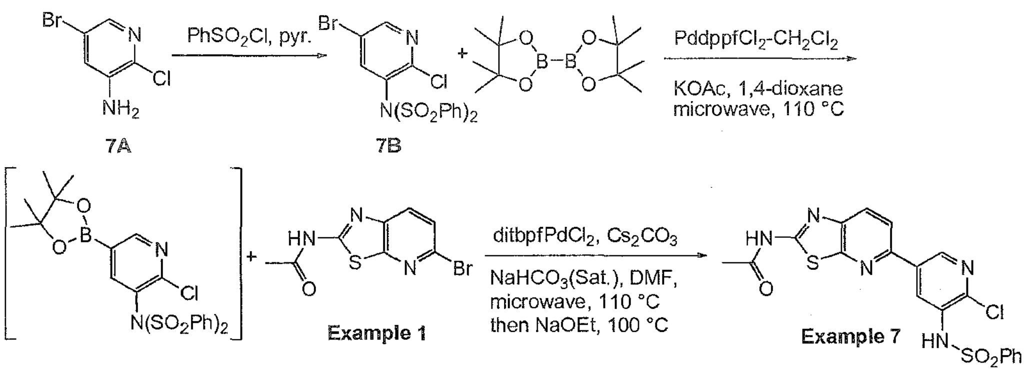

7A

7B

Example 1

Example 7

To a solution of 5-bromo-2-chloropyridin-3-amine 7A (2.87 g, 13.8 mmol) in pyridine (70 mL) was added $PhSO_2Cl$ (5.3 mL, 41.5 mmol). The mixture was heated at 50-70 ˚C for 3 h. The solvent was removed, and the resulting solid was washed with Hexanes-EtOAc (1:1, 150 mL) and water, and dried over $P_2O_5$ for 4 days to give *N*-(5-bromo-2-chloropyridin-3-yl)-*N*-(phenylsulfonyl)benzenesulfonamide as a white solid (5.1 g, 76%). The mixture of the compound obtained above (255 mg, 0.52 mmol), bispinacolatodiboron (265 mg, 1.04 mmol), $PddppfCl_2$-DCM (38 mg, 0.052 mmol), and KOAc (153 mg, 1.56 mmol) in 1,4-dioxane (5 mL, degassed) was heated in a microwave at 110 ˚C for 1 h. **Example 1** (128 mg, 0.47 mmol), ditbpfPdCl2 (cat.), $Cs_2CO_3$ (508 mg, 1.56 mmol), $NaHCO_3$ (sat., 1 mL) and DMF (dry, degassed, 1 mL) were then added to the mixture. It was heated in a microwave at 110˚C for 1 h. EtOH (2 mL, absolute) and NaOEt (1.6 mL, 21 wt% in EtOH) were then added and the mixture was heated in a microwave at 100 ˚C for 15 min. The reaction mixture was purified by HPLC to give the titled compound as a white solid (25 mg). $^1$H NMR (400 MHz, DMSO-$d_6$) δ ppm 12.67 (s, 1H), 10.55 (s, 1H), 9.02 (s, 1H), 8.48 (m, 1H), 8.24 (m, 2H), 8.20 (m, 2H), 7.84 (m, 2H), 7.75 (m, 1H),

7.66 (m, 1H), 2.32 (s, 3H); [M+H] calc'd for $C_{19}H_{14}ClN_sO_3S_2$, 460; found, 460.

**<u>Example 8:</u> Preparation of *N*-(5-(6-Chloro-5-(methylsulfonamido)pyridin-3-yl)thiazolo[5,4-*b*] pyridin-2-yl)acetamide (8)**

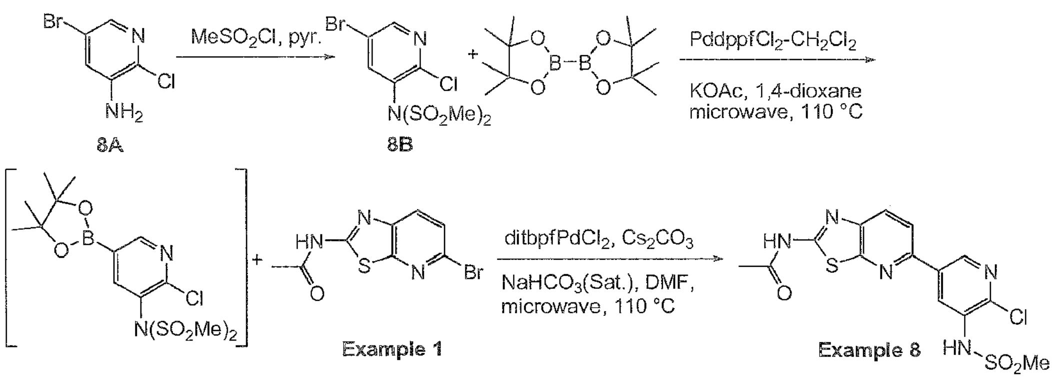


8A 8B Example 1 Example 8

To a solution of 5-bromo-2-chloropyidin-3-amine (4 g, 19.28 mmol) in pyridine (50 mL) was added $NeSO_2Cl$ (4.5 mL, 57.84 mmol). The mixture was heated at 50˚C for 4 h. The solvent was removed, and the resulting solid was washed with Hexanes-EtOAc (1:1, 150 mL) and water, and dried over $P_2O_5$ for 3 days to give N-(5-bromo-2-chloropyridin-3-yl)-N-(methylsulfonyl)methanesulfonamide as a light brown solid (2.12 g, 30%). The mixture of the bissulfonamide obtained above (263 mg, 0.723 mmol), bispinacolatodiboron (367 mg, 1.45 mmol), $PddppfCl_2$-DCM (cat.), and KOAc (213 mg, 2.17 mmol) in 1,4-dioxane (5 mL, degassed) was heated in a microwave at 110˚C for 1 h. To this mixture was then added **Example 1** (329 mg, 1.2 mmol), $ditbpfPdCl_2$ (cat.), $Cs_2CO_3$ (707 mg, 2.17 mmol), $NaHCO_3$ (sat., 1 mL) and DMF (dry, degassed, 1 mL). It was heated in a microwave at 110˚C for 1 h. The reaction mixture was purified on HPLC to give the titled compound as a white solid (66 mg). $^1H$ NMR (400 MHz, DMSO-$d_6$) δ ppm 12.50 (br s, 1H), 8.60 (s, 1H), 8.32 (s, 1H), 8.13 (d, *J*=8 Hz, 1H), 8.01 (d, *J*=8 Hz, 1H), 2.97 (s, 3H), 2.17 (s, 3H); [M+H] calc'd for $C_{14}H_{12}ClN_5O_3S_2$, 398; found, 398.

**<u>Example 9:</u> Preparation of *N*-(5-(6-Chloro-5-(methylsulfonamido)pyridin-3-yl)thiazolo[5,4-*b*] pyridin-2-yl)cyclopropanecarboxamide (9)**

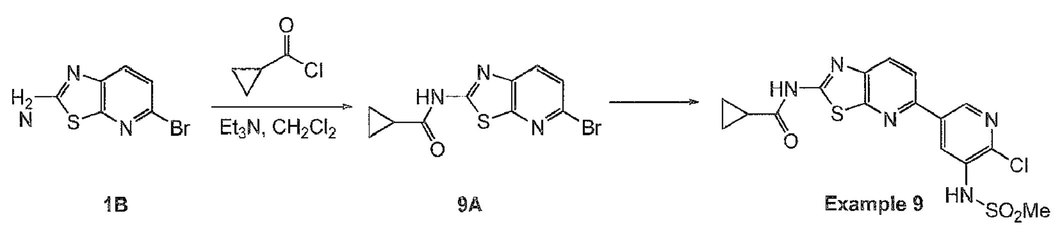


1B 9A Example 9

To **1B** (5 g, 21.6 mmol) in $CH_2Cl_2$ (220 mL) was slowly added triethylamine (37.6 mL) and cyclopropanecarbonylchloride (9 mL). The mixture was then refluxed at 70 ˚C for 1 h. The solvent was removed, and the crude product was crystallized from EtOH to give 9A as a white solid (3.5 g, 54%).

The titled compound **Example 9** was prepared following the procedure for the preparation of **Example 8** except for using compound **9A** as starting material. $^1H$ NMR (400 MHz, DMSO-$d_6$) δ ppm 8.68 (s, 1H), 8.35 (d, *J*=4 Hz, 1H), 8.14 (d, *J*=8 Hz, 1H), 8.04 (d, *J*=8 Hz, 1H), 2.97 (s, 3H), 1.98 (m, 1H), 0.93 (m, 4H); [M+H] calc'd for $C_{16}H_{14}ClN_5O_3S_2$ 424; found, 424.

**<u>Example 10:</u> Preparation of *N*-(6-Phenylbeenzo[*d*]thiazol-2-yl)acetamide (10)**

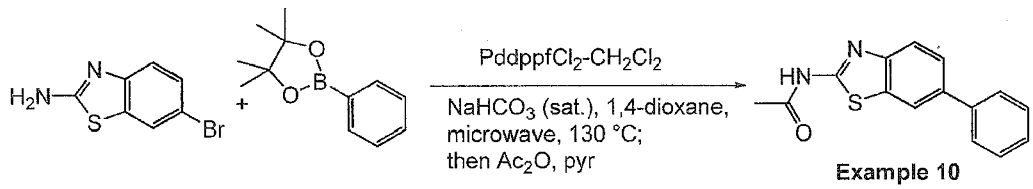


A mixture of 6-bromobenzo[d]thiazol-2-amine (61.7 mg, 0.27 mmol), phenylboronic acid (49 mg, 0.40 mmol), $PddppfCl_2$-DCM (cat.) in $NaHCO_3$ (sat., 0.75 mL) and 1,4-dioxane (1.5 mL) was heated in a microwave at 130 ˚C for 30 min. The reaction mixture was purified on HPLC to give the corresponding amine (54 mg, 89%) as a white solid. To this solid was added pyridine (2 mL) and $Ac_2O$ (1 mL). The mixture was stirred for 3 d and was purified on HPLC to give the titled compound as a white solid (26.9 mg). $^1H$ NMR (400 MHz, MeOD) δ ppm 8.01 (s, 1H), 7.69 (d, *J*=8 Hz, 1H), 7.58 (m, 3H), 7.35 (m, 2H), 7.24 (m, 1H), 2.18 (s, 3H); [M+H] calc'd for $C_{15}H_{12}N_2OS$, 269; found, 269.

**<u>Example 11:</u> Preparation of *N*-(6-(Pyridin-3-yl)benzo[*d*]thiazol-2-yl)acetamide (11)**

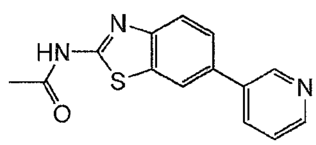

Following the same procedure for the preparation of **Example 10**, the titled compound **Example 11** was prepared using 3-(4,4,5,5-tetramethyl-1,3,2-dioxaborolan-2-yl)pyridine as the starting material. $^1H$ NMR (400 MHz, MeOD) δ ppm 9.06 (s, 1H), 8.71 d, *J*=8 Hz, 1H), 8.66 (d, *J*=8 Hz, 1H), 8.26 (s, 1H), 7.94 (m, 1H), 7.82 (d, *J*=8 Hz, 1H), 7.75 (m, 1H), 2.19 s, 3H); [M+H] calc'd for $C_{14}H_{11}N_3OS$, 270; found, 270.

**<u>Example 12:</u> Preparation of N-(6-(1*H*-Indol-5-yl)benzo[*d*]thiazol-2-yl)acetamide (12)**

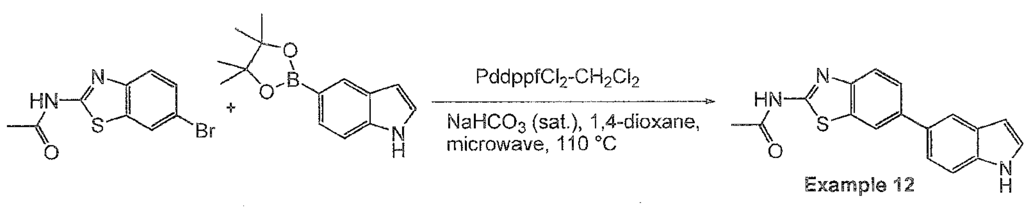


A mixture of N-(6-bromobenzo[d]thiazol-2-yl)acetamide (103 mg, 0.378 mmol), 5-(4,4,5,5-tetramethyl-1,3,2-dioxaborolan-2-yl)-1*H*-indole (138 mg, 0.568 mmol), $PddppfCl_2$-DCM (cat.) in $NaHCO_3$ (sat., 0.75 mL) and 1,4-dioxane (1.5 mL) was heated in a microwave at 110˚C for 15 min. The reaction mixture was purified by HPLC to give the titled compound as a brown solid (77 mg). $^1H$ NMR (400 MHz, MeOD) δ ppm 8.12 (s, 1H), 7.86 (s, 1H), 7.79 (d, *J*=8 Hz, 1H), 7.75 (d, *J*=8 Hz, 1H), 7.46 (m, 2H), 7.28 (s, 1H), 6.63 (, 1H), 2.29 (s, 3H); [M+H] calc'd for $C_{17}H_{13}N_3OS$, 308; found, 308.

**<u>Example 13:</u> Preparation of *N*-(6-(4-Aminophenyl)benzo[*d*]thiazol-2-yl)acetamide (13)**

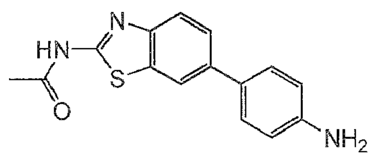

The titled compound was prepared following the procedures for the preparation of **Example 12**, except 4-(4,4,5,5-tetramethyl-1,3,2-dioxaborolan-2-yl)aniline was used as starting material. $^1$H NMR (400 MHz, MeOD) δ ppm 8.06 (s, 1H), 7.72 (m, 3H), 7.62 (d, *J*=8 Hz, 1H), 7.33 (d, *J*=8 Hz, 2H), 2.18 (s, 3H); [M+H] calc'd for $C_{15}H_{13}N_3OS$, 284; found, 284.

**Example 14: Preparation of *N*-(6-(1*H*-Indol-4-yl)benzo[*d*]thiazol-2-yl)acetamide (14)**

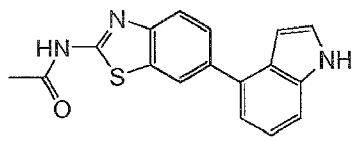

The titled compound was prepared following the procedures for the preparation of **Example 12**, except 4-(4,4,5,5-tetramethyl-1,3,2-dioxaborolan-2-yl)-1*H*-indole was used as starting material. $^1$H NMR (400 MHz, MeOD) δ ppm 8.03 (s, 1H), 7.73 (d, *J*=8 Hz, 1H), 7.67 (d, *J*=8 Hz, 2H), 7.29 (d, *J*=8 Hz, 1H), 7.20 (d, *J*=4 Hz, 1H), 7.10 (m, 1H), 7.04 (d, *J*=8 Hz, 1H), 6.52 (d, *J*=4 Hz, 1H), 2.18 (s, 3H); [M+H] calc'd for $C_{17}H_{13}N_3OS$, 308; found, 308.

**Example 15: Preparation of *N*-(6-(3-Formyl-1*H*-indol-5-yl)benzo[*d*]thiazol-2-yl)acetamide (15)**

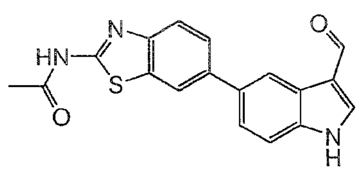

The titled compound was prepared following the procedures for the preparation of **Example 12**, except 5-(4,4,5,5-tetramethyl-1,3,2-dioxaborolan-2-yl)-1*H*-indole-3-carbaldehyde was used as starting material. [M+H] calc'd for $C_{18}H_{13}N_3O_2S$, 336; found, 336.

**Example 16: Preparation of *N*-(6-(3-Cyano-1*H*-indol-5-yl)benzo[*d*]thiazol-2-**yl)acetamide (16)

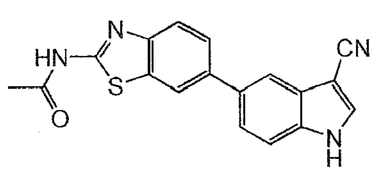

The titled compound was prepared following the procedures for the preparation of **Example 12**, except 5-(4,4,5,5-tetramethyl-1,3,2-dioxaborolan-2-yl)-1*H*-indole-3 carbonitrilewas used as starting material. [M+H] calc'd for $C_{18}H_{12}N_4OS$, 333; found, 333.

**Example 17: Preparation of 5-(2-Acetamidobenzo[*d*]thiazol-6-yl)-1*H*-indole-3-carboxamide (17)**

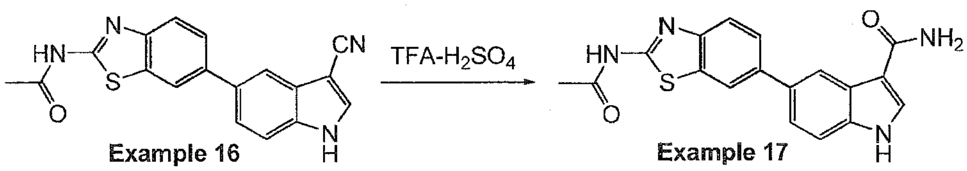


A solution of **Example 16** (1 mg, 0.003 mmol) was stirred in TFA (1 mL) and $H_2SO_4$ (0.25 mL) at room temperature for 3 h. The mixture was purified on the HPLC to give the titled compound. [M+H] calc'd for $C_{18}H_{14}N_4O_2S$, 351; found, 351.

**Example 18: Preparation of *N*-(6-(6-Chloro-5-(phenylsulfonamido)pyidin-3-yl)benzo [*d*]thiazol-2-yl)acetamide (18)**

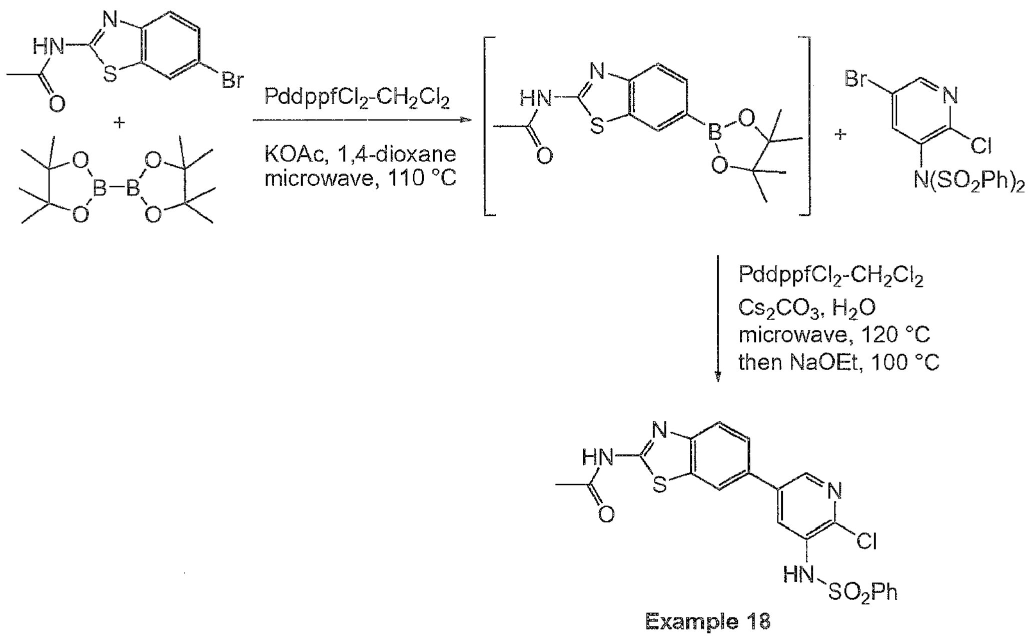


A mixture of N-(6-bromobenzo[*d*]thiazol-2-yl)acetamide (207 mg, 0.76 mmol), bispinacolatodiboron (386 mg, 1.52 mmol), PddppfCl$_2$-DCM (56 mg, 0.076 mmol), KOAc (224 mg, 2.28 mmol) in 1,4-dioxane (2 mL, degassed) was heated in a microwave at 110 ˚C for 1 h. To this mixture was then added N-(5-bromo-2-chloropyridin-3-yl)-N-(phenylsulfonyl)benzenesulfonamide (445 mg, 0.91 mmol), more PddppfCl$_2$-DCM (cat.), $Cs_2CO_3$ (495 mg, 1.52 mmol), $H_2O$ (1.3 mL) and 1,4-dioxane (1 mL). It was heated in a microwave at 120˚C for 3h. EtOH (2 mL, absolute) and NaOEt (1.5 mL, 21 wt% in EtOH) were then added and the mixture heated in a microwave at 100 ˚C for 5 min. HPLC purification of the reaction mixture gives the titled compound as a light brown solid (32.6 mg). $^1H$ NMR (400 MHz, MeOD) δ ppm 8.38 (d, *J*=4 Hz, 1H), 8.11 (s, 1H), 8.06 (s, 1H), 7.88 (m, 1H), 7.76 (d, *J*=8 Hz, 1H), 7.71 (d, *J*=8 Hz, 1H), 7.58 (d, *J*=8 Hz, 1H), 7.53 (d, *J*=8 Hz, 1H), 7.44 (m, 2H), 2.19 (s, 3H); [M+H] calc'd for $C_{20}H_{15}ClN_4O_3S_2$, 459; found, 459.

**Example 19: Preparation of *N*-(6-(4-Amino-2-(trifluoromethyl)phenyl)benzo [*d*]thiazol-2-yl)acetamide (19)**

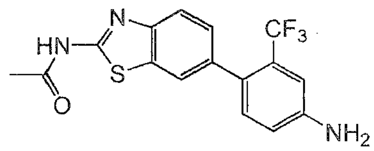

The titled compound **Example 19** was prepared following the procedure outlined for the synthesis of **Example 18,** except 4-bromo-3-(trifluoromethyl)aniline was used instead of N-(5-bromo-2-chloropyridin-3-yl)-N-(phenylsulfonyl) benzenesulfonamide to obtain the final product. $^1$H NMR (400 MHz, MeOD) δ ppm 7.93 (s, 1H), 7.66 (d, *J*=8 Hz, 1H), 7.56 (s, 1H), 7.53 (m, 2H), 6.84 (d, *J*=8 Hz, 1H), 2.17 9s, 3H); [M+H] calc'd for $C_{16}H_{12}F_3N_3OS$, 352; found, 352.

<u>Example 20:</u> **Preparation of *N*-(6-(5-Aminopyrazin-2-yl)benzo[*d*]thiazol-2-yl)acetamide (20)**

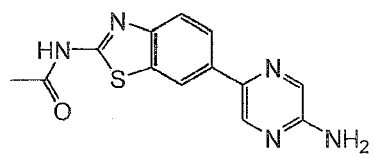

The titled compound **Example 20** was prepared according to the procedure used for the synthesis of **Example 18**, except that 5-bromopyrazin-2-amine was used instead of N-(5-bromo-2-chloropyridin-3-yl)-N-(phenylsulfonyl)benzenesulfonamide to obtain the product.
[M+H] calc'd for $C_{13}H_{11}N_5OS$, 286; found, 286.

<u>Example 21:</u> **Preparation of *N*-(6-(2,5-Dioxo-2,3,4,5-tetrahydro-1*H*-benzo[e][1,4] diazepin-8yl) benzo[*d*]thiazol-2-yl)acetamide (21)**

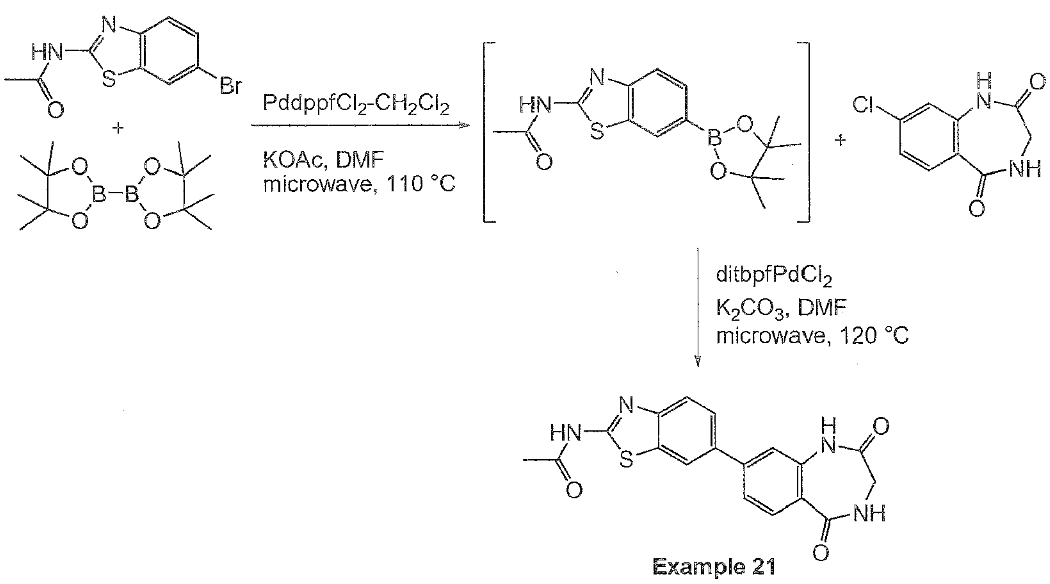


Example 21

A mixture of N-(6-bromobenzo[d]thiazol-2-yl)acetamide (45 mg, 0.16 mmol), bispinacolatodiboron (85 mg, 0.33 mmol), PddppfCl$_2$-DCM (cat.), KOAc (49 mg, 0.49 mmol) in DMF (1.5 mL, degassed) was heated in a microwave at 110 ˚C for 1 h. To this mixture was then added 8-chloro-3,4-dihydro-1H-benzo[e][1,4]diazepine-2,5-dione (35 mg, 0.16 mmol), ditbpfpdCl$_2$ (cat.), $K_2CO_3$ (69 mg, 0.49 mmol), DMF (1 mL, degassed). It was heated in a microwave at 120˚C for 3h. The reaction mixture was purified by HPLC to give the titled compound as an off-white solid (7.8 mg). $^1$H NMR

(400 MHz, DMSO-$d_6$) δ ppm 10.43 (s, 1H), 8.57 (t, *J*=8 Hz, 1H), 8.30 (d, *J*=4 Hz, 1H), 7.86 (d, *J*=8 Hz, 1H), 7.84 (d, *J*=8 Hz, 1H), 7.72 (dd, *J*=8, 4 Hz, 1H), 7.59 (dd, *J*=8, 4 Hz, 1H), 7.44 (d, *J*=4 Hz, 1H), 3.66 (d, *J*=4 Hz, 2H), 2.22 (s, 3H); [M+H] calc'd for $C_{18}H_{14}F_3N_4O_3S$, 367; found, 367.

**Example 22: Preparation of *N*-(5-(Imidazo[1,2-*a*]pyridin-6-yl)thiazolo[5,4-*b*]pyridin-2-yl)acetamide (22)**

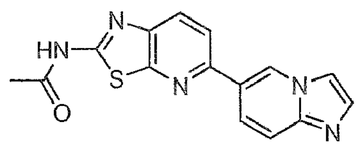

The titled compound was prepared following the procedures for the preparation of **Example 2**, except that 6-(4,4,5,5-tetramethyl-1,3,2-dioxaborlan-2-yl)imidazo[1,2-a]pyridine was used as starting material. $^1$H NMR (400 MHz, DMSO-$d_6$) δ ppm 2.25 (s, 3 H) 8.01 (d, *J*=9.35 Hz, 1 H) 8.14 (d, *J*=1.77 Hz, 1 H) 8.18 (d, *J*=8.59 Hz, 1 H) 8.30 (d, *J*=8.59 Hz, 1 H) 8.36 (d, *J*=1.52 Hz, 1 H) 8.58 (dd, *J*=9.60, 1.52 Hz, 1 H) 9.67 (s, 1 H) 12.63 (s, 1 H). [M+H] calc'd for $C_{15}H_{11}N_5OS$, 310; found, 310.

**Example 23: Preparation of *N*-(5-(6-Cyanopyridin-3-yl)thiazolo[5,4-*b*]pyridin-2-yl)acetamide (23)**

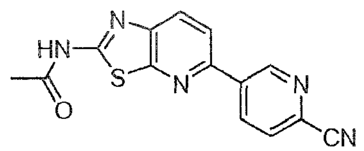

The titled compound was prepared following the procedures for the preparation of **Example 2**, except that 5-(4,4,5,5-tetramethyl-1,3,2-dioxaborolan-2-yl)picolinonitrilewas used as starting material. $^1$H NMR (400 MHz, MeOD) δ ppm 2.28 (s, 3 H) 7.98 (d, *J*=8.34 Hz, 1 H) 8.69 - 8.18 (m, 2 H) 8.68 (dd, *J*=8.34, 2.27 Hz, 1 H) 9.43 (d, *J*=1.52 Hz, 1 H). [M+H] calc'd for $C_{14}H_9N_5OS$, 296; found, 296.

**Example 24: Preparation of *N*-(5-(1*H*-Pyrrolo[2,3-*b*]pyridin-5-yl)thiazolo[5,4-*b*]pyridin-2-yl)acetamide (24)**

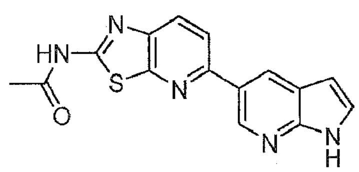

The titled compound was prepared following the procedures for the preparation of **Example 2**, except that 5-(4,4,5,5-tetramethyl-1,3,2-dioxaborolan-2-yl)-1*H*-pyrrolo[2,3-b]pyridine was used as starting material. [M+H] calc'd for $C_{15}H_{11}N_5OS$, 310; found, 310.

**Example 25: Preparation of *N*-(5-(1H-Pyrrolo[3,2-*b*]pyridin-6-yl)thiazolo[5,4-*b*]pyridin-2-yl)acetamide (25)**

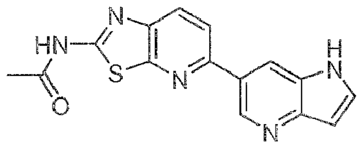

The titled compound was prepared following the procedures for the preparation of **Example 2**, except that 6-(4,4,5,5-tetramethyl-1,3,2-dioxaborolan-2-yl)-1H-pyrrolo[3,2-b]pyridine was used as starting material $^1$H NMR (400 MHz, MeOD) δ ppm 2.29 (s, 3 H) 6.90 (d, *J*=2.53 Hz, 1 H) 8.12 - 8.22 (m, 3 H) 9.19 (s, 1 H) 9.31 (s, 1 H). [M+H] calc'd for $C_{15}H_{11}N_5OS$, 310; found, 310.

**Example 26: Preparation of *N*-(5(4-(Methylsulfonyl)phenyl)thiazolo[5,4-*b*]pyridine-2-yl)acetamide (26)**

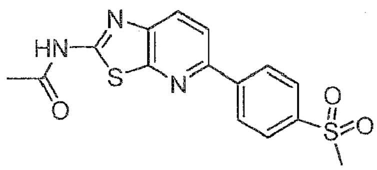

The titled compound was prepared following the procedures for the preparation of **Example 2,** except that 4,4,5,5-tetramethyl-2-(4-(methylsulfonyl)phenyl)-1,3,2-dioxaborolane was used as starting material. $^1$H NMR (400 MHz, DMSO-$d_6$) δ ppm 1.55 (s, 3 H) 1.86 (s, 3 H) 7.33 - 7.39 (m, 2 H) 7.40 - 7.48 (m, 2 H) 7.67 (d, *J*=8.34 Hz, 2 H). [M+H] calc'd for ($C_{15}H_3O_3S_2$, 348; found, 348.

**Example 27: Preparation of 3-(2-Acetamidothazolo[5,4-*b*]pyndin-5-yl)-*N*-methoxybenzamide (27)**

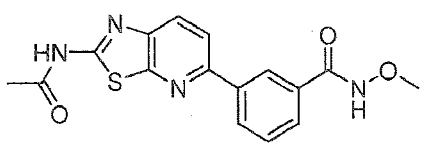

The titled compound was prepared following the procedures for the preparation of **Example 2**, except that N-methoxy-3-(4,4,5,5-tetraniethyl-1,3,2-dioxaborolan-2-yl)benzamide was used as starting material. $^1$H NMR (400 MHz, DMSO-$d_6$) δ ppm 2.24 (s, 3 H) 3.75 (s, 3 H) 7.62 (q, *J*=7.66 Hz, 1 H) 7.81 (d, *J*=8.08 Hz, 1 H) 8.12 - 8.15 (m, 1 H) 8.20 - 8.23 (m, 1 H) 8.31 (d, *J*=7.33 Hz, 1 H) 8.49 (t, *J*=1.52 Hz, 1 H) 11.92 (s, 1 H) 12.55 (s, 1 H). [M+H] calc'd for $C_{16}H_{14}N_4O_3S$, 343; found, 343.

**Example 28: Preparation of *N*-(5-(4-(*N*-Methylsulfamoyl)phenyl)thiazolo[5,4-*b*]pyridine-2-yl)acetamide (28)**

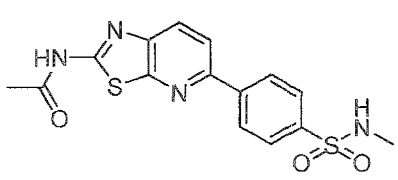

The titled compound was prepared following the procedures for the preparation of **Example 2**, except that *N*-methyl-4-(4,4,5,5-tetramethyl-1,3,2-dioxaborolan-2-yl)benzenesulfonamide was used as starting material. $^1$H NMR (400 MHz, DMSO-$d_6$) δ ppm 2.24 (s, 3 H) 2.45 (d, *J*=5.05 Hz, 3 H) 7.54 (d, *J*=5.05 Hz, 1 H) 7.88 - 7.91 (m, 2 H) 8.15 - 8.24 (m, 2 H) 8.34 - 8.38 (m, 2 H) 12.58 (s, 1 H). [M+H] calc'd for ($C_{15}H_{14}N_4O_3S_2$, 363; found, 363.

__Example 29:__ **Preparation of *N*-(5-(4-(Methylsulfonamido)pheny)thiazolo[5,4-*b*]pyridin-2-yl)acetamide (29)**

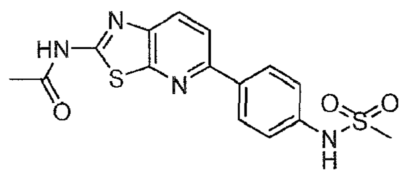

The titled compound was prepared following the procedures for the preparation of **Example 2**, except that *N*-(4-(4,4,5,5-tetramethyl-1,3,2-dioxaborolan-2-yl)phenyl)methanesulfonamide was used as starting material. $^1$H NMR (400 MHz, DMSO-$d_6$) δ ppm 2.23 (s, 3 H) 3.06 (s, 3 H) 7.32 (d, *J*=8.84 Hz, 2 H) 8.01 (d, *J*=8.59 Hz, 1 H) 8.12 (dd, *J*=13.77, 8.72 Hz, 3 H) 9.99 (s, 1 H) 12.49 (s, 1 H). [M+H] calc'd for $C_{15}H_{14}N_4O_3S_2$, 363; found, 363.

__Example 30:__ **Preparation of 4-(2Acetamidothiazolo[5,4-*b*]pyridin-5-yl)-*N*-methoxybenzamide (30)**

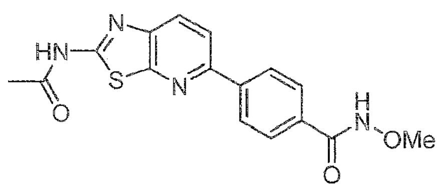

The titled compound was prepared following the procedures for the preparation of **Example 2**, except that *N*-methoxy-4-(4,4,5,5 -tetramethyl-1,3,2-dioxaborolan-2-yl)benzamide was used as starting material. $^1$H NMR (400 MHz, MeOD) δ ppm 2.28 (s, 3 H) 3.83 (s, 3 H) 7.85 - 7.92 (m, 2 H) 8.01 (d, *J*=8.59 Hz, 1 H) 8.11 (d, *J*=8.59 Hz, 1 H) 8.15 - 8.22 (m, 2H). [M+H] calc'd for $C_{16}H_{14}O_3S$, 343; found, 343.

__Example 31:__ **Preparation of *N*-(5-(2-Oxoindolin-5-yl)thiazolo[5,4-*b*]pyridin-2-yl)acetamide (31)**

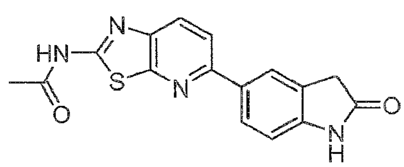

The titled compound was prepared following the procedures for the preparation of **Example 2**, except that 5-(4,4,5,5-tetramethyl-1,3,2-dioxaborolan-2-yl)indolin-2-one was used as starting material. [M+H] calc'd for $C_{16}H_{12}N_4O_2S$, 325; found, 325.

__Example 32:__ **Preparation of *N*-(6-(7-(Methylsulfonamido)-1*H*-indol-5-yl)benzo[*d*]thiazol-2-yl)acetamide (32)**

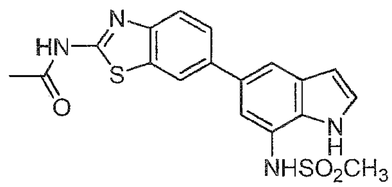

The titled compound was prepared following the procedures for the preparation of Example 18, except that N-(5-bromo-1H-indol-7-yl)methanesulfonamide was used as in the final step to obtain the product. The titled compound was synthesized following the procedures for the preparation of Example 18. [M+H] calc'd for C18H16N4O3S2, 401; found, 401.

**Example 33: Preparation of *N*-(5-(Pyridin-4-yl)thiazolo[5,4-*b*]pyridin-2-yl)acetamide (33)**

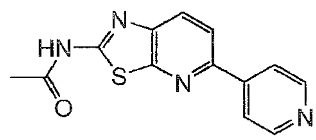

The titled compound was prepared following the procedures for the preparation of **Example 2**, except that 4-(4,4,5,5-tetramethyl-1,3,2-dioxaborolan-2-yl)pyridine was used as starting material. Yield: 2.8%. $^1$H NMR (400 MHz, MeOD) δ ppm 2.33 (s, 3 H) 8.24 (d, *J*=8.08 Hz, 1 H) 8.36 (d, *J*=8.08 Hz, 1 H) 8.69 (d, *J*=6.82 Hz, 2 H) 8.87 (d, *J*=6.82 Hz, 2 H). [M+H] calc'd for $C_{13}H_{10}N_4OS$, 271; found, 271.

**Example 34: Preparation of *N*-(5-(2-Aminophenyl)thiazolo[5,4-*b*]pyridin-2-yl)acetamide (34)**

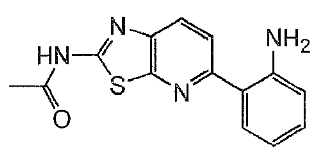

The titled compound was prepared following the procedures for the preparation of **Example 2**, except that 2-(4,4,5,5-tetramethyl-1,3,2-dioxaborolan-2-yl)aniline was used as starting material. Yield: 2.7%. $^1$H NMR (400 MHz, MeOD) δ ppm 2.30 (s., 3 H) 7.41 (br., 3 H) 7.88 (br., 2 H) 8.19 (br., 1 H). [M+H] calc'd for $C_{14}H_{12}N_4OS$, 285; found, 285.

**Example 35: Preparation of 4-(2-Acetamidothiazolo[5,4-*b*]pyridin-5-yl)-*N*-**methylbenzamide (35)

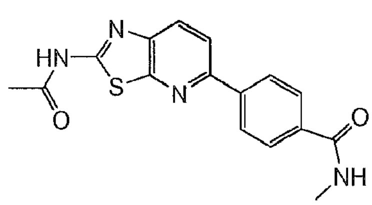

The titled compound was prepared following the procedures for the preparation of **Example 2**, except that 3-amino-N-methyl-4-(4,4,5,5-tetramethyl-1,3,2-dioxaborolan-2-yl)benzamide was used as starting material. Yield:

2.83%. $^1H$ NMR (400 MHz, MeOD) δ ppm 2.31 (s., 3 H) 2.97 (s., 3 H) 7.45-7.66 (br., 2 H) 7.96-8.20 (br., 4 H). [M+H] calc'd for $C_{16}H_{14}N_4O_2S$, 327; found, 327.

**Example 36: Preparation of *N*-(5-(4-Acetamidophenyl)thiazolo[5,4-*b*]pyridinyl)acetamide (36)**

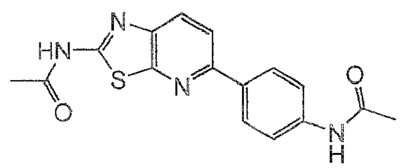

The titled compound was prepared following the procedures for the preparation of **Example 2**, except that *N*-(3-amino-4-(4,4,5,5-tetramethyl-1,3,2-dioxaborolan-2-yl)phenyl)acetamide was used as starting material. Yield: 14.5%. $^1H$ NMR (400 MHz, MeOD) δ ppm 2.20 (s., 3 H) 2.31 (s., 3 H) 7.73 (br., 2 H) 7.95-8.05 (br., 4 H). [M+H] calc'd for $C_{16}H_{14}N_4O_2S$,327; found, 327.

**Example 37: Preparation of 4-(2-Acetamidothiazolo[5,4-*b*]pyridin-5-yl)-*N*-cyclopropylbenzamide (37)**

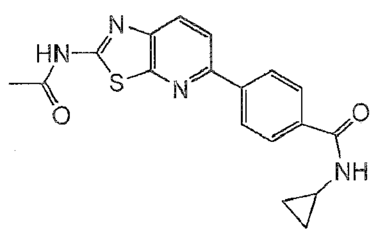

The titled compound was prepared following the procedures for the preparation of **Example 2**, except that 3-amino-N-cydopropyl-4-(4,4,5,5-tetramethyl-1,3,2-dioxaborolan-2-yl)benzamide was used as starting material. Yield: 15.5%. $^1H$ NMR (400 MHz, DMSO-$d_6$) δ ppm 0.55 - 0.65 (m, 2 H) 0.67 - 0.78 (m, 2 H) 2.30 (s, 3 H) 2.88 (m, 1 H) 7.96 (d, *J*=8.34 Hz, 2 H) 8.13 - 8.24 (m, 3 H) 8.55 (d, *J*=4.29 Hz, 1 H). [M+H] calc'd for $C_{18}H_{16}N_4O_2S$, 353; found, 353.

**Example 38: Preparation of 4-(2-Acetamidothiazolo[5,4-*b*]pyridin-5-yl)benzamide (38)**

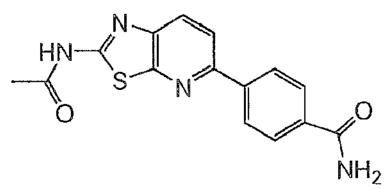

The titled compound was prepared following the procedures for the preparation of **Example 2**, except that 3-amino-4-(4,4,5,5-tetramethyl-1,3,2-dioxaborolan-2-yl)benzamide was used as starting material. Yield: 8.7%. $^1H$ NMR (400 MHz, DMSO-$d_6$) δ ppm 2.24 (s, 3 H) 8.00 (d, *J*=8.08 Hz, 2 H) 8.08 (br. s., 1 H) 8.17-8.23 (m, 3 H). [M+H] calc'd for $C_{15}H_{12}N_4O_2S$, 313; found, 313.

**Example 39: Preparation of *N*-(4-(2-Acetamidobenzo[*d*]thiazol-6-yl)phenyl)acetamide (39)**

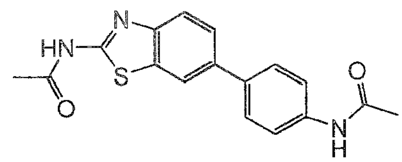

The titled compound was prepared following the procedures for the preparation of **Example 12**, using *N*-(6-bromobenzo[d]thiazol-2-yl)acetamide and *N*-(4-(4,4,5,5-tetramethyl-1,3,2-dioxaborolan-2-yl)phenyl)acetamide as starting material. Yield: 15.4%. $^1$H NMR (400 MHz, DMSO-$d_6$) δ ppm 2.08 (s, 3 H) 2.22 (s, 3 H) 7.65-7.72 (m, 5 H) 7.75 - 7.80 (m, 1 H) 8.24 (s, 1 H). [M+H] calc'd for $C_{17}H_{15}N_3O_2S$, 326; found, 326.

**Example 40: Preparation of 3-(2-Acetamidobenxo[*d*]thiaxol-6-yl)-*N*-methylbenzamide (40)**

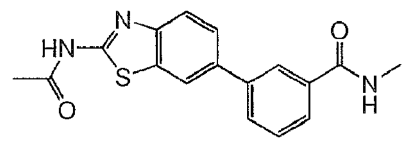

The titled compound was prepared following the procedures for the preparation of **Example 12**, using *N*-(6-bromobenzo[d]thiazol-2-yl)acetamide and *N*-methyl-3-(4,4,5,5-tetramethyl-1,3,2-dioxaborolan-2-yl)benzamide as starting material. Yield: 7.4%. $^1$H NMR (400 MHz, MeOD) δ ppm 2.30 (s, 3 H) 2.98 (s, 3 H) 7.58 (t, *J*=7.71 Hz, 1 H) 7.75 - 7.91 (m, 4 H) 8.14 - 8.22 (m, 2 H). [M+H] calc'd for $C_{17}H_{15}N_3O_2S$, 326; found, 326.

**Example 41: of *N*-(3-(2-Acetamidobetizo[*d*]thiazol-6-yl)phenyl)acetamide (41)**

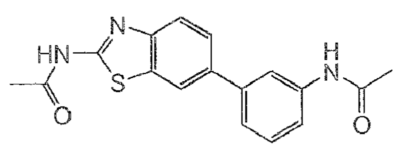

The titled compound was prepared following the procedures for the preparation of **Example 12**, using *N*-(6-broniobenzo[d]thiazol-2-yl)acetamide and *N*-(3-(4,4,5,5-tetramethyl-1,3,2-dioxaborolan-2-yl)phenyl)acetamide as starting material. Yield: 18.8%. $^1$H NMR (400 MHz, DMSO-$d_6$) δ ppm 2.08 (s, 3 H) 2.22 (s, 3 H) 7.35 - 7.43 (m, 2 H) 7.57 (d, *J*=7.07 Hz, 1 H) 7.66 (dd, *J*=8.34, 1.77 Hz, 1 H) 7.81 (d, *J*=8.34 Hz, 1 H) 7.94 (s, 1 H) 8.21 (d, *J*=1.52 Hz, 1 H). [M+H] calc'd for $C_{17}H_{15}N_3O_2S$, 326; found, 326.

**Example 42: Preparation of *N*-(5-(4-(4H-1,2,4-Triazol-3-yl)phenyl)thiazolo[5,4-*b*]pyridin-2-yl)acetamide (42)**

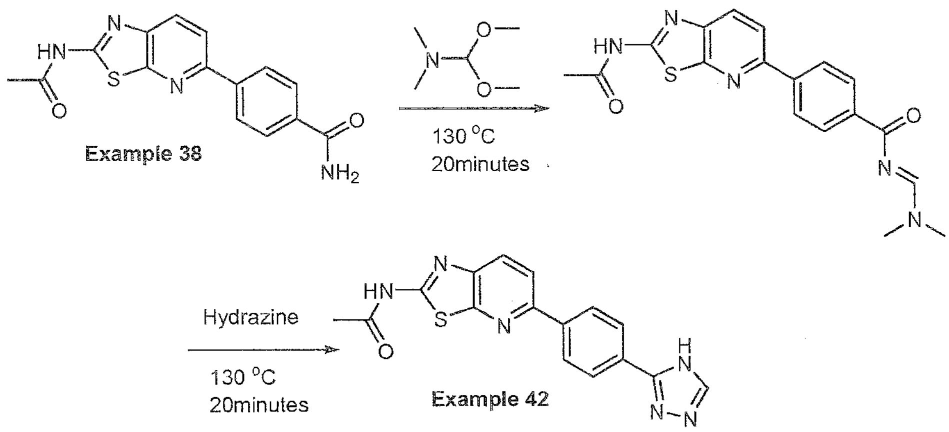


Example 38

Example 42

Example **38** (20 mg, 6.4 mmol) was dissolved in DMF-DMA (2 mL). The mixture was heated at 130˚C for 20 minutes. The volatiles were removed under vacuum, and the residue was dissolved in 2ml of acetic acid, and 8 drops of hydrazine mono hydrate were added. The mixture was heated at 130˚C for 20 minutes. The crude was purified by preparative HPLC to give the titled compound (0.5 mg, yield 2.3%). $^1$H NMR (400 MHz, MeOD) δ ppm 2.31 (s, 3 H) 8.06 (d, *J*=8.59 Hz, 1 H) 8.15 (d, *J*=8.34 Hz, 1 H) 8.17 (d, *J*=8.84 Hz, 2 H) 8.25 (d, *J*=8.84 Hz, 2 H) 8.48 (s, 1 H). [M+H] calc'd for $C_{16}H_{12}N_6OS$, 337; found, 337.

**<u>Example 43:</u> Preparation of *N*-(5-(5-Amino-6-chloropyridin-3-yl)thiazoio[5,4-d]pyrimidin-2-yl)acetamide (43)**

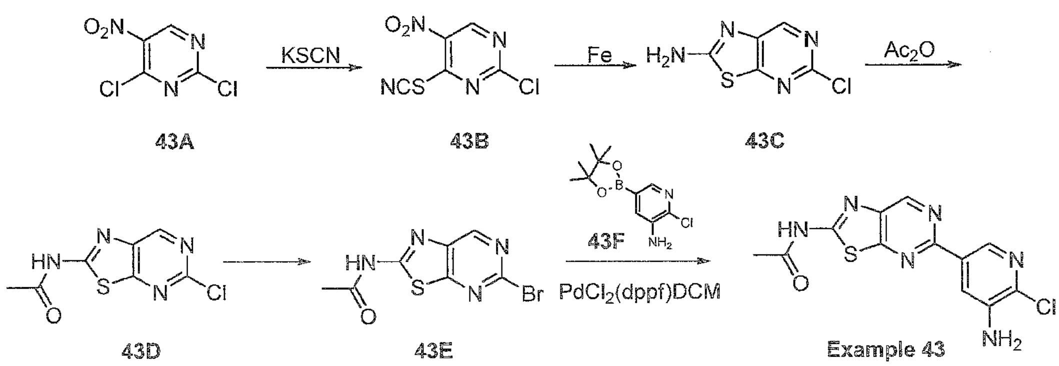


43A 43B 43C 43D 43E 43F Example 43

To a solution of 2,4-dichloro-5-nitropyrimidine (43A, 16.7 g, 85.8 mmol) in acetic acid (100 mL), was slowly added potassium thiocyanate (8.82 g, 9.08 mmol) over 1.5 h. The mixture was stirred at room temperature for 3h. An off-white precipitate started to form. The resulting suspension was poured into water (500 mL) and was stirred for 30 min. The off-white solid was collected by filtration, well washed with water, and then dried in vacuo. The crude material 2-Chloro-5-nitro-4-thiocyanatopyrimidine **(43B,** 16.5 g, 89%) was essentially pure and was used in the next step without further purification. $^1$H NMR (300 MHz, DMSO-$d_6$): δ 9.32 (s, 1H).

To a hot solution of 2-chloro-5-nitropyrimidin-4-yl thiocyanate **(43B,** 1.00 g, 4.64 mmol) in acetic acid (30 mL) at 120 ˚C, was added iron powder (1.40 g, 25.1 mmol). The mixture was stirred for 30 min at the same temperature. The mixture was allowed to cool to room temperature. Insoluble materials were removed by filtration and then well washed with acetic acid. The filtrate was concentrated under reduced pressure. The residue was dissolved in ethyl acetate (150 mL) and THF (50 mL), and was washed with saturated aqueous solution of $NH_4Cl$ (100 mL), saturated aqueous solution of $NaHCO_3$ (100 mL), and dried over anhydrous $MgSO_4$. Insoluble materials were removed by filtration and the filtrate was concentrated in vacuo. The resulting yellow crystalline material of 5-Chlorothiazolo[5,4-*d*]pyrimidin-2-amine (43C, 870 mg, quantitative) was essentially pure and was used in the next step without further purifications. $^1$H NMR (400 MHz, DMSO-$d_6$): δ 8.54 (s, 1H), 8.30 (br s, 2H).

To a solution of 43C (870 mg, ca 4.64 mmol) in pyridine (100 mL), was added acetic anhydride (1mL, 10.6 mmol) and DMAP (29.5 mg, 0.24 mmol). The mixture was stirred for 2h at room temperature. The reaction was quenched

by the addition of methanol (10 mL) and the volatiles were removed under reduced pressure. The residue was dissolved in ethyl acetate (50 mL), the solution was washed with water (30 mL), 0.1 N hydrochloric acid (30 mL) and saturated aqueous solution of $NaHCO_3$ (30 mL). The organic layer was dried over anhydrous $MgSO_4$ and the insoluble materials removed by filtration. Concentration of the filtrate afforded an off white solid, which was recrystallized from ethanol to yield the titled compound N-(5-chlorothiazolo[5,4-*d*]pyrimidin-2-yl)acetamide **(43D)** as an off-white crystalline solid (238 mg, 22% in 2 steps). A second crop **of43D** was obtained from mother liquid (250 mg, 24%). $^1$H NMR (300 MHz, DMSO-$d_6$): δ 12.86 (br s, 1H), 9.09 (s, 1H), 8.71 (s, 1H), 2.25 (s, 3H). MS (ES) [M+H] calc'd for $C_7H_5ClN_4OS$, 229; found 229.

A mixture **of 43D** (3.20 g, 14.0 mmol) and 25% hydrogen bromide in acetic acid (100 mL) was stirred for 3 h at 80 °C. After cooling to room temperature, 25% hydrogen bromide in acetic acid (20 mL) was added, and the mixture was heated to 80 °C again. After 2 h, the mixture was concentrated in vacuo. The solid formed was triturated with EtOAc, and the solid was collected by filtration and washed with EtOAc to afford the titled compound *N*-(5-bromothiazolo[5,4-*d*] pyrinidin-2-yl)acetamide (43E, 4.86 g, 98%) as a white solid. $^1$H NMR (300 MHz, DMSO-$d_6$): δ 12.87 (br s, 1H), 9.05 (s, 1H), 8.50 (br s, 1H), 2.26 (s, 3H).. MS (ES) [M+H] calc'd for $C_7H_5BrN_4OS$, 273, 275; found 273, 275.

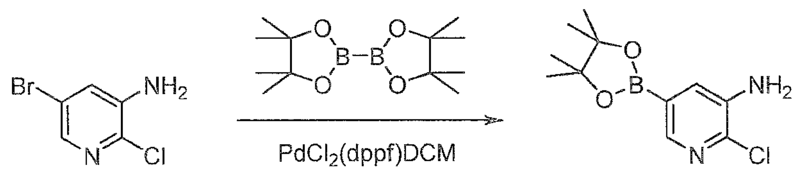


43F

To a solution of 5-bromo-2-ehloropyridin-3-amine (2.07 g, 10 mmol) in DME (50 mL) were added bis(pinaeolato) diboron (3.05 g, 12 mmol), fd(dppf)$Cl_2$ $CH_2Cl_2$ (245 mg, 0.3 mmol), and potassium acetate (2.94 g, 30 mmol). The mixture was stirred overnight at 80 °C under Ar and then allowed to cool to room temperature. Ethyl acetate (200 mL) and water (100 mL) were added to the mixture. Insoluble materials were removed by filtration. Two phases were separated and the aqueous phase was extracted with ethyl acetate (100 mL). The organic extracts were combined and washed with brine (100 mL) and dried over anhydrous $MgSO_4$ Insoluble materials were removed by filtration and the filtrate was concentrated in vacuo. The crude product was purified by silica gel column chromatography eluted with a 10%--40% mixture of ethyl acetate and hexane. After concentration of appropriate fractions, the residual solid was collected by filtration and washed with hexane to yield titled compound 2-Chloro-5-(4,4,5,5-tetramethyl-1,3,2-dioxaborolan-2-yl)pyridin-3-amine (**43F**, 1.11 g, 43%) as white crystalline solid. $^1$H NMR (300 MHz, $CDCl_3$): δ 8.13 (d, 1 H, J = 1.6 Hz), 7.40 (d, 1 H, J = 1.6 Hz), 4.02 (br s, 2 H), 1.34 (s, 12 H). MS (ES) [M+H] calc'd for CUH16BCIN202, 255; found 255.

A mixture off (4.86 g, 13.8 mmol), 2-chloro-5-(4,4,5,5-tetramethyl-l,3,2-dioxaborolan-2-yl)pyridin-3-amine (3.20 g, 12.6 mmol), 1,1'-bis(diphenylphosphino)ferrocene-palladium(II)dichloride dichloromethane complex (2.07 g, 2.51 mmol), cesium carbonate (16.4 g, 50.3 mmol), DME (113 mL) and water (30 mL) was stirred for 3 h at 80 °C under Ar. The mixture was poured onto EtOAc-THF (3:1, v/v, 400 mL) and water (200 mL). The insoluble materials were filtered off and washed with EtOAc-THF (3:1, v/v, 800 mL). The filtrate was let to stay at room temperature for 1 h and a precipitate was emerged. The precipitate was collected by filtration and washed with EtOAc and water to afford the titled compound as a pale gray solid (0.58 g, 14.38 % yield). From the filtrate, the organic layer was collected, dried over $MgSO_4$ and concentrated in vacuo. The residue was treated with THF (~30 mL) and the precipitate was collected by filtration to afford the second crop of the titled compound *N*-(5-(5-Amino-6-chloropyridin-3-yl)thiazolo[5,4-d]pyrimidin-2-yl)acetamide (Example **43,** 1.81 g, 44.9 % yield) as a pale yellow solid. $^1$H NMR (300 MHz, DMSO-$d_6$): δ 12.79 (1H, brs), 9.23 (1H, s), 8.56 (1H, d, J = 2.4 Hz),, 8.15 (1H, d, J = 3.9 Hz), 5.82 (2H, brs), 2.26 (3H, s). MS (ES) [M+H] calc'd for $C_{12}H_9ClN_6OS$, 321; found 321.

**<u>EXagmgle 44:</u> Preparation of *N*-(5-(6-CMoro-5-(methylsulfonamido)pyridin-3-yl)thiazolo[5,4-*d*]pyrimidin-2-yl) acetamide (44)**

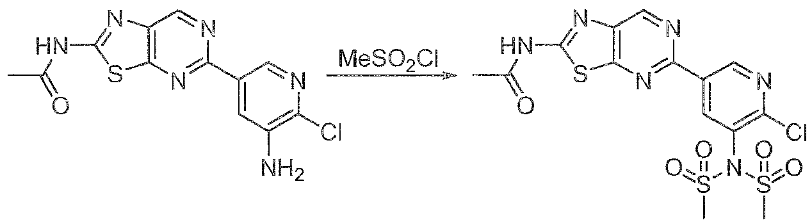


Example 43 44A

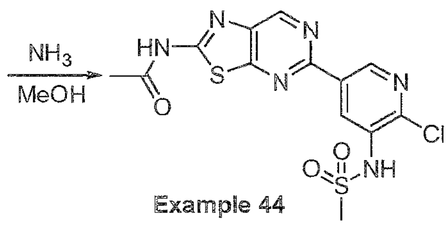


Example 44

Methanesulfonyl chloride (0.362 mL, 4.68 mmol) was added a suspension of **Example 43** (500 mg, 1.559 mmol) and $Et_3N$ (0.84 mL, 6 mmol) in THF (30 ml). After h, water (60 mL) and EtOAc (60 mL) were added. The organic layer was collected, dried over $MgSO_4$ and concentrated in vacuo. The residue was purified by column chromatography (Si60, charged with EtOAc-THF-toluene, hexane-EtOAc = 95:5 - EtOAc only, then EtOAc - EtOAc-MeOH = 7:3) then the obtained solid was washed with EtOAc-hexane (1:1, v/v) to afford the titled compound (*N*-(5-(6-Chloro-5-(N-(methylsulfonyl)methylsulfonamido)pyridin-3-yl)thiazolo[5,4-*d*]pyrimidin-2-yl)acetamide **(44A,** 291 mg, 39.1 % yield) as a pale yellow solid. $^1H$ NMR (300 MHz, DMSO-$d_6$): δ 12.86 (1H, brs), 9.43 (1H, d, J = 2.1 Hz), 9.29 (1H,s), 8.92 (1H, d, J = 2.1 Hz), 3.69 (6H, s), 2.27 (3H, s). MS (ES) [M+H] calc'd for $C_{14}H_{13}ClN_6O_5S_3$, 477; found 477.

A suspension of **44A** (291 mg, 0.610 mmol) in 8M ammonia in methanol (8 mL) was stirred for 1 h. After removal of the solvent in vacuo, EtOH (4 mL) and MeOH (4mL) were added to the residue and concentrated in vacuo. The residue was dissolved in THF (~15 mL) and a small amount of water and charged onto Si60 column (10 g). Elution with EtOAc was done. First fraction (~200 ml) was collected and concentrated in vacuo. The residue was treated with water-MeOH and the precipitate was collected. MeOH and water were added to the precipitate again and heated for 15 min at 70 °C. After cooling, the precipitate was collected by filtration and washed with MeOH to afford the titled compound *N*-(5-(6-Chloro-5-(methylsulfonamido)pyridin-3-yl)thiazolo[5,4-*d*]pyrimidin-2-yl)acetamide (Example **44,** 180 mg, 74.0%) as a pale yellow solid. $^1H$ NMR (300 MHz, DMSO-$d_6$): δ 12.83 (1H, brs), 9.91 (1H, brs), 9.29 (1H, s), 9.17 (1H, d, J = 2.4 Hz), 8.77 (1H, d, J = 1.8 Hz), 3.17 (3H, s), 2.27 (3H, s),. MS (ES) [M+H] calc'd for $C_{13}H_{11}ClN_6O_3S_2$ 399; found 399.

## <u>Example 45:</u> Preparation of *N*-(5-(6-Aminopyridin-3-yl)thiazolo[5,4-*d*]Apyrimidin-2-yl)acetamide (45)

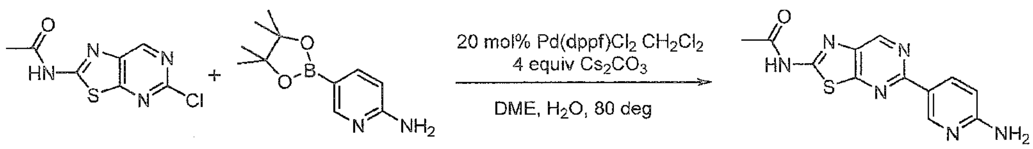


43D 45A Example 45

To a solution of **43D** (114 mg, 0.5 mmol) in DME (5 mL) was added 5-(4,4,5,5-tetramethyl-1,3,2-dioxaborolan-2-yl)pyridin-2-amine **45A** (220 mg, 1.0 mmol), Pd(dppf)$Cl_2$ $CH_2Cl_2$ (82 mg, 0.1 mmol), and 2.0 M aqueous solution of $Cs_2CO_3$ (1 mL). The mixture was stirred overnight at 80 °C under Ar and then allowed to cool to room temperature. Ethyl acetate (50 mL), THF (50 mL) and water (20 mL) were added to the mixture. Insoluble materials were removed by filtration. Two phases were separated and the aqueous phase was extracted with ethyl acetate (50 mL) and THF (50 mL). The organic extracts were combined and washed with brine (50 mL) and dried over anhydrous $MgSO_4$. Insoluble materials were removed by filtration and the filtrate was concentrated in vacuo. The crude product was purified by amino silica gel column chromatography eluted with a 0%--50% mixture of methanol and ethyl acetate. After concentration of the appropriate fractions, the residue was purified by silica gel column chromatography eluting with a 0%--20% mixture of methanol and ethyl acetate. After concentration of the appropriate fractions, the residual solid was collected by filtration

and washed with ethyl acetate to yield the titled compound (13 mg, 9%) as brown crystalline solid. $^1$H NMR (300 MHz, DMSO-$d_6$): δ 12.62 (br s, 1 H), 9.08 (s, 1 H), 8.97 (s, 1 H), 8.31 (d, 1 H, J = 8.7 Hz), 6.54 (d, 1 H, J = 8.9 Hz), 6.49 (s, 2 H), 2.24 (s, 3 H). MS (ES) [M+H] calc'd for $C_{12}H_{10}N_6OS$ 287; found 287.

**Example 46: Preparation of *N*-(5-(6-Amino-5-(trifluoromethyl)pyridin-3-yl)thiazolo[5,4-*d*]pyrimdia-2-yl)acetamde (46)**

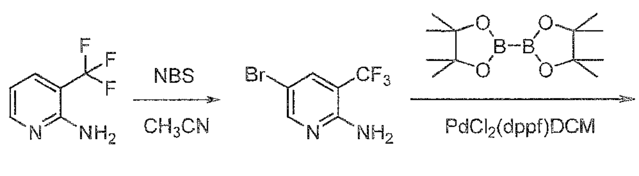


46A 46B

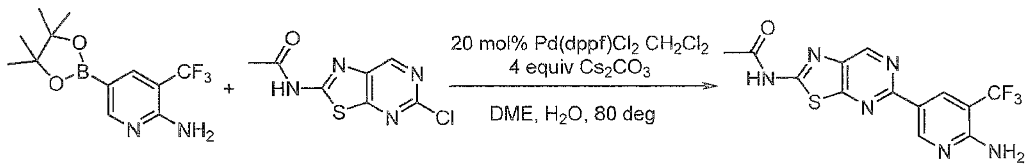


46C 43D Example 46

To a solution of 3-(trifluoromethyl)pyridm-2-amine (**46A,** 2.10 g, 13 mmol) in acetonitrile (40 mL) was added N-bromosuceinimide (2.54 g, 14.2 mmol). The mixture was stirred overnight at room temperature. The reaction mixture was poured into water (50 mL) and extracted with ethyl acetate (100 mL x 2). The organic extract was washed with brine (50 mL) and dried over anhydrous $MSO_4$. Insoluble materials were removed by filtration and the filtrate was concentrated in vacuo. The crude product was purified by silica gel column chromatography eluting with a 10%--30% mixture of ethyl acetate and hexane. Concentration of appropriate fractions afforded the titled compound 5-Bromo-3-(trifluoromethyl) pyridin-2-amW (**46B,** 2.91 g, 93%) as pale yellow crystalline solid. $^1$H NMR (300 MHz, $CDCl_3$): δ 8.26 (d, 1 H, J =1.9 Hz), 7.79 (d, 1 H, =1.9 Hz), 5.00 (br s, 2 H). MS (ES) [M+H] calc'd for $C_6H_4ErF_3N_2$ 241; found 241.

To a solution of 5-bromo-3-(trifluoromethyl)pyridin-2-amine **(46B,** 1.21 g, 5.0 mmol) in DME (50 mL) were added bis(pinacolato)diboron (1.65 g, 6.5 mmol), $Pd(dppf)Cl_2$ $CH_2Cl_2$ (122 mg, 0.15 mmol), and potassium acetate (1.47 g, 15 mmol). The mixture was stirred overnight at 80 ˚C under Ar and then allowed to cool to room temperature. Insoluble materials were removed by filtration and washed with ethyl acetate (100 mL). The filtrate was concentrated in vacuo. The crude product was purified by silica gel column chromatography eluting with a 20%--50% mixture of ethyl acetate and hexane. After concentration of appropriate fractions, the residue was dissolved in ethyl acetate (100 mL), and the solution was washed with saturated aqueous solution of $NaHCO_3$ (50 mL). The aqueous phase was extracted with ethyl acetate (50 mL). The organic extracts were combined and washed with brine (50 mL) and dried over anhydrous $MgSO_4$. Insoluble materials were removed by filtration and the filtrate was concentrated in vacuo. The residue was re-ciystallized from hexane to yield 5-(4,4,5,5-tetramethyl-1,3,2-dioxaborolan-2-yl)-3-(trifluoromethyl)pyridin-2-amine (**46C** 1.04 g, 72%) as white crystalline solid. $^1$H NMR (300 MHz, $CDCl_3$): δ 8.56 (d, 1 H, J = 0.9 Hz), 8.07 (d, 1 H, J = 0.8 Hz), 5.15 (br s, 2 H), 1.33 (s,12 H). MS (ES) [M+H] calc'd for $C_6H_6BF_3N_2O_2$ (in situ hydrolysis of the titled compound to the boronic acid on LC), 207; found 207.

To a solution of N-(5-chloro[1,3]tlilazolo[5,4-d]pyrimidin-2-yl)acetainide (343 mg, 1.5 mmol) in DME (10 mL), was added 5-(4,4,5,5-tetramefhyl-l,3,2-dioxaborolan-2-yl)-3-(trifluoromethyl)pyridin-2-amine (432 mg, 1.5 mmol), Pd $(dppf)Cl_2$ $CH_2Cl_2$ (245 mg, 0.3 mmol), and 2.0 M aqueous solution of $Cs_2CO_3$; (2.5 mL). The mixture was stirred for 8 h at 80 ˚C under Ar and then allowed to cool to room temperature. Ethyl acetate (100 mL) and water (50 mL) were added to the mixture. Insoluble materials were removed by filtration. Two phases were separated and the aqueous phase was extracted with ethyl acetate (100 mL). The organic extracts were combined and washed with brine (100 mL) and dried over anhydrous $MgSO_4$. Insoluble materials were removed by filtration and the filtrate was concentrated in vacuo. The crude product was purified by silica gel column chromatography eluting with a 0%--20% mixture of methanol and ethyl acetate. After concentration of the appropriate fractions, the residual solid was collected by filtration and washed with acetone and ethyl acetate to yield the titled compound *N*-(s-(6-amino-s-(trifluoromethyl)pyridin-3-yl)thiazolo[5,4-*d*]pyrimidin-2-yl)acetamide (**Example 46,** 301 mg, 57%) as white crystalline solid. $^1$H NMR (300 MHz, DMSO-$d_6$): δ 12.71 (br s, 1 H), 9.20 (d, 1 H, J = 1.7 Hz), 9.14 (s, 1 H), 8.61 (d, 1 H, J = 1.9 Hz), 7.05 (s, 2 H), 2.25 (s, 3 H). MS (ES) [M+H]

calc'd for $C_{13}H_9F3N_6OS$ 355; found 355.

**Example_47: Preparation of *N*-(5-(6-Chloro-5-(ethylsulfonamido)pyridin-3-yl)thiazolo[5,4-*b*]pyridin-2yl)acetamide (47)**

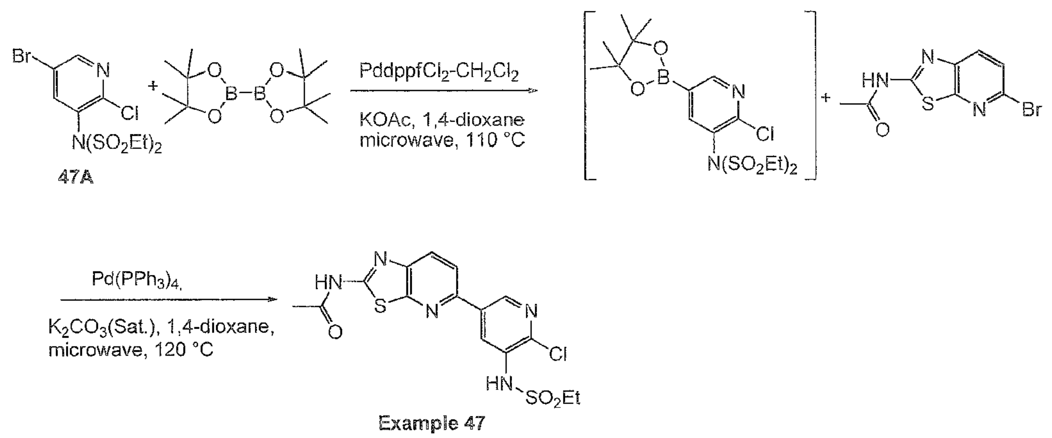


Example 47

A mixture of N-(5-bromo-2-chloropyridin-3-yl)-N-(ethylsulfonyl)ethanesulfonamide (**47A,** 1g, 2.55 mmol, prepared following the same procedure for the preparation of 7A), bispinacolatodiboron (0.65 g, 2.76 mmol), PddppfCl$_2$-DCM (0.52 g, 0.637 mmol), and KOAc (0.75 g, 7.65 mmol) in 1,4-dioxane (15 mL) was heated in a microwave at 110 ˚C for 45 minutes. The crude was used without further purification.

To 2 mL of the above crude reaction mixture was added **Example 1** (74 mg, 0.27 mmol), $Pd(PPh_3)_4$ (78 mg, 0.067 mmol), $K_2CO_3$ (sat., 1 mL). The reaction was heated in a microwave at 120 ˚C for 20 minutes. The reaction mixture was purified using preparative HPLC to give the titled compound as a yellowish solid (15 mg). $^1$H NMR (400 MHz, DMSO-*d*$_6$) δ ppm 8.98 (s, 1H), 8.54 (s, 1H), 8.22 (d, *J*=8.6 Hz, 1H), 8.17 (d, *J*=8.6 Hz, 1H), 3.25 (q, *J*= 7.33 Hz, 2H) 2.25 (s, 3H), 1.31 (t, *J*= 7.33 Hz, 3H); [M+H] calc'd for $C_{15}H_{14}ClN_5O_3S_2$, 412; found, 412.

**Example 48: Preparation of *N*-(6-(6-Chloro-5-(methylsulfonamido)pyridin-3-yl)benzo[*d*]thiazol-2-yl)acetamide (48)**

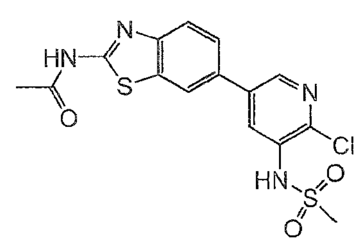

The titled compound was prepared following the procedure described in Example 8 except that N-(2-chloro-5-(4,4,5,5-tetramethyl- 1,3,2-dioxaborolan-2-yl)pyridin-3-yl)methanesulfonamide was used in the final step to yield the titled compounds **48.** $^1$H NMR (400 MHz, DMSO-*d*$_6$) δ ppm 12.35 (s, 1H), 8.20 (d, *J*=4 Hz, 1H), 8.03 (s, 1H), 7.84 (d, *J*=4 Hz, 1H), 7.75 (d, *J*=8 Hz, 1H), 7.60 (dd, *J*=8, 4 Hz, 1H), 2.80 (s, 3H), 2.15 (s, 3H); [M+H] calc'd for $C_{15}H_{13}ClN4O_3S_2$, 396; found, 396.

**Example 49: Preparation of *N*-(5-(6-Chloro-5-(cyclopropanesulfonamido)pyridin-3-yl)thiazolo[5,4-*b*]pyridin-2-yl)acetamdie (49)**

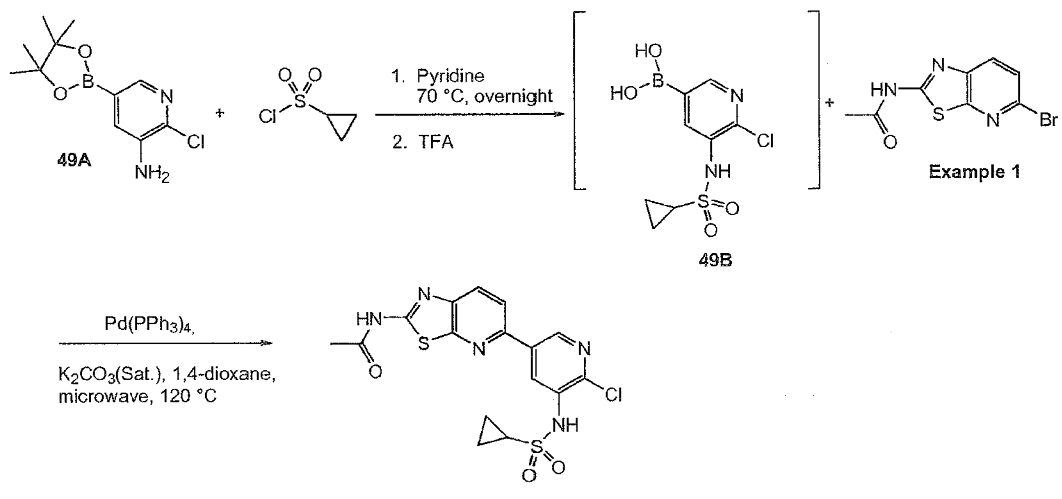


Example 49

2-Chloro-5-(4,4,5,5-tetramethyl-1,3,2-dioxaborolan-2-yl)pyridin-3-amine **(49A,** 100 mg, 0.39 mmol), cyclopropanesulfonyl chloride (221 mg, 1.6 mmol) were mixed in 2 mL pyridine. The mixture was heated at 70 ˚C overnight. The crude was purified by preparative HPLC (0.05% TFA in mobile phase) to give 6-chloro-5-(cyclopropanesulfonamido) pyridin-3-ylboronic acid **(49B,** 87 mg), which was mixed with **Example 1** (86 mg, 0.32 mmol), $Pd(PPh_3)_4$ (91.1 mg, 0.079 mmol), $K_2CO_3$ (sat., 1 mL) in 2 mL of 1,4-dioxane. The reaction mixture was heated in a microwave at 120 ˚C for 20 minutes. The reaction mixture was purified using preparative HPLC to give the titled compound as a yellowish solid (5 mg). $^1$H NMR (400 MHz, DMSO-$d_6$) δ ppm 8.39 (d, *J*=8.0 Hz, 1H), 8.35 (d, *J*=8.0 Hz, 1H), 8.22 (d, *J*=8.0 Hz, 1H), 8.04 (d, *J*=8.0 Hz, 1H), 2.79 (m, 1H) 2.25 (s, 3H), 1.02 (m, 2H), 0.94 (m, 2H); [M+H] calc'd for $C_{16}H_{14}ClN_5O_3S_2$, 424; found, 424.

**<u>Example 50.</u> Preparation of *N*-(5-(6-Chloro-5(phenylsulfonamido)pyridin-3-yl)thiazolo[5,4-*d*]pyrimidin-2yl) acetamide (50)**

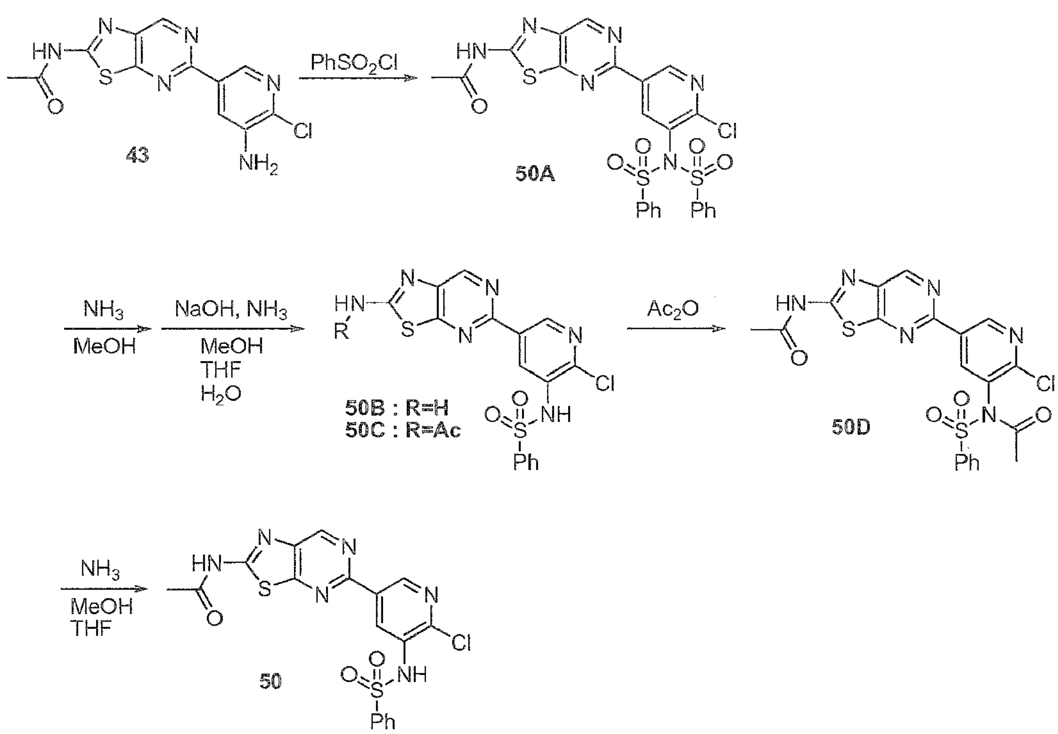

A mixture of *N*-(5-(5-Amino-6-chloropyridin-3-yl)thiazolo[5,4-d]pyrimidin-2-yl)acetamide **(43,** 642 mg, 2.0 mmol), benzenesulfonyl chloride (0.766 mL, 6.0 mmol) and pyridine (10 mL) was stirred at room temperature overnight. After removal of the solvent in vacuo, 8 M ammonia in methanol (10 mL) was added. The mixture was stirred at room temperature overnight. After removal of the solvent in vacuo, MeOH (20 mL), THF (10 mL) and 1 N sodium hydroxide solution (2 mL) were added. The mixture was stirred at room temperature overnight. 1 N Hydrochloric acid (2 mL) was added and the solvent was removed in vacuo. The residue was dissolved in MeOH (50 mL) and THF (100 mL). 1N Sodium hydroxide solution (1 mL) was added and the mixture was stirred at room temperature for 2.5 h. 28% Ammonium hydroxide solution (2 mL) was added and the mixture was stirred at room temperature for 2 h. 28% Ammonium hydroxide solution (3 mL) was added and the mixture was stirred at room temperature overnight. 28% Ammonium hydroxide solution (5 mL) was added and the mixture was stirred at room temperature for 8 h. 8 N Sodium hydroxide solution (3 mL) was added and the mixture was stirred at room temperature for 0.5 h. The mixture was neutralized with 1N hydrochloric acid and then poured into water and extracted with ethyl acetate and THF. The organic extract was washed with brine and dried over anhydrous $MgSO_4$. Insoluble materials were removed by filtration and the filtrate was concentrated in vacuo. The crude product was purified by silica gel column chromatography eluting with a 0%-5% mixture of MeOH and ethyl acetate. Concentration of the appropriate fractions afforded a mixture of **50B** and **50C** (792 mg) as a yellow solid.

To the above obtained mixture of **50B** and **50C** (792 mg) in pyridine (5 mL) was added $Ac_2O$ (0.070 mL). The mixture was stirred at 50 ˚C for 0.5 h. $Ac_2O$ (0.19 mL) was added and the mixture was stirred at 50 ˚C for 1 h. The resulting mixture was concentrated in vacuo. The residue in pyridine (5 mL) was added $Ac_2O$ (0.20 mL). The mixture was stirred at 50 ˚C for 1 h. $Ac_2O$ (0.40 mL) was added and the mixture was stirred at 50 ˚C for 1 h. After removal of the solvent in vacuo, the residue was poured into aqueous solution of $NaHCO_3$ and extracted with ethyl acetate (x2). The organic extract was washed with brine and dried over anhydrous $MgSO_4$. Insoluble materials were removed by filtration and the filtrate was concentrated in vacuo. The crude product was purified by silica gel column chromatography eluting with a 0%--20% mixture of MeOH and ethyl acetate. After concentration of the appropriate fractions, the residual solid was collected by filtration and washed with diisopropyl ether to yield **50D** (465 mg, 46%) as a pale yellow solid. A mixture of **50D** (251 mg, 0.5 mmol), 8 M ammonia in methanol (3 mL) and THF (12 mL) was stirred at room temperature for 2 h. After removal of the solvent in vacuo, the residue was poured into aqueous water and extracted with ethyl acetate (x2). The organic extract was washed with brine and dried over anhydrous $MgSO_4$. Insoluble materials were removed by filtration and the filtrate was concentrated in vacuo. The residual solid was collected by filtration and washed with ethyl acetate and diisopropyl ether to yield the titled compound N-(5-(6-Chloro-5-(phenylsulfonamido)pyridin-3-yl)thiazolo [5,4-d]pyrimidin-2-yl)acetamide **(50,**204 mg, 89 % yield) as a off-white solid. $^1$H NMR (300 MHz, DMSO-$d_6$) δ ppm 12.84 (s, 1H), 10.52 (s, 1H), 9.27 (s, 1H), 9.14 (d, *J*=2.3 Hz, 1H), 8.66 (d, *J*=2.3 Hz, 1H), 7.74-7.83 (m, 2H), 7.65-7.73 (m, 1H), 7.55-7.64 (m, 2H), 2.28 (s, 3H); [M+H] calc'd for $C_{18}H_{13}ClN_6O_3S_2$, 461; found 461.

In addition, the above reaction schemes and variations thereof can be used to prepare the following compounds. It is understood that recitation of a compound is intended to encompass all of the different possible stereoisomers.

| | |
|---|---|
| 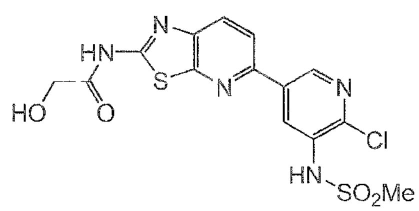<br>N-(5-(6-chloro-5-(methylsulfonamido) pyridin-3-yl) thiazolo[5,4-b]pyridin-2-yl)-2-hydroxyacetamide | 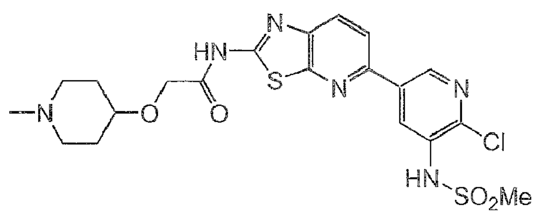<br>N-(5-(6-chloro-5-(methylsulfonamido)pyridin-3-yl) thiazolo[5,4-b]pyridin-2-yl)-2-(1-methylpiperidin-4-yloxy) acetamide |
| 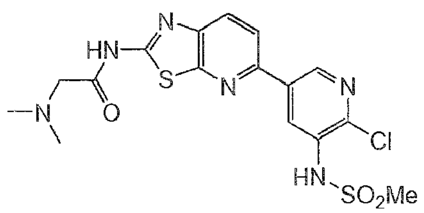<br>N-(5-(6-chloro-5-(methylsulfonamido) pyridin-3-yl) thiazolo[5,4-b]pyridin-2-yl)-2-(dimcthylamino)acctamidc | 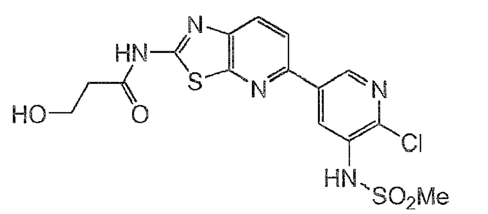<br>N-(5-(6-chloro-5-(methylsuifonaniido)pyridin-3-yl) thiazolo[5,4-b]pyridin-2-yl)-3-hydroxypropanamide |

(continued)

| | |
|---|---|
| 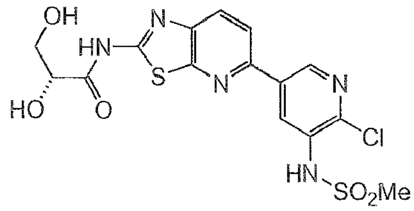<br>(R)-N-(5-(6-chloro-5-(methylsulfonamido) pyridin-3-yl) thiazolo[5,4-b]pyridin-2-yl)-2,3-dihydroxypropanamide | 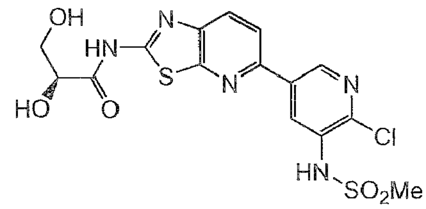<br>(S)-N-(5-(6-chloro-5-(methylsulfonamido)pyridin-3-yl) thiazolo[5,4-b]pyridin-2-yl)-2,3-dihydroxypropanamide |
| 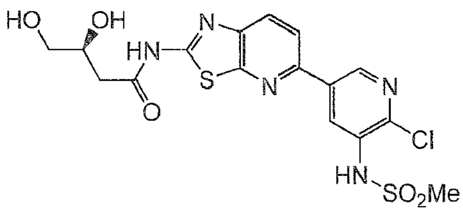<br>(S)-N-(5-(6-chloro-5-(methylsulfonamido)pyridin-3-yl) thiazolo[5,4-b]pyridin-2-yl)-3,4-dihydroxybutanamide | 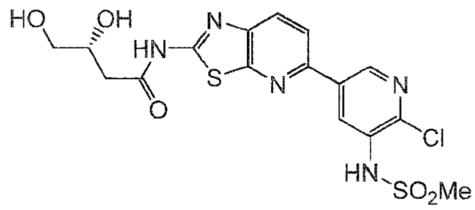<br>(R)-N-(5-(6-chloro-5-(methylsulfonamido)pyridin-3-yl) thiazolo[5,4-b]pyridin-2-yl)-3,4-dihydroxybutanamide |
| 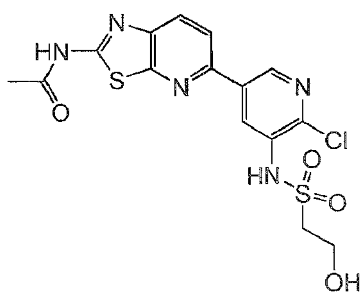<br>N-(5-(6-chloro-5-(2-hydroxyethylsulfonamnido)pyridin-3-yl)thiazolo[5,4-b]pyridin-2-yl)acetamide | 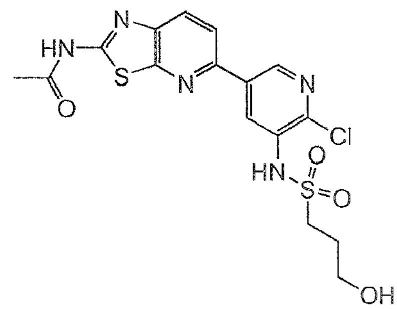<br>N-(5-(6-chloro-5-(3-hydroxypropylsulfonamido)pyridin-3-yl)thiazolo[5,4-b]pyridin-2-yl)acetamide |
| 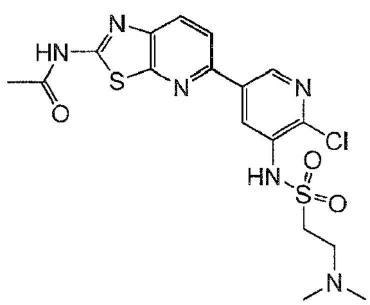<br>N-(5-(6-chloro-5-(2-(dimethylamino)ethylsulfonamido) pyridin-3-yl)thiazolo[5,4-b]pyridin-2-yl)acetamide | 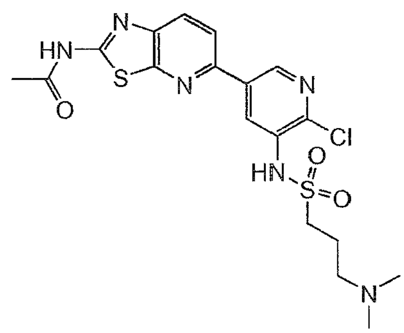<br>N-(5-(6-chloro-5-(3-(dimethylamino)propylsulfonamido) pyridin-3-yl)thiazolo[5,4-b]pyridin-2-yl)acetamide |

(continued)

| | |
|---|---|
| 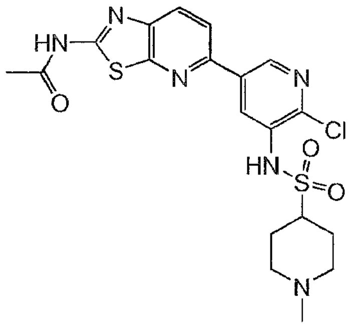<br>N-(5-(6-chloro-5-(1-methylpiperidine-4-sulfonamido) pyridin-3-yl)thiazolo[5,4-b]pyridin-2-yl)acetamide | 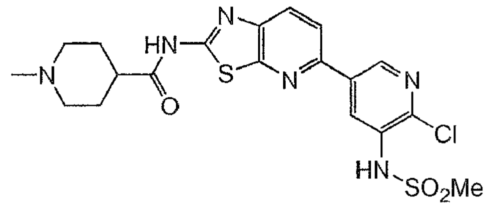<br>N-(5-(6-chloro-5-(methylsulfonamido)pyridin-3-yl) thiazolo[5,4-b]pyridin-2-yl)-1-methylpiperidine-4-carboxamide |
| 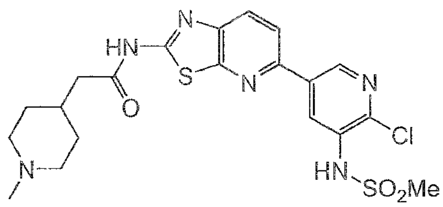<br>N-(5-(6-chloro-5-(methylsulfonamido) pyridin-3-yl) thiazolo[5,4-b]pyridin-2-yl)-2-(1-methylpiperidin-4-yl) acetamide | 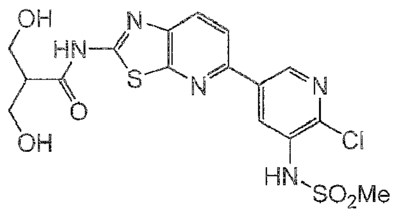<br>N-(5-(6-chloro-5-(methylsulfonamido)pyridin-3-yl) thiazolo[5,4-b]pyridin-2-yl)-3-hydroxy-2-(hydroxymethyl) propanamide |
| 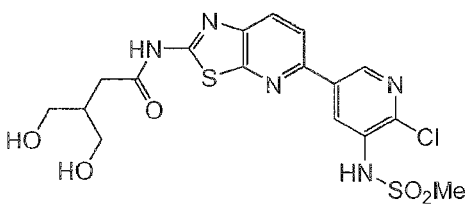<br>N-(5-(6-chloro-5-(methylsu!fonamido)pyndm-3-yl) thiazolo[5,4-b]pyridin-2-yl)-4-hydroxy-3-(hydroxymethyl) butanamide | 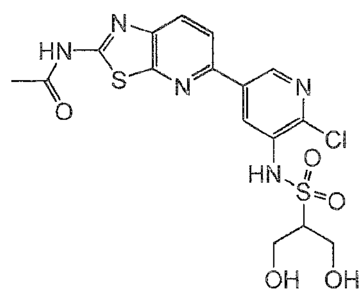<br>N-(5-(6-chloro-5-(2-bydroxy-1-(hydroxymethyl) ethylsulfonamido)pyridin-3-yl)thiazolo[5,4-b]pyridin-2-yl) acetamide |
| 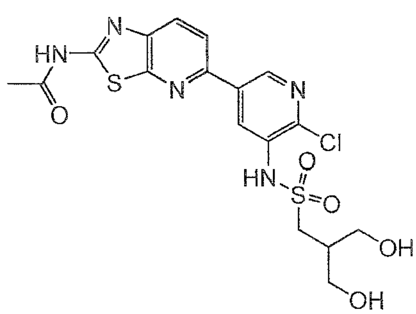<br>N-(5-(6-chloro-5-(3-hydroxy-2-(hydroxymethyl) propylsulfonamido)pyridin-3-yl)thiazolo[5,4-b]pyridin-2-yl)acetamide | 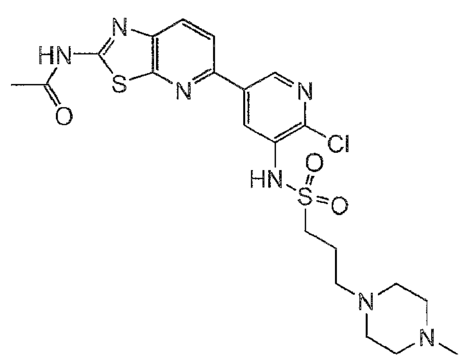<br>N-(5-(6-chloro-5-(3-(4-methylpiperazin-1-yl) propylsulfonamido)pyridin-3 -yl)thiazolo[5,4-b]pyridin-2-yl)acetamide |

(continued)

| | |
|---|---|
| 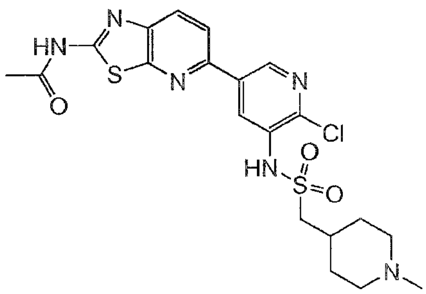<br>N-(5-(6-chloro-5-((1-methylpiperidin-4-yl) methylsulfonamido)pyridin-3-yl)thiazolo[5,4-b]pyridin-2-yl)acetamide | 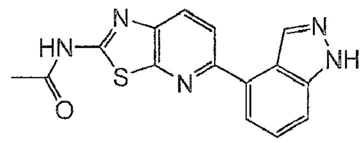<br>N-(5-(1H-indazol-4-yl)thiazolo[5,4-b]pyridin-2-yl) acetamide |
| 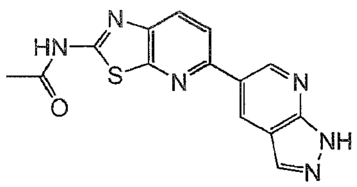<br>N-(5-(1H-pyrazolo[3,4-b]pyridin-5-yl)thiazolo[5,4-b] pyridin-2-yl)acetamide | 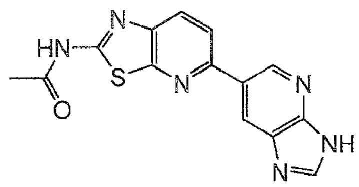<br>N-(5-(3H-imidazo[4,5-b]pyridin-6-yl)thiazolo[5,4-b] pyridin-2-yl)acetamide |
| 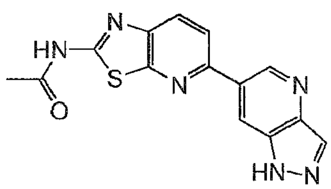<br>N-(5-(1H-pyrazolo[4,3-b]pyridin-6-yl)thiazolo[5,4-b] pyridin-2-yl)acetamide | 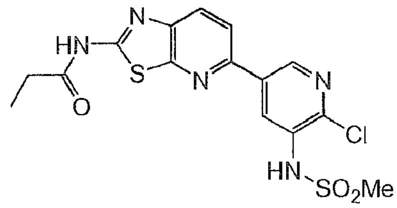<br>N-(5-(6-chloro-5-(methylsulfonamido)pyridin-3-yl) thiazolo[5,4-b]pyridin-2-yl)propionamide |
| 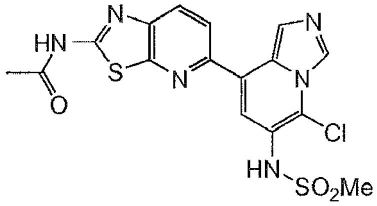<br>N-(5-(5-chloro-6-(methylsulfonamido)imidazo[1,5-a] pyridin-8-yl)thiazolo[5,4-b]pyridin-2-yl)acetamide | 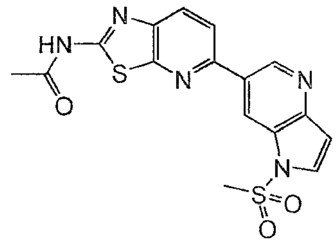<br>N-(5-(1-(methylsulfonyl)-1H-pyrrolo[3,2-b]pyridin-6-yl) thiazolo[5,4-b]pyridin-2-yl)acetamide |
| 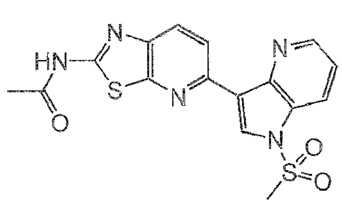<br>N-(5-(1-(methylsulfonyl)-1H-pyrrolo[3,2-b]pyridin-3-yl) thiazolo[5,4-b]pyridin-2-yl)acetamide | 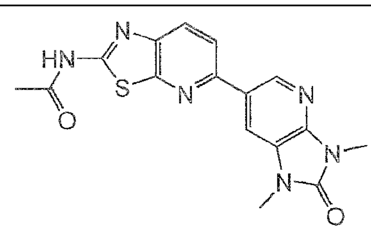<br>N-(5-(1,3-dimethyl-2-oxo-2,3-dihydro-1H-imidazo[4,5-b] pyridin-6-yl)thiazolo[5,4-b]pyridin-2-yl)acetamide |

(continued)

| | |
|---|---|
| 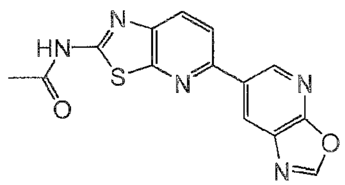<br>N-(5-(oxazolo[5,4-b]pyridin-6-yl)thiazolo[5,4-b]pyridin-2-yl)acetamide | 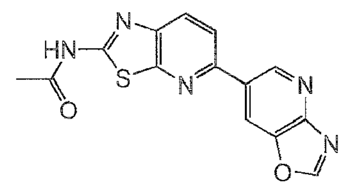<br>N-(5-(oxazolo[4,5-b]pyridin-6-yl)thiazolo[5,4-b]pyridin-2-yl)acetamide |
| 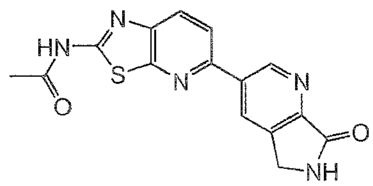<br>N-(5-(7-oxo-6,7-dihydro-5H-pynolo[3,4-b]pyridin-3-yl) thiazolo[5,4-b)pyridin-2-yl)acetamide | 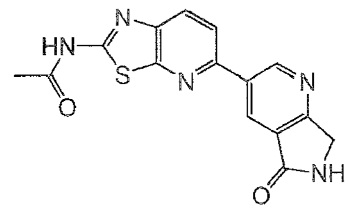<br>N-(5-(5-oxo-6,7-dihydro-5H-pyrrolo[3,4-b]pyridin-3-yl) thiazolo[5,4-b]pyridin-2-yl)acetamide |
| 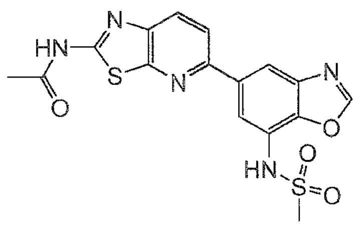<br>N-(5-(7-(methylsulfonamido)benzo[d]oxazol-5-yl) thiazolo[5,4-b]pyridin-2-yl)acetamide | 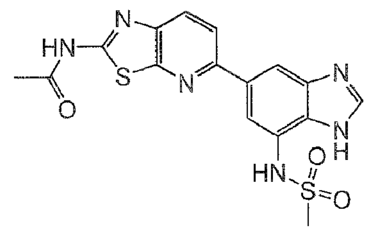<br>N-(5-(7-(methylsulfonamido)-1H-benzo[d]imidazol-5-yl) thiazolo[5,4-b]pyridin-2-yl)acetamide |

**Example A.** **Determination of the Inhibition of mTOR**

The inhibitory property of a compound relative to mTOR catalysis was determined by using the Z-Lyte™ biochemical assay conducted by Invitrogen Corporation (Madison, WI), which employs a fluorescence-based, coupled-enzyme format and is based on the differential sensitivity of phosphorylated and non-phosphorylated peptides to proteolytic cleavage. The peptide substrate is labeled with two fluorophores-one at each end-that make up a FRET pair.

In the primary reaction, the kinase transfers the gamma-phosphate of ATP to a single tyrosine, serine or threonine residue in a synthetic FRET-peptide. In the secondary reaction, a site-specific protease recognizes and cleaves non-phosphorylated FRET-peptides. Phosphorylation of FRET-peptides suppresses cleavage by the Development Reagent. Cleavage disrupts FRET between the donor (i.e., coumarin) and acceptor (i.e., fluorescein) fluorophores on the FRET-peptide, whereas uncleaved, phosphorylated FRET-peptides maintain FRET. A ratio metric method, which calculates the ratio (the Emission Ratio) of donor emission to acceptor emission after excitation of the donor fluorophore at 400 nm, is used to quantitate reaction progress, as shown in the equation below.

$$\text{Emission Ratio} = \frac{\text{Coumarin Emission (445 nM)}}{\text{Fluorescein Emission (520 nM)}}$$

A significant benefit of this ratio metric method for quantitating reaction progress is the elimination of well-to-well variations in FRET-peptide concentration and signal intensities. As a result, the assay yields very high Z'-factor values (>0.7) at a low percent phosphorylation. Both cleaved and uncleaved FRET-peptides contribute to the fluorescence signals and therefore to the Emission Ratio. The extent of phosphorylation of the FRET-peptide can be calculated from the Emission Ratio. The Emission Ratio will remain low if the FRET-peptide is phosphorylated (i.e., no kinase inhibition) and will be high if the FRET-peptide is non-phosphorylated (i.e., kinase inhibition).

Recombinant human mTOR enzyme (amino acids 1360-2549), GST-tagged, expressed in and purified from

insect cells (Invitrogen Corp. Cat. #PV4753; also known as FRAP1) was used for the assay. The 2X FRAP1 (mTOR) / Ser/Thr 11 Peptide Mixture (Invitrogen Corp.) was prepared in buffer containing 50 mM HEPES pH 7.5, 0.01% BRIJ-35, 10 mM MnCl2, 1 mM EGTA, 2 mM DTT, and 0.02% NaN3. The final 10 μL Kinase Reaction consisted of 7.2 - 58.1 ng TRAP 1 (mTOR) and 2 μM Ser/Thr11 Peptide in buffer containing 50 mM HEPES pH 7.5, 0.01% BRIJ-35, 5 mM MgC12, 5 mM MnCl2, 1 mM EGTA, 1 mM and 0.01% NaN3.

Test compounds are screened in 1% DMSO (final) in the well. For 10 point titrations, 3-fold serial dilutions are conducted from the starting concentration. The Ser/Thr 11 Peptide/Kinase Mixture was diluted to a 2X working concentration in buffer containing 50 mM HEPES pH 7.5, 0.01% BRIJ-35, 10 mM $MnCl_2$ 1 mM EGTA, 2 mM DTT, and 0.02% NaN3. The ATP solution was diluted to a 4X working concentration in buffer containing 50 mM HEPES pH 7.5, 0.01% BRIJ-35, 10 mM $MgCl_2$, and 1 mM EGTA.

The assay protocol consists of sequentially adding to the individual wells of a black 384-well, low volume NBS test plate (Corning Cat. #3676), either 2.5 μL of 4X or 100 nL of 100X test compound stock (in 100% DMSO) plus 2.4 μL kinase buffer, followed by 5 μL of 2X Ser/Thr 11 Peptide/Kinase mixture, then finally 2.5 μL of 4X ATP solution (final ATP concentration of 10 μM approximates apparent ATP Km of 11 μM), followed by a 30 sec plate shake to ensure thorough mixing of the reaction components. After the 1 hour Kinase Reaction incubation, 5 μL of a 1:16 dilution of Development Reagent B was added, and the development reaction was allowed to proceed for an additional 1 hour at room temperature. The assay plate was then read on a fluorescence plate reader and the data analyzed as outlined above.

$IC_{50}$ values were calculated by non-linear least squares curve fitting using XL*fit* from IDBS to fit the results to model number 205 (sigmoidal dose-response model). As a reference point for this assay, the PI3K inhibitor, PI-103, displayed an $IC_{50}$ of 31 nM. PI-103 (CAS number 371935-74-9) is a potent, cell-permeable, ATP-competitive inhibitor of PI3K family members with selectivity toward DNA-PK, PI3K (p110a), and mTOR.

**Example :8: Determination of The Inhibition of PI3Kalpha**

The inhibitory property of a compound relative to mTOR catalysis was determined by using the Adapta™ universal kinase assay conducted by Invitrogen Corporation (Madison, WI), which is a homogenous, fluorescent based immunoassay for the detection of ADP. In contrast to ATP depletion assays, the Adapta™ assay is extremely sensitive to ADP formation such that a majority of the signal change occurs in the first 10-20% conversion of ATP to ADP. This makes the Adapta™ universal kinase assay ideally suited for use with low activity kinases.

The principle of the Adapta™ universal kinase assay is outlined below. The assay itself can be divided into two phases: a kinase reaction phase, and an ADP detection phase. In the kinase reaction phase, all components required for the kinase reaction are added to the well, and the reaction is allowed to incubate for 60 minutes. After the reaction, a detection solution consisting of a europium labeled anti-ADP antibody, an Alexa Fluor® 647 labeled ATP tracer, and EDTA (to stop the kinase reaction) is added to the assay well. ADP formed by the kinase reaction (in the absence of an inhibitor) will displace the Alexa Fluor® 647 labeled ADP tracer from the antibody, resulting in a decrease in the TR-FRET signal. In the presence of an inhibitor, the amount of ADP formed by the kinase reaction is reduced, and the resulting intact antibody-tracer interaction results in a high TR-FRET signal.

ADP formation is determined by calculating the emission ratio from the assay well. The emission ratio is calculated by dividing the intensity of the tracer (acceptor) emission by the intensity of the Eu (donor) emission at 615 nm as shown in the equation below.

$$\text{Emission Ratio} = \frac{\text{AlexaFluor™647 Emission (665 nm)}}{\text{Europium Emission (615 nm)}}$$

Since the AdaptaTM technology measures ADP formation (i.e. conversion of ATP to ADP) it can be used to measure any type of ATP hydrolysis, including intrinsic ATPase activity of kinases. In this case, the substrate is water, not a lipid or peptide (Kashem, et al., 2007, J. Biomol. Screen. 12, 1, 70-83).

The combination of recombinant human, full length, N-terminal His-tagged phosphoinositide-3-kinase catalytic subunit alpha polypeptide (p100alpha, PI3Kalpha), expressed in and purified from insect cells along with co-expressed recombinant, full length, untagged phosphoinositide-3-kinase regulatory subunit 1 (p85alpha) (combination also known as PIK3CA/PIK3R1; Invitrogen Corp. Cat # PV4788) was used as the enzyme for this assay. The 2X PIK3CA/PIK3R1 (pl 10 alpha/p85 alpha) / PIP2:PS mixture was prepared in buffer containing 50 mM HEPES pH 7.5, 0.1% CHAPS, 1 mM EGTA. The final 10 μL Kinase Reaction consisted of 4 - 50 ng PIK3CAlPIK3R1 (p110 alpha/p85 alpha) and 50 μM PIP2:PS in buffer- containing 50 mM HEPES pH 7.5, 0.005% BRIJ-35, 0.005% CHAPS, 5 mM MgCl2, 1 mM EGTA.

The test compounds are screened at 1% DMSO (final) in the well. For 10-point titrations, 3-fold serial dilutions are conducted from the starting concentration. The PIP2:PS Substrate/Kinase Mixture was diluted 2X to working concentration in Kinase Buffer containing 50 mM HEPES pH 7.5, 0.1% CHAPS, and 1 mM EGTA. The ATP Solution was diluted to 4X working concentration in buffer containing 50 mM HEPES pH 7.5, 0.01% BRIJ-35, 10 mM MgCl2, and 1 mM EGTA. The Detection Mix was prepared in TR-FRET Dilution Buffer. The Detection Mix consists of EDTA (10 mM), Eu-anti-ADP antibody (30 nM) and 60 nM ADP tracer (for kinase reactions with 5-50 μM ATP; the PI3Kalpha assay has 25 μM ATP final concentration).

The assay protocol consists of sequentially adding to the individual wells of a white 384-well, low volume NBS test plate (Coming Cat. #3673), either 2.5 μL of4X or 100 nL of 100X test compound stock (in 100% DMSO) plus 2.4 μL kinase buffer, followed by 5 μL of PIP2:PS Substrate/Kinase mixture, then finally 2.5 μL of 4X ATP solution (final ATP concentration of 25 μM approximates apparent ATP Km of 24 μM), followed by a 30 sec plate shake to ensure thorough mixing of the reaction components. After the 1 hour Kinase Reaction incubation at room temperature, 5 μL of Detection Mix was added, followed by another 30 sec plate shake to ensure mixing, and the Detection Mix was allowed to equilibrate for 1 hour at room temperature. The assay plate was then read on a fluorescence plate reader and the data analyzed as outlined above.

Lipid substrates are prepared by creating lipid vesicles. In some cases, these vesicles include a carrier lipid, such as phosphatidylserine (PS). In the assay conditions section above, "PIP2:PS" refers to large unilamellar vesicles (LUVs) containing five mole percent L-α-Phosphatidylinositol-4,5-bisphosphate (PIP2) and ninety-five percent phosphatidylserine (PS), and the concentration listed refers only to the PIP2 substrate, not the PS carrier lipid.

**Table 1: $IC_{50}$ of Exemplified Compounds Against PBK and MOTOR**

| **Example No.** | **$IC_{50}$** (nm) **PI3Kα** | **$IC_{50}$** (nM) **mTOR** |
|---|---|---|
| Example 2 | 100-1000 | 100-1000 |
| Example 3 | <100 | 100-1000 |
| Example 4 | 100-1000 | 100-1000 |
| Example 5 | >1000 | >1000 |
| Example 6 | <100 | 100-1000 |
| Example 7 | <100 | <100 |
| Example 8 | <100 | <100 |
| Example 9 | <100 | <100 |
| Example 10 | -- | >1000 |
| Example 11 | -- | 100-1000 |
| Example 12 | 100-1000 | >1000 |
| Example 13 | 100-1000 | >1000 |
| Example 14 | >1000 | >1000 |
| Example 15 | <100 | <100 |
| Example 16 | >1000 | 100-1000 |
| Example 17 | 100-1000 | >1000 |
| Example 18 | <100 | <100 |
| Example 19 | -- | >1000 |
| Example 20 | <100 | 100-1000 |
| Example 21 | >1000 | >1000 |
| Example 22 | <100 | 100-1000 |
| Example 23 | 100-1000 | 100-1000 |
| Example 24 | <100 | <100 |
| Example 25 | <100 | <100 |
| Example 26 | >1000 | >1000 |
| Example 27 | <100 | >1000 |

(continued)

| Example No. | $IC_{50}$ (nm) PI3Kα | $IC_{50}$ (nM) mTOR |
|---|---|---|
| Example 28 | > 1000 | >1000 |
| Example 29 | 100-1000 | 100-1000 |
| **Example No.** | **$IC_{50}$ (nM) PI3Kα** | **$IC_{50}$ (nM) mTOR** |
| Example 30 | <100 | 100-1000 |
| Example 31 | <100 | 100-1000 |
| Example 32 | >1000 | >1000 |
| Example 33 | 100-1000 | >1000 |
| Example 34 | 100-1000 | >1000 |
| Example 35 | <100 | <100 |
| Example 36 | 100-1000 | 100-1000 |
| Example 37 | -- | 100-1000 |
| Example 38 | -- | <100 |
| Example 39 | -- | 100-1000 |
| Example 40 | 100-1000 | >1000 |
| Example 41 | >1000 | >1000 |
| Example 42 | <100 | <100 |
| Example 43 | 100-1000 | -- |
| Example 44 | <100 | -- |
| Example 45 | 100-1000 | -- |
| Example 46 | <100 | -- |
| Example 47 | <100 | <100 |
| Example 48 | <100 | <100) |
| Example 49 | 100-1000 | >1000 |

## Claims

**1.** A compound of the formula:

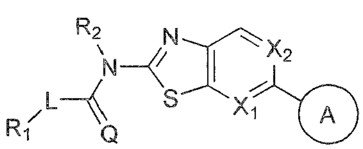

wherein

ring A is heteroaryl, each unsubstituted or substituted with 1-5 substituents which is independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, carbonylamino, sulfonylamino, $(C_{1-10})$alkylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$

alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero $(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero $(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl;

Q is O or S;

L is selected from the group consisting of $-CHR_3-$, $-NR_4-$ and -O-;

$X_1$ is selected from the group consisting of $CR_5$ and N;

$X_2$ is selected from the group consisting of $CR_{5'}$ and N;

$R_1$ and $R_3$ are each independently selected from the group consisting of hydrogen, halo, for nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl $(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino $(C_{1-10})$alkyl, $(C_{3-12})$cycloalkyl$(C_{1-10})$alkyl, hetero$(C_{3-12})$cycloalkyl$(C_{1-10})$alkyl, aryl$(C_{1-10})$alkyl, hetero$(C_{1-10})$aryl $(C_{1-10})$alkyl, $(C_{9-12})$bicycloaryl$(C_{1-10})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloaticyclyl, $(C_{4-12})$aryl, hetero$(C_{1-10})$aryl, $(C_{9-12})$bicycloaryl, and hetero$(C_{4-12})$bicycloaryl, each substituted or unsubstituted, or $R_1$ and $R_3$ are taken together to form a cyclopropyl;

$R_2$ is selected from the group consisting of hydrogen, $(C_{1-6})$alkoxy, carbonyl, oxycarbonyl, aminocarbonyl, amino, $(C_{1-6})$alkylamino, sulfonyl, sulfinyl, $(C_{1-6})$alkyl, $(C_{1-10})$alkenyl, $(C_{1-10})$alkynyl, hetero$(C_{1-6})$alkyl, halo$(C_{1-6})$alkyl, hydroxy$(C_{1-6})$alkyl, carbonyl$(C_{1-6})$alkyl, $(C_{3-6})$cycloalkyl$(C_{1-3})$alkyl, hetero$(C_{1-5})$cycloalkyl$(C_{1-3})$alkyl, $(C_{3-6})$alicyclyl, and hetero$(C_{1-5})$alicyclyl, each substituted or unsubstituted;

$R_4$ is selected from the group consisting of hydrogen, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, imino, sulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$allcyl, aza$(C_{1-10})$alkyl, $(C_{1-10})$oxaalkyl, $(C_{1-10})$oxoalkyl, imino $(C_{1-10})$alkyl, $(C_{3-12})$cycloalkyl$(C_{1-10})$alkyl, hetero$(C_{3-12})$cycloalkyl$(C_{1-10})$alkyl, aryl$(C_{1-10})$alkyl, hetero$(C_{1-10})$aryl $(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted; and

$R_5$ and $R_{5'}$ are each independently selected from the group consisting of hydrogen, halo, nitro, cyano, thio, oxy, hydroxy, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, sulfonylamino, imino, sulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl $(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-10})$alkyl, $(C_{9-12})$bicycloaryl $(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each substituted or unsubstituted;

provided that

when $X_1$ is N or CH, $X_2$ is CH, Q is O, L is $-CH_2-$, -NH- or O, $R_1$ is hydrogen, $(C_{1-4})$alkyl or hetero$(C_6)$cycloalkyl, and ring A is a pyridyl, pyrimidinyl or pyrazinyl, ring A does not have an amino, substituted amino, substituted oxy, or fluoro attached to position 4 of ring A where the ring atom through which ring A attaches to the ring containing $X_1$ and $X_2$ is counted as position 1;

when $X_1$ is CH, $X_2$ is CH, Q is O, L is $-CHR_3-$, $R_1$ and $R_3$ are both hydrogen, and ring A is a five- or six-membered heteroaryl comprising one nitrogen ring atom, said one nitrogen ring atom is not located at a position adjacent to the ring atom through which said heteroaryl attaches to the ring containing $X_1$ and $X_2$;

when $X_1$ is CH, $X_2$ is CH, Q is O, and L is $-CHR_3-$, $R_1$ and $R_3$ are both hydrogen, and ring A is a pyrimidinyl, the ring atom located at position 4 of said pyrimidinyl is not a nitrogen where the ring atom through which said pyrimidinyl attaches to the ring containing $X_1$ and $X_2$ is counted as at position 1; and

when $X_1$ is CH, $X_2$ is CH, Q is O, L is -NH-, and ring A is as defined above, $R_1$ is not selected from the group consisting of alkyl, carbonyl, and phenoxy substituted pyridinyl.

2. The compound according to claim 1, of the formula:

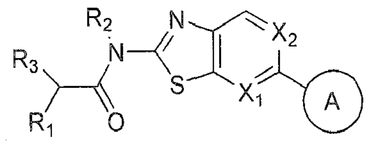

provided that

when $X_1$ is N or CH, $X_2$ is CH, $R_3$ is hydrogen, $R_1$ is hydrogen, unsubstituted or substituted $(C_{1-4})$alkyl, or hetero $(C_6)$cycloalkyl, and ring A is a pyridyl, pyrimidinyl or pyrazinyl, ring A does not have an amino, substituted amino, substituted oxy, or fluoro attached to position 4 of ring A where the ring atom through which ring A attaches to the ring containing $X_1$ and $X_2$ is counted as position 1;
when $X_1$ is CH, $X_2$ is CH, $R_1$ and $R_3$ are both hydrogen, and ring A is a five- or six-membered heteroaryl comprising one nitrogen ring atom, said one nitrogen ring atom is not located at a position adjacent to the ring atom through which said heteroaryl attaches to the ring containing $X_1$ and $X_2$;
when $X_1$ is CH, $X_2$ is CH, $R_1$ and $R_3$ are both hydrogen, and ring A is a pyrimidinyl, the ring atom located at position 4 of said pyrimidinyl is not a nitrogen where the ring atom through which said pyrimidinyl attaches to the ring containing $X_1$ and $X_2$ is counted as at position 1; and
when $X_1$ is N, $X_2$ is CH, and ring A is as defined in claim 1, $R_1$ and $R_3$ are both not a substituted phenyl.

3. The compound according to claim 1, of the formula:

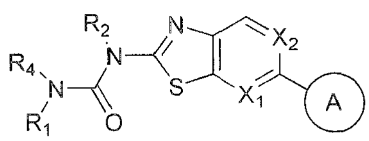

provided that

when $X_1$ is N or CH, $X_2$ is CH, $R_4$ is hydrogen, $R_1$ is unsubstituted or substituted $(C_{1-4})$alkyl, and ring A is a pyridyl, pyrimidinyl or pyrazinyl, ring A does not have an amino, substituted amino, substituted oxy, or fluoro attached to position 4 of ring A where the ring atom through which ring A attaches to the ring containing $X_1$ and $X_2$ is counted as position 1; and
when $X_1$ is CH and $X_2$ is CH, and ring A is as defined in claim 1, $R_1$ is not selected from the group consisting of alkyl, carbonyl, and phenoxy substituted pyridinyl.

4. The compound according to claim 1, of the formula:

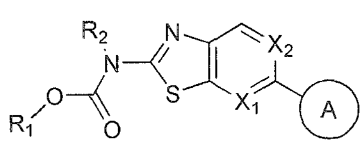

provided that

when $X_1$ is N or CH, $X_2$ is CH, $R_1$ is unsubstituted or substituted $(C_{1-4})$alkyl, and ring A is a pyridyl, pyrimidinyl or pyrazinyl, ring A does not have an amino, substituted amino, substituted oxy, or fluoro attached to position 4 of ring A where the ring atom through which ring A attaches to the ring containing $X_1$ and $X_2$ is counted as position 1.

5. The compound according to any one of claims 1-4, wherein ring A is selected from the group consisting of

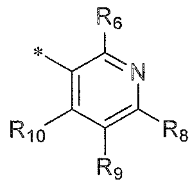
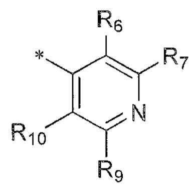
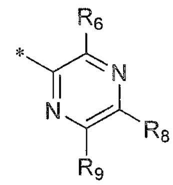

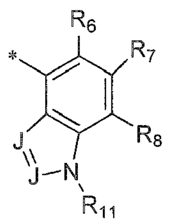
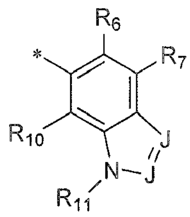
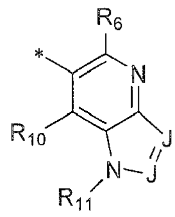
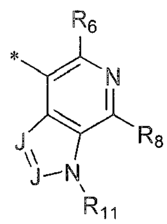
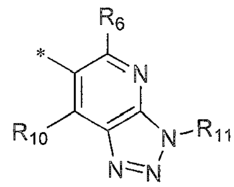

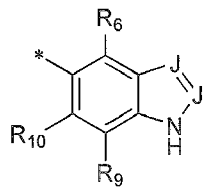
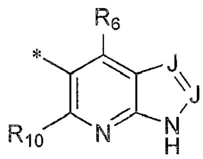
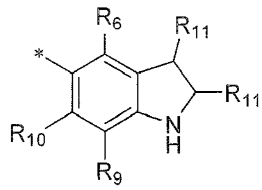
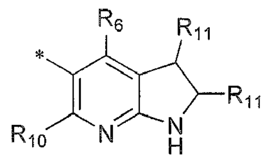

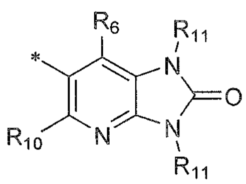
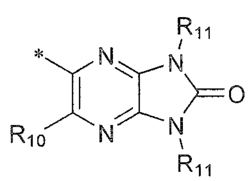
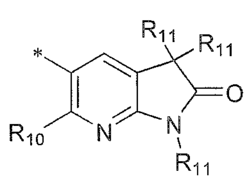
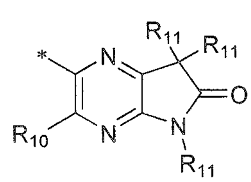

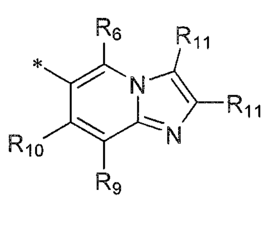
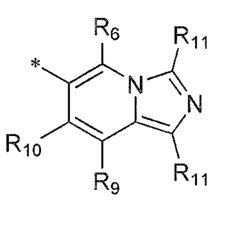
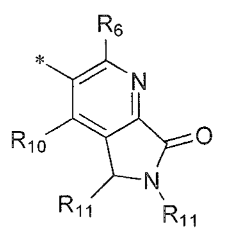
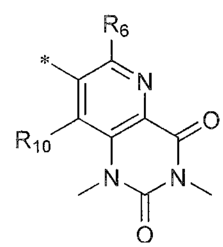

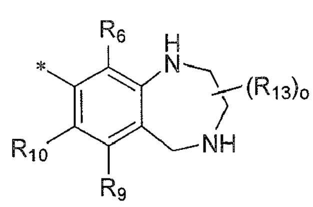

wherein

n is 1, 2, 3, or 4;
o is 1 , 2, 3, 4, 5, or 6;
each J is independently N or $CR_{11}$;
each $R_6$, $R_7$, $R_8$, $R_9$, and $R_{10}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxy-

carbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl, or one of $R_6$ and $R_7$, $R_7$ and $R_8$, $R_8$ and $R_9$, and $R_9$ and $R_{10}$, are taken together to from a saturated, unsaturated, or aromatic ring, each unsubstituted or substituted;

each $R_{11}$ and $R_{13}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl; and

each $R_6$, $R_7$, $R_8$, $R_9$, and $R_{10}$ is independently selected from the group consisting of hydrogen, halo, nitro, cyano, thio, oxy, hydroxy, oxo, carbonyloxy, $(C_{1-10})$alkoxy, $(C_{4-12})$aryloxy, hetero$(C_{1-10})$aryloxy, carbonyl, oxycarbonyl, aminocarbonyl, amino, $(C_{1-10})$alkylamino, carbonylamino, sulfonylamino, imino, sulfonyl, aminosulfonyl, sulfinyl, $(C_{1-10})$alkyl, $(C_{1-10})$alkenyl, $(C_{1-10})$alkynyl, halo$(C_{1-10})$alkyl, hydroxy$(C_{1-10})$alkyl, carbonyl$(C_{1-10})$alkyl, thiocarbonyl$(C_{1-10})$alkyl, sulfonyl$(C_{1-10})$alkyl, sulfinyl$(C_{1-10})$alkyl, aza$(C_{1-10})$alkyl, $(C_{1-10})$oxaalkyl, $(C_{1-10})$oxoalkyl, imino$(C_{1-10})$alkyl, $(C_{3-12})$cycloalkyl$(C_{1-5})$alkyl, hetero$(C_{3-12})$cycloalkyl$(C_{1-10})$alkyl, aryl$(C_{1-10})$alkyl, hetero$(C_{1-10})$aryl$(C_{1-5})$alkyl, $(C_{9-12})$bicycloaryl$(C_{1-5})$alkyl, hetero$(C_{8-12})$bicycloaryl$(C_{1-5})$alkyl, hetero$(C_{1-10})$alkyl, $(C_{3-12})$alicyclyl, hetero$(C_{3-12})$alicyclyl, $(C_{9-12})$bicycloalicyclyl, hetero$(C_{3-12})$bicycloalicyclyl, $(C_{4-12})$aryl, hetero$(C_{1-10})$aryl, $(C_{9-12})$bicycloaryl, and hetero$(C_{4-12})$bicycloaryl, each unsubstituted or further substituted with 1-2 substituents independently selected from the group consisting of halo, cyano, $(C_{1-6})$alkyl, hydroxyl$(C_{1-6})$alkyl, halo$(C_{1-6})$alkyl, amino$(C_{1-6})$alkyl, $(C_{3-6})$alicyclyl$(C_{1-6})$alkyl, hetero$(C_{1-5})$alicyclyl$(C_{1-6})$alkyl, $(C_{4-6})$aryl$(C_{1-6})$alkyl, hetero$(C_{1-5})$aryl$(C_{1-6})$alkyl, amino, $(C_{1-6})$alkoxy, $(C_{3-6})$alicyclyl, hetero$(C_{1-5})$alicyclyl, $(C_{4-6})$aryl, hetero$(C_{1-5})$aryl, or one of $R_6$ and $R_7$, $R_7$ and $R_8$, $R_8$ and $R_9$, and $R_9$ and $R_{10}$, are taken together to from a saturated, unsaturated, or aromatic ring, each unsubstituted or substituted; and

provided that

when $X_1$ is N or CH, $X_2$ is CH, Q is O, L is-$CH_2$-, -NH- or -O-, $R_1$ is hydrogen, unsubstituted or substituted $(C_{1-4})$alkyl, or hetero$(C_6)$cycloalkyl, and ring A is a pyridyl, pyrimidinyl or pyrazinyl, $R_8$ is not amino, substituted amino, substituted oxy, or fluoro;

when $X_1$ is CH, $X_2$ is CH, Q is O, L is -$CHR_3$-, $R_1$ and $R_3$ are both hydrogen, and ring A is a pyridyl, $A_1$ is $CR_6$ and $A_5$ is $CR_{10}$;

when $X_1$ is CH, $X_2$ is CH, Q is O, L is -$CH_2$-, $R_1$ is hydrogen, and ring A is a pyrimidinyl, $A_3$ is not N; and

when $X_1$ is CH, $X_2$ is CH, Q is O, and L is -NH-, $R_1$ is not selected from the group consisting of alkyl, carbonyl, and phenoxy substituted pyridinyl.

**6.** The compound according to any one of claims 1-4, wherein ring A is selected from the group consisting of furanyl, thiophenyl, pyrrolyl, oxazolyl, thiazolyl, imidazolyl, pyrazolyl, isoxazolyl, isothiazolyl, oxadiazolyl, triazolyl, thiadiazolyl, indolizinyl, indolyl, isoindolyl, benzofuranyl, benzothiophenyl, indazolyl, benzimidazolyl, benzthiazolyl, pyridinylfuranyl, pyridinylthiophenyl, pyridinylpyrazolyl, pyridinylimidazolyl, pyridinylthiazolyl, pyrimidinylfuranyl, pyrimidinylthiophenyl, pyrimidinylpyrazolyl, pyrimidinylimidazolyl, pyrimidinylthiazolyl, and purinyl.

**7.** The compound according to claim 10, wherein ring A is

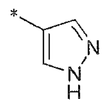
.

**8.** The compound according to any one of claims 1-7, wherein $R_2$ is hydrogen.

**9.** The compound according to any one of claims 1-8, wherein $R_1$ is independently selected from the group consisting of hydrogen, hydroxy, halo, nitro, cyano, thio, oxy, amino, carbonyloxy, $(C_{1-6})$alkoxy, $(C_{4-12})$aryloxy, hetero$(C_{1-10})$aryloxy, carbonyl, oxycarbonyl, sulfonyl, sulfinyl, $(C_{1-6})$alkyl, $(C_{1-10})$alkenyl, $(C_{1-10})$alkynyl, hetero$(C_{1-6})$alkyl, halo$(C_{1-6})$alkyl, hydroxy$(C_{1-6})$alkyl, carbonyl$(C_{1-10})$alkyl, sulfonyl$(C_{1-6})$alkyl, $(C_{3-6})$cycloalkyl$(C_{1-3})$alkyl, hetero$(C_{1-5})$cycloalkyl$(C_{1-3})$alkyl, aryl$(C_{1-3})$alkyl, hetero$(C_{1-5})$aryl$(C_{1-3})$alkyl, $(C_{3-6})$alicyclyl, hetero$(C_{1-5})$alicyclyl, $(C_{4-6})$aryl, and hetero$(C_{1-5})$aryl, each unsubstituted or substituted.

**10.** The compound according to any one of claims 1-9, wherein $R_1$ is hydrogen.

**11.** The compound according to any one of claims 1-2 and 5-10, wherein $R_3$ is independently selected from the group consisting of hydrogen, hydroxy, halo, nitro, cyano, thio, oxy, amino, carbonyloxy, $(C_{1-6})$alkoxy, $(C_{4-12})$aryloxy, hetero$(C_{1-10})$aryloxy, carbonyl, oxycarbonyl, sulfonyl, sulfinyl, $(C_{1-6})$alkyl, $(C_{1-10})$alkenyl, $(C_{1-10})$alkynyl, hetero$(C_{1-6})$alkyl, halo$(C_{1-6})$alkyl, hydroxy$(C_{1-6})$alkyl, carbonyl$(C_{1-10})$alkyl, sulfonyl$(C_{1-6})$alkyl, $(C_{3-6})$cycloalkyl$(C_{1-3})$alkyl, hetero$(C_{1-5})$cycloalkyl$(C_{1-3})$alkyl, aryl$(C_{1-3})$alkyl, hetero$(C_{1-5})$aryl$(C_{1-3})$alkyl, $(C_{3-6})$alicyclyl, hetero$(C_{1-5})$alicyclyl, $(C_{4-6})$aryl, and hetero$(C_{1-5})$aryl, each unsubstituted or substituted.

**12.** The compound according to any one of claims 1, 3, and 6-11, wherein $R_4$ is selected from the group consisting of hydrogen, $(C_{1-6})$alkyl, hetero$(C_{1-6})$alkyl, $(C_{3-6})$cycloalkyl, hetero$(C_{1-5})$cycloalkyl, phenyl, and $(C_{1-5})$heteroaryl, each unsubstituted or substituted.

**13.** The compound according to any one of claims 1, 3, and 6-12, wherein $R_5$ and $R_5$, are each independently selected from the group consisting of hydrogen, $(C_{1-6})$alkyl, $(C_{1-10})$alkenyl, $(C_{1-10})$alkynyl, hetero$(C_{1-6})$alkyl, $(C_{3-6})$cyclloalkyl, and hetero$(C_{1-5})$cycloalkyl, each unsubstituted or substituted.

**14.** The compound according any one of claims 1, 3, and 6-13, wherein $R_5$ and $R_{5'}$, when present, are each hydrogen.

**15.** The compound according to any one of claims 6-14, wherein $R_6$ is selected from the group consisting of hydrogen, halo, hydroxyl, amino, $(C_{1-6})$alkoxy, $(C_{1-6})$alkyl, $(C_{1-6})$heteroalkyl, halo$(C_{1-6})$alkyl, $(C_{3-6})$cycloalkyl, and hetero$(C_{1-5})$cycloalkyl, each unsubstituted or substituted with said 1-2 substituents.

**16.** The compound according to any one of claims 6-15, wherein $R_6$ is hydrogen or $-NH_2$.

**17.** The compound according to any one of claims 6-16, wherein $R_7$ is hydrogen, trifluoromethyl, and $-C(O)NHOR_{14}$ where $R_{14}$ is $(C_{1-6})$alkyl.

**18.** A compound of claim 1 selected from the group consisting of

*N*-(5-(1*H*-Indol-5-yl)thiazolo[5,4-*b*]pyridin-2-yl)acetamide;
*N*-(5-(1*H*-Indazol-5-yl)thiazolo[5,4-*b*]pyridin-2-yl)acetamide;
*N*-(5-(6-Acetamidopyridin-3-yl)thiazolo[5,4-*b*]pyridin-2-yl)acetamide;
5-(2-Acetarnidothiazolo[5,4-*b*]pyridin-5-yl)-N-methylpicolinamide;
*N*-(5-(6-Chloro-5-(phenylsulfonamido)pyridin-3-yl)thiazolo[5,4-*b*] pyridin-2-yl) acetamide;
*N*-(5-(6-Chloro-5-(methylsulfonamido)pyridin-3-yl)thiazolo[5,4-*b*] pyridin-2-yl)acetamide;
*N*-(5-(6-Chloro-5-(methylsulfonamido)pyridin-3-yl)thiazolo[5,4-*b*] pyridin-2-yl)cyclopropanecarboxamide;
*N*-(6-(Pyridin-3-yl)benzo[*d*]thiazol-2-yl)acetamide;
*N*-(6-(1*H*-Indol-5-yl)benzo[*d*]thiazol-2-yl)acetamide;
*N*-(6-(1*H*-Indol-4-yl)benzo[*d*]thiazol-2-yl)acetamide;
*N*-(6-(3-Formyl-1*H*-indol-5-yl)benzo[*d*]thiazol-2-yl)acetamide;
*N*-(6-(3-Cyano-1*H*-indol-5-yl)benzo[*d*]thiazol-2-yl)acetamide;
5-(2-Acetamidobenzo[*d*]thiazol-6-yl)-1*H*-indole-3-carboxamide;

*N*-(6-(5-Aminopyrazin-2-yl)benzo[*d*]thiazol-2-yl)acetamide;
*N*-(6-(2,5-Dioxo-2,3,4,5-tetrahydro-1*H*-benzo[*e*][1,4]diazepin-8-yl) benzo[*d*]thiazol-2-yl)acetamide;
*N*-(5-(Imidazo[1,2-*a*]pyridin-6-yl)thiazolo[5,4-*b*]pyridin-2-yl)acetamide;
*N*-(5-(6-Cyanopyridin-3-yl)thiazolo[5,4-*b*]pyridin-2-yl)acetamide;
*N*-(5-(1*H*-Pyrrolo[2,3-*b*]pyridin-5-yl)thiazolo[5,4-*b*]pyridin-2-yl)aectamide;
*N*-(5-(1*H*-Pyrrolo[3,2-*b*]pyridin-6-yl)thiazolo[5,4-*b*]pyridin-2-yl)acetamide;
*N*-(5-(2-Oxoindolin-5-yl)thiazolo[5,4-*b*]pyridin-2-yl)acetamide;
*N*-(6-(7-(Methylsulfonamido)-1*H*-indol-5-yl)benzo[*d*]thiazol-2-yl)acetamide;
*N*-(5-(Pyridin-4-yl)thiazolo[5,4-*b*]pyridin-2-yl)acetamide;
*N*-(5-(5-Amino-6-chloropyridin-3-yl)thiazolo[5,4-d]pyrimidin-2-yl)acetamide;
*N*-(5-(6-Chloro-5-(methylsulfonamido)pyridin-3-yl)thiazolo[5,4-*d*]pyrimidin-2-yl)acetamide;
*N*-(5-(6-Aminopyridin-3-yl)thiazolo[5,4-*d*]pyrimidin-2-yl)acetamide;
*N*-(5-(6-Amino-5-(trifluoromethyl)pyridin-3-yl)thiazolo[5,4-*d*]pyrimidin-2-yl)acetamide;
*N*-(5-(6-chloro-5-(ethylsulfonamido)pyridin-3-yl)thiazolo[5,4-*b*]pyridin-2-yl)acetamide;
*N*-(6-(6-chloro-5-(methylsulfonamido)pyridin-3-yl)benzo [*d*]thiazol-2-yl)acetamide;
*N*-(5-(6-chloro-5-(cyclopropanesulfonamido)pyridin-3-yl)thiazolo[5,4-*b*]pyridin-2-yl)acetamide;
*N*-(5-(6-Chloro-5-(phenylsulfonamido)pyridin-3-yl)thiazolo[5,4-*d*]pyrimidin-2-yl)acetamide; and
*N*-(5-(6-chloro-5-(methylsulfonamido)pyridin-3-yl)thiazolo[5,4-*b*]pyridin-2-yl)-2-hydroxyacetamide;
*N*-(5-(6-chloro-5-(methylsulfonamido)pyridin-3-yl)thiazolo[5,4-b]pyridin-2-yl)-2-(1 -methylpiperidin-4-yloxy) acetamide
*N*-(5-(6-chloro-5-(methylsulfonamido)pyridin-3-yl)thiazolo[5,4-*b*]pyridin-2-yl)-2-(dimethylamino)acetamide;
N-(5 -(6-chloro-5 -(methylsulfonamido)pyridin-3 -yl)thiazolo [5,4-b]pyridin-2-yl)-3 - hydroxypropanamide;
(R)-*N*-(5-(6-chloro-5-(methylsulfonamido)pyridin-3-yl)thiazolo[5,4-*b*]pyridin-2-yl)-2,3-dihydroxypropanamide;
(S)-*N*-(5-(6-chloro-5-(methylsulfonamido)pyridin-3-yl)thiazolo[5,4-*b*]pyridin-2-yl)-2,3-dihydroxypropanamide;
(S)-*N*-(5-(6-chloro-5-(methylsulfonamido)pyridin-3-yl)thiazolo[5,4-*b*]pyridin-2-yl)-3,4-dihydroxybutanamide;
(R)-*N*-(5-(6-chloro-5-(methylsulfonamido)pyridin-3-yl)thiazolo[5,4-*b*]pyridin-2-yl)-3,4-dihydroxybutanamide;
*N*-(5-(6-chloro-5-(2-hydroxyethylsulfonamido)pyridin-3-yl)thiazolo[5,4-*b*]pyridin-2-yl)acetamide;
*N*-(5-(6-chloro-5-(3-hydroxypropylsulfonamido)pyridin-3-yl)thiazolo[5,4-*b*]pyridin-2-yl)acetamide;
*N*-(5-(6-chloro-5-(2-(dimethylamino)ethylsulfonamido)pyridin-3-yl)thiazolo[5,4-*b*]pyridin-2-yl)acetamide;
*N*-(5-(6-chloro-5-(3-(dimethylamino)propylsulfonamido)pyridin-3-yl)thiazolo[5,4-b]pyridin-2-yl)acetamide;
*N*-(5-(6-chloro-5-(1-methylpiperidine-4-sulfonamido)pyridin-3-yl)thiazolo[5,4-*b*]pyridin-2-yl)acetamide;
*N*-(5-(6-chloro-5-(methylsulfonamido)pyridin-3-yl)thiazolo[5,4-*b*]pyridin-2-yl)-1-methylpiperidine-4-carboxamide;
*N*-(5-(6-chloro-5-(methylsulfonamido)pyridin-3-yl)thiazolo[5,4-*b*]pyridin-2-yl)-2-(1-methylpiperidin-4-yl)acetamide;
*N*-(5-(6-chloro-5-(methylsulfonamido)pyridin-3-yl)thiazolo[5,4-*b*]pyridin-2-yl)-3-hydroxy-2-(hydroxymethyl)propanamide;
*N*-(5-(6-chloro-5-(methylsulfonamido)pyridin-3-yl)thiazolo[5,4-*b*]pyridin-2-yl)-4-hydroxy-3-(hydroxymethyl)butanamide;
*N*-(5-(6-chloro-5-(2-hydroxy-1-(hydroxymethyl)ethylsulfonamido)pyridin-3-yl)thiazolo[5,4-*b*]pyridin-2-yl)acetamide;
*N*-(5-(6- chloro- 5-(3- hydroxy- 2-(hydroxymethyl) propylsulfonamido) pyridin- 3- yl) thiazolo [5,4-*b*] pyridin- 2- yl) acetamide;
*N*-(5-(6-chloro-5-(3-(4-methylpiperazin-1-yl)propylsulfonamido)pyridin-3-yl)thiazolo[5,4-*b*]pyridin-2-yl)acetamide;
*N*-(5-(6- chloro- 5-((1- methylpiperidin- 4- yl) methylsulfonamido) pyridin- 3- yl) thiazolo [5,4-*b*] pyridin- 2- yl) acetamide;
*N*-(5-(1*H*-indazol-4-yl)thiazolo[5,4-*b*]pyridin-2-yl)acetamide;
*N*-(5-(1*H*-pyrazolo[3,4-*b*]pyridin-5-yl)thiazolo[5,4-*b*]pyridin-2-yl)acetamide;
*N*-(5-(3*H*-imidazo[4,5-b]pyridin-6-yl)thiazolo[5,4-*b*]pyridin-2-yl)acetamide;
*N*-(5-(1*H*-pyrazolo[4,3-b]pyridin-6-yl)thiazolo[5,4-*b*]pyridin-2-yl)acetamide;
*N*-(5-(6-chloro-5-(methylsulfonamido)pyridin-3-yl)thiazolo[5,4-*b*]pyridin-2-yl)propionamide;
*N*-(5-(5-chloro-6-(methylsulfonamido)imidazo[1,5-*a*]pyridin-8-yl)thiazolo[5,4-*b*]pyridin-2-yl)acetamide;
*N*-(5-(1-(methylsulfonyl)-1*H*-pyrrolo[3,4-*b*]pyridin-6-yl)thiazolo[5,4-*b*]pyridin-2-yl)acetamide;
*N*-(5-(1-(methylsulfonyl)-1*H*-pyrrolo[3,4-*b*]pyridin-3-yl)thiazolo[5,4-*b*]pyridin-2-yl)acetamide;
*N*-(5-(1,3-dimethyl-2-oxo-2,3-dihydro-1*H*-imidazo[4,5-*b*]pyridin-6-yl)thiazolo[5,4-*b*]pyridin-2-yl)acetamide;
*N*-(5-(oxazolo[5,4-*b*]pyridin-6-yl)thiazolo[5,4-*b*]pyridin-2-yl)acetamide;
*N*-(5-(oxazolo[4,5-*b*]pyridin-6-yl)thiazolo[5,4-*b*]pyridin-2-yl)acetamide;

*N*-(5-(7-oxo-6,7-dihydro-5*H*-pyrrolo[3,4-*b*]pyridin-3-yl)thiazolo[5,4-*b*]pyridin-2-yl)acetamide;
*N*-(5-(5-oxo-6,7-dihydro-5*H*-pyrrolo[3,4-*b*]pyridin-3-yl)thiazolo[5,4-*b*]pyridin-2-yl)acetamide;
*N*-(5-(7-(methylsulfonamido)benzo[*d*]oxazol-5-yl)thiazolo[5,4-*b*]pyridin-2-yl)acetamide; and
*N*-(5-(7-(methylsulfonamido)-1*H*-benzo[*d*]imidazol-5-yl)thiazolo[5,4-*b*]pyridin-2-yl)acetamide.

**19.** The compound according to any one of claims 1-18, wherein the compound is in the form of a pharmaceutically acceptable salt.

**20.** A pharmaceutical composition comprising as an active ingredient a compound according to any one of claims 1-19.

**21.** A compound of any one of claims 1-19 for use as a medicament.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4328245 A
- US 4409239 A
- US 4410545 A
- US RE28819 E
- US 4358603 A
- US 3710795 A
- US 4044126 A
- US 4414209 A
- US 4364923 A

### Non-patent literature cited in the description


- **VANHAESEBROECK, B. et al.** *Ann. Rev. Biochem.,* 2001, vol. 70, 535-602
- **VIVANCO, I. ; SAWYERS, C.L.** *Nature Rev. Cancer,* 2002, vol. 2, 489-501
- **LUO, J. et al.** *Cancer Cell,* 2003, vol. 4, 257-262
- **SAAL, L.H. et al.** *Cancer Res.,* 2005, vol. 65, 2554-2559
- **SHAYESTEH, L. et al.** *Nature Genetics,* 1999, vol. 21, 99-102
- **MA, Y.-Y. et al.** *Oncogene,* 2000, vol. 19, 2739-2744
- **SAMUEL, Y. et al.** *Science,* 2004, vol. 304, 554
- **BADER, A.G. et al.** *Nature Rev. Cancer,* 2005, vol. 5, 921-929
- **PARSONS, W.D. et al.** *Nature,* 2005, vol. 36, 792
- **PHILP, A.J. et al.** *Cancer Res.,* 2001, vol. 61, 7426-7429
- **SAMUELS, Y. et al.** *Science,* 2004, vol. 304, 554
- **KANG, S. et al.** *Proc. Natl. Acad. Sci USA,* 2005, vol. 102, 802-807
- **IKENOUE, T. et al.** *Cancer Res,* 2005, vol. 65, 4562-4567
- **ISAKOF, S.J. et al.** *Cancer Res.,* 2005, vol. 65, 10992-11000
- **GYMNOPOULOS, M. et al.** *Proc. Natl. Acad. Sci. USA,* 2007, vol. 104, 5569-5574
- **FRESNO VARA, J.A. et al.** *Cancer Treatment Rev.,* 2004, vol. 30, 193-204
- **HARARI, D. et al.** *Oncogene,* 2000, vol. 19, 6102-6114
- **KAUFFMANN-ZEH, A. et al.** *Nature,* 1997, vol. 385, 544-548
- **SIMPSON, L. ; PARSONS, R.** *Exp. Cell. Res.,* 2001, vol. 264, 29-41
- **ABID, M.R. et al.** *Arterioscler. Thromb. Vasc. Biol.,* 2004, vol. 24, 294-300
- **SAWYER, T.K.** *Expert Opin. Investig. Drugs,* 2004, vol. 13, 1-19
- **GUERTIN, D.A. et al.** *Cancer Cell,* 2007, vol. 12, 9-22
- **WAN, X. ; HELMAN, L.J.** *The Oncologist,* 2007, vol. 12, 1007-1018
- **JACINTO, E. ; HALL, M.N.** *Nature Rev. Mol. Cell Biol.,* 2003, vol. 4, 117-126
- **WULLSCHLEGER, S. et al.** *Cell,* 2006, vol. 124, 471-484
- **GWINN, D.M. et al.** *Molecular Cell,* 2008, vol. 30, 214-226
- **SMITH, E.M.** *J. Biol. Chem.,* 2005, vol. 280, 18717-18727
- **LONG, X. et al.** *Curr. Biol.,* 2005, vol. 15, 702-713
- **HAY, N. ; SONENBERG, N.** *Genes Dev.,* 2004, vol. 18, 1926-1945
- **VEZINA, C. et al.** *J. Antibiot. (Tokyo,* 1975, vol. 28, 721-726
- **EASTON, J.B. ; HOUGHTON, P.J.** *Oncogene,* 2006, vol. 25, 6436-6446
- **FAIVRE, S. et al.** *Nature Rev. Drug Discovery,* 2006, vol. 5, 671-688
- **GRANVILLE, C.A. et al.** *Clin. Cancer Res.,* 2006, vol. 12, 679-689
- **LOEWITH, R. et al.** *Molecular Cell,* 2002, vol. 10, 457-468
- **HARA, K. et al.** *Cell,* 2002, vol. 110, 177-189
- **JACINTO, E. et al.** *Nature Cell. Biol.,* 2004, vol. 6, 1122-1128
- **KIM, D.H. et al.** *Cell,* 2002, vol. 110, 163-175
- **SARBASSOV, D.D. et al.** *Curr. Biol,* 2004, vol. 14, 1296-1302
- **GUERTIN, D.A. ; SABATINI, D.M.** *Cancer Cell,* 2007, vol. 12, 9-22
- **DANCEY, J.E.** *Expert Opinion on Invest. Drugs,* 2005, vol. 14, 313-328
- **HUDSON, C.C. et al.** *Molec. Cell. Biol.,* 2002, vol. 22, 7004-7014

- **TEE, A.R. ; BLENIS, J.** *Semin. Cell Dev. Biol.,* 2005, vol. 16, 29-37
- **INOKI, K. et al.** *Nature Genetics,* 2005, vol. 37, 19-24
- **EASTON, J.B. ; HOUGHTON, P.J.** *Expert Opin. Ther. Targets,* 2004, vol. 8, 551-564
- **SEHGAL, S.N.** *Transplantation Proceedings,* 2003, vol. 35, 7S-14S
- **MORICE, M.C. et al.** *J. Amer. Coll. Cardiol.,* 2007, vol. 50, 1299-1304
- **EISEN, H.J. et al.** *New England Journal of Medicine,* 2003, vol. 349, 847-858
- **VIGANO, M. et al.** *J. Heart Lung Transplant,* 2007, vol. 26, 584-592
- **FAN, Q-W. et al.** *Cancer Cell,* 2006, vol. 9, 341-349
- **FAN, Q-W. et al.** *Cancer Res.,* 2007, vol. 67, 7960-7965
- **FAN, Q-W. et al.** *Cancer Cell,* 2006, vol. 9, 341-239
- **CHEN, J.S. et al.** *Mol. Cancer Ther.,* 2008, vol. 7, 841-850
- **CAREY ; SUNDBERG.** ADVANCED ORGANIC CHEMISTRY 5TH ED. Springer Science+Business Media, 2007, vol. A
- **MARCH, JERRY.** Advanced Organic Chemistry. John Wiley & Sons, 2001
- **P.G.M. WUTS ; T.W. GREENE.** Greene's Protecting Groups in Organic Synthesis. John Wiley & Sons, Inc, 2007
- **SAULNIER et al.** *Bioorganic and Medicinal Chemistry Letters,* 1985, vol. 4
- **P.G.M. WUTS ; T.W. GREENE.** Greene's Protective Groups in Organic Synthesis. John Wiley and Sons, 2007
- **ABID et al.** *Arterioscler. Thromb. Vasco BioI.,* 2004, vol. 24, 294-300
- **SAWYER.** *Expert Opinion Investing. Drugs,* 2004, vol. 13, 1-19
- **HANNIGAN et al.** *Proc. Nat. Acad. of Sciences of U.S.A.,* 2002, vol. 99 (6), 3603-8
- **COUSSENS ; WERB.** *Nature,* 2002, vol. 420, 860867
- **KOYASU.** *Nature Immunology,* 2003, vol. 4, 313-319
- **DANCEY.** *Expert Opinion on Investigational Drugs,* 2005, vol. 14, 313-328
- **HUDSON et al.** *Molecular and Cellular Biology,* 2002, vol. 22, 7004-7014
- **TEE ; BLENIS.** *Seminars in Cell and Developmental Biology,* 2005, vol. 16, 29-37
- **SEHGAL.** *Transplantation Proceedings,* 2003, vol. 35, 7S-14S
- **EASTON ; HOUGHTON.** *Expert Opinion on Therapeutic Targets,* 2004, vol. 8, 551-564
- **MORICE et al.** *New England Journal of Medicine,* 2002, vol. 346, 1773-1780
- **NAKAGAWA et al.** *Nature,* 2000, vol. 403, 98-103
- **IMAI et al.** *Gell,* 2001, vol. 105, 891-902
- **NISHITOH et al.** *Genes Dev.,* 2002, vol. 16, 1345-1355
- **FERRARA N. ; ALITALO, K.** Clinical application of angiogenic growth factors and their inhibitors. *Nature Medicine,* 1999, vol. 5, 1359-1364
- Remington: The Science and Practices of Pharmacy. Lippincott Williams, and Wilkins Publisher, 2005
- **SAULNIER et al.** *Bioorganic and Medicinal Chemistry Letters,* 1994, vol. 4, 1985
- Fieser and Fieser's Reagents for Organic Synthesis. John Wiley and Sons, 2006, vol. 1-23
- Rodd's Chemistry of Carbon Compounds. Elsevier Science Publishers, 1998, vol. 1-5
- Organic Reactions. John Wiley and Sons, 2007, vol. 1-68
- **MARCH J.** Advanced Organic Chemistry. John Wiley and Sons, 2001
- **LAROCK.** Comprehensive Organic Transformations. John Wiley and Sons, 1999
- **JEAN JACQUES ; ANDRE COLLET ; SAMUEL H. WILEN.** Enantiomers, Racemates and Resolutions. John Wiley & Sons, Inc, 1981
- **KASHEM et al.** *J. Biomol. Screen.,* 2007, vol. 12, 70-83